(19) Europäisches Patentamt
European Patent Office
Office européen des brevets

(11) EP 2 459 591 B1

(12) **EUROPEAN PATENT SPECIFICATION**

(45) Date of publication and mention of the grant of the patent:
**20.08.2014 Bulletin 2014/34**

(21) Application number: **10740103.6**

(22) Date of filing: **30.07.2010**

(51) Int Cl.:
*C07K 16/18* (2006.01) *C07K 16/24* (2006.01)
*A61K 39/395* (2006.01) *A61P 35/04* (2006.01)
*G01N 33/50* (2006.01) *G01N 33/574* (2006.01)
*C07K 16/22* (2006.01) *C07K 16/28* (2006.01)

(86) International application number:
**PCT/US2010/043872**

(87) International publication number:
**WO 2011/014750 (03.02.2011 Gazette 2011/05)**

(54) **INHIBITION OF TUMOR METASTASIS USING ANTI-G-CSF-ANTIBODIES**

HEMMUNG VON TUMORMETASTASE MITTELS ANTI-G-CSF-ANTIKÖRPERN

INHIBITION DE MÉTASTASE TUMORALE UTILISANT DES ANTICORPS ANTI-G-CSF

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **31.07.2009 US 230571 P**
**02.06.2010 US 350558 P**

(43) Date of publication of application:
**06.06.2012 Bulletin 2012/23**

(73) Proprietor: **Genentech, Inc.**
**South San Francisco, CA 94080 (US)**

(72) Inventors:
- **FERRARA, Napoleone**
**San Francisco**
**California 94109 (US)**
- **KOWANETZ, Marcin Leszek**
**San Francisco**
**California 94116 (US)**

(74) Representative: **Brodbeck, Michel**
**F. Hoffmann-La Roche AG**
**Patent Department CLP**
**Grenzacherstrasse 124**
**4070 Basel (CH)**

(56) References cited:
**WO-A2-2009/039337**

- **YANG LI ET AL: "Abrogation of TGF beta signaling in mammary carcinomas recruits Gr-1+CD11b+ myeloid cells that promote metastasis." CANCER CELL JAN 2008 LNKD-PUBMED:18167337, vol. 13, no. 1, January 2008 (2008-01), pages 23-35, XP002602433 ISSN: 1535-6108**
- **ISHIHARA YOKO ET AL: "Expression of matrix metalloproteinase, tissue inhibitors of metalloproteinase and adhesion molecules in silicotic mice with lung tumor metastasis." TOXICOLOGY LETTERS 30 APR 2003 LNKD-PUBMED:12765241, vol. 142, no. 1-2, 30 April 2003 (2003-04-30), pages 71-75, XP002602434 ISSN: 0378-4274**
- **BOYD JOHN H ET AL: "S100A8 and S100A9 mediate endotoxin-induced cardiomyocyte dysfunction via the receptor for advanced glycation end products." CIRCULATION RESEARCH, vol. 102, no. 10, 23 May 2008 (2008-05-23), pages 1239-1246, XP002602435 ISSN: 1524-4571 -& BOYD JOHN H ET AL: "Materials and methods: S100A8 and S100A9 mediate endotoxin-induced cardiomyocyte dysfunction via the receptor for advanced glycation end products." CIRCULATION RESEARCH - DATA SUPPLEMENT, vol. 102, no. 10, 23 May 2008 (2008-05-23), pages 1-5, XP002606888**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- MOON AREE ET AL: "Global gene expression profiling unveils S100A8/A9 as candidate markers in H-ras-mediated human breast epithelial cell invasion." MOLECULAR CANCER RESEARCH : MCR OCT 2008 LNKD- PUBMED: 18922970, vol. 6, no. 10, October 2008 (2008-10), pages 1544-1553, XP002602436 ISSN: 1541-7786
- NINOMIYA S ET AL: "Effect of Bevacizumab, a Humanized Monoclonal Antibody to Vascular Endothelial Growth Factor, on Peritoneal Metastasis of MNK-45P Human Gastric Cancer in Mice" JOURNAL OF SURGICAL RESEARCH, ACADEMIC PRESS INC., SAN DIEGO, CA, US LNKD- DOI:10.1016/J.JSS.2008.08.017, vol. 154, no. 2, 15 June 2009 (2009-06-15), pages 196-202, XP026149413 ISSN: 0022-4804 [retrieved on 2008-09-16]
- CHANG PU-YUAN ET AL: "Local radiotherapy suppresses granulocyte colony-stimulating factor induced tumor angiogenesis and metastasis" PROCEEDINGS OF THE ANNUAL MEETING OF THE AMERICAN ASSOCIATION FOR CANCER RESEARCH; 100TH ANNUAL MEETING OF THE AMERICAN-ASSOCIATION-FOR-CANCER-RESEARCH; DENVER, CA, USA; APRIL 18 20090401 AMERICAN ASSOCIATION FOR CANCER RESEARCH, US, vol. 50, 1 April 2009 (2009-04-01), page 168, XP008127229 ISSN: 0197-016X
- YAN HANNAH H ET AL: "Gr-1+CD11b+ myeloid cells tip the balance of immune protection to tumor promotion in the premetastatic lung." CANCER RESEARCH 1 AUG 2010 LNKD-PUBMED:20631080, vol. 70, no. 15, 1 August 2010 (2010-08-01), pages 6139-6149, XP002602437 ISSN: 1538-7445

Sequence Listing

<110> GENENTECH, INC. FERRARA, Napoleone KOWANETZ, Marcin Leszek

<120> INHIBITION OF TUMOR METASTASIS

<130> P4348R1 WO

<141> 2010-07-30

<150> US 61/230,571
<151> 2009-07-31

<150> US 61/350,558
<151> 2010-06-02

<160> 19

<210> 1
<211> 427
<212> DNA
<213> Homo sapiens

<400> 1

tgagggcgcc atgaggagcc tgtgctgcgc cccactcctg ctcctcttgc 50
tgctgccgcc gctgctgctc acgccccgcg ctggggacgc cgccgtgatc 100
accggggctt gtgacaagga ctcccaatgt ggtggaggca tgtgctgtgc 150
tgtcagtatc tgggtcaaga gcataaggat ttgcacacct atgggcaaac 200
tgggagacag ctgccatcca ctgactcgta aaaacaattt tggaaatgga 250
aggcaggaaa gaagaaagag gaagagaagc aaaaggaaaa aggaggttcc 300
attttttggg cggaggatgc atcacacttg cccatgtctg ccaggcttgg 350
cctgtttacg gacttcattt aaccgattta tttgtttagc ccaaaagtaa 400
tcgctctgga gtagaaacca aatgtga 427

<210> 2
<211> 129
<212> PRT
<213> Homo sapiens

<400> 2

Met Arg Ser Leu Cys Cys Ala Pro Leu Leu Leu Leu Leu Leu Leu
1 5 10 15

Pro Pro Leu Leu Leu Thr Pro Arg Ala Gly Asp Ala Ala Val Ile
20 25 30

Thr Gly Ala Cys Asp Lys Asp Ser Gln Cys Gly Gly Gly Met Cys
35 40 45

Cys Ala Val Ser Ile Trp Val Lys Ser Ile Arg Ile Cys Thr Pro
50 55 60

Met Gly Lys Leu Gly Asp Ser Cys His Pro Leu Thr Arg Lys Asn
65 70 75

Asn Phe Gly Asn Gly Arg Gln Glu Arg Arg Lys Arg Lys Arg Ser
80 85 90

Lys Arg Lys Lys Glu Val Pro Phe Phe Gly Arg Arg Met His His
95 100 105

Thr Cys Pro Cys Leu Pro Gly Leu Ala Cys Leu Arg Thr Ser Phe
110 115 120

Asn Arg Phe Ile Cys Leu Ala Gln Lys
125

<210> 3
<211> 363
<212> DNA
<213> Homo sapiens

<400> 3

gagggcgcca tgaggagcct gtgctgcgcc ccactcctgc tcctcttgct 50
gctgccgccg ctgctgctca cgccccgcgc tggggacgcc gccgtgatca 100
ccggggcttg tgacaaggac tcccaatgtg gtggaggcat gtgctgtgct 150
gtcagtatct gggtcaagag cataaggatt tgcacaccta tgggcaaact 200
gggagacagc tgccatccac tgactcgtaa agttccattt tttgggcgga 250
ggatgcatca cacttgccca tgtctgccag gcttggcctg tttacggact 300
tcatttaacc gatttatttg tttagcccaa aagtaatcgc tctggagtag 350
aaaccaaatg tga 363

<210> 4
<211> 108
<212> PRT
<213> Homo sapiens

<400> 4

Met Arg Ser Leu Cys Cys Ala Pro Leu Leu Leu Leu Leu Leu Leu
1 5 10 15

Pro Pro Leu Leu Leu Thr Pro Arg Ala Gly Asp Ala Ala Val Ile
20 25 30

Thr Gly Ala Cys Asp Lys Asp Ser Gln Cys Gly Gly Gly Met Cys
35 40 45

Cys Ala Val Ser Ile Trp Val Lys Ser Ile Arg Ile Cys Thr Pro
50 55 60

Met Gly Lys Leu Gly Asp Ser Cys His Pro Leu Thr Arg Lys Val
65 70 75

Pro Phe Phe Gly Arg Arg Met His His Thr Cys Pro Cys Leu Pro
80 85 90

Gly Leu Ala Cys Leu Arg Thr Ser Phe Asn Arg Phe Ile Cys Leu
95 100 105

Ala Gln Lys

<210> 5
<211> 1338
<212> DNA
<213> Mouse

<400> 5

cggacgcgtg ggcgtcccct aaccgccacc gcgtccccgg gacgccatgg 50
gggacccgcg ctgtgccccg ctactgctac ttctgctgct accgctgctg 100
ttcacaccgc ccgccgggga tgccgcggtc atcaccgggg cttgcgacaa 150
ggactctcag tgcggaggag gcatgtgctg tgctgtcagt atctgggtta 200
agagcataag gatctgcaca cctatgggcc aagtgggcga cagctgccac 250
cccctgactc ggaaagttcc attttggggg cggaggatgc accacacctg 300
cccctgcctg ccaggcttgg cgtgtttaag gacttctttc aaccggttta 350
tttgcttggc ccggaaatga tcactctgaa gtaggaactt gaaatgcgac 400
cctccgctgc acaatgtccg tcgagtctca cttgtaattg tggcaaacaa 450
agaatactcc agaaagaaat gttctccccc ttccttgact ttccaagtaa 500
cgtttctatc tttgattttt gaagtggctt tttttttttt ttttttttcc 550
tttccttgaa ggaaagtttt gatttttgga gagatttata gaggactttc 600
tgacatggct tctcatttcc ctgtttatgt tttgccttga catttttgaa 650
tgccaataac aactgttttc acaaatagga gaataagagg gaacaatctg 700
ttgcagaaac ttccttttgc cctttgcccc actcgccccg ccccgccccg 750
ccccgccctg cccatgcgca gacagacaca cccttactct tcaaagactc 800
tgatgatcct caccttactg tagcattgtg ggtttctaca cttccccgcc 850
ttgctggtgg acccactgag gaggctcaga gagctagcac tgtacaggtt 900
tgaaccagat cccccaagca gctcatttgg ggcagacgtt gggagcgctc 950
caggaacttt cctgcaccca tctggcccac tggctttcag ttctgctgtt 1000
taactggtgg gaggacaaaa ttaacgggac cctgaaggaa cctggcccgt 1050
ttatctagat ttgtttaagt aaaagacatt ttctccttgt tgtggaatat 1100
tacatgtctt tttcttttt atctgaagct tttttttttt ttctttaagt 1150
cttcttgttg gagacatttt aaagaacgcc actcgaggaa gcattgattt 1200
tcatytggca tgacaggagt catcatttta aaaaatcggt gttaagttat 1250
aatttaaact ttatttgtaa cccaaaggty taatgtaaat ggatttcctg 1300
atatcctgcc atttgtactg gtatcaatat ttytatgt 1338

<210> 6
<211> 107
<212> PRT
<213> Mouse

<400> 6

Met Gly Asp Pro Arg Cys Ala Pro Leu Leu Leu Leu Leu Leu Leu
1 5 10 15

Pro Leu Leu Phe Thr Pro Pro Ala Gly Asp Ala Ala Val Ile Thr
20 25 30

Gly Ala Cys Asp Lys Asp Ser Gln Cys Gly Gly Gly Met Cys Cys
35 40 45

Ala Val Ser Ile Trp Val Lys Ser Ile Arg Ile Cys Thr Pro Met
50 55 60

Gly Gln Val Gly Asp Ser Cys His Pro Leu Thr Arg Lys Val Pro
65 70 75

Phe Trp Gly Arg Arg Met His His Thr Cys Pro Cys Leu Pro Gly
80 85 90

Leu Ala Cys Leu Arg Thr Ser Phe Asn Arg Phe Ile Cys Leu Ala
95 100 105

Arg Lys

<210> 7
<211> 624
<212> DNA
<213> Homo sapiens

<400> 7

atggctggac ctgccaccca gagccccatg aagctgatgg ccctgcagct 50

gctgctgtgg cacagtgcac tctggacagt gcaggaagcc accccctgg 100

gccctgccag ctccctgccc cagagcttcc tgctcaagtg cttagagcaa 150

gtgaggaaga tccagggcga tggcgcagcg ctccaggaga agctggtgag 200

tgagtgtgcc acctacaagc tgtgccaccc cgaggagctg gtgctgctcg 250

gacactctct gggcatcccc tgggctcccc tgagcagctg ccccagccag 300

gccctgcagc tggcaggctg cttgagccaa ctccatagcg gccttttcct 350

ctaccagggg ctcctgcagg ccctggaagg gatctccccc gagttgggtc 400

ccaccttgga cacactgcag ctggacgtcg ccgactttgc caccaccatc 450

tggcagcaga tggaagaact gggaatggcc cctgccctgc agcccaccca 500

gggtgccatg ccggccttcg cctctgcttt ccagcgccgg gcaggagggg 550

tcctggttgc ctcccatctg cagagcttcc tggaggtgtc gtaccgcgtt 600

ctacgccacc ttgcccagcc ctga 624

<210> 8
<211> 207
<212> PRT
<213> Homo sapiens

<400> 8

```
Met Ala Gly Pro Ala Thr Gln Ser Pro Met Lys Leu Met Ala Leu
 1               5                  10                  15

Gln Leu Leu Leu Trp His Ser Ala Leu Trp Thr Val Gln Glu Ala
                20                  25                  30

Thr Pro Leu Gly Pro Ala Ser Ser Leu Pro Gln Ser Phe Leu Leu
                35                  40                  45

Lys Cys Leu Glu Gln Val Arg Lys Ile Gln Gly Asp Gly Ala Ala
                50                  55                  60

Leu Gln Glu Lys Leu Val Ser Glu Cys Ala Thr Tyr Lys Leu Cys
                65                  70                  75

His Pro Glu Glu Leu Val Leu Leu Gly His Ser Leu Gly Ile Pro
                80                  85                  90

Trp Ala Pro Leu Ser Ser Cys Pro Ser Gln Ala Leu Gln Leu Ala
                95                 100                 105

Gly Cys Leu Ser Gln Leu His Ser Gly Leu Phe Leu Tyr Gln Gly
               110                 115                 120

Leu Leu Gln Ala Leu Glu Gly Ile Ser Pro Glu Leu Gly Pro Thr
               125                 130                 135

Leu Asp Thr Leu Gln Leu Asp Val Ala Asp Phe Ala Thr Thr Ile
               140                 145                 150

Trp Gln Gln Met Glu Glu Leu Gly Met Ala Pro Ala Leu Gln Pro
               155                 160                 165

Thr Gln Gly Ala Met Pro Ala Phe Ala Ser Ala Phe Gln Arg Arg
               170                 175                 180

Ala Gly Gly Val Leu Val Ala Ser His Leu Gln Ser Phe Leu Glu
               185                 190                 195

Val Ser Tyr Arg Val Leu Arg His Leu Ala Gln Pro
               200                 205
```

<210> 9
<211> 603
<212> DNA
<213> Homo sapiens

<400> 9

atgtctcctg agcccgctct gtccccagcc ctgcagctgc tgctgtggca 50

cagtgcactc tggacagtgc aggaagccac cccccctgggc cctgccagct 100

ccctgcccca gagcttcctg ctcaagtgct tagagcaagt gaggaagatc 150

cagggcgatg gcgcagcgct ccaggagaag ctgtgtgcca cctacaagct 200

gtgccacccc gaggagctgg tgctgctcgg acactctctg ggcatcccct 250

gggctcccct gagcagctgc cccagccagg ccctgcagct ggcaggctgc 300

ttgagccaac tccatagcgg ccttttcctc taccaggggc tcctgcaggc 350

cctggaaggg atctcccccg agttgggtcc caccttggac acactgcagc 400

tggacgtcgc cgactttgcc accaccatct ggcagcagat ggaagaactg 450

ggaatggccc ctgccctgca gcccacccag ggtgccatgc cggccttcgc 500

ctctgctttc cagcgccggg caggaggggt cctggttgcc tcccatctgc 550

agagcttcct ggaggtgtcg taccgcgttc tacgccacct tgcccagccc 600

tga 603

<210> 10
<211> 200
<212> PRT
<213> Homo sapiens

<400> 10

```
Met Ser Pro Glu Pro Ala Leu Ser Pro Ala Leu Gln Leu Leu Leu
1               5                   10                  15

Trp His Ser Ala Leu Trp Thr Val Gln Glu Ala Thr Pro Leu Gly
                20                  25                  30

Pro Ala Ser Ser Leu Pro Gln Ser Phe Leu Leu Lys Cys Leu Glu
                35                  40                  45

Gln Val Arg Lys Ile Gln Gly Asp Gly Ala Ala Leu Gln Glu Lys
                50                  55                  60

Leu Cys Ala Thr Tyr Lys Leu Cys His Pro Glu Glu Leu Val Leu
                65                  70                  75

Leu Gly His Ser Leu Gly Ile Pro Trp Ala Pro Leu Ser Ser Cys
                80                  85                  90

Pro Ser Gln Ala Leu Gln Leu Ala Gly Cys Leu Ser Gln Leu His
                95                  100                 105

Ser Gly Leu Phe Leu Tyr Gln Gly Leu Leu Gln Ala Leu Glu Gly
                110                 115                 120

Ile Ser Pro Glu Leu Gly Pro Thr Leu Asp Thr Leu Gln Leu Asp
                125                 130                 135

Val Ala Asp Phe Ala Thr Thr Ile Trp Gln Gln Met Glu Glu Leu
                140                 145                 150

Gly Met Ala Pro Ala Leu Gln Pro Thr Gln Gly Ala Met Pro Ala
                155                 160                 165

Phe Ala Ser Ala Phe Gln Arg Arg Ala Gly Gly Val Leu Val Ala
                170                 175                 180

Ser His Leu Gln Ser Phe Leu Glu Val Ser Tyr Arg Val Leu Arg
                185                 190                 195

His Leu Ala Gln Pro
                200
```

<210> 11
<211> 615
<212> DNA
<213> Homo sapiens

<400> 11

```
atggctggac ctgccaccca gagccccatg aagctgatgg ccctgcagct 50
gctgctgtgg cacagtgcac tctggacagt gcaggaagcc acccccctgg 100
gccctgccag ctccctgccc cagagcttcc tgctcaagtg cttagagcaa 150
```

gtgaggaaga tccagggcga tggcgcagcg ctccaggaga agctgtgtgc 200

cacctacaag ctgtgccacc ccgaggagct ggtgctgctc ggacactctc 250

tgggcatccc ctgggctccc ctgagcagct gccccagcca ggccctgcag 300

ctggcaggct gcttgagcca actccatagc ggccttttcc tctaccaggg 350

gctcctgcag gccctggaag ggatctcccc cgagttgggt cccaccttgg 400

acacactgca gctggacgtc gccgactttg ccaccaccat ctggcagcag 450

atggaagaac tgggaatggc ccctgccctg cagcccaccc agggtgccat 500

gccggccttc gcctctgctt tccagcgccg ggcaggaggg gtcctggttg 550

cctcccatct gcagagcttc ctggaggtgt cgtaccgcgt tctacgccac 600

cttgcccagc cctga 615

<210> 12
<211> 204
<212> PRT
<213> Homo sapiens

<400> 12

```
Met Ala Gly Pro Ala Thr Gln Ser Pro Met Lys Leu Met Ala Leu
1               5                   10                  15

Gln Leu Leu Leu Trp His Ser Ala Leu Trp Thr Val Gln Glu Ala
                20                  25                  30

Thr Pro Leu Gly Pro Ala Ser Ser Leu Pro Gln Ser Phe Leu Leu
                35                  40                  45

Lys Cys Leu Glu Gln Val Arg Lys Ile Gln Gly Asp Gly Ala Ala
                50                  55                  60

Leu Gln Glu Lys Leu Cys Ala Thr Tyr Lys Leu Cys His Pro Glu
                65                  70                  75

Glu Leu Val Leu Leu Gly His Ser Leu Gly Ile Pro Trp Ala Pro
                80                  85                  90

Leu Ser Ser Cys Pro Ser Gln Ala Leu Gln Leu Ala Gly Cys Leu
                95                  100                 105

Ser Gln Leu His Ser Gly Leu Phe Leu Tyr Gln Gly Leu Leu Gln
                110                 115                 120

Ala Leu Glu Gly Ile Ser Pro Glu Leu Gly Pro Thr Leu Asp Thr
                125                 130                 135

Leu Gln Leu Asp Val Ala Asp Phe Ala Thr Thr Ile Trp Gln Gln
                140                 145                 150

Met Glu Glu Leu Gly Met Ala Pro Ala Leu Gln Pro Thr Gln Gly
                155                 160                 165

Ala Met Pro Ala Phe Ala Ser Ala Phe Gln Arg Arg Ala Gly Gly
                170                 175                 180

Val Leu Val Ala Ser His Leu Gln Ser Phe Leu Glu Val Ser Tyr
                185                 190                 195

Arg Val Leu Arg His Leu Ala Gln Pro
                200
```

<210> 13
<211> 516
<212> DNA
<213> Homo sapiens

<400> 13

atggctggac ctgccaccca gagccccatg aagctgatgg ccctgcagct 50

gctgctgtgg cacagtgcac tctggacagt gcaggaagcc acccccctgg 100

gccctgccag ctccctgccc cagagcttcc tgctcaagtg cttagagcaa 150

gtgaggaaga tccagggcga tggcgcagcg ctccaggaga agctggtgag 200

tgaggcaggc tgcttgagcc aactccatag cggccttttc ctctaccagg 250

ggctcctgca ggccctggaa gggatctccc ccgagttggg tcccaccttg 300

gacacactgc agctggacgt cgccgacttt gccaccacca tctggcagca 350

gatggaagaa ctgggaatgg cccctgccct gcagcccacc cagggtgcca 400

tgccggcctt cgcctctgct ttccagcgcc gggcaggagg ggtcctggtt 450

gcctcccatc tgcagagctt cctggaggtg tcgtaccgcg ttctacgcca 500

ccttgcccag ccctga 516

<210> 14
<211> 171
<212> PRT
<213> Homo sapiens

<400> 14

Met Ala Gly Pro Ala Thr Gln Ser Pro Met Lys Leu Met Ala Leu
1 5 10 15

Gln Leu Leu Leu Trp His Ser Ala Leu Trp Thr Val Gln Glu Ala
20 25 30

Thr Pro Leu Gly Pro Ala Ser Ser Leu Pro Gln Ser Phe Leu Leu
35 40 45

Lys Cys Leu Glu Gln Val Arg Lys Ile Gln Gly Asp Gly Ala Ala
50 55 60

Leu Gln Glu Lys Leu Val Ser Glu Ala Gly Cys Leu Ser Gln Leu
65 70 75

His Ser Gly Leu Phe Leu Tyr Gln Gly Leu Leu Gln Ala Leu Glu
80 85 90

Gly Ile Ser Pro Glu Leu Gly Pro Thr Leu Asp Thr Leu Gln Leu
95 100 105

Asp Val Ala Asp Phe Ala Thr Thr Ile Trp Gln Gln Met Glu Glu
110 115 120

Leu Gly Met Ala Pro Ala Leu Gln Pro Thr Gln Gly Ala Met Pro
125 130 135

Ala Phe Ala Ser Ala Phe Gln Arg Arg Ala Gly Gly Val Leu Val
140 145 150

Ala Ser His Leu Gln Ser Phe Leu Glu Val Ser Tyr Arg Val Leu
155 160 165

Arg His Leu Ala Gln Pro
170

<210> 15
<211> 19
<212> DNA
<213> Artificial sequence

<220>
<223> sequence is synthesized

<400> 15
ccgcaaggaa tcggtcaat 19

<210> 16
<211> 20
<212> DNA
<213> Artificial sequence

<220>
<223> sequence is synthesized

<400> 16
gatcagcaat cgcgcatatg 20

<210> 17
<211> 17
<212> DNA
<213> Artificial sequence

<220>
<223> sequence is synthesized

<400> 17
cactacatgg cgtgatt 17

<210> 18
<211> 6
<212> PRT
<213> Artificial sequence

<220>
<223> sequence is synthesized

<400> 18
Gly Ser Gly Ser Gly Ser 5

<210> 19
<211> 4
<212> PRT
<213> Artificial sequence

<220>
<223> sequence is synthesized

<400> 19
Gly Gly Gly Ser

**Description**

## FIELD OF THE INVENTION

The present invention relates generally to compositions that are useful for treatment of conditions and diseases associated with angiogenesis and tumor metastasis. In particular, the invention concerns the prevention or treatment of tumor metastasis using G-CSF antagonists and/or Bv8 antagonists.

## BACKGROUND OF THE INVENTION

It is well established that angiogenesis plays an important role in tumor progression and metastasis and anti-angiogenesis represents a clinically validated anti-cancer strategy (Folkman, Nat Med 1, 27-31 (1995); Ferrara and Kerbel, Nature 438, 967-974 (2005); Carmeliet, Nat Med 9, 653-660 (2003)).

Over the last several years, the contribution of various bone marrow (BM)-derived cell types to tumor angiogenesis has been the object of intense investigation (Coussens and Werb, Nature 420, 860-867 (2002); Rafii et al., Nat Rev Cancer 2:826-35 (2002); De Palma et al., Trends Immunol 28:519-524 (2007); Shojaei et al., Trends Cell Biol 18:372-378 2008)). Among these cell types, $CD11b^+Gr1^+$ cells are frequently increased in the tumors and in the peripheral blood (PB) of tumor-bearing animals, and have been shown to promote tumor angiogenesis (Yang et al., Cancer Cell 6, 409-421 (2004)), and to suppress immune functions, hence, the denomination of myeloid-derived suppressor cells (MDSC) (Bronte et al., Blood 96:3838-3846 (200)). However, the initiating mechanisms responsible for peripheral mobilization, tumor-homing, and acquisition of proangiogenic properties in $CD11b^+Gr1^+$ cells remain to be elucidated.

$CD11b^+Gr1^+$ cells produce several angiogenic factors, including Bv8, a secreted protein previously characterized as a mitogen for specific endothelial cell type, as a growth factor for hematopoietic progenitors (LeCouter et al., Proc Natl Acad Sci USA 100, 2685-2690 (2003); LeCouter et al., Proc Natl Acad Sci USA 101, 16813-16818 (2004)), and as a neuromodulator (Cheng et al., Nature 417, 405-410 (2002); Matsumoto et al., Proc Natl Acad Sci USA 103:4140-4145 (2006)). Analysis of several xenografts, as well as of a transgenic cancer model (RIP-Tag), suggested that Bv8 promotes tumor angiogenesis through increased peripheral mobilization of myeloid cells and local stimulation of angiogenesis (Shojaei et al., Nature 450:825-831 (2007); Shojaei et al., Proc Natl Acad Sci USA 105:2640-2645 (2008)). It has been reported that granulocyte colony stimulating factor (G-CSF) as a strong inducer of Bv8 expression, both *in vitro* and *in vivo* (Shojaei et al., Proc Natl Acad Sci USA 106(16):6742-7 (2009)). Physiologically, G-CSF has an important role in mobilization of hematopoietic stem cells, progenitors, and mature cells, particularly neutrophils, into the blood circulation (Rapoport et al., Blood Rev 6:43-57 (1992); Lieschke et al., Blood 84:1737-1746 (1994)). G-CSF is also necessary for differentiation of progenitors to cells of granulocytic lineage, such as neutrophils, eosinophils and basophils. Although a few reports have suggested that G-CSF administration enhances tumor angiogenesis and growth (Natori et. al., Biochem Biophys Res Commun 297:1058-1061 (2002); Okazaki, T. et al., Int Immunol 18, 1-9 (2006)), the evidence implicating this factor in tumorigenesis is far from conclusive.

Yang, L. et al. Cancer Cell 13, 23-35 (2008) describes that Cd11b+Gr1+ cells promote invasion and metastasis at the primary tumor site through increased production of MMPs and TGF beta 1.

WO 2009/039337 relates to the involvement of Cd11b+Gr1+ cells in angiogenesis and the growth of primary tumor. This reference elucidates the role of Bv8 and G-CSF in regulating mobilization of Cd11b+Gr1+ cells from bone marrow. It is shown that an anti-G-CSF antibody reduced circulating- as well as bone marrow-CD11b+Gr1+ cells. However, there is no disclosure as to the inhibition/reduction of tumor metastasis by an anti-G-CSF antibody.

Ninomiya S. et al. Journal of Surgical Research 154(2) 196-202 (2009) relates to the effect of bevacizumab on gastric cancer with peritoneal metastasis in nude mice. It is shown that intraperitoneal administration of bevacizumab inhibits peritoneal metastasis and reduces malignant ascites in tumor-bearing mice.

Metastasis is a complex series of steps in which cancer cells leave the original tumor site and migrate to other parts of the body via the bloodstream or the lymphatic system. Metastatic tumors are very common in the late stages of cancer. Metastasis is a major cause of death from solid tumors. Unfortunately, the treatment options currently available are rarely able to cure metastatic cancer.

Thus, there is a need to discover and understand how angiogenesis and tumor metastatic progression can be effectively inhibited, prevented and treated. The present invention addresses these and other needs, as will be apparent upon review of the following disclosure.

## SUMMARY OF THE INVENTION

The present invention is based, at least in part, on the discovery that G-CSF and Bv8 are involved in angiogenesis and tumor metastasis.

In one aspect, the invention provides compositions for use in methods of inhibiting or reducing tumor metastasis comprising administering to a subject an effective amount of a G-CSF antagonist. In certain embodiments, the method further comprises administering to the subject an effective amount of Bv8 antagonist. In certain embodiments, the G-CSF antagonist inhibits or reduces the spread of a primary tumor to a pre-metastatic organ of the subject. In certain embodiments, reducing tumor metastasis comprises reducing size or number of lung metastases. In certain embodiments, inhibiting or reducing tumor metastasis comprises reducing one or more expression levels of the following molecules: G-CSF, Bv8, PKR1, MMP-9, S100A8 or S100A9. In certain embodiments, inhibiting or reducing tumor metastasis comprises reducing expression levels of G-CSF and PKR1. In certain embodiments, inhibiting or reducing tumor metastasis comprises reducing expression levels of MMP-9, S100A8 and S100A9. In certain embodiments, inhibiting or reducing tumor metastasis comprises reducing expression levels of Bv8, MMP-9, S100A8 and S100A9. In certain embodiments, inhibiting or reducing tumor metastasis comprises reducing expression levels of G-CSF, Bv8 and PKR1. In certain embodiments, the expression levels are mRNA expression levels. In certain embodiments, the expression levels are protein expression levels. In certain embodiments, expression levels of one or more of these molecules are reduced in a pre-metastatic organ of the subject. In certain embodiments, the pre-metastatic organ is lung. In certain embodiments, the pre-metastatic organ is liver.

In certain embodiments, the methods further comprise administering to the subject an effective amount of VEGF antagonist. In certain embodiments, the subject has been previously treated with VEGF antagonist. In certain embodiments, the VEGF antagonist is an anti-VEGF antibody or a fragment thereof. In certain embodiments, the anti-VEGF antibody is bevacizumab or a fragment or variant thereof. In certain embodiments, the methods further comprise administering to the subject an effective amount of a chemotherapeutic agent.

In one aspect, the description includes methods of reducing expression level of G-CSF, Bv8, PKR1, MMP-9, S100A8 or S100A9 in a pre-metastatic organ comprising administering to the subject an effective amount of a G-CSF antagonist and/or Bv8 antagonist. In another aspect, the description includes methods of reducing expression level of one or more of the following molecules, G-CSF, Bv8, PKR1, MMP-9, S100A8 or S100A9, in a pre-metastatic organ comprising administering to the subject an effective amount of a G-CSF antagonist and/or Bv8 antagonist. In certain embodiments, expression levels of PKR1, MMP-9, S100A8 and/or S100A9 are reduced in the pre-metastatic organ of the subject when G-CSF antagonist is administered to the subject. In certain embodiments, expression levels of PKR1, MMP-9, S 100A8 and/or S100A9 are reduced in the pre-metastatic organ of the subject when Bv8 antagonist is administered to the subject. In certain embodiments, expression levels of Bv8, MMP-9, S100A8 and/or S100A9 are reduced in the pre-metastatic organ of the subject when G-CSF antagonist is administered to the subject. In certain embodiments, expression levels of Bv8, MMP-9, S100A8 and/or S 100A9 are reduced in the pre-metastatic organ of the subject when Bv8 antagonist is administered to the subject. In certain embodiments, expression levels of MMP-9, S100A8 and S100A9 are reduced in the pre-metastatic organ of the subject when G-CSF antagonist is administered to the subject. In certain embodiments, expression levels of MMP-9, S100A8 and S100A9 are reduced in the pre-metastatic organ of the subject when Bv8 antagonist is administered to the subject. In certain embodiments, expression levels of G-CSF and PKR1 are reduced in the pre-metastatic organ of the subject when G-CSF antagonist is administered to the subject. In certain embodiments, expression levels of G-CSF, PKR1 and MMP-9 are reduced in the pre-metastatic organ of the subject when G-CSF antagonist is administered to the subject.

In one aspect, the description includes methods of inhibiting migration of metastatic tumor cells to a pre-metastatic organ comprising administering to the subject an effective amount of a G-CSF antagonist or Bv8 antagonist. In another aspect, the description includes methods of inhibiting migration of neutrophils to a pre-metastatic organ comprising administering to the subject an effective amount of a G-CSF antagonist or Bv8 antagonist. In certain embodiments, the methods comprise administering to the subject an effective amount of the G-CSF antagonist and an effective amount of the Bv8 antagonist.

In certain embodiments, the pre-metastatic organ of the subject is lung, liver, brain, bone or lymph node. In certain embodiments, the pre-metastatic organ of the subject is lung or liver. In certain embodiments, the pre-metastatic organ of the subject is lung.

In certain embodiments, the G-CSF antagonist is an anti-G-CSF antibody. In certain embodiments, the anti-G-CSF antibody is a monoclonal antibody. In certain embodiments, the anti-G-CSF antibody is a humanized antibody. In certain embodiments, the anti-G-CSF antibody is a human antibody.

In certain embodiments, the Bv8 antagonist is an anti-Bv8 antibody. In certain embodiments, the Bv8 antagonist is an anti-PKR1 antibody. In certain embodiments, the anti-Bv8 antibody or anti-PKR1 antibody is a monoclonal antibody. In certain embodiments, the anti-Bv8 antibody or anti-PKR1 antibody is a humanized antibody. In certain embodiments, the anti-Bv8 antibody or anti-PKR1 antibody is a human antibody.

In another aspect, the description includes methods of predicting whether a tumor in a subject will respond effectively to treatment with a G-CSF antagonist and/or Bv8 antagonist comprising determining whether a sample from the subject comprises a cell that expresses Bv8, PKR1 and/or G-CSF at a level greater than the expression level in a reference sample, wherein detection of said cell indicates that the subject has the tumor that will respond effectively to treatment with said G-CSF antagonist and/or Bv8 antagonist. In certain embodiments, the detection of a cell that expresses Bv8, PKR1 and G-CSF at levels greater than the expression levels in a reference sample indicates that the subject has the tumor cells that will respond effectively to treatment with said G-CSF antagonist and/or Bv8 antagonist. In certain embodiments, the mRNA expression levels of Bv8, PKR1 and/or G-CSF are measured. In certain embodiments, the protein expression levels of Bv8, PKR1 and/or G-CSF are measured. In certain embodiments, the tumor is metastatic tumor.

In another aspect, the description includes methods of predicting whether a tumor in a subject will respond effectively to treatment with a G-CSF antagonist and/or Bv8 antagonist comprising determining whether a sample from the subject comprises a functional human counterpart of CD11b+Gr1+ cell that expresses Bv8 at a level greater than the expression level in a reference sample, wherein detection of said functional human counterpart of CD11b+Gr1+ cell indicates that the subject has the tumor that will respond effectively to treatment with said G-CSF antagonist and/or Bv8 antagonist. In certain embodiments, the mRNA expression level of Bv8 is measured. In certain embodiments, the protein expression level of Bv8, is measured. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cells are human neutrophils, monocytes or macrophages. In certain embodiments, the tumor is metastatic tumor.

In yet another aspect, the description includes methods of predicting whether a tumor in a subject will respond effectively to treatment with a G-CSF antagonist and/or Bv8 antagonist comprising determining whether a sample from the subject has increased number or frequency of functional human counterpart of CD11b+Gr1+ cells compared to a reference sample, wherein the increased number or frequency of functional human counterpart of CD11b+Gr1+ cells indicates that the subject has the tumor that will respond effectively to treatment with said G-CSF antagonist and/or Bv8 antagonist. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cells are neutrophils, monocytes or macrophages. In certain embodiments, the tumor is metastatic tumor.

In one aspect, the description includes methods of treating a tumor in a subject with a G-CSF antagonist and/or Bv8 antagonist comprising (a) determining whether a sample from the subject comprises a cell that expresses Bv8, PKR1 and/or G-CSF at a level greater than the expression level in a reference sample, and (b) if the sample comprises a cell that expresses Bv8, PKR1 and/or G-CSF at a level greater than the expression level in the reference sample, administering to the subject an effective amount of said G-CSF antagonist and/or Bv8 antagonist. In certain embodiments, the cell is a functional human counterpart of CD11b+Gr1+ cell. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cell is neutrophils, monocytes or macrophages. In certain embodiments, the cell is metastatic tumor cell. In certain embodiments, the cells are from a pre-metastatic organ of the subject. In certain embodiments, if the sample comprises a cell that expresses Bv8, PKR1 and G-CSF at levels greater than the expression levels in the reference sample, then an effective amount of said G-CSF antagonist and/or Bv8 antagonist is administered to the subject.

In certain embodiments, the mRNA expression levels of Bv8, PKR1 and/or G-CSF are measured. In certain embodiments, the protein expression levels of Bv8, PKR1 and/or G-CSF are measured.

In another aspect, the description includes methods of treating a tumor in a subject with a G-CSF antagonist and/or Bv8 antagonist comprising (a) determining whether a sample from the subject has increased number or frequency of functional human counterpart of CD11b+Gr1+ cells compared to a reference sample, and (b) if the sample has the increased number or frequency of functional human counterpart of CD11b+Gr1+ cells compared to the reference sample, administering to the subject an effective amount of said G-CSF antagonist and/or Bv8 antagonist. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cells are neutrophils, monocytes or macrophages. In certain embodiments, the tumor is metastatic tumor.

In another aspect, the description includes methods of treating tumor, comprising (a) administering to a tumor-bearing subject an effective amount of a G-CSF antagonist, and (b) monitoring the efficacy of said treatment by determining the number or frequency of functional human counterpart of CD11b+Gr1+ cells in a sample obtained from the subject after the treatment, relative to the number or frequency of functional human counterpart of CD11b+Gr1+ cells in a sample obtained from the subject before the treatment, wherein a reduced number or frequency of functional human counterpart of CD11b+Gr1+ cells in the sample obtained from the subject after the treatment indicates that the treatment is effective. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cells are neutrophils, monocytes or macrophages. In certain embodiments, the tumor is metastatic tumor.

In another aspect, the description includes methods of treating tumor, comprising (a) administering to a tumor-bearing subject an effective amount of a G-CSF antagonist and/or Bv8 antagonist, and (b) monitoring the efficacy of said treatment by determining the expression level of Bv8, MMP-9, S100A8 or S100A9 in a sample obtained from the subject after the treatment, relative to the expression level of Bv8, MMP-9, S100A8 or S100A9 in a sample obtained from the subject before the treatment, wherein a reduced expression level of Bv8, MMP-9, S 100A8 or S 100A9 in the sample obtained from the subject after the treatment indicates that the treatment is effective.

In certain embodiments, expression levels of at least two of the following four molecules, Bv8, MMP-9, S100A8 and S100A9, are reduced in the sample obtained from the subject after the treatment compared to the sample obtained from the subject before the treatment. In certain embodiments, expression levels of at least three of the following four molecules, Bv8, MMP-9, S100A8 and S100A9, are reduced in the sample obtained from the subject after the treatment compared to the sample obtained from the subject before the treatment. In certain embodiments, the mRNA expression levels of Bv8, MMP-9, S100A8 and/or S100A9 are measured. In certain embodiments, the protein expression levels of Bv8, MMP-9, S100A8 and/or S100A9 are measured. In certain embodiments, the tumor is metastatic tumor.

In certain embodiments, the mRNA expression levels of Bv8, MMP-9, S100A8 and/or S100A9 are increased in pre-metastatic lung and/or metastatic lung. In certain embodiments, the protein expression levels of Bv8, MMP-9, S100A8 and/or S100A9 are increased in pre-metastatic lung and/or metastatic lung. In certain embodiments, the mRNA expression levels of Bv8, MMP-9, S100A8 and/or S100A9 are increased in pre-metastatic tissue or tumor tissue. In certain embodiments, the protein expression levels of Bv8, MMP-9, S100A8 and/or S100A9 are increased in pre-metastatic tissue or tumor tissue. In certain embodiments, the treatment with anti-Bv8 antibody is efficacious if the mRNA expression level of Bv8 and/or MMP-9 is decreased after the treatment with anti-Bv8 antibody. In certain embodiments, the treatment with anti-Bv8 antibody decreases the mRNA expression level of Bv8 and/or MMP-9 in pre-metastatic lungs. In certain embodiments, the treatment with anti-Bv8 antibody is efficacious if the protein expression level of Bv8 and/or MMP-9 is decreased after the treatment with anti-Bv8 antibody. In certain embodiments, the treatment with anti-Bv8 antibody decreases the protein expression level of Bv8 and/or MMP-9 in pre-metastatic lungs. In certain embodiments, the treatment with anti-G-CSF antibody is efficacious if the mRNA expression level of Bv8 and/or MMP-9 is decreased after the treatment with anti-G-CSF antibody. In certain embodiments, the treatment with anti-G-CSF antibody decreases the mRNA expression level of Bv8 and/or MMP-9 in pre-metastatic lungs and/or metastatic lungs. In certain embodiments, the treatment with anti-G-CSF antibody is efficacious if the protein expression level of Bv8 and/or MMP-9 is decreased after the treatment with anti-G-CSF antibody. In certain embodiments, the treatment with anti-G-CSF antibody decreases the protein expression level of Bv8 and/or MMP-9 in pre-metastatic lungs and/or metastatic lungs. In certain embodiments, the treatment with anti-PKR1 antibody is efficacious if the mRNA expression level of Bv8 and/or MMP-9 is decreased after the treatment with anti-PKR1 antibody. In certain embodiments, the treatment with anti-PKR1 antibody decreases the mRNA expression level of Bv8 and/or MMP-9 in pre-metastatic lungs and/or metastatic lungs. In certain embodiments, the treatment with anti-PKR1 antibody is efficacious if the protein expression level of Bv8 and/or MMP-9 is decreased after the treatment with anti-PKR1 antibody. In certain embodiments, the treatment with anti-PKR1 antibody decreases the protein expression level of Bv8 and/or MMP-9 in pre-metastatic lungs and/or metastatic lungs.

In certain embodiments, the tumor is a metastatic tumor. In certain embodiments, the treatment with G-CSF antagonists and/or Bv8 antagonists prevents the metastatic tumor from metastasizing to pre-metastatic tissues or pre-metastatic organs elsewhere in the body. In certain embodiments, the sample from the subject is a tissue, plasma, serum, or any combinations thereof. In certain embodiments, the samples used in these methods are from a pre-metastatic or metastatic organ. In certain embodiments, the pre-metastatic or metastatic organ is lung, liver, brain, bone, lymph node or ovary. In certain embodiments, the samples are from a pre-metastatic or metastatic tissue. In certain embodiments, the pre-metastatic or metastatic tissue is from subject's lung, liver, brain, ovary, lymph node, bone or bone marrow. In certain embodiments, the sample is a tissue sample from a pre-metastatic organ. In certain embodiments, if the sample is from pre-metastatic organ or pre-metastatic tissue, the sample does not contain any tumor cells. In certain embodiments, the sample is primary tumor tissue.

In certain embodiments, the mRNA expression level of a gene is measured using qRT-PCR or qPCR. In certain embodiments, the mRNA expression level is measured using microarrary. In certain embodiments, the mRNA expression level is measured using ISH (in situ hybridization). In certain embodiments, the protein expression level of a gene is measured using IHC assay.

In another aspect, the description includes methods of inhibiting mobilization of functional human counterpart of CD11b+Gr1+ cells from the bone marrow to a pre-metastatic or metastatic organ comprising administering to a subject an effective amount of a G-CSF antagonist and/or Bv8 antagonist. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cell is neutrophils, monocytes or macrophages.

In one aspect, the description includes methods of treating tumor in a subject relapsed from or refractory to anti-cancer therapy comprising administering to a subject an effective amount of a G-CSF antagonist. In certain embodiments, the anti-cancer therapy comprises a VEGF antagonist. In certain embodiments, the VEGF antagonist is an anti-VEGF antibody or fragment thereof. In certain embodiments, the anti-VEGF antibody is bevacizumab or a fragment or variant thereof.

In one aspect, the description includes methods of treating a benign, pre-cancerous, or non-metastatic cancer in a subject, comprising administering to the subject an effective amount of a G-CSF antagonist and/or Bv8 antagonist. In certain embodiments, administering of the G-CSF antagonist and/or Bv8 antagonist prevents the benign, pre-cancerous, or non-metastatic cancer from becoming an invasive or metastatic cancer. In certain embodiments, the benign, pre-cancerous, or non-metastatic cancer is a stage 0, stage I, or stage II cancer. In certain embodiments, administering of the G-CSF antagonist and/or Bv8 antagonist prevents the benign, pre-cancerous or non-metastatic cancer from progressing to a stage III or stage IV cancer. In certain embodiments, administering of the G-CSF antagonist and/or Bv8 antagonist reduces tumor size.

In another aspect, the description includes methods of treating a subject with operable cancer comprising administering to the subject an effective amount of a G-CSF antagonist and/or Bv8 antagonist and performing surgery whereby the cancer is resected. In certain embodiments, the G-CSF antagonist and/or Bv8 antagonist is administered to the subject prior to surgery. In certain embodiments, the methods further comprise the step of administering to the subject an effective amount of the G-CSF antagonist and/or Bv8 antagonist after surgery to prevent recurrence of the cancer. In certain embodiments, administering of the G-CSF antagonist and/or Bv8 antagonist prevents proliferation of micrometastases.

In another aspect, the description includes methods of neoadjuvant therapy in a subject with operable cancer comprising administering to the subject a G-CSF antagonist and/or Bv8 antagonist.

In another aspect, the description includes methods of preventing recurrence of cancer in a subject, comprising administering to the subject a G-CSF antagonist and/or Bv8 antagonist, wherein said administering prevents cancer recurrence in the subject. In yet another aspect, the description includes methods of reducing the likelihood of cancer recurrence in a subject, comprising administering to the subject a G-CSF antagonist and/or Bv8 antagonist, wherein said administering reduces the likelihood of cancer recurrence in the subject. In certain embodiments, the administering of the G-CSF antagonist and/or Bv8 antagonist prevents or reduces the likelihood of reoccurrence of a clinically detectable tumor, or metastasis thereof. In certain embodiments, the subject has had definitive surgery prior to the administering the G-CSF antagonist and/or Bv8 antagonist.

In another aspect, the description includes methods of preventing the regrowth of a tumor in a subject comprising the steps of removing the tumor and thereafter administering to the subject a G-CSF antagonist and/or Bv8 antagonist. In yet another aspect, the description includes methods of preventing the recurrence of cancer in a subject having a tumor comprising the steps of removing the tumor and thereafter administering to the subject a G-CSF antagonist and/or Bv8 antagonist. In certain embodiments, the methods further comprise a period of time between removal of the tumor and administering the G-CSF antagonist and/or Bv8 antagonist, wherein the period of time is sufficient for the surgical incision to be fully healed or to reduce the risk of wound dehiscence.

In certain embodiments, the G-CSF antagonist administered is an anti-G-CSF antibody or a fragment thereof. In certain embodiments, the Bv8 antagonist administered is an anti-Bv8 antibody or a fragment thereof. In certain embodiments, the Bv8 antagonist administered is an anti-PKR1 antibody or a fragment thereof.

In certain embodiments, the antagonists and antibodies of the present invention are administered sequentially. In certain embodiments, the antagonists and antibodies of the present invention are administered concurrently.

In one aspect, the invention provides compositions for use in methods of inhibiting lung metastasis, liver metastasis, brain metastasis, ovary metastasis, lymph node metastasis and/or bone metastasis comprising administered to a subject an effective amount of an anti-G-CSF antibody and/or anti-Bv8 antibody. In another aspect, the invention provides compositions for use in methods of inhibiting lung metastasis comprising administered to a subject an effective amount of an anti-G-CSF antibody, wherein the anti-G-CSF antibody inhibits tumor from metastasizing to a pre-metastatic lung. In another aspect, the description includes methods of inhibiting lung metastasis comprising administered to a subject an effective amount of an anti-Bv8 antibody, wherein the anti-Bv8 antibody inhibits tumor from metastasizing to a pre-metastatic lung. In another aspect, the description includes methods of inhibiting lung metastasis comprising administered to a subject an effective amount of an anti-PKR1 antibody, wherein the anti-PKR1 antibody inhibits tumor from metastasizing to a pre-metastatic lung. In another aspect, the invention provides compositions for use in methods of inhibiting lung metastasis comprising administered to a subject an effective amount of an anti-G-CSF antibody and an effective amount of an anti-Bv8 antibody. In another aspect, the invention provides compositions for use in methods of inhibiting lung metastasis comprising administered to a subject an effective amount of an anti-G-CSF antibody and an effective amount of an anti-PKR1 antibody. In another aspect, the description includes methods of inhibiting lung metastasis comprising administered to a subject an effective amount of an anti-Bv8 antibody and an effective amount of an anti-PKR1 antibody.

In another aspect, the invention provides compositions for use in methods of inhibiting liver metastasis comprising administered to a subject an effective amount of an anti-G-CSF antibody, wherein the anti-G-CSF antibody inhibits tumor from metastasizing to a pre-metastatic liver. In another aspect, the description includes methods of inhibiting liver metastasis comprising administered to a subject an effective amount of an anti-Bv8 antibody, wherein the anti-Bv8 antibody inhibits tumor from metastasizing to a pre-metastatic liver. In another aspect, the description includes methods of inhibiting liver metastasis comprising administered to a subject an effective amount of an anti-PKR1 antibody, wherein the anti-PKR1 antibody inhibits tumor from metastasizing to a pre-metastatic liver.

In certain embodiments, the antibodies or antibody fragments can be chimeric, humanized or human.

In certain embodiments, the methods of the present invention further comprise administering to the subject an effective amount of a VEGF antagonist. In certain embodiments, the subject has a tumor previously treated with a VEGF antagonist. In certain embodiments, the subject is relapsed from or refractory to a VEGF antagonist. In certain embodiments, the VEGF antagonist is an anti-VEGF antibody or a fragment thereof. In certain embodiments, the anti-VEGF antibody is bevacizumab or a fragment or variant thereof. In certain embodiments, the methods of the present invention further comprise administering to the subject an effective amount of a chemotherapeutic agent. In certain embodiments, the subject being treated is subjected to chemotherapy and/or radiation therapy, where the chemotherapy may, for example, comprise the administration of a cytotoxic agent. In certain embodiments, the additional treatment is a treatment known as "standard of care" for the treatment of the particular tumor targeted. In certain embodiments, the G-CSF antagonist is administered in combination with a different anti-tumor agent and/or treatment regiment, such as chemotherapy and/or radiation therapy. In certain embodiments, the Bv8 antagonist is administered in combination with a different anti-tumor agent and/or treatment regiment, such as chemotherapy and/or radiation therapy. In certain embodiments, the methods of the present invention further comprise administration of an additional inhibitor of angiogenesis, such as, for example, an antibody to an angiogenic factor.

In certain embodiments, the subject is mammal. In certain embodiments, the subject is human. In certain embodiments, the subject is diagnosed with cancer. In certain embodiments, the tumor is cancer. In certain embodiments, the metastatic tumor is cancer. In certain embodiments, the cancer is colon cancer, lung cancer, breast cancer, renal cell cancer, ovarian cancer, prostate cancer, bladder cancer, melanoma or glioblastoma.

Any embodiment described above or any combination thereof applies to any and all methods of the inventions described herein.

## BRIEF DESCRIPTION OF THE DRAWINGS

**Figure 1. *Bv8* is strongly up-regulated in pre-metastatic lungs of mice bearing metastatic tumors. A.** Design and results of the microarray study comparing gene expression in lungs from Balb/c naive mice, mice bearing non-metastatic tumors 67NR and metastatic 4T1 tumors. The "metastatic" gene expression profile is clearly separated from both naive from "non-metastatic" profiles. Each profile column represents one individual mouse. **B.** Schematic representation of the top 24 up- and down-regulated genes in the pre-metastatic lungs from mice bearing 4T1 tumors compared to naive or 67NR-bearing mice. **C.** Bv8 protein concentrations in the pre-metastatic lungs of mice bearing various tumors (n=3 per group). **D.** FACS analysis of Cd11b+Gr1+ cells in the pre-metastatic lungs of Balb/c mice bearing various tumors. Graphs present Means ± SEM.

**Figure 2. Increased levels of G-CSF and Bv8 are associated with a metastatic phenotype. A.** Plasma levels of SDF1α, PIGF, VEGF-A, M-CSF, GM-CSF, G-CSF and Bv8 two weeks after inoculation of non-metastatic or metastatic tumor cells. Asterisk (*) indicates significant difference when compared to naive group. **B.** Analysis of total numbers of Cd11b+Gr1+ cells in lungs from mice bearing 4T1 tumors and treated with anti-Bv8, anti-G-CSF antibodies, or combination (n=10 per group). 4T1 cells were orthotopically inoculated into the $4^{th}$ mammary fat pad of female CB6F1 mice. Treatment with antibodies began 2 days after inoculation of the cells, and was performed as described in Materials and Methods section in Example 1. Lungs were perfused with PBS and harvested 1 week (pre-metastatic phase) or 5.5 weeks (metastatic phase) after inoculation of cells. Asterisk (*) indicates significant difference when compared to corresponding ISO group. **C.** Numbers of tumors in lungs of mice bearing 4T1 tumors and treated as in panel **B.** Analysis was performed 5.5 weeks after tumor inoculation. ISO - isotype control antibody (n=5 per group). Asterisk (*) indicates significant difference relative to Naive group. **D.** Numbers of lung tumors analyzed by micro-CT in Balb-c mice bearing 66c14 tumors and treated with control (ISO) or anti-Bv8 antibody (n=8 for ISO group, and n=10 for anti-Bv8 group). Renderings (2 per group) of representative micro-CT scanned lungs demonstrating metastatic nodules (red) in ISO and anti-Bv8 groups are shown below the graph. Data shown are Means ± SEM.

**Figure 3. G-CSF initiates the pre-metastatic "niche" and enhances the metastatic potential of several tumors.**

**A.** FACS analysis of Cd11b+Gr1+ cells in tissues isolated from mice treated with vehicle or human rG-CSF (for 5 days, 10μg/mouse). Tissues were harvested twenty-four hours after the last dose of rG-CSF. Mice were perfused with PBS prior to the tissue harvest (n=5 per group). Asterisk (*) indicates significant difference when compared to Vehicle group. Data are represented as a percentage of Cd11b+Gr1+ (or Cd11b+Gr1-) cells among live cells. **B.** Bv8 levels measured by ELISA in tissues matching those in panel **A.** Values were normalized to the total protein content and are plotted on a logarithmic scale. Asterisk (*) indicates significant difference when compared to Vehicle group (n=5 per group). **C.** Numbers of tumors in lungs of mice pre-treated for 5 days with human rG-CSF (10μg/mouse, grey bars) or vehicle (white bars). Following G-CSF treatment, mice (5 per group) were injected intravenously (through tail-vein) with tumor cells (10,000 cells per mouse). 66c14 and 4T1 cells were injected into Balb/c mice, whereas B16F10 cells into C57BL/6 mice. rG-CSF treatment followed for another 5 days. Lungs were analyzed for the presence of visible tumors 3 weeks after cell inoculation. Asterisk (*) indicates significant difference between vehicle and G-CSF treated mice (n=5 per group). **D.** Representative images of lungs from mice injected with B16F10 cells and pre-treated with either vehicle or rG-CSF as in panel **C.** The lung from a mouse treated with rG-CSF has the higher number of tumors (black spots). **E.** Number of lung tumors in Balb/c mice intravenously injected with non-metastatic 67NR cells and treated with human rG-CSF as in panel **C.** Mice were analyzed for the presence of visible lung tumors 3 weeks after cell inoculation. Asterisk (*) indicates significant difference. Frequency denotes numbers of mice with detectable tumors in lungs (n=5 per group). **F.** Numbers of tumors in lungs of mice treated daily with mouse rG-CSF (2.5μg/mouse) and then treated with control (ISO), anti-Bv8, anti-Gr1 or anti-G-CSF antibody and injected intravenously with 66c14 cells as described in Materials and Methods section in Example 1. Lungs were analyzed for the presence of tumors 3 weeks after cells inoculation. Data shown are Means ± SEM.

**Figure 4. Bv8 mediates G-CSF induced metastasis through enhancement of cancer cell migration. A.** qRT-PCR analysis of *PKR1* and *PKR2* expression by non- and metastatic cancer cells *in vitro.* Data are presented as values normalized to *Hprt1* expression (n=6 per group). Asterisk (*) indicates significant difference when compared to 67NR cells. **B.** Migration of non-metastatic and metastatic cells in response to increasing concentrations of human Bv8. 1% FBS served as positive control. Cells that had migrated through the collagen-coated 8μm pore well were counted 16 hours after assay initiation (n=5 per group). Asterisk (*) indicates significant difference when compared to untreated group. **C.** *In vivo* extravasation of 4T1 cells assessed 36 hours after intravenous inoculation (tail-vein) of cells labeled with CellTracker Green. Balb/c Nude mice were pre-treated with mouse rG-CSF (2.5μg/mouse, daily) for 5 consecutive days and then were treated with control (ISO), anti-Bv8, anti-Gr1 or anti-G-CSF antibodies. "No label" denotes a group of mice which was inoculated with cells that had not been labeled with CellTracker Green. Images show 4T1 tumor cells (indicated with white arrow heads) in representative lung sections. Scale bar = 50μm. **D.** Quantification of the *in vivo* extravasation assay from panel **C.** On average, three mice per group were used and 15 images (5 random sections per mouse) were included into the analysis. **E.** Kaplan-Meier curves representing the probability of overall survival of breast cancer patients from Pawitan Breast dataset stratified based on the expression of G-CSF. Curves shows a strong correlation between high levels of G-CSF and reduced survival. **F.** Schematic model of the role of G-CSF and Bv8 in metastasis. LU - lung, T - primary tumor, BM - bone marrow. Data are Means ± SEM.

**Figure 5. Pre-metastatic lungs are tumor-free at the time of tissue harvest. Additional analysis of the pre-metastatic lungs from mice bearing tumors. A.** Schematic representation of 4T1-related cell lines used in the study, with known metastatic sites. **B.** qRT-PCR expression analysis of *Hygromycin* gene in lungs or primary tumors showing absence of *Hygromycin* signal in lungs from mice bearing 4T1-Luc-zsGreen-Hygro tumors. Note positive signal from the primary tumor. Lungs were harvested 6 days after tumor cell inoculation. **C.** BLI imaging of mice bearing 4T1-Luc-zsG-Hygro tumors. Note the lack of signal in the lung area and positive signal in the primary tumor. **D.** *Bv8* qRT-PCR analysis of lung tissues used for microarray analysis. **E.** qRT-PCR analysis of Bv8 expression in the pre-metastatic lungs of mice bearing non-and metastatic tumors. **F.** qRT-PCR analysis of *Bv8* transcript levels in Cd11b+Gr1+ and Cd11b+Gr1- cells isolated from lung tissue from Naive mice or mice with 4T1 tumors. **G.** Example of Bv8 IHC staining in the pre-metastatic lungs from mice bearing 67NR or 4T1 tumors. Dark staining in the pre-metastatic lung tissue bearing 4T1 tumors indicates Bv8-positive cells. 67NR or 4T1 cells were orthotopically inoculated in the $4^{th}$ mammary fat pad and lungs were harvested 1 week later and Bv8 was detected by IHC as described in Materials and Methods section in Example 1. Scale bar represents 50μm. **H.** FACS analysis of Cd11b+Gr1+ in pre-metastatic lungs from mice bearing B16F10 and LLC tumors (Matrigel-injected mice served as control group), as well as in MMTV-PyMT mice (PyMT-negative siblings served as control group) (n=5 per group). **I.** Bv8 expression levels in the pre-metastatic lungs from mice with LLC (Bv8 protein measured by ELISA), B16F10 and MMTV-PyMT (Bv8 RNA measured by qRT-PCR) tumors. Data are shown as expression relative to Naive tissue. Values shown are Means ± SEM.

**Figure 6. Additional analysis of G-CSF and Bv8 expression in tumor-bearing mice. Additional analysis of tumor burden. A.** G-CSF concentrations in cell culture supernatants after 48h-incubation, as described in Materials ands Methods section in Example 1 (n=3 per group). Asterisk (*) indicates significant difference when compared to Naive group. **B.** Bv8 levels in lungs and plasma in mice bearing 4T1 tumors and treated with ISO control or anti-G-CSF antibody (n=5 per group). Asterisk (*) indicates significant difference when compared to Naive group. **C.** Plasma and tumor G-CSF levels in mice bearing 4T1 tumors in the pre-metastatic and metastatic phases (n=5 per group). Asterisk (*) indicates significant difference when compared to Naive group. **D.** 4T1 primary tumor growth in mice treated with control (ISO), anti-Bv8, anti-G-CSF or both antibodies (n=10 per group). 4T1 cells were orthotopically inoculated into the right $4^{th}$ mammary fat pad of female CB6F1 mice. Asterisk (*) next to anti-Bv8 treatment group indicates significant difference when compared to ISO group. **E.** 66c14 primary tumor growth in mice treated with control (ISO), anti-Bv8, anti-G-CSF and both antibodies (n=10 per group). 66c14 cells were orthotopically inoculated into the right $4^{th}$ mammary fat pad of female Balb/c mice. Asterisk (*) next to anti-Bv8 treatment group indicates significant difference when compared to ISO group. **F.** Lung tumor multiplicity in mice bearing 66c14 tumors 6 weeks after tumor inoculation. Tumors were implanted and treatment performed as in panel **E** (n=10 per group). Asterisk (*) indicates significant difference when compared to ISO group. Graphs show Means ± SEM.

**Figure 7. Analysis of G-CSF induced metastasis in mice intravenously (tail-vein) injected with cancer cells. A.** Increases in lung mass (compared to lungs from Naive mice) following inoculation of tumor cells and treatment with human rG-CSF or Vehicle. Data correspond to the groups in **Fig. 3C.** Asterisk (*) indicates significant difference (n=5 per group). **B.** H&E staining of lungs from mice injected with 67NR cells and treated with Vehicle or G-CSF. Note presence of tumors in the G-CSF-treated group. Scale bar in magnified images = 50μm. T - tumor area, L - normal lung area. C. FACS analysis of Cd11b+Gr1+ cells in lungs of mice treated daily (for 3 days) with mouse rG-CSF (1μg/mouse) or vehicle and treated with control antibody (ISO), anti-Bv8, anti-Gr1 or anti-G-CSF antibody as described in Materials and Methods section in Example 1 (n=5 per group). **D.** Number of lung tumors in mice 3 weeks after inoculation of 4T1 cells. Mice were pre-treated (daily) with vehicle or human rG-CSF and treated with control (ISO) or anti-Bv8 or anti-Gr1 antibody. Treatment with both, rG-CSF and antibodies, continued for additional 5 days after inoculation of cancer cells. **E.** Number of lung tumors in mice inoculated with 4T1 cells. Lungs were analyzed 3 weeks later. Animals were also treated with control (ISO), anti-Bv8, anti-VEGF, anti-Gr1, or anti-G-CSF antibody. **F.** FACS analysis of VEGFR1+ cells in lungs in mice treated daily with mouse rG-CSF or vehicle for 3 consecutive days. G. FACS analysis of Cd11b+Gr1+ (and Gr1-) cells in lung in mice treated daily with mouse rG-CSF or vehicle as in panel **F. H.** Distribution of VEGFR1+ cells among all Cd11b+Gr1+ (or Gr1-) cells in mice dosed with vehicle or mouse rG-CSF as in panels **F** and **G.** For panels **F, G,** and **H** asterisk (*) indicates significant difference when compared to Vehicle group, NS - not significant (n=5 per group). Values shown are Means ± SEM.

**Figure 8. Mechanisms underlying the G-CSF-initiated pre-metastatic microenvironment. A.** qRT-PCR gene expression analysis of *MMP-9, S100A8* and *S100A9* in total lung tissue from mice pre-treated with vehicle or mouse rG-CSF (1μg/mouse, daily, for 3 days), and then treated with control (ISO), anti-Bv8, anti-Gr1 or anti-G-CSF antibodies. Asterisk (*) indicates significant difference when compared to G-CSF+ISO group (n=5 per group). **B.** *Bv8, S100A8, S100A9* and *MMP9* gene expression measured by qRT-PCR in Cd11b+Gr1+ (or Gr1-) cells sorted from lungs from mice pre-treated with vehicle or rG-CSF. Single cell suspensions were generated from the tissues, cells were stained with specific anti-Cd11b and anti-Gr1 antibodies, and double-positive cells (Cd11b+Gr1+), Cd11b+Gr1-, and double-negative cells (Cd11b-Gr1-) cells populations were separated by FACS. Total RNA was isolated from the cells and gene expression was measured by qRT-PCR. Data are presented as relative expression to *Hprt1* (n=5 per group). Asterisk (*) indicates significant difference when compared to Vehicle group. **C.** MMP-9 concentrations measured by ELISA in the pre-metastatic or metastatic lungs from mice bearing orthotopically inoculated 4T1 tumors and treated with control (ISO), anti-Bv8, anti-G-CSF or combination of both anti-Bv8 and anti-G-CSF antibodies (n=5 per group). Naïve - lungs from mice without tumor. Samples are from the corresponding experiment in **Fig. 2B.** Asterisk (*) indicates significant difference when compared to ISO group. **D.** Immunoblot analysis showing phosphorylation of ERK1/2 (p-ERK1/2) upon stimulation with Bv8 in 67NR and 66c14 cells. Cells were stimulated with Bv8 (5ng/ml) or with 1% FBS for the indicated times. Duplicate samples are shown. **E.** Quantification of *in vivo* extravasation of 66c14 cells assessed 36 hours after intravenous inoculation (tail-vein) of cells labeled with CellTracker Green. Mice were pre-treated with mouse rG-CSF (2.5μg/mouse, daily) for 5 consecutive days and then treated with control (ISO), anti-Bv8, anti-Gr1 or anti-G-CSF antibodies. "No label" denotes a group of mice which was inoculated with cells that had not been labeled with CellTracker Green. Values shown are Means ± SEM.

**Figure 9. High G-CSF expression in cancer patients is associated with reduced survival. A.** Kaplan-Meier curves representing the probability of overall survival of breast cancer patients from Chin Breast group stratified based on the expression of G-CSF. Graph shows strong correlation between high levels of G-CSF and reduced survival. **B.** Kaplan-Meier curve representing the probability of overall survival of breast cancer patients from Blaveri Bladder group stratified on the basis of G-CSF. Graph shows significant correlation between high levels of G-CSF and shorter survival. C. Summary of datasets used in this study to analyze clinical significance of G-CSF.

**Figure 10. Pre-metastatic lungs are tumor-free at the time of tissue harvest. Additional analysis of the pre-metastatic lungs from tumor-bearing mice.** Timing of development of lung metastasis in 4T1 tumor bearing mice, qRT-PCR expression analysis of *Hygromycin* gene in lungs or primary tumors showing absence of *Hygromycin* signal in lungs up to 2 weeks after inoculation of 4T1-Luc-zsGreen-Hygro tumors. Note positive signal from the primary tumor. Lungs were harvested every 7 days after tumor inoculation and analyzed for the presence of *Hygromycin* that correlates with the presence of cancer cells in the tissue.

**Figure 11. FACS analysis of Cd11b+Gr1+ cells in pre-metastatic tissues. Additional analysis of Bv8 and G-CSF expression. A.** FACS analysis of different cell populations in pre-metastatic lungs from mice bearing 4T1 tumors, 2 weeks after tumor inoculation. **B.** G-CSF concentrations in cell culture supernatants after 48h-incubation (n=3 per group). Asterisk (*) indicates significant difference when compared to Naive and non-metastatic groups. **C.** Number of Ly6G+Ly6C+ cells in peripheral blood of Naive or PyMT-tumor bearing FVB mice treated with isotype IgG (ISO), anti-Bv8 (2D3) or anti-GCSF antibody (upper panel). FACS analysis of Ly6G+Ly6C+ cells in lung, primary tumor and bone marrow of Naive or PyMT-tumor bearing FVB mice (lower panel). Asterisk (*) indicates significant difference when compared to Naive group, while double asterisk (**) indicates significant difference when compared to ISO group.

**Figure 12. Increased levels of G-CSF and Bv8 are associated with a metastatic phenotype. A.** Numbers of tumor foci in lungs of SCID/bg mice bearing MDA-MB-231-X1.1 tumors and treated with anti-Bv8 (2D3) or anti-G-CSF for 6 weeks (n=10 per group). Asterisk (*) indicates significant difference relative to ISO group. **B.** MDA-MB-231-X1.1 primary tumor growth in SCID/bg mice treated with control (ISO), anti-Bv8 (2D3), or anti-G-CSF (n=10 per group). **C.** Numbers of lung tumors in Balb-c Nude mice bearing 66c14 tumors and treated with indicated antibody (n=10 per group). Asterisk (*) indicates significant difference relative to ISO group. Data shown are Means $\pm$ SEM.

**Figure 13. Analysis of lung tumor burden and primary tumor growth in mice bearing orthotopically inoculated tumors. A** and **B.** Number of lung metastases in mice with Bv8 WT or KO BMMNCs obtained by fetal liver transplantation. Mice were inocluated with 66c14, panel **A,** or 4T1, panel **B,** tumors (orthotopic model) and treated with isotype control IgG (ISO) or anti-Bv8 (2B9+3F1) antibody. Asterisk (*) indicates significant difference when compared to WT-ISO group. **C.** Summary of statistical analysis of Bv8 WT and KO expereiments from panels **A** and **B.** Note that comparison is significant only if linear function values are less than 0. Statistical significance was achieved only for the following comparisons: WT anti-Bv8 vs WT ISO, KO ISO vs WT ISO, KO anti-Bv8 vs WT ISO and ISO vs all other groups. **D.** Number of metastases per lung in FVB mice bearing MMTV-PyMT tumors 7 weeks after tumor inoculation. Tumors were implanted and treatment performed (n=6 per group). Asterisk (*) indicates significant difference when compared to ISO group. **E.** MMTV-PyMT primary tumor growth in FVB mice treated with control (ISO), anti-Bv8 (2D3), or anti-G-CSF (n=10 per group). **F.** Expression level of mouse and human G-CSF (mG-CSF and hG-CSF) in plasma and primary tumor in mice bearing MDA-MB-231-X1.1 tumors. Asterisk (*) indicates significant difference when compared to Naive group. Data shown are Means $\pm$ SEM.

**Figure 14. G-CSF initiates the pre-metastatic "niche" and enhances the metastatic potential of several tumors. A.** Numbers of tumors in lungs of mice treated daily with vehicle or mouse rG-CSF (2.5 $\mu$g/mouse) and treated with control (ISO), anti-Bv8 (2D3), or anti-G-CSF antibody and injected intravenously with MDA-MB-231-L1.1 cells (n=5 per group). **B.** Numbers of tumors in lungs of mice treated daily with vehicle or rG-CSF (2.5 $\mu$g/mouse) and then treated with control (ISO), anti-Bv8 (2D3), anti-Ly6G, anti-Gr1 antibody or anti-G-CSF antibody and injected intravenously with 66c14 cells. Lungs were analyzed for the presence of tumors 3 weeks after cells inoculation (n=10 per group). In panels **A** and **B,** asterisk (*) indicates significant difference when compared to Vehicle-ISO group, whereas double asterisk (**) indicates significant difference when compared to G-CSF-ISO group. Data shown are Means $\pm$ SEM.

**Figure 15. Bv8 mediates G-CSF induced metastasis through enhancement of cancer cell migration. A.** Number of lung tumors in Balb/c mice intravenously injected with 67NR or 67NR-PKR1 cells. Mice were pre-treated with vehicle or human rG-CSF. Mice were analyzed for the presence of visible lung tumors 3 weeks after cell inoculation. Asterisk (*) indicates significant difference between vehicle and G-CSF treated mice. Frequency denotes numbers of mice with detectable tumors in lungs (n=10 per group). **B.** Gene expression analysis of MDA-MB-231 cells that were isolated either from lung metastases (Lung) or from prmary tumors (Tumor). Three independent cell lines were analyzed per each group (for Lung: cell lines L1.1, L2.1, and L3.1; for Tumor: cell lines T1.1, T2.1, and T3.1). Expression was compared to the parental MDA-MB-231-D3H1 cell line. Asterisk (*) indicates significant difference when compared to parental cell line, whereas double asterisk (**) indicates significant difference when compared to Tumor or parental cell lines. Data are Means ± SEM. C. Schematic presentation of MDA-MB-231 clones used for gene expression analysis in **Fig. 15B.** MDA-MB-231-D3H1 were injected into 4th mammary fat pad or through tail-vein of SCID/bg mice. Subsequently, cancer cells from established tumors (either in breast or lung) were isolated and expanded *in vitro.* From each tissue, three independent cell lines (L1.1, L2.1, and L3.1 from three seperate lungs and T1.1, T2.1, and T3.1 from three independent primary tumor) were established.

**Figure 16. Mechanisms underlying the G-CSF-initiated pre-metastatic microenvironment. A.** qRT-PCR analysis of *PKR1* expression in 67NR cells over-expressing PKR1. Note that the expression level is comparable to 66c14 cells. **B.** qRT-PCR analysis of *PKR1* and *G-CSF* expression in 66c14 cells expressing shRNA targeting *PKR1* (shPKR1). Note that *G-CSF* is not affected by shPKR1. sh(Control) is a scrambled shRNA used as control. Asterisk (*) indicates significant difference when compared to sh(Control) group. **C.** Number of tumors in lungs of mice that were pre-treated with vehicle or G-CSF and injected with 66c14 or 66c14-shPKR1 cells. Analysis was performed 3 weeks after cell inoculation. Asterisk (*) indicates significant difference when compared to 66c14-WT ISO group, while double asterisk (**) indicates significant difference when compared to 66c14-WT G-CSF group (n=10 per group). Data shown are Means ± SEM.

## DETAILED DESCRIPTION OF THE INVENTION

Before describing the present invention in detail, it is to be understood that this invention is not limited to particular compositions or biological systems, which can, of course, vary. It is also to be understood that the terminology used herein is for the purpose of describing particular embodiments only, and is not intended to be limiting.

Unless defined otherwise, technical and scientific terms used herein have the same meaning as commonly understood by one of ordinary skill in the art to which this invention belongs. Singleton *et al.,* Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### A. Definitions

For purposes of interpreting this specification, the following definitions will apply and whenever appropriate, terms used in the singular will also include the plural and vice versa.

The terms "granulocyte colony-stimulating factor", "G-CSF", "colony-stimulating factor 3" and "CSF3" are used herein interchangeably, and refer to the full-length polypeptide and/or the active fragments of the full-length polypeptide.

"Native sequence G-CSF" comprises a polypeptide having the same amino acid sequence as G-CSF derived from nature, regardless of its mode of preparation. Thus, native sequence G-CSF can have the amino acid sequence of naturally occurring human G-CSF, murine G-CSF, or G-CSF from any other mammalian species. Examples of native sequence human G-CSF amino acid sequences are shown in SEQ ID NOs: 8, 10, 12 and 14. Human and murine G-CSF sequences are also disclosed, for example, in Nagata et al. Nature 319, 415-418(1986); Nagata et al. EMBO J. 5, 575-581 (1986), and Tsuchiya et al. Proc Natl Acad Sci 83(20), 7633-7637 (1986). Such native sequence G-CSF can be isolated from nature or can be produced by recombinant and/or synthetic means. The term "native sequence G-CSF" specifically encompasses naturally occurring prepro, pro and mature forms and truncated forms of G-CSF, naturally occurring variant forms and naturally occurring allelic variants.

"G-CSF variants" are biologically active G-CSF polypeptides having an amino acid sequence which differs from the sequence of a native sequence G-CSF polypeptide for human and murine G-CSF, by virtue of an insertion, deletion, modification and/or substitution of one or more amino acid residues within the native sequence. G-CSF variants generally have less than 100% sequence identity with a native sequence G-CSF, such as the human G-CSF shown in SEQ ID NOs: 8, 10, 12 and 14. Ordinarily, however, a biologically active G-CSF variant will have an amino acid sequence with at least about 70% amino acid sequence identity with the amino acid sequence of a naturally occurring G-CSF. In certain embodiments, a biologically active G-CSF variant will have an amino acid sequence with at least about 75%, about 80%, about 85%, about 90%, about 95%, or at least about 99% amino acid sequence identity, in 1% increments, with the amino acid sequence of a naturally occurring G-CSF. In certain embodiments, the G-CSF variants include peptide fragments of at least 5 amino acids that retain a biological activity of the corresponding native sequence G-CSF polypeptide. G-CSF variants also include G-CSF polypeptides wherein one or more amino acid residues are added at the N- or C-terminus of, or within, a native G-CSF sequence. G-CSF variants also include G-CSF polypeptides where a number of amino acid residues are deleted and optionally substituted by one or more amino acid residues. G-CSF variants also may be covalently modified, for example by substitution with a moiety other than a naturally occurring amino acid or by modifying an amino acid residue to produce a non-naturally occurring amino acid.

The term "G-CSF antagonist" when used herein refers to a molecule which binds to G-CSF and inhibits or substantially reduces a biological activity of G-CSF. Non-limiting examples of G-CSF antagonists include antibodies or antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of G-CSF, antisense molecules directed to G-CSF, RNA aptamers, and ribozymes against G-CSF. In one embodiment of the invention, the G-CSF antagonist is an antibody, especially an anti- G-CSF antibody or fragment thereof which binds human G-CSF.

By "G-CSF antagonist antibody" is meant an antibody that is a G-CSF antagonist, as hereinabove defined, and thus partially or fully blocks, inhibits, or neutralizes the ability to modulate Cd11b+Gr1+ cell mobilization or functional human counterpart of CD11b+Gr1+ cell mobilization, modulate expression of Bv8, promote tumor angiogenesis and/or promote tumor metastasis. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cell is neutrophils, monocytes or macrophages.

The terms "Bv8," "Bv8 homologue," "prokineticin-2," (also known as "PK2," KAL4," and "MIT1") are used herein interchangeably, the full-length polypeptide and/or the active fragments of the full-length polypeptide.

"Native sequence Bv8" comprises a polypeptide having the same amino acid sequence as Bv8 derived from nature, regardless of its mode of preparation. Thus, native sequence Bv8 can have the amino acid sequence of naturally occurring human Bv8, murine Bv8, or Bv8 from any other mammalian species. For example a full-length native sequence human Bv8 amino acid sequence is shown in SEQ ID NO: 2. A second full-length native sequence human Bv8 is shown in SEQ ID NO: 4. These two sequences are the result of the alternative splicing of an exon that encodes a canonical heparin binding domain. Thus the native sequence human Bv8 whose amino acid sequence is shown in SEQ ID NO: 2 comprises a heparin binding domain, while the native sequence Bv8 depicted in SEQ ID NO: 4 does not. A native sequence mouse Bv8 amino acid sequence is shown in SEQ ID NO: 6. Human and murine Bv8 sequences are also disclosed, for example, in Wechselberger et al. (FEBS Lett. 462:177-181 (1999)) and Li et al. (Mol. Pharm. 59:692-698 (2001)). Such native sequence Bv8 can be isolated from nature or can be produced by recombinant and/or synthetic means. The term "native sequence Bv8" specifically encompasses naturally occurring prepro, pro and mature forms and truncated forms of Bv8, naturally occurring variant forms (*e.g.* alternatively spliced forms, such as that shown in SEQ ID NO: 4, and naturally occurring allelic variants. In certain embodiments, native sequence Bv8 is a full-length native sequence human Bv8 as shown in SEQ ID NO: 2. In certain embodiments, native sequence Bv8 is a full-length native sequence human Bv8 as shown in SEQ ID NO: 4.

"Bv8 variants" are biologically active Bv8 polypeptides having an amino acid sequence which differs from the sequence of a native sequence Bv8 polypeptide, such as those shown in (SEQ ID NOs: 2, 4 and 6 for human and murine Bv8, by virtue of an insertion, deletion, modification and/or substitution of one or more amino acid residues within the native sequence. Bv8 variants generally have less than 100% sequence identity with a native sequence Bv8, such as the human Bv8 of SEQ ID NO: 2 or SEQ ID NO:4. Ordinarily, however, a biologically active Bv8 variant will have an amino acid sequence with at least about 70% amino acid sequence identity with the amino acid sequence of a naturally occurring Bv8. In certain embodiments, a biologically active Bv8 variant will have an amino acid sequence with at least about 75%, about 80%, about 85%, about 90%, about 95%, and to at least about 99% amino acid sequence identity, in 1% increments, with the amino acid sequence of a naturally occurring Bv8. The Bv8 variants include peptide fragments of at least 5 amino acids that retain a biological activity of the corresponding native sequence Bv8 polypeptide. Bv8 variants also include Bv8 polypeptides wherein one or more amino acid residues are added at the N- or C-terminus of, or within, a native Bv8 sequence. Bv8 variants also include Bv8 polypeptides where a number of amino acid residues are deleted and optionally substituted by one or more amino acid residues. Bv8 variants also may be covalently modified, for example by substitution with a moiety other than a naturally occurring amino acid or by modifying an amino acid residue to produce a non-naturally occurring amino acid. Bv8 variants may comprise a heparin binding domain.

The term "Bv8 antagonist," as used herein, refers to any molecule that partially or fully blocks, inhibits, or neutralizes the ability of a native sequence Bv8 to modulate mobilization of Cd11b+Gr1+ cells or functional human counterpart of CD11b+Gr1+ cells, to promote angiogenesis during tumor development and/or to promote tumor metastasis. Suitable antagonist molecules specifically include antagonist antibodies or antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of Bv8, and fusions proteins, receptor molecules and derivatives which bind specifically to Bv8 thereby sequestering its binding to its target, antagonist variants of Bv8, antisense molecules directed to Bv8, RNA aptamers, and ribozymes against Bv8.

In particular, Bv8 antagonists include, without limitation, antibodies and antibody fragments specifically binding to a native sequence Bv8 polypeptide, or a native sequence Bv8 receptor (PKR1/EG-VEGFR1 or PKR2/EG-VEGFR2) polypeptide. In certain embodiments, Bv8 antagonist is PKR1 antagonist. In certain embodiments, PKR1 antagonist is anti-PKR1 antibody. Methods for identifying antagonists of a Bv8 polypeptide may comprise contacting a Bv8 polypeptide with a candidate antagonist molecule and measuring a detectable change in the ability of Bv8 to modulate Cd11b+Gr1+ cell mobilization or functional human counterpart of CD11b+Gr1+ cell mobilization, promote tumor angiogenesis and/or promote tumor metastasis.

By "Bv8 antagonist antibody" is meant an antibody that is a Bv8 antagonist, as hereinabove defined, and thus partially or fully blocks, inhibits, or neutralizes the ability to modulate Cd11b+Gr1+ cell mobilization or functional human counterpart of CD11b+Gr1+ cell mobilization, promote tumor angiogenesis and/or promote tumor metastasis.

"Bv8 receptor" is a molecule to which Bv8 binds and which mediates the biological properties of Bv8. Therefore, the term "Bv8 receptor" includes within its meaning PKR1/GPR73/EG-VEGF receptor-1/ PROKR1 and PKR2/ GPR73L1/EG-VEGF receptor-2/PROKR2 (LeCouter et al., 2003, Proc. Natl. Acad. Sci. USA, 100:2685-2690; Lin et al., 2002, J. Biol. Chem., 277:19276-19280; Masuda et al., 2002, Biochem. Biophys. Res. Commun., 293:396-402).

The term "biological activity" and "biologically active" with regard to a polypeptide refer to the ability of a molecule to specifically bind to and regulate cellular responses, e.g., proliferation, migration, etc. Cellular responses also include those mediated through a receptor, including, but not limited to, migration, and/or proliferation. In this context, the term "modulate" includes both promotion and inhibition.

"Active" or "activity," in connection with Bv8 or G-CSF, for the purposes herein refers to form(s) of Bv8 or G-CSF which retain a biological and/or an immunological activity of native or naturally-occurring Bv8 or G-CSF, wherein "biological" activity refers to a biological function (either inhibitory or stimulatory) caused by a native or naturally-occurring Bv8 or G-CSF, other than the ability to induce the production of an antibody against an antigenic epitope, possessed by a native or naturally-occurring Bv8 or G-CSF, and an "immunological" activity refers to the ability to induce the production of an antibody against an antigenic epitope possessed by a native or naturally-occurring Bv8 or G-CSF. In certain embodiments, the biological activity of G-CSF is the ability to modulate Cd11b+Gr1+ cell (or functional human counterpart of CD11b+Gr1+ cell) mobilization, modulate expression of Bv8, promote tumor angiogenesis and/or promote tumor metastasis. In certain embodiments, the biological activity of Bv8 is the ability to modulate Cd11b+Gr1+ cell (or functional human counterpart of CD11b+Gr1+ cell) mobilization, promote tumor angiogenesis and/or promote tumor metastasis. In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cells are human neutrophils, monocytes or macrophages.

A "hematopoietic stem/progenitor cell" or "primitive hematopoietic cell" is one which is able to differentiate to form a more committed or mature blood cell type. "Lymphoid blood cell lineages" are those hematopoietic precursor cells which are able to differentiate to form lymphocytes (B-cells or T-cells). Likewise, "lymphopoeisis" is the formation of lymphocytes. "Erythroid blood cell lineages" are those hematopoietic precursor cells which are able to differentiate to form erythrocytes (red blood cells) and "erythropoeisis" is the formation of erythrocytes.

The phrase "myeloid blood cell lineages", for the purposes herein, encompasses all hematopoietic progenitor cells, other than lymphoid and erythroid blood cell lineages as defined above, and "myelopoiesis" involves the formation of blood cells (other than lymphocytes and erythrocytes).

In certain embodiments, a myeloid cell population can be enriched in myeloid immune cells that are Gr1+/CD11b+ (or CD11b+Gr1+) or Gr1+/Mac-1+. In certain embodiments, a human myeloid cell population can be enriched in myeloid immune cells that are functional human counterpart of Gr1+/CD11b+ (or functional human counterpart of CD11b+Gr1+). *See e.g.*, Almand, B. et al. J Immunol 166, 678-689 (2001); Young, M. R. & Lathers, D. M. Int J Immunopharmacol 21, 241-252 (1999). In certain embodiments, the human counterpart cells are human immature myeloid cells. In certain embodiments, the human counterpart cells are human myeloid derived suppressor cells. In certain embodiments, the human counterpart cells are precursors of human neutrophils, monocytes or macrophages. In certain embodiments, the human counterpart cells are neutrophils, monocytes or macrophages. In certain embodiments, the myeloid immune cells express a marker for myeloid cells of the macrophage lineage, CD11b, and a marker for granulocytes, Gr1. A Gr1+/CD11b+ can be selected by immunoadherent panning, for example, with an antibody to Gr1+.

The term "Gr1 antagonist" when used herein refers to a molecule which binds to Gr1 and inhibits or substantially reduces a biological activity of Gr1. Non-limiting examples of Gr1 antagonists include antibodies, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of Gr1, antisense molecules directed to Gr1, RNA aptamers, and ribozymes against Gr1. In one embodiment of the invention, the Gr1 antagonist is an antibody, especially an anti-Gr1 antibody or fragment thereof which binds human Gr1. In certain embodiments, an anti-Gr1 antibody is capable of inhibiting the biological activity of G-CSF.

The term "VEGF" or "VEGF-A" as used herein refers to the 165-amino acid human vascular endothelial cell growth factor and related 121-, 189-, and 206- amino acid human vascular endothelial cell growth factors, as described by Leung et al. (1989) Science 246:1306, and Houck et al. (1991) Mol. Endocrin, 5:1806, together with the naturally occurring allelic and processed forms thereof. The term "VEGF" also refers to VEGFs from non-human species such as mouse, rat or primate. Sometimes the VEGF from a specific species are indicated by terms such as hVEGF for human VEGF, mVEGF for murine VEGF, and etc. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "$VEGF_{165}$." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

"VEGF biological activity" includes binding to any VEGF receptor or any VEGF signaling activity such as regulation of both normal and abnormal angiogenesis and vasculogenesis (Ferrara and Davis-Smyth (1997) Endocrine Rev. 18:4-25; Ferrara (1999) J. Mol. Med. 77:527-543); promoting embryonic vasculogenesis and angiogenesis (Carmeliet et al. (1996) Nature 380:435-439; Ferrara et al. (1996) Nature 380:439-442); and modulating the cyclical blood vessel proliferation in the female reproductive tract and for bone growth and cartilage formation (Ferrara et al. (1998) Nature Med. 4:336-340; Gerber et al. (1999) Nature Med. 5:623-628). In addition to being an angiogenic factor in angiogenesis and vasculogenesis, VEGF, as a pleiotropic growth factor, exhibits multiple biological effects in other physiological processes, such as endothelial cell survival, vessel permeability and vasodilation, monocyte chemotaxis and calcium influx (Ferrara and Davis-Smyth (1997), *supra* and Cebe-Suarez et al. Cell. Mol. Life Sci. 63:601-615 (2006)). Moreover, studies have reported mitogenic effects of VEGF on a few non-endothelial cell types, such as retinal pigment epithelial cells, pancreatic duct cells, and Schwann cells. Guerrin et al. (1995) J. Cell Physiol. 164:385-394; Oberg-Welsh et al. (1997) Mol. Cell. Endocrinol. 126:125-132; Sondell et al. (1999) J. Neurosci. 19:5731-5740.

A "VEGF antagonist" refers to a molecule capable of neutralizing, blocking, inhibiting, abrogating, reducing or interfering with VEGF activities including, but not limited to, its binding to one or more VEGF receptors. VEGF antagonists include, without limitation, anti-VEGF antibodies and antigen-binding fragments thereof, receptor molecules and derivatives which bind specifically to VEGF thereby sequestering its binding to one or more receptors, anti-VEGF receptor antibodies and VEGF receptor antagonists such as small molecule inhibitors of the VEGFR tyrosine kinases. The term "VEGF antagonist," as used herein, specifically includes molecules, including antibodies, antibody fragments, other binding polypeptides, peptides, and non-peptide small molecules, that bind to VEGF and are capable of neutralizing, blocking, inhibiting, abrogating, reducing or interfering with VEGF activities. Thus, the term "VEGF activities" specifically includes VEGF mediated biological activities of VEGF.

An "anti-VEGF antibody" is an antibody that binds to VEGF with sufficient affinity and specificity. In one embodiment, the anti-VEGF antibody can be used as a therapeutic agent in targeting and interfering with diseases or conditions wherein the VEGF activity is involved. An anti-VEGF antibody will usually not bind to other VEGF homologues such as VEGF-B or VEGF-C, nor other growth factors such as PlGF, PDGF or bFGF. In one embodiment, anti-VEGF antibodies include a monoclonal antibody that binds to the same epitope as the monoclonal anti-VEGF antibody A4.6.1 produced by hybridoma ATCC HB 10709; a recombinant humanized anti-VEGF monoclonal antibody (*see* Presta et al. (1997) Cancer Res. 57:4593-4599), including but not limited to the antibody known as "bevacizumab (BV)," also known as "rhuMAb VEGF" or "AVASTIN®." AVASTIN® is presently commercially available. Bevacizumab comprises mutated human $IgG_1$ framework regions and antigen-binding complementarity-determining regions from the murine antibody A.4.6.1 that blocks binding of human VEGF to its receptors. Approximately 93% of the amino acid sequence of bevacizumab, including most of the framework regions, is derived from human $IgG_1$, and about 7% of the sequence is derived from A4.6.1. Bevacizumab has a molecular mass of about 149,000 daltons and is glycosylated. Bevacizumab and other humanized anti-VEGF antibodies are further described in U.S. Pat. No. 6,884,879, issued February 26, 2005. Additional anti-VEGF antibodies include the G6 or B20 series antibodies (*e.g.*, G6-23, G6-31, B20-4.1), as described in PCT Application Publication No. WO2005/012359. For additional antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., Journal of Immunological Methods 288:149-164 (2004).

The term "B20 series polypeptide" as used herein refers to a polypeptide, including an antibody that binds to VEGF. B20 series polypeptides includes, but not limited to, antibodies derived from a sequence of the B20 antibody or a B20-derived antibody described in US Publication No. 20060280747, US Publication No. 20070141065 and/or US Publication No. 20070020267. In one embodiment, B20 series polypeptide is B20-4.1 as described in US Publication No. 20060280747, US Publication No. 20070141065 and/or US Publication No. 20070020267. In another embodiment, B20 series polypeptide is B20-4.1.1 described in US Publication No. 20090142343.

The term "G6 series polypeptide" as used herein refers to a polypeptide, including an antibody that binds to VEGF. G6 series polypeptides includes, but not limited to, antibodies derived from a sequence of the G6 antibody or a G6-derived antibody described in US Publication No. 20060280747, US Publication No. 20070141065 and/or US Publication No. 20070020267. G6 series polypeptides, as described in US Publication No. 20060280747, US Publication No. 20070141065 and/or US Publication No. 20070020267 include, but not limited to, G6-8, G6-23 and G6-31.

By "metastasis" is meant the spread of http://en.wikipedia.org/wiki/Cancercancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream or lymphatics, and stopping at a distant site. After the tumor cells come to rest at another site, they re-penetrate through the blood vessels or lymphatic walls, continue to multiply, and eventually another tumor is formed. At the new site, the cells establish a blood supply and can grow to form a life-threatening mass. In certain embodiments, this new tumor is referred to as a metastatic (or *secondary*) tumor. In certain embodiments, the term metastatic tumor refers to a tumor that is capable of metastasizing, but has not yet metastasized to tissues or organs elsewhere in the body. In certain embodiments, the term metastatic tumor refers to a tumor that has metastasized to tissues or organs elsewhere in the body. In certain embodiments, metastatic tumors are comprised of metastatic tumor cells.

The "metastatic organ" or "metastatic tissue" is used in the broadest sense, refers to an organ or a tissue in which the cancer cells from a primary tumor or the cancer cells from another part of the body have spread. Examples of metastatic organ and metastatic tissue include, but not limited to, lung, liver, brain, ovary, bone, bone marrow and lymph node.

The "pre-metastatic organ" or "pre-metastatic tissue" as used herein, refers to an organ or a tissue in which no cancer cells from a primary tumor or from another part of the body have been detected. In certain embodiments, the pre-metastatic organ or pre-metastatic tissue as used herein, refers to an organ or tissue that is in the phase before the spread of cancer cells from a primary tumor or from another part of the body to this organ or tissue have occurred. Examples of pre-metastatic organ or pre-metastatic tissue include, but not limited to, lung, liver, brain, ovary, bone, bone marrow and lymph node.

By "micrometastasis" is meant a small number of cells that have spread from the primary tumor to other parts of the body. Micrometastasis may or may not be detected in a screening or diagnostic test.

By "non-metastatic" is meant a cancer that is benign or that remains at the primary site and has not penetrated into the lymphatic or blood vessel system or to tissues other than the primary site. In certain embodiments, a non-metastatic cancer is any cancer that is a Stage 0, I, or II cancer, and occasionally a Stage III cancer.

Reference to a tumor or cancer as a "Stage 0," "Stage I," "Stage II," "Stage III," or "Stage IV" indicates classification of the tumor or cancer using the Overall Stage Grouping or Roman Numeral Staging methods known in the art. Although the actual stage of the cancer is dependent on the type of cancer, in general, a Stage 0 cancer is an in situ lesion, a Stage I cancer is small localized tumor, a Stage II and III cancer is a local advanced tumor which exhibits involvement of the local lymph nodes, and a Stage IV cancer represents metastatic cancer. The specific stages for each type of tumor is known to the skilled clinician.

By "primary tumor" or "primary cancer" is meant the original cancer and not a metastatic lesion located in another tissue, organ, or location in the subject's body. In certain embodiments, primary tumor is comprised of primary tumor cells.

By "benign tumor" or "benign cancer" is meant a tumor that remains localized at the site of origin and does not have the capacity to infiltrate, invade, or metastasize to a distant site.

"Cancer recurrence" herein refers to a return of cancer following treatment, and includes return of cancer in the primary organ, as well as distant recurrence, where the cancer returns outside of the primary organ.

By "tumor dormancy" is meant a prolonged quiescent state in which tumor cells are present but tumor progression is not clinically apparent. A dormant tumor may or may not be detected in a screening or diagnostic test.

By "tumor burden" is meant the number of cancer cells, the size of a tumor, or the amount of cancer in the body. Tumor burden is also referred to as tumor load.

By "tumor number" is meant the number of tumors.

A "URMCNAs" refers to nucleic acids that are upregulated in tumors, pre-metastatic organs and/or metastatic organs. URMCNAs include, but are not limited to, Stfa3, CAMP, Bv8, TNFSF14, MGAM, Ngp, S100A8, S100A9, LRP1B, CEACAM4, HAO1, C10orf46, CLECSF9, Mcpt8, PRTN3, Slfn3 and MMP-9. In certain embodiment, the URMCNAs refer to Bv8, S100A8, S100A9 and MMP-9.

A "URMCPs" refers to proteins that are upregulated in tumors, pre-metastatic organs and/or metastatic organs. URMCPs include, but are not limited to, Stfa3, CAMP, Bv8, TNFSF14, MGAM, Ngp, S100A8, S100A9, LRP1B, CEACAM4, HAO1, C10orf46, CLECSF9, Mcpt8, PRTN3, Slfn3 and MMP-9. In certain embodiment, the URMCPs refer to Bv8, S100A8, S 100A9 and/or MMP-9.

A "DRMCNAs" refers to nucleic acids that are downregulated in tumors, pre-metastatic organs and/or metastatic organs. DRMCNAs include, but are not limited to TYMS, CRIPTO CR-1, MPHOSPH1, CDH6, WWP2, DMC1, BCHE, NSLE16484, F9, GDAP1, LOC375188, DNTT, PPIL3, KCND2, ZNF597, IGSF4D and GTF3C4.

A "DRMCPs" refers to proteins that are downregulated in tumors, pre-metastatic organs and/or metastatic organs. DRMCPs include, but are not limited to TYMS, CRIPTO CR-1, MPHOSPH1, CDH6, WWP2, DMC1, BCHE, NSLE16484, F9, GDAP1, LOC375188, DNTT, PPIL3, KCND2, ZNF597, IGSF4D and GTF3C4.

The term "sample" as used herein, refers to a composition that is obtained or derived from a subject of interest that contains a cellular and/or other molecular entity that is to be characterized and/or identified, for example based on physical, biochemical, chemical and/or physiological characteristics. In certain embodiments, the definition encompasses blood and other liquid samples of biological origin and tissue samples such as a biopsy specimen or tissue cultures or cells derived therefrom. The source of the tissue sample may be solid tissue as from a fresh, frozen and/or preserved organ or tissue sample or biopsy or aspirate; blood or any blood constituents; bodily fluids; and cells from any time in gestation or development of the subject or plasma.

In another embodiment, the definition includes biological samples that have been manipulated in any way after their procurement, such as by treatment with reagents, solubilization, or enrichment for certain components, such as proteins or polynucleotides, or embedding in a semi-solid or solid matrix for sectioning purposes. In certain embodiments, a "section" of a tissue sample is meant a single part or piece of a tissue sample, e.g. a thin slice of tissue or cells cut from a tissue sample.

Samples include, but not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, urine, cerebro-spinal fluid, saliva, sputum, tears, perspiration, mucus, tumor lysates, and tissue culture medium, as well as tissue extracts such as homogenized tissue, tumor tissue, and cellular extracts.

In one embodiment, the sample is a clinical sample. In another embodiment, the sample is used in a diagnostic assay. In certain embodiments, the sample is obtained from a pre-metastatic organ or a pre-metastatic tissue. In certain embodiments, the sample is obtained from a primary tumor or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood.

In certain embodiments, a sample is obtained from a subject or patient prior to treatment with G-CSF antagonist and/or Bv8 antagonist. In certain embodiments, a sample is obtained from a subject or patient after the treatment with G-CSF antagonist and/or Bv8 antagonist. In certain embodiments, a sample is obtained from a subject or patient prior to VEGF antagonist therapy. In certain embodiments, a sample is obtained from a subject or patient after VEGF antagonist therapy. In certain embodiments, a sample is obtained from a subject or patient prior to anti-VEGF antibody therapy. In certain embodiments, a sample is obtained from a subject or patient after anti-VEGF antibody therapy. In certain embodiments, a sample is obtained before a cancer has metastasized. In certain embodiments, a sample is obtained after a cancer has metastasized.

A "reference sample" as used herein, refers to any sample, standard, or level that is used for comparison purposes. In one embodiment, a reference sample is obtained from a healthy and/or non-diseased part of the body of the same subject or patient. In another embodiment, a reference sample is obtained from an untreated tissue and/or cell of the body of the same subject or patient.

In certain embodiments, a reference sample is a single sample or combined multiple samples from the same subject or patient that are obtained at one or more different time points than when a sample is obtained. For example, a reference sample is obtained at an earlier time point from the same subject or patient than when a sample is obtained. Such reference sample may be useful if the reference sample is obtained during initial diagnosis of cancer and the sample is later obtained when the cancer becomes metastatic.

In one embodiment, a reference sample is obtained from a healthy and/or non-diseased part of the body of an individual who is not the subject or patient. In another embodiment, a reference sample is obtained from an untreated tissue and/or cell part of the body of an individual who is not the subject or patient.

In certain embodiments, a reference sample includes all types of biological samples as defined above under the term "sample" that is obtained from one or more individuals who is not the subject or patient. In certain embodiments, a reference sample is obtained from one or more individuals with cancer who is not the subject or patient.

In certain embodiments, a reference sample is a combined multiple samples from one or more healthy individuals who are not the subject or patient. In certain embodiments, a reference sample is a combined multiple samples from one or more individuals with cancer who are not the subject or patient. In certain embodiments, a reference sample is pooled RNA samples from pre-metastatic organs or pre-metastatic tissues from one or more individuals who are not the subject or patient. In certain embodiments, a reference sample is pooled RNA samples from pre-metastatic organs or pre-metastatic tissues from one or more individuals with cancer who are not the subject or patient.

Expression level/amount of a gene, protein or biomarker in a first sample is increased as compared to expression level/amount in a second sample if the expression level/amount of the gene, gene product, e.g., protein or biomarker in the first sample is greater than the expression level/amount of the gene, gene product, e.g., protein or biomarker in the second sample. Expression levels/amount can be determined based on any suitable criterion known in the art, including but not limited to mRNA, cDNA, proteins, protein fragments and/or gene copy. Expression levels/amounts can be determined qualitatively and/or quantitatively.

In certain embodiments, the increase in expression level/amount of the gene, gene product, e.g., protein or biomarker in first sample is at least about 5%, 10%, 20%, 25%, 30%, 40%, 50%, 60%, 70%, 80%, 85%, 90% or 100% the expression level/amount of the respective gene, gene product, e.g., protein or biomarker, detected by standard art known methods such as those described herein, as compared to second sample. In certain embodiments, the increase in expression level/amount of the gene, gene product, e.g., protein or biomarker in first sample is at least about 1.5X, 1.75X, 2X, 3X, 4X, 5X, 6X, 7X, 8X, 9X, 10X, 25X, 50X, 75X, or 100X the expression level/amount of the respective gene, gene product, e.g., protein or biomarker in second sample. In certain embodiments, the first sample is the sample obtained from the subject and the second sample is the reference sample.

In certain embodiments, the term "decrease" or "reduce" herein refers to an overall reduction in expression level/amount of the gene, gene product, e.g., protein or biomarker in first sample of about 5%, 10%, 20%, 25%, 30%, 40%, 50%, 60%, 70%, 80%, 85%, 90%, 95%, 100% compared to the expression level/amount of the respective gene, gene product, e.g., protein or biomarker, detected by standard art known methods such as those described herein, as compared to second sample. In certain embodiments, the term reduce refers to the decrease in expression level/amount of a gene or biomarker in first wherein the decrease is at least about 0.9X, 0.8X, 0.7X, 0.6X, 0.5X, 0.4X, 0.3X, 0.2X, 0.1X, 0.05X, or 0.01X the expression level/amount of the respective gene or biomarker in second sample. In certain embodiments, the first sample is the sample obtained from the subject and the second sample is the reference sample.

In certain embodiments, the samples are normalized for both differences in the amount of RNA or protein assayed and variability in the quality of the RNA or protein samples used. Such normalization may be accomplished by measuring and incorporating the expression of certain normalizing genes, including well known housekeeping genes, such as GAPDH. Alternatively, normalization can be based on the mean or median signal of all of the assayed genes or a large subset thereof (global normalization approach). On a gene-by-gene basis, measured normalized amount of a patient tumor mRNA or protein is compared to the amount found in a reference set. Normalized expression levels for each mRNA or protein per tested tumor per patient can be expressed as a percentage of the expression level measured in the reference set. The expression level measured in a particular patient sample to be analyzed will fall at some percentile within this range, which can be determined by methods well known in the art.

The term "detecting" or "detection" is used in the broadest sense to include both qualitative and quantitative measurements of a target molecule. Detection includes any means of detecting, including direct and indirect detection.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs.

"Oligonucleotide," as used herein, generally refers to short, generally single-stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

In certain embodiments, polynucleotides are capable of specifically hybridizing to a gene under various stringency conditions. "Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, *see* Ausubel et al., Current Protocols in Molecular Biology, Wiley Interscience Publishers, (1995).

Stringent conditions or high stringency conditions may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

Moderately stringent conditions may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

An "isolated" nucleic acid molecule is a nucleic acid molecule that is identified and separated from at least one contaminant nucleic acid molecule with which it is ordinarily associated in the natural source of the polypeptide nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the polypeptide where, for example, the nucleic acid molecule is in a chromosomal location different from that of natural cells.

A "primer" is generally a short single stranded polynucleotide, generally with a free 3'-OH group, that binds to a target potentially present in a sample of interest by hybridizing with a target sequence, and thereafter promotes polymerization of a polynucleotide complementary to the target.

The term "housekeeping gene" refers to a group of genes that codes for proteins whose activities are essential for the maintenance of cell function. These genes are typically similarly expressed in all cell types.

The term "biomarker" as used herein refers generally to a molecule, including a gene, protein, carbohydrate structure, or glycolipid, the expression of which in or on a mammalian tissue or cell can be detected by standard methods (or methods disclosed herein) and is predictive, diagnostic and/or prognostic for a mammalian cell's or tissue's sensitivity to treatment regimes based on inhibition of angiogenesis e.g., an anti-angiogenic agent such as a VEGF-specific inhibitor, Bv8-specific inhibitor, G-CSF-specific inhibitor. In certain embodiments, the expression of such a biomarker is determined to be higher or lower than that observed for a reference sample. Expression of such biomarkers can be determined using a high-throughput multiplexed immunoassay such as those commercially available from Rules Based Medicine, Inc. or Meso Scale Discovery. Expression of the biomarkers may also be determined using, e.g., PCR or FACS assay, an immunohistochemical assay or a gene chip-based assay.

The term "array" or "microarray," as used herein refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane. The nucleotide sequences can be DNA, RNA, or any permutations thereof.

A "target gene," "target biomarker," "target sequence," "target nucleic acid" or "target protein," as used herein, is a polynucleotide or protein of interest, the detection of which is desired. Generally, a "template," as used herein, is a polynucleotide that contains the target nucleotide sequence. In some instances, the terms "target sequence," "template DNA," "template polynucleotide," "target nucleic acid," "target polynucleotide," and variations thereof, are used interchangeably.

"Amplification," as used herein, generally refers to the process of producing multiple copies of a desired sequence. "Multiple copies" mean at least 2 copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

A "native sequence" polypeptide comprises a polypeptide having the same amino acid sequence as a polypeptide derived from nature. Thus, a native sequence polypeptide can have the amino acid sequence of naturally occurring polypeptide from any mammal. Such native sequence polypeptide can be isolated from nature or can be produced by recombinant or synthetic means. The term "native sequence" polypeptide specifically encompasses naturally occurring truncated or secreted forms of the polypeptide (e.g., an extracellular domain sequence), naturally occurring variant forms (e.g., alternatively spliced forms) and naturally occurring allelic variants of the polypeptide.

An "isolated" polypeptide or "isolated" antibody is one that has been identified and separated and/or recovered from a component of its natural environment. Contaminant components of its natural environment are materials that would interfere with diagnostic or therapeutic uses for the polypeptide, and may include enzymes, hormones, and other proteinaceous or nonproteinaceous solutes. In certain embodiments, the polypeptide will be purified (1) to greater than 95% by weight of polypeptide as determined by the Lowry method, or more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue, or silver stain. Isolated polypeptide includes the polypeptide in situ within recombinant cells since at least one component of the polypeptide's natural environment will not be present. Ordinarily, however, isolated polypeptide will be prepared by at least one purification step.

A "polypeptide chain" is a polypeptide wherein each of the domains thereof is joined to other domain(s) by peptide bond(s), as opposed to non-covalent interactions or disulfide bonds.

A polypeptide "variant" means a biologically active polypeptide having at least about 80% amino acid sequence identity with the corresponding native sequence polypeptide. Such variants include, for instance, polypeptides wherein one or more amino acid (naturally occurring amino acid and/or a non-naturally occurring amino acid) residues are added, or deleted, at the N- and/or C-terminus of the polypeptide. Ordinarily, a variant will have at least about 80% amino acid sequence identity, or at least about 90% amino acid sequence identity, or at least about 95% or more amino acid sequence identity with the native sequence polypeptide. Variants also include polypeptide fragments (e.g., subsequences, truncations, etc.), typically biologically active, of the native sequence.

The term "protein variant" as used herein refers to a variant as described above and/or a protein which includes one or more amino acid mutations in the native protein sequence. Optionally, the one or more amino acid mutations include amino acid substitution(s). Protein and variants thereof for use in the invention can be prepared by a variety of methods well known in the art. Amino acid sequence variants of a protein can be prepared by mutations in the protein DNA. Such variants include, for example, deletions from, insertions into or substitutions of residues within the amino acid sequence of protein. Any combination of deletion, insertion, and substitution may be made to arrive at the final construct having the desired activity. The mutations that will be made in the DNA encoding the variant must not place the sequence out of reading frame and preferably will not create complementary regions that could produce secondary mRNA structure. EP 75,444A.

In certain embodiments, a polypeptide "variant" (*i.e.* a variant of any polypeptide disclosed herein) means a biologically active polypeptide having at least about 80% amino acid sequence identity with the corresponding native sequence polypeptide. Such variants include, for instance, polypeptides wherein one or more amino acid (naturally occurring amino acid and/or a non-naturally occurring amino acid) residues are added, or deleted, at the N- and/or C-terminus of the polypeptide. Ordinarily, a variant will have at least about 80% amino acid sequence identity, or at least about 90% amino acid sequence identity, or at least about 95% or more amino acid sequence identity with the native sequence polypeptide. Variants also include polypeptide fragments (e.g., subsequences, truncations, *etc.*), typically biologically active, of the native sequence.

"Percent (%) amino acid sequence identity" herein is defined as the percentage of amino acid residues in a candidate sequence that are identical with the amino acid residues in a selected sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2. The ALIGN-2 sequence comparison computer program was authored by Genentech, Inc. has been filed with user documentation in the U.S. Copyright Office, Washington D.C., 20559, where it is registered under U.S. Copyright Registration No. TXU510087, and is publicly available through Genentech, Inc., South San Francisco, California. The ALIGN-2 program should be compiled for use on a UNIX operating system, e.g., digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

The term "antagonist" when used herein refers to a molecule capable of neutralizing, blocking, inhibiting, abrogating, reducing or interfering with the activities of a protein including its binding to one or more receptors in the case of a ligand or binding to one or more ligands in case of a receptor. Antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors, and fusions proteins, receptor molecules and derivatives which bind specifically to a protein thereby sequestering its binding to its target, antagonist variants of the protein, antisense molecules directed to a protein, RNA aptamers, and ribozymes against a protein.

The term "antibody" is used in the broadest sense and specifically covers monoclonal antibodies (including full length or intact monoclonal antibodies), polyclonal antibodies, multivalent antibodies, multispecific antibodies (e.g., bispecific antibodies) formed from at least two intact antibodies, and antibody fragments (see below) so long as they exhibit the desired biological activity.

Unless indicated otherwise, the expression "multivalent antibody" is used throughout this specification to denote an antibody comprising three or more antigen binding sites. The multivalent antibody is typically engineered to have the three or more antigen binding sites and is generally not a native sequence IgM or IgA antibody.

A "blocking" antibody or an "antagonist" antibody is one which inhibits or reduces biological activity of the antigen it binds. Certain blocking antibodies or antagonist antibodies substantially or completely inhibit the biological activity of the antigen.

An "agonist antibody," as used herein, is an antibody which partially or fully mimics at least one of the functional activities of a polypeptide of interest.

"Antibody fragments" comprise only a portion of an intact antibody, generally including an antigen binding site of the intact antibody and thus retaining the ability to bind antigen. Examples of antibody fragments encompassed by the present definition include: (i) the Fab fragment, having VL, CL, VH and CH1 domains; (ii) the Fab' fragment, which is a Fab fragment having one or more cysteine residues at the C-terminus of the CH1 domain; (iii) the Fd fragment having VH and CH1 domains; (iv) the Fd' fragment having VH and CH1 domains and one or more cysteine residues at the C-terminus of the CH1 domain; (v) the Fv fragment having the VL and VH domains of a single arm of an antibody; (vi) the dAb fragment (Ward et al., Nature 341, 544-546 (1989)) which consists of a VH domain; (vii) isolated CDR regions; (viii) F(ab')2 fragments, a bivalent fragment including two Fab' fragments linked by a disulphide bridge at the hinge region; (ix) single chain antibody molecules (e.g. single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (VH) connected to a light chain variable domain (VL) in the same polypeptide chain (see, e.g., EP 404,097; WO 93/11161; and Hollinger et al., Proc. Natl. Acad. Sci. USA, 90:6444-6448 (1993)); (xi) "linear antibodies" comprising a pair of tandem Fd segments (VH-CH1-VH-CH1) which, together with complementary light chain polypeptides, form a pair of antigen binding regions (Zapata et al. Protein Eng. 8(10):1057 1062 (1995); and US Patent No. 5,641,870).

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. Monoclonal antibodies are highly specific, being directed against a single antigen. In certain embodiments, a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations that typically include different antibodies directed against different determinants (epitopes), each monoclonal antibody is directed against a single determinant on the antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler and Milstein, Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage-display technologies *(see, e.g.,* Clackson et al., Nature, 352: 624-628 (1991); Marks et al., J. Mol. Biol. 222: 581-597 (1991); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (*see, e.g.,* WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intem. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also, *e.g.,* Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409. *See also* van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, *e.g.,* U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. PNAS (USA) 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized *in vitro). See, e.g.,* Cole et al., Monoclonal Antibodies and Cancer Therapy, Alan R. Liss, p. 77 (1985); Boerner et al., J. Immunol., 147 (1):86-95 (1991); and US Pat No. 5,750,373.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody-dependent cellular toxicity.

The term "hypervariable region," "HVR," or "HV," when used herein refers to the amino acid residues of an antibody which are responsible for antigen-binding. For example, the term hypervariable region refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, *e.g.,* Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, e.g., Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat *et al., supra,* for each of these definitions.

"Framework Region" or "FR" residues are those variable domain residues other than the hypervariable region residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

Throughout the present specification and claims, the Kabat numbering system is generally used when referring to a residue in the variable domain (approximately, residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, MD (1991). Unless stated otherwise herein, references to residues numbers in the variable domain of antibodies means residue numbering by the Kabat numbering system. Unless stated otherwise herein, references to residue numbers in the constant domain of antibodies means residue numbering by the EU numbering system (e.g., see United States Provisional Application No. 60/640,323, Figures for EU numbering).

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., $IgG_1$ (including non-A and A allotypes), $IgG_2$, $IgG_3$, $IgG_4$, $IgG_1$, and $IgA_2$. The heavy chain constant domains that correspond to the different classes of immunoglobulins are called $\alpha$, $\delta$, $\varepsilon$, $\gamma$, and $\mu$, respectively. The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The "light chains" of antibodies (immunoglobulins) from any vertebrate species can be assigned to one of two clearly distinct types, called kappa ($\kappa$) and lambda ($\lambda$), based on the amino acid sequences of their constant domains.

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. The Fc region of an immunoglobulin generally comprises two constant domains, a CH2 domain and a CH3 domain, and optionally comprises a CH4 domain.

Unless indicated otherwise herein, the numbering of the residues in an immunoglobulin heavy chain is that of the EU index as in Kabat et *al.,* supra. The "EU index as in Kabat" refers to the residue numbering of the human IgG1 EU antibody.

By "Fc region chain" herein is meant one of the two polypeptide chains of an Fc region.

The "CH2 domain" of a human IgG Fc region (also referred to as "Cg2" domain) usually extends from an amino acid residue at about position 231 to an amino acid residue at about position 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec. Immunol.22:161-206 (1985). The CH2 domain herein may be a native sequence CH2 domain or variant CH2 domain.

The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protroberance" in one chain thereof and a corresponding introduced "cavity" in the other chain thereof; see US Patent No. 5,821,333. Such variant CH3 domains may be used to make multispecific (e.g. bispecific) antibodies as herein described.

"Hinge region" is generally defined as stretching from about Glu216, or about Cys226, to about Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions. The hinge region herein may be a native sequence hinge region or a variant hinge region. The two polypeptide chains of a variant hinge region generally retain at least one cysteine residue per polypeptide chain, so that the two polypeptide chains of the variant hinge region can form a disulfide bond between the two chains. The preferred hinge region herein is a native sequence human hinge region, e.g. a native sequence human IgG1 hinge region.

A "functional Fc region" possesses at least one "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (e.g. B cell receptor; BCR), etc. Such effector functions generally require the Fc region to be combined with a binding domain (e.g. an antibody variable domain) and can be assessed using various assays known in the art for evaluating such antibody effector functions.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

An "intact" antibody is one which comprises an antigen-binding variable region as well as a light chain constant domain ($C_L$) and heavy chain constant domains, $C_H1$, $C_H2$ and $C_H3$. The constant domains may be native sequence constant domains (*e.g.* human native sequence constant domains) or amino acid sequence variant thereof. Preferably, the intact antibody has one or more effector functions.

A "parent antibody" or "wild-type" antibody is an antibody comprising an amino acid sequence which lacks one or more amino acid sequence alterations compared to an antibody variant as herein disclosed. Thus, the parent antibody generally has at least one hypervariable region which differs in amino acid sequence from the amino acid sequence of the corresponding hypervariable region of an antibody variant as herein disclosed. The parent polypeptide may comprise a native sequence (i.e. a naturally occurring) antibody (including a naturally occurring allelic variant), or an antibody with pre-existing amino acid sequence modifications (such as insertions, deletions and/or other alterations) of a naturally occurring sequence. Throughout the disclosure, "wild type," "WT," "wt," and "parent" or "parental" antibody are used interchangeably.

As used herein, "antibody variant" or "variant antibody" refers to an antibody which has an amino acid sequence which differs from the amino acid sequence of a parent antibody. Preferably, the antibody variant comprises a heavy chain variable domain or a light chain variable domain having an amino acid sequence which is not found in nature. Such variants necessarily have less than 100% sequence identity or similarity with the parent antibody. In a preferred embodiment, the antibody variant will have an amino acid sequence from about 75% to less than 100% amino acid sequence identity or similarity with the amino acid sequence of either the heavy or light chain variable domain of the parent antibody, more preferably from about 80% to less than 100%, more preferably from about 85% to less than 100%, more preferably from about 90% to less than 100%, and most preferably from about 95% to less than 100%. The antibody variant is generally one which comprises one or more amino acid alterations in or adjacent to one or more hypervariable regions thereof.

A "variant Fc region" comprises an amino acid sequence which differs from that of a native sequence Fc region by virtue of at least one amino acid modification. In certain embodiments, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide, e.g. from about one to about ten amino acid substitutions, and preferably from about one to about five amino acid substitutions in a native sequence Fc region or in the Fc region of the parent polypeptide. The variant Fc region herein will typically possess, e.g., at least about 80% sequence identity with a native sequence Fc region and/or with an Fc region of a parent polypeptide, or at least about 90% sequence identity therewith, or at least about 95% sequence or more identity therewith.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (e.g. Natural Killer (NK) cells, neutrophils, and macrophages) enable these cytotoxic effector cells to bind specifically to an antigen-bearing target cell and subsequently kill the target cell with cytotoxins. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998).

"Human effector cells" are leukocytes which express one or more FcRs and perform effector functions. In certain embodiments, the cells express at least FcγRIII and perform ADCC effector function(s). Examples of human leukocytes which mediate ADCC include peripheral blood mononuclear cells (PBMC), natural killer (NK) cells, monocytes, cytotoxic T cells and neutrophils; with PBMCs and NK cells being generally preferred. The effector cells may be isolated from a native source thereof, e.g. from blood or PBMCs as described herein.

"Fc receptor" or "FcR" describes a receptor that binds to the Fc region of an antibody. In some embodiments, an FcR is a native human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, e.g., Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton *et al.*).

Binding to human FcRn *in vivo* and serum half life of human FcRn high affinity binding polypeptides can be assayed, *e.g.*, in transgenic mice or transfected human cell lines expressing human FcRn, or in primates to which the polypeptides with a variant Fc region are administered. WO 2000/42072 (Presta) describes antibody variants with improved or diminished binding to FcRs. See also, *e.g.*, Shields et al. J. Biol. Chem. 9(2):6591-6604 (2001).

"Complement dependent cytotoxicity" or "CDC" refers to the lysis of a target cell in the presence of complement. Activation of the classical complement pathway is initiated by the binding of the first component of the complement system (Clq) to antibodies (of the appropriate subclass), which are bound to their cognate antigen. To assess complement activation, a CDC assay, *e.g.*, as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, e.g., in US Patent No. 6,194,551 B1 and WO 1999/51642. See also, e.g., Idusogie et al. J. Immunol. 164: 4178-4184 (2000).

An "affinity matured" antibody is one with one or more alterations in one or more CDRs thereof which result an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "flexible linker" herein refers to a peptide comprising two or more amino acid residues joined by peptide bond(s), and provides more rotational freedom for two polypeptides (such as two Fd regions) linked thereby. Such rotational freedom allows two or more antigen binding sites joined by the flexible linker to each access target antigen(s) more efficiently. Examples of suitable flexible linker peptide sequences include gly-ser, gly-ser-gly-ser (SEQ ID NO: 18), ala-ser, and gly-gly-gly-ser (SEQ ID NO: 19).

A "dimerization domain" is formed by the association of at least two amino acid residues (generally cysteine residues) or of at least two peptides or polypeptides (which may have the same, or different, amino acid sequences). The peptides or polypeptides may interact with each other through covalent and/or non-covalent association(s). Examples of dimerization domains herein include an Fc region; a hinge region; a CH3 domain; a CH4 domain; a CH1-CL pair; an "interface" with an engineered "knob" and/or "protruberance" as described in US Patent No. 5,821,333; a leucine zipper (e.g. a jun/fos leucine zipper, see Kostelney et al., J. Immunol., 148: 1547-1553 (1992); or a yeast GCN4 leucine zipper); an isoleucine zipper; a receptor dimer pair (e.g., interleukin-8 receptor (IL-8R); and integrin heterodimers such as LFA-1 and GPIIIb/IIIa), or the dimerization region(s) thereof; dimeric ligand polypeptides (e.g. nerve growth factor (NGF), neurotrophin-3 (NT-3), interleukin-8 (IL-8), vascular endothelial growth factor (VEGF), VEGF-C, VEGF-D, PDGF members, and brain-derived neurotrophic factor (BDNF); see Arakawa et al. J. Biol. Chem. 269(45): 27833-27839 (1994) and Radziejewski et al. Biochem. 32(48): 1350 (1993)), or the dimerization region(s) thereof; a pair of cysteine residues able to form a disulfide bond; a pair of peptides or polypeptides, each comprising at least one cysteine residue (e.g. from about one, two or three to about ten cysteine residues) such that disulfide bond(s) can form between the peptides or polypeptides (hereinafter "a synthetic hinge"); and antibody variable domains. The most preferred dimerization domain herein is an Fc region or a hinge region.

A "functional antigen binding site" of an antibody is one which is capable of binding a target antigen. The antigen binding affinity of the antigen binding site is not necessarily as strong as the parent antibody from which the antigen binding site is derived, but the ability to bind antigen must be measurable using any one of a variety of methods known for evaluating antibody binding to an antigen. Moreover, the antigen binding affinity of each of the antigen binding sites of a multivalent antibody herein need not be quantitatively the same. For the multimeric antibodies herein, the number of functional antigen binding sites can be evaluated using ultracentrifugation analysis. According to this method of analysis, different ratios of target antigen to multimeric antibody are combined and the average molecular weight of the complexes is calculated assuming differing numbers of functional binding sites. These theoretical values are compared to the actual experimental values obtained in order to evaluate the number of functional binding sites.

An antibody having a "biological characteristic" of a designated antibody is one which possesses one or more of the biological characteristics of that antibody which distinguish it from other antibodies that bind to the same antigen.

In order to screen for antibodies which bind to an epitope on an antigen bound by an antibody of interest, a routine cross-blocking assay such as that described in Antibodies, A Laboratory Manual, Cold Spring Harbor Laboratory, Ed Harlow and David Lane (1988), can be performed.

The term "epitope" is used to refer to binding sites for (monoclonal or polyclonal) antibodies on protein antigens.

As used herein, "treatment" is an approach for obtaining beneficial or desired clinical results. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. "Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. Specifically, the treatment may directly prevent, slow down or otherwise decrease the pathology of cellular degeneration or damage, such as the pathology of a disease or conditions associated with tumor metastasis, tumor cell migration and/or the mobilization of Cd11b+Gr1+ cells.

The term "effective amount" or "therapeutically effective amount" refers to an amount of a drug effective to treat a disease or disorder in a subject. In certain embodiments, an effective amount is an amount sufficient to effect beneficial or desired therapeutic (including preventative) results. An effective amount can be administered in one or more administrations. In certain embodiments, an effective amount refers to an amount effective, at dosages and for periods of time necessary, to achieve the desired therapeutic or prophylactic result. A therapeutically effective amount of a substance/molecule of the invention may vary according to factors such as the disease state, age, sex, and weight of the individual, and the ability of the substance/molecule, to elicit a desired response in the individual. A therapeutically effective amount encompasses an amount in which any toxic or detrimental effects of the substance/molecule are outweighed by the therapeutically beneficial effects. In the case of cancer, the effective amount of the drug may reduce the number of cancer cells; reduce the tumor size; inhibit (*i.e.*, slow to some extent and typically stop) cancer cell infiltration into peripheral organs; inhibit (*i.e.,* slow to some extent and typically stop) tumor metastasis; inhibit, to some extent, tumor growth; allow for treatment of the tumor, and/or relieve to some extent one or more of the symptoms associated with the disorder. To the extent the drug may prevent growth and/or kill existing cancer cells, it may be cytostatic and/or cytotoxic.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effective amount.

In the case of pre-cancerous, benign, early or late-stage tumors, the therapeutically effective amount of the angiogenic inhibitor may reduce the number of cancer cells; reduce the primary tumor size; inhibit (i.e., slow to some extent and preferably stop) cancer cell infiltration into peripheral organs; inhibit (*i.e.*, slow to some extent and preferably stop) tumor metastasis; inhibit or delay, to some extent, tumor growth or tumor progression; and/or relieve to some extent one or more of the symptoms associated with the disorder. To the extent the drug may prevent growth and/or kill existing cancer cells, it may be cytostatic and/or cytotoxic.

An "individual," "subject," or "patient" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, a mammal is a human.

By "maintenance therapy" is meant a therapeutic regimen that is given to reduce the likelihood of disease recurrence or progression. Maintenance therapy can be provided for any length of time, including extended time periods up to the life-span of the subject. Maintenance therapy can be provided after initial therapy or in conjunction with initial or additional therapies. Dosages used for maintenance therapy can vary and can include diminished dosages as compared to dosages used for other types of therapy.

"Neoadjuvant therapy" or "preoperative therapy" herein refers to therapy given prior to surgery. The goal of neoadjuvant therapy is to provide immediate systemic treatment, potentially eradicating micrometastases that would otherwise proliferate if the standard sequence of surgery followed by systemic therapy were followed. Neoadjuvant therapy may also help to reduce tumor size thereby allowing complete resection of initially unresectable tumors or preserving portions of the organ and its functions. Furthermore, neoadjuvant therapy permits an in vivo assessment of drug efficacy, which may guide the choice of subsequent treatments.

"Adjuvant therapy" herein refers to therapy given after surgery, where no evidence of residual disease can be detected, so as to reduce the risk of disease recurrence. The goal of adjuvant therapy is to prevent recurrence of the cancer, and therefore to reduce the chance of cancer-related death.

Herein, "standard of care" chemotherapy refers to the chemotherapeutic agents routinely used to treat a particular cancer.

"Definitive surgery" refers to complete removal of tumor and surrounding tissue as well as any involved lymph nodes.

"Operable" cancer is cancer which is confined to the primary organ and suitable for surgery.

"Tumor," as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The tumor can be a solid tumor or a non-solid or soft tissue tumor. Examples of soft tissue tumors include leukemia (*e.g.,* chronic myelogenous leukemia, acute myelogenous leukemia, adult acute lymphoblastic leukemia, acute myelogenous leukemia, mature B-cell acute lymphoblastic leukemia, chronic lymphocytic leukemia, polymphocytic leukemia, or hairy cell leukemia), or lymphoma (e.g., non-Hodgkin's lymphoma, cutaneous T-cell lymphoma, or Hodgkin's disease). A solid tumor includes any cancer of body tissues other than blood, bone marrow, or the lymphatic system. Solid tumors can be further separated into those of epithelial cell origin and those of non-epithelial cell origin. Examples of solid tumors include tumors of colon, breast, prostate, lung, kidney, liver, pancreas, ovary, head and neck, oral cavity, stomach, duodenum, small intestine, large intestine, gastrointestinal tract, anus, gall bladder, labium, nasopharynx, skin, uterus, male genital organ, urinary organs, bladder, and skin. Solid tumors of non-epithelial origin include sarcomas, brain tumors, and bone tumors.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include, but not limited to, squamous cell cancer (e.g., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, superficial spreading melanoma, lentigo maligna melanoma, acral lentiginous melanomas, nodular melanomas, multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); hairy cell leukemia; chronic myeloblastic leukemia; and post-transplant lymphoproliferative disorder (PTLD), as well as abnormal vascular proliferation associated with phakomatoses, edema (such as that associated with brain tumors), Meigs' syndrome, brain, as well as head and neck cancer, and associated metastases. In certain embodiments, cancers that are amenable to treatment by the anti-G-CSF antibody, anti-Bv8-antibody, anti-PKR1 antibody or any combination thereof include breast cancer, colorectal cancer, rectal cancer, non-small cell lung cancer, glioblastoma, non-Hodgkins lymphoma (NHL), renal cell cancer, prostate cancer, melanoma, liver cancer, pancreatic cancer, soft-tissue sarcoma, kaposi's sarcoma, carcinoid carcinoma, head and neck cancer, ovarian cancer and multiple myeloma. In some embodiments, the cancer is selected from the group consisting of small cell lung cancer, gliblastoma, neuroblastomas, melanoma, breast carcinoma, gastric cancer, colorectal cancer (CRC), and hepatocellular carcinoma. Yet, in some embodiments, the cancer is selected from the group consisting of non-small cell lung cancer, colorectal cancer, renal cell cancer, ovarian cancer, prostate cancer, glioblastoma and breast carcinoma, including metastatic forms of those cancers.

The term cancer embraces a collection of proliferative disorders, including but not limited to pre-cancerous growths, benign tumors, malignant tumors and dormant tumors. Benign tumors remain localized at the site of origin and do not have the capacity to infiltrate, invade, or metastasize to distant sites. Malignant tumors will invade and damage other tissues around them. They can also gain the ability to break off from where they started and spread to other parts of the body (metastasize), usually through the bloodstream or through the lymphatic system where the lymph nodes are located. Dormant tumors are quiescent tumors in which tumor cells are present but tumor progression is not clinically apparent. Primary tumors are classified by the type of tissue from which they arise; metastatic tumors are classified by the tissue type from which the cancer cells are derived. Over time, the cells of a malignant tumor become more abnormal and appear less like normal cells. This change in the appearance of cancer cells is called the tumor grade and cancer cells are described as being well-differentiated, moderately-differentiated, poorly-differentiated, or undifferentiated. Well-differentiated cells are quite normal appearing and resemble the normal cells from which they originated. Undifferentiated cells are cells that have become so abnormal that it is no longer possible to determine the origin of the cells.

Epithelial cancers generally evolve from a benign tumor to a preinvasive stage (*e.g.,* carcinoma *in situ*), to a malignant cancer, which has penetrated the basement membrane and invaded the subepithelial stroma.

By "dysplasia" is meant any abnormal growth or development of tissue, organ, or cells. In certain embodiments, the dysplasia is high grade or precancerous.

The term "resistant tumor" or "VEGF independent tumor" refers to cancer, cancerous cells, or a tumor that does not respond completely, or loses or shows a reduced response over the course of cancer therapy to a cancer therapy comprising at least a VEGF antagonist. In certain embodiments, resistant tumor is a tumor that is resistant to anti-VEGF antibody therapy. In one embodiment, the anti-VEGF antibody is bevacizumab. In certain embodiments, resistant tumor is a tumor that is unlikely to respond to a cancer therapy comprising at least a VEGF antagonist. In certain embodiments, resistant tumor is a tumor that is intrinsically non-responsive or resistant to a cancer therapy comprising at least a VEGF antagonist. In certain embodiments, resistant tumor is a tumor, after an initial strong response to cancer therapy comprising at least a VEGF antagonist, reinitiates tumor growth despite ongoing cancer therapy.

"Refractory" refers to the resistance or non-responsiveness of a disease or condition to a treatment (e.g., the number of neoplastic plasma cells increases even though treatment if given). In certain embodiments, the term "refractory" refers a resistance or non-responsiveness to any previous treatment including, but not limited to, VEGF antagonist, anti-angiogenic agents and chemotherapy treatments. In certain embodiments, the term "refractory" refers an intrinsically non-responsiveness of a disease or condition to any previous treatment comprising a VEGF antagonist, anti-angiogenic agents and/or chemotherapy treatments. In certain embodiments, the VEGF antagonist is an anti-VEGF antibody.

"Relapsed" refers to the regression of the patient's illness back to its former diseased state, especially the return of symptoms following an apparent recovery or partial recovery. In certain embodiments, relapsed state refers to the process of returning to or the return to illness before the previous treatment including, but not limited to, VEGF antagonist, anti-angiogenic agents and chemotherapy treatments. In certain embodiments, relapsed state refers to the process of returning to or the return to illness after an initial strong response to a cancer therapy comprising a VEGF antagonist, anti-angiogenic agents and/or chemotherapy treatments. In certain embodiments, the VEGF antagonist is an anti-VEGF antibody.

The term "anti-cancer therapy" or "cancer therapy" refers to a therapy useful in treating cancer. Examples of anti-cancer therapeutic agents include, but are limited to, e.g., chemotherapeutic agents, growth inhibitory agents, cytotoxic agents, agents used in radiation therapy, anti-angiogenic agents, apoptotic agents, anti-tubulin agents, and other agents to treat cancer, such as anti-HER-2 antibodies, anti-CD20 antibodies, an epidermal growth factor receptor (EGFR) antagonist (e.g., a tyrosine kinase inhibitor), HER1/EGFR inhibitor (e.g., erlotinib (TARCEVA®), platelet derived growth factor inhibitors (e.g., GLEEVEC® (Imatinib Mesylate)), a COX-2 inhibitor (e.g., celecoxib), ERBITUX® (cetuximab, Imclone), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

The term "anti-neoplastic composition" refers to a composition useful in treating cancer comprising at least one active therapeutic agent, e.g., "anti-cancer agent." Examples of therapeutic agents (anti-cancer agents) include, but are limited to, e.g., chemotherapeutic agents, growth inhibitory agents, cytotoxic agents, agents used in radiation therapy, anti-angiogenesis agents, apoptotic agents, anti-tubulin agents, toxins, and other-agents to treat cancer, e.g., anti-VEGF neutralizing antibody, VEGF antagonist, anti-HER-2, anti-CD20, an epidermal growth factor receptor (EGFR) antagonist (e.g., a tyrosine kinase inhibitor), HER1/EGFR inhibitor, erlotinib, a COX-2 inhibitor (e.g., celecoxib), interferons, cytokines, antagonists (e.g., neutralizing antibodies) that bind to one or more of the ErbB2, ErbB3, ErbB4, or VEGF receptor(s), inhibitors for receptor tyrosine kinases for platelet-derived growth factor (PDGF) and/or stem cell factor (SCF) (e.g., imatinib mesylate (Gleevec ® Novartis)), TRAIL/Apo2, and other bioactive and organic chemical agents, etc. Combinations thereof are also included in the invention.

The term "cytotoxic agent" as used herein refers to a substance that inhibits or prevents the function of cells and/or causes destruction of cells. The term is intended to include radioactive isotopes (*e.g.,* $^{211}At$, $^{131}I$, $^{125}I$, $^{90}Y$, $^{186}Re$, $^{188}Re$, $^{153}Sm$, $^{212}Bi$, $^{32}P$ and radioactive isotopes of Lu), chemotherapeutic agents, and toxins such as small molecule toxins or enzymatically active toxins of bacterial, fungal, plant or animal origin, including fragments and/or variants thereof.

A "growth inhibitory agent" when used herein refers to a compound or composition which inhibits growth of a cell in vitro and/or in vivo. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e. g.,* calicheamicin, especially calicheamicin gamma1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; pemetrexed (ALIMTA®); gemicitabine (GEMZAR®); anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as amino glutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., paclitaxel (TAXOL®), albumin-engineered nanoparticle formulation of paclitaxel (ABRAXANE®), and doxetaxel (TAXOTERE®); chloranbucil; 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), raloxifene (EVISTA®), droloxifene, 4-hydroxytamoxifen, trioxifene, keoxifene, LY117018, onapristone, and toremifene (FARESTON®); anti-progesterones; estrogen receptor down-regulators (ERDs); agents that function to suppress or shut down the ovaries, for example, leutinizing hormone-releasing hormone (LHRH) agonists such as leuprolide acetate (LUPRON® and ELIGARD®), goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, megestrol acetate (MEGASE®), exemestane (AROMASIN®), formestanie, fadrozole, vorozole (RIVISOR®), letrozole (FEMARA®), and anastrozole (ARIMIDEX®). In addition, such definition of chemotherapeutic agents includes bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); lapatinib ditosylate (an ErbB-2 and EGFR dual tyrosine kinase small-molecule inhibitor also known as GW572016); COX-2 inhibitors such as celecoxib (CELEBREX®; 4-(5-(4-methylphenyl)-3-(trifluoromethyl)-1H-pyrazol-1-yl) benzenesulfonamide; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH);

hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factors (e.g., VEGF, VEGF-B, VEGF-C, VEGF-D, VEGF-E); placental derived growth factor (PlGF); platelet derived growth factors (PDGF, e.g., PDGFA, PDGFB, PDGFC, PDGFD); integrin; thrombopoietin (TPO); nerve growth factors such as NGF-alpha; platelet-growth factor; transforming growth factors (TGFs) such as TGF-alpha and TGF-beta; insulin-like growth factor-I and -II; erythropoietin (EPO); osteoinductive factors; interferons such as interferon-alpha, -beta and -gamma, colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-1beta, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20-IL-30; secretoglobin/uteroglobin; oncostatin M (OSM); a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

An "angiogenic factor or agent" is a growth factor which stimulates the development of blood vessels, e.g., promotes angiogenesis, endothelial cell growth, stability of blood vessels, and/or vasculogenesis, etc. For example, angiogenic factors, include, but are not limited to, e.g., VEGF and members of the VEGF family, PlGF, PDGF family, fibroblast growth factor family (FGFs), TIE ligands (Angiopoietins), ephrins, ANGPTL3, ANGPTL4, etc. It would also include factors that accelerate wound healing, such as growth hormone, insulin-like growth factor-I (IGF-I), VIGF, epidermal growth factor (EGF), CTGF and members of its family, and TGF-$\alpha$ and TGF-$\beta$. *See, e.g.,* Klagsbrun and D'Amore, Annu. Rev. Physiol., 53:217-39 (1991); Streit and Detmar, Oncogene, 22:3172-3179 (2003); Ferrara & Alitalo, Nature Medicine 5(12):1359-1364 (1999); Tonini et al., Oncogene, 22:6549-6556 (2003) (e.g., Table 1 listing angiogenic factors); and, Sato Int. J. Clin. Oncol., 8:200-206 (2003).

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. For example, an anti-angiogenesis agent is an antibody or other antagonist to an angiogenic agent as defined above, e.g., antibodies to VEGF, antibodies to VEGF receptors, small molecules that block VEGF receptor signaling (e.g., PTK787/ZK2284, SU6668, SUTENT/SU11248 (sunitinib malate), AMG706, sorafenib (NEXAVAR®), axitinib, and pazopanib). Anti-angiogensis agents also include native angiogenesis inhibitors, e.g., angiostatin, endostatin, etc. *See, e.g.,* Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5(12):1359-1364 (1999); Tonini et al., Oncogene, 22:6549-6556 (2003) (e.g., Table 2 listing antiangiogenic factors); and, Sato Int. J. Clin. Oncol., 8:200-206 (2003) (e.g., Table 1 lists Anti-angiogenic agents used in clinical trials).

The term "immunosuppressive agent" as used herein refers to substances that act to suppress or mask the immune system of the mammal being treated herein. This would include substances that suppress cytokine production, down-regulate or suppress self-antigen expression, or mask the MHC antigens. Examples of such agents include 2-amino-6-aryl-5-substituted pyrimidines (see U.S. Pat. No. 4,665,077); nonsteroidal antiinflammatory drugs (NSAIDs); ganciclovir, tacrolimus, glucocorticoids such as cortisol or aldosterone, anti-inflammatory agents such as a cyclooxygenase inhibitor, a 5-lipoxygenase inhibitor, or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Pat. No. 4,120,649); anti-idiotypic antibodies for MHC antigens and MHC fragments; cyclosporin A; steroids such as corticosteroids or glucocorticosteroids or glucocorticoid analogs, e.g., prednisone, methylprednisolone, and dexamethasone; dihydrofolate reductase inhibitors such as methotrexate (oral or subcutaneous); hydroxycloroquine; sulfasalazine; leflunomide; cytokine or cytokine receptor antibodies including anti-interferon-alpha, -beta, or -gamma antibodies, anti-tumor necrosis factor-alpha antibodies (infliximab or adalimumab), anti-TNF-alpha immunoahesin (etanercept), anti-tumor necrosis factor-beta antibodies, anti-interleukin-2 antibodies and anti-IL-2 receptor antibodies; anti-LFA-1 antibodies, including anti-CD11a and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 1990/08187 published Jul. 26, 1990); streptokinase; TGF-beta; streptodornase; RNA or DNA from the host; FK506; RS-61443; deoxyspergualin; rapamycin; T-cell receptor (Cohen et al., U.S. Pat. No. 5,114,721); T-cell-receptor fragments (Offner et al., Science, 251: 430-432 (1991); WO 1990/11294; laneway, Nature, 341: 482 (1989); and WO 1991/01133); and T-cell-receptor antibodies (EP 340,109) such as T10B9.

Examples of "nonsteroidal anti-inflammatory drugs" or "NSAIDs" are acetylsalicylic acid, ibuprofen, naproxen, indomethacin, sulindac, tolmetin, including salts and derivatives thereof, etc.

The "pathology" of a disease includes all phenomena that compromise the well-being of the patient. For cancer, this includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

Administration "in combination with" one or more further therapeutic agents includes simultaneous (concurrent) and consecutive administration in any order.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Carners" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

A "liposome" is a small vesicle composed of various types of lipids, phospholipids and/or surfactant which is useful for delivery of a drug (such as a Bv8 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

An "amino acid alteration" refers to a change in the amino acid sequence of a predetermined amino acid sequence. Exemplary alterations include insertions, substitutions and deletions. An "amino acid substitution" refers to the replacement of an existing amino acid residue in a predetermined amino acid sequence; with another different amino acid residue.

A "replacement" amino acid residue refers to an amino acid residue that replaces or substitutes another amino acid residue in an amino acid sequence. The replacement residue may be a naturally occurring or non-naturally occurring amino acid residue.

An "amino acid insertion" refers to the introduction of one or more amino acid residues into a predetermined amino acid sequence. The amino acid insertion may comprise a "peptide insertion" in which case a peptide comprising two or more amino acid residues joined by peptide bond(s) is introduced into the predetermined amino acid sequence. Where the amino acid insertion involves insertion of a peptide, the inserted peptide may be generated by random mutagenesis such that it has an amino acid sequence which does not exist in nature. An amino acid alteration "adjacent a hypervariable region" refers to the introduction or substitution of one or more amino acid residues at the N-terminal and/or C-terminal end of a hypervariable region, such that at least one of the inserted or replacement amino acid residue(s) form a peptide bond with the N-terminal or C-terminal amino acid residue of the hypervariable region in question.

A "naturally occurring amino acid residue" is one encoded by the genetic code, generally selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val).

A "non-naturally occurring amino acid residue" herein is an amino acid residue other than those naturally occurring amino acid residues listed above, which is able to covalently bind adjacent amino acid residues(s) in a polypeptide chain. Examples of non-naturally occurring amino acid residues include norleucine, ornithine, norvaline, homoserine and other amino acid residue analogues such as those described in Ellman et al. Meth. Enzym. 202:301-336 (1991). To generate such non-naturally occurring amino acid residues, the procedures of Noren et al. Science 244:182 (1989) and Ellman et al., supra, can be used. Briefly, these procedures involve chemically activating a suppressor tRNA with a non-naturally occurring amino acid residue followed by in vitro transcription and translation of the RNA.

As used herein, an antibody with a "high-affinity" is an antibody having a $K_d$, or dissociation constant, in the nanomolar (nM) range or better. A $K_d$ in the "nanomolar range or better" may be denoted by *X* nM, where *X* is a number less than about 10.

"Binding affinity" generally refers to the strength of the sum total of noncovalent interactions between a single binding site of a molecule (*e.g.*, an antibody) and its binding partner (*e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant (Kd), the reciprocal of the association constant (Ka). Affinity can be measured by common methods known in the art, including those described herein. Low-affinity antibodies generally bind antigen slowly and/or tend to dissociate readily, whereas high-affinity antibodies generally bind antigen faster and/or tend to remain bound longer. A variety of methods of measuring binding affinity are known in the art, any of which can be used for purposes of the present invention. Specific illustrative and exemplary embodiments for measuring binding affinity are described in the following.

In certain embodiments, the "$K_D$," "$K_d$," "Kd" or "Kd value" according to this invention is measured by using surface plasmon resonance assays using a BIACORE®-2000 or a BIACORE ®-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (-0.2 μM) before injection at a flow rate of 5 μl/minute to achieve approximately 10 response units (RU) of coupled protein. Following the injection of antigen, 1 M ethanolamine is injected to block unreacted groups. For kinetics measurements, serial dilutions of polypeptide, *e.g.*, full length antibody, are injected in PBS with 0.05% TWEEN-20™ surfactant (PBST) at 25°C at a flow rate of approximately 25 μl/min. Association rates (kon) and dissociation rates (koff) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio koff/kon. *See, e.g.,* Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

The term "filamentous phage" refers to a viral particle capable of displaying a heterogenous polypeptide on its surface, and includes, without limitation, f1, fd, Pfl, and M13. The filamentous phage may contain a selectable marker such as tetracycline (e.g., "fd-tet"). Various filamentous phage display systems are well known to those of skill in the art (see, e.g., Zacher et al. Gene 9: 127-140 (1980), Smith et al. Science 228: 1315-1317 (1985); and Parmley and Smith Gene 73: 305-318 (1988)).

The term "panning" is used to refer to the multiple rounds of screening process in identification and isolation of phages carrying compounds, such as antibodies, with high affinity and specificity to a target.

The term "short-interfering RNA (siRNA)" refers to small double-stranded RNAs that interfere with gene expression. siRNAs are an intermediate of RNA interference, the process double-stranded RNA silences homologous genes. siRNAs typically are comprised of two single-stranded RNAs of about 15-25 nucleotides in length that form a duplex, which may include single-stranded overhang(s). Processing of the double-stranded RNA by an enzymatic complex, for example by polymerases, results in the cleavage of the double-stranded RNA to produce siRNAs. The antisense strand of the siRNA is used by an RNA interference (RNAi) silencing complex to guide mRNA cleavage, thereby promoting mRNA degradation. To silence a specific gene using siRNAs, for example in a mammalian cell, the base pairing region is selected to avoid chance complementarity to an unrelated mRNA. RNAi silencing complexes have been identified in the art, such as, for example, by Fire et al., Nature 391:806-811 (1998) and McManus et al., Nat. Rev. Genet. 3(10):737-47 (2002).

The term "interfering RNA (RNAi)" is used herein to refer to a double-stranded RNA that results in catalytic degradation of specific mRNAs, and thus can be used to inhibit/lower expression of a particular gene.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. The term "progeny" refers to any and all offspring of every generation subsequent to an originally transformed cell or cell line. Mutant progeny that have the same function or biological activity as screened for in the originally transformed cell are included. Where distinct designations are intended, it will be clear from the context.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired identity between the probe and hybridizable sequence, the higher the relative temperature which can be used. As a result, it follows that higher relative temperatures would tend to make the reaction

conditions more stringent, while lower temperatures less so. For additional details and explanation of stringency of hybridization reactions, see Ausubel et al., Current Protocols in Molecular Biology, Wiley Interscience Publishers, (1995).

"High stringency conditions", as defined herein, are identified by those that: (1) employ low ionic strength and high temperature for washing; 0.015 M sodium chloride/0.0015 M sodium citrate/0.1% sodium dodecyl sulfate at 50°C; (2) employ during hybridization a denaturing agent; 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

### B. Detailed Description

The present invention is based, at least in part, on the recognition that G-CSF and Bv8 plays an important role in the cellular and molecular events leading to tumor metastasis.

Copending U.S. Publication No. 20070264193 filed on March 28, 2007, describes a correlation between recruitment of hematopoietic bone marrow-derived cells and the development of tumor resistance to anti-VEGF treatment. Copending PCT Publication No. WO 2009/039337 filed on September 19, 2008, describes the role of Bv8 in the cellular and molecular events leading to tumor resistance to anti-VEGF treatment.

Metastasis is a major cause of death from solid tumors. In order to metastasize, tumor cells need to degrade and invade the extracellular matrix, intravasate, be carried through blood or lymphatic vessels, extravasate at the secondary site, and finally establish secondary tumors (Nguyen, D. X. & Massague, J. Nat Rev Genet 8, 341-352 (2007)). In addition, mounting evidence suggests that tumors are able to modify the distant microenvironment prior to arrival of metastatic tumor cells to create the so-called "pre-metastatic niche" (Psaila, B. & Lyden, D. Nat Rev Cancer 9, 285-293 (2009)). This ability of tumors to affect distant tissues enables cancer cells to target specific organs in which they can initiate secondary tumor growth and supports the "seed and soil" hypothesis (Paget, S. Cancer Metastasis Rev. 8(2),98-101 (1989)). Bone marrow derived cells (BMDC) are thought to be a major cell type populating the niche (Kaplan, R. N. et al. Nature 438, 820-827 (2005); Hiratsuka, S. et al. Nat Cell Biol 8, 1369-1375 (2006)).

Although several molecules have been implicated (Kaplan, R. N. et al. Nature 438, 820-827 (2005); Hiratsuka, S. et al. Nat Cell Biol 8, 1369-1375 (2006); Yamamoto, M. et al. Cancer Res 68, 9754-9762 (2008); Kim, S. et al. Nature 457, 102-106 (2009); Erler, J. T. et al. Cancer Cell 15, 35-44 (2009), the mechanisms by which tumors initiate the niche and the precise role of the niche in metastasis are incompletely understood.

Priming of the organ-specific pre-metastatic sites is an important step during metastasis, but the mechanisms underlying the initiation of the pre-metastatic niche are incompletely understood. The data provided herein show that tumors having the ability to metastasize secrete granulocyte-colony stimulating factor (G-CSF), which mobilizes Cd11b+Gr1+ cells from the bone marrow and facilitates their subsequent homing at distant organs prior to the arrival of the tumor cells. G-CSF expression correlated with the metastatic potential of the tumors examined. Moreover, the data presented herein show that G-CSF-mobilized Cd11b+Gr1+ cells locally produce the pro-angiogenic Bv8 protein. Treatment with anti-G-CSF or anti-Bv8 antibodies significantly reduces lung metastasis. The data also identified a novel role for Bv8, the ability to stimulate tumor cell migration, an effect related to the expression of one of the two Bv8 receptors (PKR-1/GPR73). Finally, the data provided herein show that recombinant G-CSF is sufficient to initiate the niche and increase the numbers of Cd11b+Gr1+ cells in organ-specific metastatic sites. This results in enhanced metastatic ability of several tumors. Therefore, the data provided herein suggest that G-CSF is a key regulator of metastasis.

**C. Making anti-G-CSF Antibodies, anti-Bv8 Antibodies and anti-PKR1 Antibodies Acting as Inhibitors of Tumor Angiogenesis and Tumor Metastasis**

The antibodies identified by the binding and activity assays of the present invention can be produced by methods known in the art, including techniques of recombinant DNA technology.

i) Antigen Preparation

Soluble antigens or fragments thereof, optionally conjugated to other molecules, can be used as immunogens for generating antibodies. For transmembrane molecules, such as receptors, fragments of these (e.g. the extracellular domain of a receptor) can be used as the immunogen. Alternatively, cells expressing the transmembrane molecule can be used as the immunogen. Such cells can be derived from a natural source (e.g. cancer cell lines) or may be cells which have been transformed by recombinant techniques to express the transmembrane molecule. Other antigens and forms thereof useful for preparing antibodies will be apparent to those in the art.

(ii) Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, $SOCl_2$, or $R_1N=C=NR$, where R and $R_1$ are different alkyl groups.

Animals are immunized against the antigen, immunogenic conjugates, or derivatives by combining, e.g., 100 μg or 5 μg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

(iii) Monoclonal Antibodies

Monoclonal antibodies may be made using the hybridoma method first described by Kohler et al., Nature, 256:495 (1975), or may be made by recombinant DNA methods (U.S. Pat. No. 4,816,567). In the hybridoma method, a mouse or other appropriate host animal, such as a hamster or macaque monkey, is immunized as hereinabove described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, Calif. USA, and SP-2 or X63-Ag8-653 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984); Brodeur et al, Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

After hybridoma cells are identified that produce antibodies of the desired specificity, affinity, and/or activity, the clones may be subloned by limiting dilution procedures and grown by standard methods (Goding, MonoclonalAntibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

In a further embodiment, antibodies or antibody fragments can be isolated from antibody phage libraries generated using the techniques described in McCafferty et al., Nature, 348:552-554 (1990).

Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Pat. No. 4,816,567; Morrison, et al., Proc. Natl. Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

(iv) Humanized and Human Antibodies

A humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad Sci. USA, 89:4285 (1992); Presta et al., J. Immnol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J.sub.H) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al, Proc. Natl. Acad. Sci. USA, 90:2551 (1993); Jakobovits et al., Nature, 362:255-258 (1993); Bruggermann et al., Year in Immuno., 7:33 (1993); and Duchosal et al. Nature 355:258 (1992). Human antibodies can also be derived from phage-display libraries (Hoogenboom et al, J. Mol. Biol., 227:381 (1991); Marks et al, J. MoL Biol., 222:581-597 (1991); Vaughan et al. Nature Biotech 14:309 (1996)). Generation of human antibodies from antibody phage display libraries is further described below.

(v) Antibody Fragments

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form $F(ab')_2$ fragments (Carter et al., Bio/Technology 10:163-167 (1992)). In another embodiment as described in the example below, the $F(ab')_2$ is formed using the leucine zipper GCN4 to promote assembly of the $F(ab')_2$ molecule. According to another approach, $F(ab')_2$ fragments can be isolated directly from recombinant host cell culture. Other techniques for the production of antibody fragments will be apparent to the skilled practitioner. In other embodiments, the antibody of choice is a single chain Fv fragment (scFv). *See* WO 93/16185.

(vi) Multispecific Antibodies

Multispecific antibodies have binding specificities for at least two different epitopes, where the epitopes are usually from different antigens. While such molecules normally will only bind two different epitopes (i.e. bispecific antibodies, BsAbs), antibodies with additional specificities such as trispecific antibodies are encompassed by this expression when used herein. Examples of BsAbs include those with one arm directed against Bv8 or G-CSF and another arm directed against VEGF or EG-VEGF.

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991). According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In certain embodiments of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in WO96/27011, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/200373). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229: 81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab'-SH fragments can also be directly recovered from E. coli, and can be chemically coupled to form bispecific antibodies. Shalaby et al., J. Exp. Med., 175: 217-225 (1992) describe the production of a fully humanized bispecific antibody $F(ab')_2$ molecule. Each Fab' fragment was separately secreted from *E. coli* and subjected to directed chemical coupling in vitro to form the bispecific antibody.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Nati. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al, J. Immunol, 152:5368 (1994).

Antibodies with more than two valencies are contemplated. For example, trispecific antibodies can be prepared. Tuft et al. J. Immunol. 147: 60 (1991).

(vii) Effector Function Engineering

It may be desirable to modify the antibody of the invention with respect to effector function, so as to enhance the effectiveness of the antibody. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctonal cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al Anti-Cancer Drug Design 3:219-230 (1989).

(viii) Antibody-Salvage Receptor Binding Epitope Fusions.

In certain embodiments of the invention, it may be desirable to use an antibody fragment, rather than an intact antibody, to increase tumor penetration, for example. In this case, it may be desirable to modify the antibody fragment in order to increase its serum half life. This may be achieved, for example, by incorporation of a salvage receptor binding epitope into the antibody fragment (e.g. by mutation of the appropriate region in the antibody fragment or by incorporating the epitope into a peptide tag that is then fused to the antibody fragment at either end or in the middle, e.g., by DNA or peptide synthesis).

The salvage receptor binding epitope preferably constitutes a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (e.g., of an IgG) and transferred to the CH1, CH3, or V.sub.H region, or more than one such region, of the antibody. Alternatively, the epitope is taken from the CH2 domain of the Fc region and transferred to the CL region or VL region, or both, of the antibody fragment.

(ix) <u>Other Covalent Modifications of Antibodies</u>

Covalent modifications of antibodies are included within the scope of this invention. They may be made by chemical synthesis or by enzymatic or chemical cleavage of the antibody, if applicable. Other types of covalent modifications of the antibody are introduced into the molecule by reacting targeted amino acid residues of the antibody with an organic derivatizing agent that is capable of reacting with selected side chains or the N- or C-terminal residues. Examples of covalent modifications are described in U.S. Pat. No. 5,534,615 . A preferred type of covalent modification of the antibody comprises linking the antibody to one of a variety of nonproteinaceous polymers, e.g., polyethylene glycol, polypropylene glycol, or polyoxyalkylenes, in the manner set forth in U.S. Pat. Nos. 4,640,835; 4,496,689; 4,301,144; 4,670,417; 4,791,192 or 4,179,337.

The invention also pertains to immunoconjugates comprising the antibody described herein conjugated to a cytotoxic agent such as a chemotherapeutic agent, toxin (*e.g.* an enzymatically active toxin of bacterial, fungal, plant or animal origin, or fragments thereof), or a radioactive isotope (*i.e.*, a radioconjugate). A variety of radionuclides are available for the production of radioconjugate antibodies. Examples include, but are not limited to, e.g., $^{212}Bi$, $^{131}I$, $^{131}In$, $^{90}Y$ and $^{186}Re$.

Chemotherapeutic agents useful in the generation of such immunoconjugates have been described above. For example, BCNU, streptozoicin, vincristine, 5-fluorouracil, the family of agents known collectively LL-E33288 complex described in U.S. patents 5,053,394, 5,770,710, esperamicins (U.S. patent 5,877,296), etc. (see also the definition of chemotherapeutic agents herein) can be conjugated to antibodies of the invention or fragments thereof.

For selective destruction of the tumor, the antibody may comprise a highly radioactive atom. A variety of radioactive isotopes are available for the production of radioconjugated antibodies or fragments thereof. Examples include, but are not limited to, *e.g.,* $^{211}At$, $^{131}I$, $^{125}I$, $^{90}Y$, $^{186}Re$, $^{188}Re$, $^{153}Sm$, $^{212}Bi$, $^{32}P$, $^{212}Pb$, $^{111}In$, radioactive isotopes of Lu, etc. When the conjugate is used for diagnosis, it may comprise a radioactive atom for scintigraphic studies, for example $^{99m}tc$ or $^{123}I$, or a spin label for nuclear magnetic resonance (NMR) imaging (also known as magnetic resonance imaging, MRI), such as iodine-123, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as $^{99m}tc$ or $^{123}I$, $^{186}Re$, $^{188}Re$ and $^{111}In$ can be attached via a cysteine residue in the peptide. Yttrium-90 can be attached via a lysine residue. The IODOGEN method (Fraker et al. Biochem. Biophys. Res. Commun. 80: 49-57 (1978) can be used to incorporate iodine-123. *See, e.g.,* Monoclonal Antibodies in Immunoscintigraphy (Chatal, CRC Press 1989) which describes other methods in detail.

Enzymatically active toxins and fragments thereof which can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolacca americana* proteins (PAPI, PAPII, and PAP-S), momordica charantia inhibitor, curcin, crotin, sapaonaria officinalis inhibitor, gelonin, mitogellin, restrictocin, phenomycin, neomycin, and the tricothecenes. *See, e.g.,* WO 93/21232 published October 28, 1993.

Conjugates of the antibody and cytotoxic agent are made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein comprising the anti-VEGF, and/or the anti-protein of the invention antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In certain embodiments, the antibody is conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.* avidin) which is conjugated to a cytotoxic agent (*e.g.* a radionucleotide). In certain embodiments, an immunoconjugate is formed between an antibody and a compound with nucleolytic activity (e.g., a ribonuclease or a DNA endonuclease such as a deoxyribonuclease; Dnase).

The invention provides an antibody of the invention, which is conjugated to one or more maytansinoid molecules. Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

An antibody of the invention can be conjugated to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. In one embodiment, maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disulfide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Typical coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. The linkage is formed at the C-3 position of maytansinol or a maytansinol analogue.

Another immunoconjugate of interest comprises an antibody of the invention conjugated to one or more calicheamicin molecules. The calicheamicin family of antibiotics is capable of producing double-stranded DNA breaks at sub-picomolar concentrations. For the preparation of conjugates of the calicheamicin family, *see* U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, $\gamma_1^I$, $\alpha_2^I$, $\alpha_3^I$, N-acetyl-$\gamma_1^I$, PSAG and $\theta^I_1$ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

(x) Generation of Antibodies From Synthetic Antibody Phage Libraries

In certain embodiments, the invention provides a method for generating and selecting novel antibodies using a unique phage display approach. The approach involves generation of synthetic antibody phage libraries based on single framework template, design of sufficient diversities within variable domains, display of polypeptides having the diversified variable domains, selection of candidate antibodies with high affinity to target the antigen, and isolation of the selected antibodies.

Details of the phage display methods can be found, for example, WO03/102157 published December 11, 2003.

In one aspect, the antibody libraries used in the invention can be generated by mutating the solvent accessible and/or highly diverse positions in at least one CDR of an antibody variable domain. Some or all of the CDRs can be mutated using the methods provided herein. In some embodiments, it may be preferable to generate diverse antibody libraries by mutating positions in CDRH1, CDRH2 and CDRH3 to form a single library or by mutating positions in CDRL3 and CDRH3 to form a single library or by mutating positions in CDRL3 and CDRH1, CDRH2 and CDRH3 to form a single library.

A library of antibody variable domains can be generated, for example, having mutations in the solvent accessible and/or highly diverse positions of CDRH1, CDRH2 and CDRH3. Another library can be generated having mutations in CDRL1, CDRL2 and CDRL3. These libraries can also be used in conjunction with each other to generate binders of desired affinities. For example, after one or more rounds of selection of heavy chain libraries for binding to a target antigen, a light chain library can be replaced into the population of heavy chain binders for further rounds of selection to increase the affinity of the binders.

Preferably, a library is created by substitution of original amino acids with variant amino acids in the CDRH3 region of the variable region of the heavy chain sequence. The resulting library can contain a plurality of antibody sequences, wherein the sequence diversity is primarily in the CDRH3 region of the heavy chain sequence.

In one aspect, the library is created in the context of the humanized antibody 4D5 sequence, or the sequence of the framework amino acids of the humanized antibody 4D5 sequence. Preferably, the library is created by substitution of at least residues 95-100a of the heavy chain with amino acids encoded by the *DVK* codon set, wherein the *DVK* codon set is used to encode a set of variant amino acids for every one of these positions. An example of an oligonucleotide set that is useful for creating these substitutions comprises the sequence $(DVK)_7$, In some embodiments, a library is created by substitution of residues 95-100a with amino acids encoded by both *DVK and NNK* codon sets. An example of an oligonucleotide set that is useful for creating these substitutions comprises the sequence $(DVK)_6$ *(NNK).* In another embodiment, a library is created by substitution of at least residues 95-100a with amino acids encoded by both *DVK* and *NNK* codon sets. An example of an oligonucleotide set that is useful for creating these substitutions comprises the sequence $(DVK)_5$ (*NNK*). Another example of an oligonucleotide set that is useful for creating these substitutions comprises the sequence $(NNK)_6$. Other examples of suitable oligonucleotide sequences can be determined by one skilled in the art according to the criteria described herein.

In another embodiment, different CDRH3 designs are utilized to isolate high affinity binders and to isolate binders for a variety of epitopes. The range of lengths of CDRH3 generated in this library is 11 to 13 amino acids, although lengths different from this can also be generated. H3 diversity can be expanded by using *NNK, DVK and NVK* codon sets, as well as more limited diversity at N and/or C-terminal.

Diversity can also be generated in CDRH1 and CDRH2. The designs of CDR-H1 and H2 diversities follow the strategy of targeting to mimic natural antibodies repertoire as described with modification that focus the diversity more closely matched to the natural diversity than previous design.

For diversity in CDRH3, multiple libraries can be constructed separately with different lengths of H3 and then combined to select for binders to target antigens. The multiple libraries can be pooled and sorted using solid support selection and solution sorting methods as described previously and herein below. Multiple sorting strategies may be employed. For example, one variation involves sorting on target bound to a solid, followed by sorting for a tag that may be present on the fusion polypeptide (e.g., anti-gD tag) and followed by another sort on target bound to solid. Alternatively, the libraries can be sorted first on target bound to a solid surface, the eluted binders are then sorted using solution phase binding with decreasing concentrations of target antigen. Utilizing combinations of different sorting methods provides for minimization of selection of only highly expressed sequences and provides for selection of a number of different high affinity clones.

High affinity binders for the target antigen can be isolated from the libraries. Limiting diversity in the H1/H2 region decreases degeneracy about $10^4$ to $10^5$ fold and allowing more H3 diversity provides for more high affinity binders. Utilizing libraries with different types of diversity in CDRH3 (e.g., utilizing DVK or NVT) provides for isolation of binders that may bind to different epitopes of a target antigen.

Of the binders isolated from the pooled libraries as described above, it has been discovered that affinity may be further improved by providing limited diversity in the light chain. Light chain diversity is generated in this embodiment as follows in CDRL1: amino acid position 28 is encoded by RDT; amino acid position 29 is encoded by RKT; amino acid position 30 is encoded by RVW; amino acid position 31 is encoded by ANW; amino acid position 32 is encoded by THT; optionally, amino acid position 33 is encoded by CTG ; in CDRL2: amino acid position 50 is encoded by KBG; amino acid position 53 is encoded by AVC; and optionally, amino acid position 55 is encoded by GMA ; in CDRL3: amino acid position 91 is encoded by TMT or SRT or both; amino acid position 92 is encoded by DMC; amino acid position 93 is encoded by RVT; amino acid position 94 is encoded by NHT; and amino acid position 96 is encoded by TWT or YKG or both.

In another embodiment, a library or libraries with diversity in CDRH1, CDRH2 and CDRH3 regions is generated. In this embodiment, diversity in CDRH3 is generated using a variety of lengths of H3 regions and using primarily codon sets *XYZ and NNK or NNS.* Libraries can be formed using individual oligonucleotides and pooled or oligonucleotides can be pooled to form a subset of libraries. The libraries of this embodiment can be sorted against target bound to solid. Clones isolated from multiple sorts can be screened for specificity and affinity using ELISA assays. For specificity, the clones can be screened against the desired target antigens as well as other nontarget antigens. Those binders to the target antigen can then be screened for affinity in solution binding competition ELISA assay or spot competition assay. High affinity binders can be isolated from the library utilizing *XYZ* codon sets prepared as described above. These binders can be readily produced as antibodies or antigen binding fragments in high yield in cell culture.

In some embodiments, it may be desirable to generate libraries with a greater diversity in lengths of CDRH3 region. For example, it may be desirable to generate libraries with CDRH3 regions ranging from about 7 to 19 amino acids.

High affinity binders isolated from the libraries of these embodiments are readily produced in bacterial and eukaryotic cell culture in high yield. The vectors can be designed to readily remove sequences such as gD tags, viral coat protein component sequence, and/or to add in constant region sequences to provide for production of full length antibodies or antigen binding fragments in high yield.

A library with mutations in CDRH3 can be combined with a library containing variant versions of other CDRs, for example CDRL1, CDRL2, CDRL3, CDRH1 and/or CDRH2. Thus, for example, in one embodiment, a CDRH3 library is combined with a CDRL3 library created in the context of the humanized 4D5 antibody sequence with variant amino acids at positions 28, 29, 30,31, and/or 32 using predetermined codon sets. In another embodiment, a library with mutations to the CDRH3 can be combined with a library comprising variant CDRH1 and/or CDRH2 heavy chain variable domains. In one embodiment, the CDRH1 library is created with the humanized antibody 4D5 sequence with variant amino acids at positions 28, 30, 31, 32 and 33. A CDRH2 library may be created with the sequence of humanized antibody 4D5 with variant amino acids at positions 50, 52, 53, 54, 56 and 58 using the predetermined codon sets.

(xi) Antibody Variants

The novel antibodies generated from phage libraries can be further modified to generate antibody mutants with improved physical, chemical and or biological properties over the parent antibody. Where the assay used is a biological activity assay, the antibody mutant preferably has a biological activity in the assay of choice which is at least about 10 fold better, preferably at least about 20 fold better, more preferably at least about 50 fold better, and sometimes at least about 100 fold or 200 fold better, than the biological activity of the parent antibody in that assay. In certain embodiments, an anti-Bv8 antibody mutant or an anti-G-CSF mutant has a binding affinity for Bv8 or G-CSF, respectively, which is at least about 10 fold stronger, preferably at least about 20 fold stronger, more preferably at least about 50 fold stronger, and sometimes at least about 100 fold or 200 fold stronger, than the binding affinity of the parent antibody.

To generate the antibody mutant, one or more amino acid alterations (e.g. substitutions) are introduced in one or more of the hypervariable regions of the parent antibody. Alternatively, or in addition, one or more alterations (e.g. substitutions) of framework region residues may be introduced in the parent antibody where these result in an improvement in the binding affinity of the antibody mutant for the antigen from the second mammalian species. Examples of framework region residues to modify include those which non-covalently bind antigen directly (Amit et al. (1986) Science 233:747-753); interact with/effect the conformation of a CDR (Chothia et al. (1987) J. Mol. Biol. 196:901-917); and/or participate in the $V_L$ - $V_H$ interface (EP 239 400B1). In certain embodiments, modification of one or more of such framework region residues results in an enhancement of the binding affinity of the antibody for the antigen from the second mammalian species. For example, from about one to about five framework residues may be altered in this embodiment of the invention. Sometimes, this may be sufficient to yield an antibody mutant suitable for use in preclinical trials, even where none of the hypervariable region residues have been altered. Normally, however, the antibody mutant will comprise additional hypervariable region alteration(s).

The hypervariable region residues which are altered may be changed randomly, especially where the starting binding affinity of the parent antibody is such that such randomly produced antibody mutants can be readily screened.

One useful procedure for generating such antibody mutants is called "alanine scanning mutagenesis" (Cunningham and Wells (1989) Science 244:1081-1085). Here, one or more of the hypervariable region residue(s) are replaced by alanine or polyalanine residue(s) to affect the interaction of the amino acids with the antigen from the second mammalian species. Those hypervariable region residue(s) demonstrating functional sensitivity to the substitutions then are refined by introducing further or other mutations at or for the sites of substitution. Thus, while the site for introducing an amino acid sequence variation is predetermined, the nature of the mutation *per se* need not be predetermined. The ala-mutants produced this way are screened for their biological activity as described herein.

Normally one would start with a conservative substitution such as those shown below under the heading of "preferred substitutions". If such substitutions result in a change in biological activity (e.g. binding affinity), then more substantial changes, denominated "exemplary substitutions" in the following table, or as further described below in reference to amino acid classes, are introduced and the products screened.

Preferred substitutions:

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Even more substantial modifications in the antibodies' biological properties are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr, asn, gln;
(3) acidic: asp, glu;
(4) basic: his, lys, arg;
(5) residues that influence chain orientation: gly, pro; and
(6) aromatic: trp, tyr, phe.

Non-conservative substitutions will entail exchanging a member of one of these classes for another class.

In another embodiment, the sites selected for modification are affinity matured using phage display (see above).

Nucleic acid molecules encoding amino acid sequence mutants are prepared by a variety of methods known in the art. These methods include, but are not limited to, oligonucleotide-mediated (or site-directed) mutagenesis, PCR mutagenesis, and cassette mutagenesis of an earlier prepared mutant or a non-mutant version of the parent antibody. The preferred method for making mutants is site directed mutagenesis (see, *e.g.,* Kunkel (1985) Proc. Natl. Acad. Sci. USA 82:488).

In certain embodiments, the antibody mutant will only have a single hypervariable region residue substituted. In other embodiments, two or more of the hypervariable region residues of the parent antibody will have been substituted, e.g. from about two to about ten hypervariable region substitutions.

Ordinarily, the antibody mutant with improved biological properties will have an amino acid sequence having at least 75% amino acid sequence identity or similarity with the amino acid sequence of either the heavy or light chain variable domain of the parent antibody, more preferably at least 80%, more preferably at least 85%, more preferably at least 90%, and most preferably at least 95%. Identity or similarity with respect to this sequence is defined herein as the percentage of amino acid residues in the candidate sequence that are identical (*i.e* same residue) or similar (*i.e.* amino acid residue from the same group based on common side-chain properties, see above) with the parent antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

Following production of the antibody mutant, the biological activity of that molecule relative to the parent antibody is determined. As noted above, this may involve determining the binding affinity and/or other biological activities of the antibody. In a preferred embodiment of the invention, a panel of antibody mutants is prepared and screened for binding affinity for the antigen or a fragment thereof. One or more of the antibody mutants selected from this initial screen are optionally subjected to one or more further biological activity assays to confirm that the antibody mutant(s) with enhanced binding affinity are indeed useful, e.g. for preclinical studies.

The antibody mutant(s) so selected may be subjected to further modifications, oftentimes depending on the intended use of the antibody. Such modifications may involve further alteration of the amino acid sequence, fusion to heterologous polypeptide(s) and/or covalent modifications such as those elaborated below. With respect to amino acid sequence alterations, exemplary modifications are elaborated above. For example, any cysteine residue not involved in maintaining the proper conformation of the antibody mutant also may be substituted, generally with serine, to improve the oxidative stability of the molecule and prevent aberrant cross linking. Conversely, cysteine bond(s) may be added to the antibody to improve its stability (particularly where the antibody is an antibody fragment such as an Fv fragment). Another type of amino acid mutant has an altered glycosylation pattern. This may be achieved by deleting one or more carbohydrate moieties found in the antibody, and/or adding one or more glycosylation sites that are not present in the antibody. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Where the antibody comprises an Fc region, the carbohydrate attached thereto may be altered. For example, antibodies with a mature carbohydrate structure that lacks fucose attached to an Fc region of the antibody are described in US Pat Publication No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/011878, Jean-Mairet *et al.* and US Patent No. 6,602,684, Umana et al. Antibodies with at least one galactose residue in the oligosaccharide attached to an Fc region of the antibody are reported in WO 1997/30087, Patel *et al.* See, also, WO 1998/58964 (Raju, S.) and WO 1999/22764 (Raju, S.) concerning antibodies with altered carbohydrate attached to the Fc region thereof. See also US 2005/0123546 (Umana et al.) on antigen-binding molecules with modified glycosylation.

The preferred glycosylation variant herein comprises an Fc region, wherein a carbohydrate structure attached to the Fc region lacks fucose. Such variants have improved ADCC function. Optionally, the Fc region further comprises one or more amino acid substitutions therein which further improve ADCC, for example, substitutions at positions 298, 333, and/or 334 of the Fc region (Eu numbering of residues). Examples of publications related to "defucosylated" or "fucose-deficient" antibodies include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; Okazaki et al. J. Mol. Biol. 336:1239-1249 (2004); Yamane-Ohnuki et al. Biotech. Bioeng. 87: 614 (2004). Examples of cell lines producing defucosylated antibodies include Lec13 CHO cells deficient in protein fucosylation (Ripka et al. Arch. Biochem. Biophys. 249:533-545 (1986); US Pat Publication No US 2003/0157108 A1, Presta, L; and WO 2004/056312 A1, Adams et al., especially at Example 11), and knockout cell lines, such as alpha-1,6-fucosyltransferase gene, *FUT8*,knockout CHO cells (Yamane-Ohnuki et al. Biotech. Bioeng. 87: 614 (2004)).

(xii) Recombinant Production of Antibodies

For recombinant production of an antibody, the nucleic acid encoding it is isolated and inserted into a replicable vector for further cloning (amplification of the DNA) or for expression. DNA encoding the monoclonal antibody is readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence (e.g. as described in U.S. Pat. No. 5,534,615).

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, e.g., E. coli, Enterobacter, Erwinia, Klebsiella, Proteus, Salmonella, e.g., Salmonella typhimurium, Serrafia, e.g., Serratia marcescans, and Shigeila, as well as Bacilli such as B. subtilis and B. licheniformis (e.g., B. licheniformis 41P disclosed in DD 266,710 published Apr. 12, 1989), Pseudomonas such as P. aeruginosa, and Streptomyces. One preferred E. coli cloning host is E. coli 294 (ATCC 31,446), although other strains such as E. coli B, E. coli X 1776 (ATCC 31,537), and E coil W3110 (ATCC 27,325) are suitable. These examples are illustrative rather than limiting.

In addition to prokaryotes, eukaryotic microbes such as filamentous fungi or yeast are suitable cloning or expression hosts for antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e.g., K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-1 variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subloned for growth in suspension culture, Graham et al, J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

The host cells used to produce the antibody of this invention may be cultured in a variety of media. Commercially available media such as Ham's F10 (Sigma-Aldrich, St. Louis, MO), Minimal Essential Medium ((MEM), (Sigma-Aldrich, St. Louis, MO), RPMI-1640 (Sigma-Aldrich, St. Louis, MO), and Dulbecco's Modified Eagle's Medium ((DMEM), Sigma-Aldrich, St. Louis, MO) are suitable for culturing the host cells. In addition, any of the media described in Ham et al.,

Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. No. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMICIN™), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, as a first step, the particulate debris, either host cells or lysed cells, is removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human $\gamma$, $\gamma 2$, or $\gamma 4$ heavy chains (Lindmark et al., J. Immunol. Meth. 62:1-13 (1983)). Protein G is recommended for all mouse isotypes and for human $\gamma 3$ (Guss et al., EMBO J. 5:1567-1575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH 3 domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

**D. Uses of G-CSF antagonists and Bv8 antagonists**

The G-CSF antagonists and Bv8 antagonists of the present invention can be used, alone or in combination with other therapeutic agent(s) for the inhibition of angiogenesis, tumor metastasis, tumor cell migration and/or the modulation of mobilization of Cd11b+Gr1+ cells or functional human counterpart of Cd11b+Gr1+ cells.

Primary targets for the treatment methods of the present invention are metastatic tumors and tumors that are capable of metastasizing to a pre-metastatic organs or pre-metastatic tissues. The treatment methods of the present invention also target tumors that secrete G-CSF, which mobilizes Cd11b+Gr1+ cells or functional human counterpart of Cd11b+Gr1+ cells from bone marrow, thus initiating the pre-metastasis niche. Examples of human counterpart cells include human immature myeloid cells, human myeloid suppressor cells, precursors of human neutrophils, monocytes and macrophages, and human neutrophils, monocytes and macrophages. The treatment methods of the present invention also target tumors that have increased expression of G-CSF, Bv8, PKR1, MMP-9, S100A8 and/or S100A9.

Examples of diseases and disorders to be treated by the methods of the present invention include neoplastic disorders, such as those described herein under the terms "cancer" and "cancerous." In certain embodiments, a disease to be treated by the methods of the present invention is a metastatic tumor or metastatic cancer.

The treatment methods of the present invention also provides for treatment of benign, pre-cancerous or non-metastatic cancer, wherein the treatment prevents the benign, pre-cancerous, or non-metastatic cancer from becoming a metastatic cancer.

The invention further provides methods for predicting whether a metastatic tumor in a patient will respond effectively to a treatment with G-CSF antagonists and/or Bv8 antagonists. Examples of such methods include determining whether a sample obtained from the patient comprises a cell that expresses Bv8, PKR1 and/or G-CSF. In certain embodiments, the expression is mRNA expression. In certain embodiments, the expression is protein expression. In certain embodiments, the mRNA expression level of the gene is measured using qRT-PCR or qPCR. In another embodiment, the mRNA expression level is measured using microarrary. In another embodiment, the mRNA expression level is measured using ISH (in situ hybridization). In certain embodiments, the protein expression level of the gene is measured using IHC assay. In certain embodiments, the treatment prevents the benign, pre-cancerous, or non-metastatic cancer from becoming a metastatic cancer. In certain embodiments, the treatment with G-CSF antagonists and/or Bv8 antagonists prevents the metastatic cancer from metastasizing to pre-metastatic tissues or organs elsewhere in the body.

In certain embodiments, efficacy of the treatment with G-CSF antagonists and/or Bv8 antagonists, can be monitored by determining the expression level of G-CSF, Bv8, PKR1, MMP-9, S100A8 or S 100A9 in pre-metastatic organ or pre-metastatic tissue. In certain embodiments, the mRNA expression levels and/or protein expression levels of Bv8, MMP-9, S100A8 and/or S100A9 are up-regulated in pre-metastatic lung and/or metastic lung. In certain embodiments, the treatment with anti-Bv8 antibody is efficacious if the mRNA expression level and/or protein expression level of Bv8 and/or MMP-9 is decreased after the treatment with anti-Bv8 antibody. In certain embodiments, the treatment with anti-Bv8 antibody reduces the mRNA expression level and/or protein of Bv8 and/or MMP-9 in pre-metastatic lungs. In certain embodiments, the treatment with anti-G-CSF antibody is efficacious if the mRNA expression level and/or protein expression level of Bv8 and/or MMP-9 is decreased after the treatment with anti-G-CSF antibody. In certain embodiments, the treatment with anti-G-CSF antibody reduces the mRNA expression level and/or protein expression level of Bv8 and/or MMP-9 in pre-metastatic lungs and/or metastatic lungs.

The invention provides combined therapies in which a G-CSF antagonist or Bv8 antagonist of the present invention is administered in combination with another therapy. Combination treatment specifically includes the administration of a G-CSF antagonist and/or Bv8 antagonist herein in combination with a VEGF antagonist, such as an anti-VEGF antibody. In addition, or alternatively, the G-CSF antagonists and/or Bv8 antagonists herein can be administered in combination with one or more further agents, e.g., anti-cancer agents or therapeutics, anti-angiogenesis agents, or anti-neovascularization therapeutics to treat various neoplastic conditions.

The G-CSF antagonist or Bv8 antagonist of the invention can be administered serially or in combination with another agent that is effective for those purposes, either in the same composition or as separate compositions.

The administration of the antagonist and/or agents can be done simultaneously, *e.g.,* as a single composition or as two or more distinct compositions using the same or different administration routes. Alternatively, or additionally, the administration can be done sequentially, in any order. In certain embodiments, intervals ranging from minutes to days, to weeks to months, can be present between the administrations of the two or more compositions. For example, the VEGF antagonist may be administered first, followed by a different antagonist or agent. However, simultaneous administration or administration of the different antagonist or agent of the invention first is also contemplated.

The effective amounts of therapeutic agents administered will be at the physician's or veterinarian's discretion. Dosage administration and adjustment is done to achieve maximal management of the conditions to be treated. The dose will additionally depend on such factors as the type of therapeutic agent to be used and the specific patient being treated. Suitable dosages for the VEGF antagonist are those presently used and can be lowered due to the combined action (synergy) of the VEGF antagonist and the different antagonist of the invention. In certain embodiments, the combination of the inhibitors potentiates the efficacy of a single inhibitor. The term "potentiate" refers to an improvement in the efficacy of a therapeutic agent at its common or approved dose. *See also* the section entitled Pharmaceutical Compositions herein.

Anti-angiogenic therapy in relationship to cancer is a cancer treatment strategy aimed at inhibiting the development of tumor blood vessels required for providing nutrients to support tumor growth. Because angiogenesis is involved in both primary tumor growth and metastasis, the antiangiogenic treatment provided by the invention is capable of inhibiting the neoplastic growth of tumor at the primary site as well as preventing metastasis of tumors at the secondary sites, therefore allowing attack of the tumors by other therapeutics. In one embodiment of the invention, anti-cancer agent or therapeutic is an anti-angiogenic agent. In another embodiment, anti-cancer agent is a chemotherapeutic agent.

Many anti-angiogenic agents have been identified and are known in the arts, including those listed herein, *e.g.,* listed under Definitions, and by, *e.g.,* Carmeliet and Jain, Nature 407:249-257 (2000); Ferrara et al., Nature Reviews:Drug Discovery, 3:391-400 (2004); and Sato Int. J. Clin. Oncol., 8:200-206 (2003). *See also,* US Patent Publication US20030055006. In one embodiment, a G-CSF antagonist and/or Bv8 antagonist is used in combination with an anti-VEGF neutralizing antibody (or fragment) and/or another VEGF antagonist or a VEGF receptor antagonist including, but not limited to, for example, soluble VEGF receptor (e.g., VEGFR-1, VEGFR-2, VEGFR-3, neuropillins (e.g., NRP1, NRP2)) fragments, aptamers capable of blocking VEGF or VEGFR, neutralizing anti-VEGFR antibodies, low molecule weight inhibitors of VEGFR tyrosine kinases (RTK), antisense strategies for VEGF, ribozymes against VEGF or VEGF receptors, antagonist variants of VEGF; and any combinations thereof. Alternatively, or additionally, two or more angiogenesis inhibitors may optionally be co-administered to the patient in addition to VEGF antagonist and G-CSF antagonist and/or Bv8 antagonist. In certain embodiment, one or more additional therapeutic agents, *e.g.,* anti-cancer agents, can be administered in combination with G-CSF antagonist, Bv8 antagonist, the VEGF antagonist, and/or an anti-angiogenesis agent.

In certain aspects of the invention, other therapeutic agents useful for combination tumor therapy with the G-CSF antagonists or Bv8 antagonists include other cancer therapies, (e.g., surgery, radiological treatments (e.g., involving irradiation or administration of radioactive substances), chemotherapy, treatment with anti-cancer agents listed herein and known in the art, or combinations thereof). Alternatively, or additionally, two or more antibodies binding the same or two or more different antigens disclosed herein can be co-administered to the patient. Sometimes, it may be beneficial to also administer one or more cytokines to the patient.

In certain aspects, the invention provides a method of inhibiting metastasis by administering effective amounts of an antagonist of VEGF and a G-CSF antagonist and/or Bv8 antagonist and one or more chemotherapeutic agents to a patient susceptible to, or diagnosed with, cancer. A variety of chemotherapeutic agents may be used in the combined treatment methods of the invention. An exemplary and non-limiting list of chemotherapeutic agents contemplated is provided herein under "Definition."

As will be understood by those of ordinary skill in the art, the appropriate doses of chemotherapeutic agents will be generally around those already employed in clinical therapies wherein the chemotherapeutics are administered alone or in combination with other chemotherapeutics. Variation in dosage will likely occur depending on the condition being treated. The physician administering treatment will be able to determine the appropriate dose for the individual subject.

The invention provides methods and compositions for treating a patient who is relapsed from or refractory to previous anti-cancer therapy. The invention also provides methods and compositions for treating relapse tumor growth or relapse cancer cell growth. Relapse tumor growth or relapse cancer cell growth is used to describe a condition in which patients undergoing or treated with one or more currently available therapies (e.g., cancer therapies, such as chemotherapy, radiation therapy, surgery, hormonal therapy and/or biological therapy/immunotherapy, anti-VEGF antibody therapy, particularly a standard therapeutic regimen for the particular cancer) is not clinically adequate to treat the patients or the patients are no longer receiving any beneficial effect from the therapy such that these patients need additional effective therapy. In certain embodiments, the phrase can also refer to a condition of the "non-responsive/refractory" patient, *e.g.,* which describe patients who respond to therapy yet suffer from side effects, develop resistance, do not respond to the therapy, do not respond satisfactorily to the therapy, etc. In various embodiments, a cancer is relapse tumor growth or relapse cancer cell growth where the number of cancer cells has not been significantly reduced, or has increased, or tumor size has not been significantly reduced, or has increased, or fails any further reduction in size or in number of cancer cells. The determination of whether the cancer cells are relapse tumor growth or relapse cancer cell growth can be made either *in vivo* or *in vitro* by any method known in the art for assaying the effectiveness of treatment on cancer cells, using the art-accepted meanings of "relapse" or "refractory" or "non-responsive" in such a context as well as using the definitions provided herein under Definitions.

In addition, the G-CSF antagonists or Bv8 antagonists can be administered in combination with hormonal, radiation and chemotherapeutic agents thereby resensitizing the cancer cells to one or more of these agents, which can then be administered (or continue to be administered) to treat or manage cancer, including metastatic cancer, and to prevent metastasis.

The invention is also based partly on the identification of biomarkers that are useful for identifying patients who will respond effectively to treatment with a G-CSF antagonist and/or Bv8 antagonist. These biomarkers are also useful for predicting and monitoring the efficacy of such treatment. For example, a cancer patient could have a biopsy performed to obtain a tissue or cell sample, and the sample could be examined by various *in vitro* assays to determine whether the expression of G-CSF, PKR1, or one or more genes listed as URMCNAs or DRMCNAs is increased or decreased as compared to a reference sample. A sample comprising a target biomarker can be obtained by methods well known in the art, and that are appropriate for the particular type and location of the cancer of interest. Tissue biopsy is often used to obtain a representative piece of cancerous tissue. Comparison can be performed on samples and reference samples measured concurrently or at temporally distinct times. Thus, the disclosed methods and assays provide convenient, efficient, and potentially cost-effective means to obtain data and information useful in assessing appropriate or effective therapies for treating cancer patients.

In the methods of the invention, a mammalian tissue or cell sample is obtained and examined for expression of one or more biomarkers. Expression of various biomarkers in a sample can be analyzed by a number of methodologies, many of which are known in the art and understood by the skilled artisan, including but not limited to, immunohistochemical and/or Western blot analysis, immunoprecipitation, molecular binding assays, ELISA, ELIFA, fluorescence activated cell sorting (FACS) and the like, quantitative blood based assays (as for example Serum ELISA) (to examine, for example, levels of protein expression), biochemical enzymatic activity assays, *in situ* hybridization, Northern analysis and/or PCR analysis of mRNAs, as well as any one of the wide variety of assays that can be performed by gene and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery (MSD) may also be used.

Methods of the invention further include protocols which examine the presence and/or expression of mRNAs of the one ore more target genes listed as URMCNAs or DRMNAs in a tissue or cell sample. The target genes also include G-CSF and PKR1. Methods for the evaluation of mRNAs in cells are well known and include, for example, hybridization assays using complementary DNA probes (such as *in situ* hybridization using labeled riboprobes specific for the one or more genes listed as URMCNAs, DRMCNAs, G-CSF or PKR1. Northern blot and related techniques) and various nucleic acid amplification assays (such as RT-PCR using complementary primers specific for one or more of the genes listed as URMCNAs, DRMCNAs, G-CSF or PKR1, and other amplification type detection methods, such as, for example, branched DNA, SISBA, TMA and the like).

Tissue or cell samples from mammals can be conveniently assayed for mRNAs using Northern, dot blot or PCR analysis. For example, RT-PCR assays such as quantitative PCR assays are well known in the art. In an illustrative embodiment of the invention, a method for detecting a target mRNA in a biological sample comprises producing cDNA from the sample by reverse transcription using at least one primer; amplifying the cDNA so produced using a target polynucleotide as sense and antisense primers to amplify target cDNAs therein; and detecting the presence of the amplified target cDNA. In addition, such methods can include one or more steps that allow one to determine the levels of target mRNA in a biological sample (e.g. by simultaneously examining the levels a comparative control mRNA sequence of a "housekeeping" gene such as an actin family member). Optionally, the sequence of the amplified target cDNA can be determined. In certain embodiments, methods of the invention include protocols which examine or detect mRNAs, such as target mRNAs, in a tissue or cell sample by microarray technologies.

In some embodiments of the invention, the expression of target proteins in a sample is examined using immunohistochemistry and staining protocols. Immunohistochemical staining of tissue sections has been shown to be a reliable method of assessing or detecting presence of proteins in a sample. Immunohistochemistry ("IHC") techniques utilize an antibody to probe and visualize cellular antigens *in situ,* generally by chromogenic or fluorescent methods.

A diagnostic marker set for target proteins can include one, two or more, three or more, four or more, five or more, six or more, seven or more, eight or more, nine or more, ten or more, twelve or more, thirteen or more, fourteen or more, fifteen or more, or the entire set, of molecules listed under URMCPs or DRMCPs. The molecule is a nucleic acid encoding a protein or a protein with an altered expression and/or activity, and is selected from the following: C-CSF, Bv8, PKR1, Stfa3, CAMP, TNFSF14, MGAM, Ngp, S100A8, S100A9, LRP1B, CEACAM4, HAO1, C10orf46, CLECSF9, Mcpt8, PRTN3, Slfn3, MMP-9, TYMS, CRIPTO CR-1, MPHOSPH1, CDH6, WWP2, DMC1, BCHE, NSLE16484, F9, GDAP1, LOC375188, DNTT, PPIL3, KCND2, ZNF597, IGSF4D and GTF3C4. In certain embodiments, one or more antibodies are provided that detects one or more of the target proteins.

### E. Pharmaceutical Compositions and Administration

The G-CSF antagonists and Bv8 antagonists, such as anti-G-CSF antibodies, anti-Bv8 antibodies and anti-PKR1 antibodies of the present invention, alone or in combination with other therapeutic agents, are administered to a human patient, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes, and/or subcutaneous administration.

In certain embodiments, the treatment of the invention involves the combined administration of a G-CSF antagonist or Bv8 antagonist and a VEGF antagonist and/or chermotherapeutic agent. In one embodiment, additional anti-cancer agents are present, e.g., one or more different anti-angiogenesis agents, one or more chemotherapeutic agents, etc. The invention also contemplates administration of multiple inhibitors, e.g., multiple antibodies to the same antigen or multiple antibodies to different proteins of the invention. In one embodiment, a cocktail of different chemotherapeutic agents is administered with the Bv8 antagonist herein. The combined administration includes coadministration, using separate formulations or a single pharmaceutical formulation, and/or consecutive administration in either order. For example, a VEGF antagonist may precede, follow, alternate with administration of the G-CSF antagonist or Bv8 antagonist, or may be given simultaneously therewith. In one embodiment, there is a time period while both (or all) active agents simultaneously exert their biological activities.

For the prevention or treatment of disease, the appropriate dosage of the agent of the invention will depend on the type of disease to be treated, as defined above, the severity and course of the disease, whether the inhibitor is administered for preventive or therapeutic purposes, previous therapy, the patient's clinical history and response to the inhibitor, and the discretion of the attending physician. The inhibitor is suitably administered to the patient at one time or over a series of treatments. In a combination therapy regimen, the compositions of the invention are administered in a therapeutically effective amount or a therapeutically synergistic amount. As used herein, a therapeutically effective amount is such that administration of a composition of the invention and/or co-administration of VEGF antagonist and one or more other therapeutic agents, results in reduction or inhibition of the targeting disease or condition. The effect of the administration of a combination of agents can be additive. In one embodiment, the result of the administration is a synergistic effect. A therapeutically synergistic amount is that amount of VEGF antagonist and one or more other therapeutic agents, *e.g.,* a G-CSF antagonist and/or Bv8 antagonist and optionally a chemotherapeutic agent and/or an anti-cancer agent, necessary to synergistically or significantly reduce or eliminate conditions or symptoms associated with a particular disease.

Depending on the type and severity of the disease, about 1 μg/kg to 50 mg/kg *(e.g.* 0.1-20mg/kg) of G-CSF antagonist, Bv8 antagonist, VEGF antagonist, a chemotherapeutic agent, and/or an anti-cancer agent is an initial candidate dosage for administration to the patient, whether, for example, by one or more separate administrations, or by continuous infusion. A typical daily dosage might range from about 1 μg/kg to about 100 mg/kg or more, depending on the factors mentioned above. For repeated administrations over several days or longer, depending on the condition, the treatment is sustained until a desired suppression of disease symptoms occurs. However, other dosage regimens may be useful. Typically, the clinician will administered a molecule(s) of the invention until a dosage(s) is reached that provides the required biological effect. The progress of the therapy of the invention is easily monitored by conventional techniques and assays.

For example, preparation and dosing schedules for angiogenesis inhibitors, *e.g.,* anti-VEGF antibodies, such as AVASTIN® (Genentech, South San Francisco, CA), may be used according to manufacturers' instructions or determined empirically by the skilled practitioner. In another example, preparation and dosing schedules for such chemotherapeutic agents may be used according to manufacturers' instructions or as determined empirically by the skilled practitioner. Preparation and dosing schedules for chemotherapy are also described in Chemotherapy Service Ed., M.C. Perry, Williams & Wilkins, Baltimore, MD (1992).

The efficacy of the treatment of the invention can be measured by various endpoints commonly used in evaluating neoplastic disorders. For example, cancer treatments can be evaluated by, e.g., but not limited to, tumor regression, tumor weight or size shrinkage, time to progression, duration of survival, progression free survival, overall response rate, duration of response, quality of life, protein expression and/or activity. Because the anti-angiogenic agents described herein target the tumor vasculature and not necessarily the neoplastic cells themselves, they represent a unique class of anticancer drugs, and therefore can require unique measures and definitions of clinical responses to drugs. For example, tumor shrinkage of greater than 50% in a 2-dimensional analysis is the standard cut-off for declaring a response. However, the inhibitors of the invention may cause inhibition of metastatic spread without shrinkage of the primary tumor, or may simply exert a tumouristatic effect. Accordingly, approaches to determining efficacy of the therapy can be employed, including for example, measurement of plasma or urinary markers of angiogenesis and measurement of response through radiological imaging.

In another embodiment of the invention, an article of manufacture containing materials useful for the treatment of the disorders or diagnosing the disorders described above is provided. The article of manufacture comprises a container, a label and a package insert. Suitable containers include, for example, bottles, vials, syringes, etc. The containers may be formed from a variety of materials such as glass or plastic. In one embodiment, the container holds a composition which is effective for treating the condition and may have a sterile access port (for example the container may be an intravenous solution bag or a vial having a stopper pierceable by a hypodermic injection needle). At least one active agent in the composition is a G-CSF antagonist or Bv8 antagonist. In certain embodiments, the active agent is anti-G-CSF antibody. In certain embodiments, the active agent is anti-Bv8 antibody. The label on, or associated with, the container indicates that the composition is used for treating the condition of choice. In certain embodiments, the containers hold a marker set which is diagnostic for detecting metastatic tumors that will respond effectively to treatment with a G-CSF antagonist and/or Bv8 antagonist. In certain embodiments, at least one agent in the composition is a marker for detecting G-CSF, PKR1, a URMCNA, a URMCP, a DRMCNA and/or a DRMCP. The article of manufacture may further comprise a second container comprising a pharmaceutically-acceptable buffer, such as phosphate-buffered saline, Ringer's solution and dextrose solution.

Further details of the invention are illustrated by the following non-limiting examples.

## Example 1

### Materials and Methods

Cell lines. Mouse mammary tumor cell lines 67NR, 168FARN, 4TO7, and 66c14 (Dexter, DL et al Cancer Res 38, 3174 (1978); Aslakson, CJ and Miller, FR Cancer Res 52, 1399 (1992)) were from F. Miller (Karmanos Cancer Institute, Detroit, MI). 4T1 breast carcinoma, Lewis Lung Carcinoma (LLC), and B16F10 mouse melanoma were purchased from the ATCC. MDA-MB-231-D3H1 (MDA-MB-231 cells stably expressing *luciferase)* were from Xenogen (Alameda, CA). MDA-MB-231-X1.1 are GFP expressing cells, previosuly passaged through mice in order to generate highly tumorgenic cell line. They were generated by tranfecting parental MDA-MB-231 cells with vector expressing GFP and puromycin. Subsequently, GFP-positive cells were injected subqutenously (in Matrigel) into SCID/bg mice. Visible tumors were harvested and tumorgenic cells were isolated and expanded. All breast cancer cell lines were cultured in IMDM, except MDA-MB-231, LLC and B16F10 cells which were grown in DMEM (Invitrogen, Carlsbad, CA). Both IMDM and DMEM were supplemented with 10% FBS (Sigma, St. Louis, MO). All cells were maintained at 37°C in a 5% $CO_2$, 80% humidity incubator.

Mice. Mouse mammary carcinoma MMTV-PyMT mice were obtained from McMaster University, Ontario. Female Balb/c, Balb/c Nude, CB6F1 (a cross between female BALB/c and male C57BL/6) were from Charles River Laboratory (Hollister, CA). Female C57BL/6 mice were obtained from The Jackson Laboratory (Sacramento, CA). Maintenance of animals and experimental protocols were conducted following federal regulations and approved by Institutional Animal Care and Use Committee.

**Tumor models.** 67NR, 168FARN, 4TO7, 66c14, and 4T1 cells were injected orthotopically into the right $4^{th}$ mammary fat pad of female Balb/c or CB6F1 at the concentration of 200,000 cells per 10μl of PBS. Anti-Bv8 treatment was performed as described previously (Shojaei, F. et al. Nature 450, 825-831 (2007)). Briefly, anti-Bv8 treatment was started (5mg/kg of each 2B9 and 3F1 monoclonal antibodies) 2 days after tumor cell inoculation by the intraperitoneal route of administration. Anti-ragweed monoclonal antibody (10mg/kg) served as controls. Antibodies were administered to tumor-bearing mice twice weekly. Neutralizing anti-mouse G-CSF mAb (MAB414) and the matching isotype IgG control (R&D Systems, Minneapolis, MN) were administered at the dose of 25μg per mouse every other day. Tumor volumes were calculated every other day using the ellipsoid volume formulas ($0.5 \times L \times W^2$, where L is length and W is width).

B16F10 and LLC cells were resuspended at a concentration of $1 \times 10^8$ cells per ml Matrigel (growth factor-reduced; BD Pharmingen, San Jose, CA) and injected (100 μl) subcutaneously into the dorsal flank of C57BL/6 mice. Tissues from B16F10 or LLC bearing mice were analyzed for the content of Cd11b+Gr1+ cells and Bv8 expression 15 days after tumor inoculation, at which point cancer cells were not detected in the lung tissue.

Tissues from female MMTV-PyMT mice were analyzed at week 8 (no visible lung metastasis were detected) and week 12 (metastatic nodules were clearly visible) of age for the expression of G-CSF and Bv8 in plasma and lung tissue. MMTV-PyMT negative siblings (littermates that lacked the transgene as assessed by genotyping) were used as control animals and are referred to as naive. For anti-Bv8 and anti-G-CSF study, MMTV-PyMT cells were isolated from primary tumors of 12-week old females. All isolated cells (tumor and stroma) were counted and resuspended at a concentration of $2 \times 10^7$ cells per ml of PBS:Matrigel (1:1 mix) (growth factor-reduced; BD Pharmingen, San Jose, CA) and injected (50 μl) into the 4th mammary fat pad of FVB mice. Isolated cells were also cultured in DMEM supplemented with FBS. Treatment with anti-Bv8 (10mg/kg of 2D3 monoclonal antibody) or anti-mouse G-CSF (anti-G-CSF, 2.5mg/kg, MAB414 from R&D Systems, Minneapolis, MN) was initiated 2 days after tumor inoculation by the intraperitoneal route of administration. At the end of the study, lungs were perfused with PBS and fixed in formalin. Subsequently, 7-μm-thick sections were H&E stained and tumors present in lungs were counted. On average, three sections (seperated by 250 μm) per each lung were analyzed.

MDA-MB-231-X1.1 cells were resuspended at a concentration of $4 \times 10^7$ cells per ml of PBS:Matrigel (1:1 mix) (BD Pharmingen, San Jose, CA) and injected (50 μl) into the 4th mammary fat pad of SCID/bg mice. Treatment with anti-Bv8 (10mg/kg of 2D3 monoclonal antibody) or anti-human G-CSF (anti-hG-CSF, 2.5mg/kg, MAB214 from R&D Systems, Minneapolis, MN) was initiated 2 days after tumor cell inoculation by the intraperitoneal route of administration. Matching isotype IgG control served as controls. Antibodies were administered to tumor-bearing mice three times a week. At the end of the study, lungs were perfused with PBS and fixed in para-formaldehyde for 16 hours. Subsequently, lungs were transferred to 30% sucrose/PBS solution. The number of metastatic foci was generated by placing whole lungs under the flourescent microscope and counting GFP-positive foci within the tissue (consisting of more than 2 cells to avoid counting single cancer cells trapped in the lung vasculature).

Alternatively, for studies involving injection of recombinant G-CSF into female Balb/c Nude mice, hamster anti-mouse Bv8 mAb 2D3 (Shojaei, F. et al. Proc Natl Acad Sci U S A (2009)), an antibody suitable for immunoneutralization and immunohistochemistry (Genentech, South San Francisco, CA), was administered at the dose of 10mg/kg twice weekly.

**Generation of Bv8 null mice.** To assess the impact of absence of endogenous Bv8 in tumor models, we generated mice lacking Bv8 in bone marrow derived mononulear cells (BMMNCs) through transplantation of Bv8 null fetel liver cells. To generate Bv8 null mice, a BAC clone mVRPA BAC 133 N12 (Research Genetics) containing the mouse gene Bv8, and a targeting vector TNLOX1-3 with three *loxP* sites were used to build a targeting construct designed to generate mice with a knock-out and a conditional knock-out deletion allele of Bv8. ES clones with PGK-Neo cassette correctly targeted at exon2 were screened by Southern blotting and subjected to electroporation of Cre recombinase to excise both PGK-Neo selection cassette and exon 2 for Bv8 knock-out. Six Bv8 KO clones were injected into blastocysts from C57BL/6 mice. Chimeras transmitted the disruptive mutation through the germline were crossed with C57BL/6 mice. Mice were kept on inbred 129SvEv background and backcrossed to C57BL/6 background.

**Fetal liver transplantation and tumor inoculation into a lethally irradiated host.** Fetal liver (FL) cells were isolated from embryos of Bv8 WT or KO mice at days E13.5-14.5. Pregnant mice were euthanized at days 13.5-14.5 of pregnancy and FL cells were isolated in a microdissection microscope and immediately placed in Petri dish containing cold plain DMEM. Single cell suspension was generated by vigorously pipetting the FLs and by passing cells through a 40 micron cell strainer (BD Falcon, CA). FL cells were then subjected to red blood cell lysis using ACK lysis buffer (Cambrex, MA), kept in plain DMEM and subsequently used for transplantation into lethally irradiated mice.

Before transplantation, CB6F1 mice were lethally irradiated with 1080 Rad, in a Cs-irradiator, to ablate endogenous BMMNCs in the host, followed by iv injection of $4 \times 10^5$ of whole fetal liver cells in 100μl of PBS.

Four weeks after FL tranplantation, 4T1 or 66c14 cells were inoculated into the 4th mammary fat pad of CB6F1 mice as described. Treatment with isotype control or anti-Bv8 antibody (2B9 and 3F1) was perforemd as described (Shojaei, F. et al. Nature 450, 825-831 (2007)). Lungs were analyzed for the presence of metastases either by micro-CT or by counting visible tumors on the lungs surface.

**Generation of Luc-zsGreen-Hygro cells lines.** pMSCV vector encoding a fusion protein Luciferase-zsGreen (a functional fusion between firefly luciferase and zsGreen) was generated by inserting a luciferase cDNA in frame with zsGreen cDNA into pMSCV-zsGreen vector. Hygromycin resistance gene was encoded by pMSCV vector.

To generate 67NR and 4T1 cells stably expressing Luciferase-zsGreen fusion, cells were transfected with pMSCV-Luc-zsGreen-Hygro vector using FuGENE transfection reagent, according to the manufacturer's protocol (Roche, Indianapolis, IN). Hygromycin-resistant cells were selected by culturing transfected cells in the presence of 200 μg/ml of Hygromycin for 10 days. Luciferase-zsGreen positive cells were sorted by Flow Cytometry based on their zsGreen expression and further validated to express luciferase by measuring luciferase activity with Dual-Luciferase Reporter Assay System (Promega, San Luis Obispo, CA). Cells expressing both zsGreen and Luciferase were further expanded in the presence of Hygromycin (100 μg/ml) and used for subsequent experiments.

***In vivo* selection of MDA-MB-231 cells.** To generate MDA-MB-231 cell line that shows enhanced metastatic potential in lungs, $5x10^5$ MDA-MB-231-D3H1 cells in PBS were injected through the tail-vein of SCID/bg mice. 6 weeks later (when bioluminescence imaging showed presence of growing tumors in lungs), lungs from three mice were harvested and tumor cells were disected and expanded in culture. Three independent cell lines (obtain from three different mice) were generated and named MDA-MB-231-L1.1, -L2.1 and -L3.1. To obtain cells that are tumorgenic at the primary tumor site, $1x10^6$ MDA-MB-231-D3H1 cells were inoculated in Matrigel into the 4th mammary fat pad of SCID/bg mice. Primary tumors were harvested and cells diasected and expanded in culture. Three independent cell lines (obtain from three different mice) were generated and named MDA-MB-231-T1.1, -T2.1 and -T3.1. For gene expression analysis, newly established cell lines were seeded at density of 500,000 cells/well (in 6-well plates) and RNA was collected 48 hours later and used for subsequent qRT-PCR expression analysis.

**Production of 66c14-shPKR1 and 67NR-PKR1 cells.** To generate 66c14 cells with reduced expression of *PKR1,* 66c14 cells were transfected with shRNA vector targeting mouse *PKR1* (shPKR1, Open Biosystems, Huntsville, AL), and clones stably expressing shPKR1 were isolated through cultering cells in the presence of puromycin (5μg/ml). As a control, 66c14 cells were transfected with vector expressing non-targeting shRNA (sh(Control)). To generate 67NR cells over-expressing PKR1, 67NR cells were co-transfected with vector containing CMV-PKR1 cassette together with vector carrying Zeocin-resistance gene. Cells stably expressing PKR1 were selected in the presence of zeocin (200ng/ml). Susequently, cells were injected intravenously through tail-vein into Balb/c mice pre-treated with vehicle or G-CSF. Three weeks later, lungs were isolated and checked for the presence of visible tumors. Authenticity of tumors in lungs was confirmed by H&E staining.

**RNA sample preparation and quantitative reverse transcriptase-PCR (qRT-PCR) analysis.** Total DNA-free RNA was prepared from PBS-perfused lungs with RNeasy kit (Qiagen, Germany) according to the manufacturer's protocol. One-step quantitative reverse transcription-PCR was done in a total volume of 50 μL with SuperScript III Platinum One-Step qRT-PCR Kit (Invitrogen, Carlsbad, CA) or with TaqMan One-Step RT-PCR Master Mix (Applied Biosystems, Foster City, CA), and 100 ng of total RNA. The following TaqMan Gene Expression Assay primers and probe mixes were used: Bv8 (assay ID: Mm00450080_ml), *MMP9* (assay ID: Mm00442991_m1), *S100A8* (assay ID: Mm00496696_g1), *S100A9* (assay ID: Mm00656925_ml), *G-CSF* (asay ID: Hs99999083_m1), *M-CSF* (assay ID: Hs99999084_m1), *GM-CSF* (assay ID: Hs00929873_m1) *VEGF-A* (assay ID: Hs00900054_m1), *PlGF* (assay ID: Hs01119262_m1), *SDF1α*(assay ID: Hs00171022_m1), *Cxcl1* (assay ID: Hs00236937_m1), *MMP1* (assay ID: Hs00899658_m1), *PKR1* (assay ID: Mm00517546_m1 and Hs03044877_m1), *PKR2* (assay ID: Mm00769571_m1 and Hs03046077_m1), *Hprt1* (assay ID: Mm00446968_m1 and Hs99999909_m1), *GAPDH* (assay ID: Mm99999915_g1 and Hs99999905_m1). *Hygromycin* expression was detected using custom made primers and TaqMan probe (Applied Biosystems, Foster City, CA): forward primer: CCGCAAGGAATCGGTCAAT (SEQ ID NO:15); reverse primer: GATCAGCAATCGCGCATATG (SEQ ID NO:16); TaqMan probe: 6FAM - CACTACATGGCGTGATT - MGBNFQ (SEQ ID NO: 17)

(Molecular-Groove Binding Non-fluorescence Quencher, Applied Biosystems, Foster City, CA):.

Reactions were carried out using Applied Biosystems 7500 Real-time PCR System under the following conditions: a reverse transcription step (15 min at 48°C) followed by denaturation step at 95°C and 40 cycles of 15 s at 95°C and 1 min at 60°C. Levels of gene expression in each sample were determined with the relative quantification method using *GAPDH* or *Hprt1* mRNA as an endogenous control.

**Microarray sample preparation and analysis.** Balb/c mice were injected with PBS (naive), non-metastatic 67NR cells (200,000 cells in PBS per mouse), or metastatic 4T1 cells (200,000 cells in PBS per mouse). Five mice were used per each condition. Six days after tumor cell inoculation (or when tumors reached about $50mm^3$), mice were euthanized, and lungs were perfused with PBS to completely remove blood from the lung vasculature. Cleaned lung lobes were immersed in RNAlater (Qiagen, Germany) to stabilize RNA. Total RNA was isolated as described in RNA Preparation section and used for subsequent microarray analysis. Affymetrix microarrays (Mouse 430-v2) were used to analyze the expression profile of tissue samples. One sample from 4T1 group was excluded from the final analysis due to very poor quality of the data. Raw data were processed using a program that implemented the recommendations of Choe, et al. Genome Biol 6, R16 (2005).

**mG-CSF cloning and expression.** To generate a murine G-CSF transient expression construct, a PCR fragment containing the consensus Kozak sequence and the coding region of mG-CSF with a 6xHis tag at its C-terminus was amplified from IMAGE mouse cDNA clone # 40129682 (OPEN BIOSYSTEMS) and inserted into the ClaI and EcoRI sites of pRK vector. The resulting construct was then used to transiently transfect CHO cells. The supernatants were harvested and recombinant mG-CSF protein was purified using Ni-NTA column followed by size exclusion column.

***In vivo* G-CSF and anti-G-CSF studies.** To analyze the effect of G-CSF on the mobilization and homing of myeloid cells at distant organs, eight-week-old female Balb/c or Balb/c Nude mice were injected subcutaneously with 10μg of recombinant human G-CSF (Neupogen, Amgen, Thousand Oaks, CA) or recombinant mouse G-CSF daily for five consecutive days. The G-CSF stock was diluted to the desired concentration with sterile PBS (Invitrogen, Carlsbad, CA). Control animals were given PBS as vehicle. At the end of study (24 hours after last G-CSF injection), mice were perfused with PBS and tissues (lung, bone marrow, whole-blood, spleen, liver, and kidney) were taken for analysis. Cd11b+Gr1+ or Ly6G+Ly6C+ cells were analyzed as described in Flow Cytometry section. Levels of Bv8 in the tissues were measured by ELISA as described.

To determine the role of Bv8 in G-CSF-induced mobilization of Cd11b+Gr1+ cells, Balb/c Nude mice received daily doses (for five days) of anti-Bv8 2D3 antibody (2.5mg/kg), followed by mouse G-CSF (Genentech, South San Francisco, CA) 6 h after the treatment. Anti-gp120 antibody (Genentech, South San Francisco, CA) was used as an isotype control. In some experiments, mice were treated with rat anti-Gr1 antibody (50μg per mouse, clone RB6-8C5, eBioscience, San Diego, CA) or anti-Ly6G antibody (50μg per mouse, clone 1A8, BD Biosciences, San Jose, CA). As a positive control, we used a rat anti-mouse G-CSF monoclonal antibody (50μg per mouse, MAB414, R&D Systems, Minneapolis, MN) given at the same interval as anti-Bv8 antibody, and followed by mouse G-CSF.

To determine the role of G-CSF in metastasis, Balb/c, Balb/c Nude, C57BL/6 or SCID/bg mice were pre-treated with human or mouse G-CSF (10μg per mouse) as described above. This was followed by tail-vein injections of 10,000 cells (66c14, 4T1, 67NR, B16F10 or MDA-MB-231-L1.1) in 100μl of PBS. G-CSF administration continued for another 5 days after injection of cancer cells. Mice were analyzed for the presence of lung tumors 3 weeks (4 weeks for 67NR and 5 weeks for MDA-MB-231-L1.1 tumors) after injection of cancer cells. Lungs were perfused with PBS, fixed in formalin and visible tumors were counted. Authenticity of tumors in lungs was confirmed by H&E staining.

To determine the role of Bv8, Gr1+ or Ly6G+ cells in G-CSF-induced metastasis, mice were pre-treated with vehicle or mouse G-CSF (2.5μg per mouse) and further treated with anti-Bv8, anti-Gr1, anti-Ly6G or anti-G-CSF antibodies for 5 days as described above. Anti-VEGF antibody (G6.31, Genentech, South San Francisco, CA) was given at 5mg/kg, every other day. Antibody treatments were discontinued after inoculation of tumor cells.

**Migration assay.** Cells were trypsynized, washed with FBS-free IMDM and resuspended at the concentration of 100,000 cells/ml. 300μl of the cell suspension was loaded to the upper well of FluoroBlok 24-Multiwell Insert System plate (8.0 μm, BD Biosciences) that was coated with collagen type I (BD Biosciences, San Jose, CA) for 1 hour prior to setting up the assay. The lower well was filled with 1 ml of IMDM with the indicated concentration of human Bv8 (Peprotech, Rocky Hill, NJ). IMDM with 1% FCS served as positive control. Plates were incubated for 16 hours. Cells that migrated through the membrane were fluorescently labeled with Calcein AM (BD Biosciences, San Jose, CA). Images of labeled cells were acquired (Zeiss microscope system) and cells were counted using ImageJ software (NIH).

**Collection of condition media from tumor cells.** 67NR, 168FARN, 4TO7, and 66C14, and 4T1 isolated MMTV-PyMT and MDA-MB-231-X1.1 cells were grown as described in Cell lines section. After reaching 90% confluence, media were changed to serum-free IMDM. The conditioned media were collected after a two-day incubation, and cell viability and total cell number were measured using Vi-Cell XR (Beckman Coulter, Fullerton, CA). Collected media were analyzed for the presence of cytokines as described in ELISA section. All data were normalized to the total cell number.

***In vivo* extravasation assay.** 66c14 or 4T1 cells were grown until they reached 90% confluence and were labelled with 10μM CellTracker Green CMFDA (Invitrogen, Carlsbad, CA) for 30 minutes according to the manufacturer protocol. Following the labelling, cells were trypsinized, washed with PBS, counted and 100,000 labelled cells in 100μl of PBS were injected intravenously through tail-vein of Balb/c Nude mice that were pre-treated with mouse G-CSF (2.5μg per mouse, daily for 5 days) in the presence of anti-Bv8 (2B9+3F1), anti-Gr1, anti-G-CSF, or isotype control (ISO) antibody as described in the G-CSF section. For a negative control, unlabeled 66c14 or 4T1 cells were injected into mice pre-treated with G-CSF and isotype control antibody (ISO). Treatment with G-CSF and antibody continued for another day after cell inoculation. Mice were euthanized 36 hours after cell inoculation, lungs were perfused with PBS, harvested and fixed in paraformaldehyde. Fixed lungs were embedded into O.C.T. compound (Tissue-Tek, Torrance, CA), frozen and 5μm sections were generated. Sections were counterstained with DAPI and coverslipped with ProLong Gold mounting media (Invitrogen, Carlsbad, CA). Images were acquired and fluorescently labelled cells were manually counted. On average, three mice per group were used and 15 images (5 random sections per mouse) were included into the analysis. The final values were presented as an average number of cells per field.

**Histological analysis (H&E).** Formalin-fixed tissue was dehydrated and embedded in paraffin. 5-μm-thick

sections were dewaxed, rehydrated and stain with hematoxylin and eosin following standard protocol.

**Bv8 immunohistochemistry (IHC).** Bv8 IHC was performed as described previously (Shojaei, F. et al. Proc Natl Acad Sci U S A (2009)). Briefly, pre-metastatic lungs from PBS-perfused mice bearing 67NR or 4T1 tumors were harvested and fixed in neutral-buffered formalin. 5-μm-thick, paraffin embedded sections were dewaxed and rehydrated. After antigen retrieval using universal decloaker buffer (Biocare Medical, Concord, CA) in a pressure cooker (Biocare Medical, Concord, CA), the sections were blocked with peroxidase blocking reagent (DAKO, Glostrup, Denmark) for 5 min, followed blocked with protein block solution (DAKO, Glostrup, Denmark) for 30 min. Sections were then incubated with hamster anti-mBv8 mAb 2D3 (Genentech, South San Francisco, CA), or with hamster IgG1 (BD Pharmingen, San Jose, CA) at 10 μg/mL for 1 h at room temperature. Next, sections were stained with biotinylated goat anti-hamster antibody (Jackson Immuno Research, West Grove, PA) for 30 min at room temperature, followed by incubation with Vectastain ABC Elite reagents (Vector Laboratories, Burlingame, CA). Sections were then incubated with peroxidase substrate solution (metal enhanced DAB; Pierce Chemical, Rockford, IL), until the desired intensity was developed. Last, sections were light counterstained with hematoxylin, dehydrated, and coverslipped.

ELISA. Plasma was collected using EDTA-treated tubes. Mice were perfused with PBS and small pieces of lungs, spleens, tumors, kidneys, and bone marrow tissues were collected and immediately frozen in liquid nitrogen. Tissues were subsequently lysed in RIPA buffer and total protein content was measured by the Bradford method, according to the manufacturer protocol (Pierce). Levels of Bv8 protein in plasma and tissue lysates were measured by ELISA as described (Shojaei, F. et al. Nature 450, 825-831 (2007); Shojaei, F. et al. Proc Natl Acad Sci U S A (2009)). Levels of G-CSF, GM-CSF, M-CSF, SDF1α, P1GF, VEGF and MMP9 were measured by species-specific ELISA kits (R&D System, Minneapolis, MN). All values were normalized to the total protein content.

**Flow Cytometry (FACS).** Mice were perfused with PBS. Lungs, spleens, tumors, kidneys and bone marrow tissues were collected, minced into small pieces and filtered to generate single cell suspensions. For several experiments, tumor and lung tissue pieces were enzymatically digested with a blend of collagenase and dispase (Roche), followed by mechanical dissociation using a 16-gauge needle tituration. Red blood cells were lysed using ACK lysis buffer (Lonza, Basel, Switzerland). Cells were incubated with rat anti-mouse CD16/CD32 mAb (BD Biosciences, San Jose, CA) to prevent non-specific antibody binding, prior to staining with the following fluorophore-conjugated antibodies: anti-cd45 (30-F11), anti-cd11b (M1/70), anti-F4/80 (BM8), anti-cd11c (N418), anti-cd117 (2B8) (all from eBiosciences, San Diego, CA), anti-Ly6G (1A8), anti-Ly6C (AL-21), anti-Gr1 (RB6-8C5), anti-SiglecF (E50-2440) (all from BD Biosciences, San Jose, CA), and anti-Flt-1 (141522) (R&D Systems, Minneapolis, MN). Propidium iodide (Sigma-Aldrich, St. Louis, MO) was used to distinguish viable and dead cells. Data were acquired using the FACSCalibur or BD LSR II instruments (BD Biosciences, San Jose, CA) and analyzed using FlowJo software (Tree Star, Ashland, OR) or BD FACSDiva program.

**Sorting of Cd11b+Gr1+ cells.** Lungs were prepared and stained with Cd11b-APC and Gr1-FITC antibodies as described in the "Flow Cytometry" section. Desired cells were sorted on FACS instrument, their purity was confirmed and RNA was immediately isolated as described in the RNA isolation section. On average, the purity of the sorted populations was 99%.

**p-ERK Immunoblotting.** Cells were seeded at a density of 10,000 per 100μl of 10%FCS/IMDM. Twenty-four hours later, media were changed to serum-free IMDM, and cells were starved for 24 hours. Cells were stimulated with 5ng/ml of human Bv8 (Peprotech, Rocky Hill, NJ) for 5, 10, 15, and 20 minutes and immediately lysed and boiled in Tris-Glycine SDS Sample Buffer (Invitrogen, Carlsbad, CA). Equal volumes of the lysates were subjected to SDS-PAGE electrophoresis, and immunoblotted for phosphorylated or total p44/p42 MAP kinase (ERK1 and ERK2) with specific antibodies (Cell Signaling Technology, Danvers, MA).

**Micro-CT Analysis.** Lung preparation, imaging and analysis were previously described in detail (Caunt, M. et al. Cancer Cell 13, 331-342 (2008)). Briefly, mice were anesthetized with 5% isouflurane anesthesia. The heart and lungs were exposed and the left and right atria are removed. A blunt 24 gauge needle was inserted into the right ventricle and 10 ml of PBS was perfused to remove blood from the lung vasculature. Lungs were dissected and inflated with 10% neutral buffered formalin (NBF). Lungs were immersed in 10% NBF for 24 hours, then immersed in a 20% solution of an iodine-based x-ray computed tomography contrast agent, Isovue370 (Bracco Diagnostics Inc, Princeton, NJ), diluted with PBS for 24 hours. Lungs were then perfused via the trachea with 20 ml of soy bean oil (Sigma-Aldrich, St. Louis, MO) to remove excess contrast agent. Lungs were imaged in soybean oil to provide a background media for imaging.

The mouse lungs were imaged ex-vivo with a μCT 40 (SCANCO Medical, Basserdorf, Switzerland) x-ray micro-computed tomography (micro-CT) system. The micro-CT images were generated by operating the x-ray tube at an energy level of 45 kV, a current of 177 μA and an integration time of 450 milliseconds. Axial images were obtained at an isotropic resolution of 20 μm. The lung tumor estimates (number and volume) were obtained by a semi-automated image analysis algorithm that includes an inspection step by a trained reader. Potential tumor masses were extracted by a series of image processing steps (Caunt, M. et al. Cancer Cell 13, 331-342 (2008)). The image analysis software was coded in C++ and employed the Analyze (AnalyzeDirect Inc., Lenexa, KS, USA) image analysis software libraries. The extracted objects were then evaluated by a trained reader with the Analyze 3D visualization software. Individual objects were accepted or rejected as possible tumors based on the appearance of the object and its location within the lung. The tumor count, individual tumor volume and total tumor volume were determined for each lung.

**Bioluminescence imaging (BLI).** Mice were injected intraperitoneally with 200 μL of 200 mg/ml D-luciferin (Invitrogen, Carlsbad, CA) and were anesthetized during imaging using isoflurane (Henry Schein, Sparks, NV) via nose cone and body temperature maintained using a warming pad. Bioluminescence images were acquired using a cooled intensified charge-coupled device camera fixed to a light-tight imaging chamber (Stanford Photonics, Palo Alto, CA). Image acquisition times were typically less than 5 minutes. Images were processed by co-registering a reference image with the bioluminescence data image.

**Analysis of public microarray data sets.** The Blaveri bladder cancer data set (Blaveri, E. et al. Clin Cancer Res 11, 7012-7022 (2005)) was downloaded from http://waldman.ucsf.edu/bladder/blaveri.cgh/. The Chin breast cancer data set (Chin, K. et al. Cancer Cell 10, 529-541 (2006)) was downloaded from caArray (http://caarray.nci.nih.gov/) and the Pawitan breast cancer data (Pawitan, Y. et al. Breast Cancer Res 7, R953-964 (2005)) was downloaded from Gene Expression Omnibus (http://www.ncbi.nlm.nih.gov/geo/GSE1456). Two methods were employed in the survival analysis: 1) The Cox proportional hazards model to assess the significance of the association of *G-CSF* gene expression, as a continuous variable, with the survival outcome, 2) Within each dataset, an optimal threshold of *G-CSF* gene expression was chosen by testing a series of quantile cutoffs of *G-CSF* gene expression values using the log rank test. The Kaplan-Meier survival curves were then plotted for the two subgroups based on the identified optimal cutoffs and then the Cox model was used to estimate the Hazard ratios between the two groups.

**Statistical Analysis.** Unless otherwise stated, Student's t-test was used for all of the statistical analysis, and p-value $\leq$ 0.05 was considered to be significant. Graphs present Means $\pm$ SEM. All experiments were repeated at least twice and representative data are presented. For analysis of tumor study in fetal liver transplant experiments, a generalized linear model with Poisson errors and distrbution was fit to the data.

Results and Discussion

Metastasis is a major cause of death from solid tumors. In order to metastasize, tumor cells need to degrade and invade the extracellular matrix, intravasate, be carried through blood or lymphatic vessels, extravasate at the secondary site, and finally establish secondary tumors (Nguyen, D. X. & Massague, J. Nat Rev Genet 8, 341-352 (2007)). In addition, mounting evidence suggests that tumors are able to modify the distant microenvironment prior to arrival of metastatic tumor cells to create the so-called "pre-metastatic niche" (Psaila, B. & Lyden, D. Nat Rev Cancer 9, 285-293 (2009)). This ability of tumors to affect distant tissues enables cancer cells to target specific organs in which they can initiate secondary tumor growth and supports the "seed and soil" hypothesis (Paget, S. Cancer Metastasis Rev. 8(2),98-101 (1989)). Bone marrow derived cells (BMDC) are thought to be a major cell type populating the niche (Kaplan, R. N. et al. Nature 438, 820-827 (2005); Hiratsuka, S. et al. Nat Cell Biol 8, 1369-1375 (2006)).

Although several molecules have been implicated (Kaplan, R. N. et al. Nature 438, 820-827 (2005); Hiratsuka, S. et al. Nat Cell Biol 8, 1369-1375 (2006); Yamamoto, M. et al. Cancer Res 68, 9754-9762 (2008); Kim, S. et al. Nature 457, 102-106 (2009); Erler, J. T. et al. Cancer Cell 15, 35-44 (2009), the mechanisms by which tumors initiate the niche and the precise role of the niche in metastasis are incompletely understood. To characterize the changes triggered by primary tumors at distant sites, we performed cDNA microarray analysis of total lung tissues from mice without any tumors (naive) versus mice bearing non-metastatic (67NR) or metastatic (4T1) breast carcinomas in the pre-metastatic phase (Fig. 1A). As a model, we used the 4T1-related lines of mouse breast carcinoma (Dexter, D. L. et al. Cancer Res 38, 3174-3181 (1978); Aslakson, C. J. & Miller, F. R. Cancer Res 52, 1399-1405 (1992)) which provide a phenotypic spectrum ranging from non-metastatic cells (67NR, 168FARN) to cells able to complete all steps of metastasis (4TO7, 66c14 and 4T1) (Fig. 5A). Recently, analysis of these cell lines enabled the identification of novel genes regulating metastasis (Yang, J. et al. Cell 117, 927-939 (2004)). To ensure that the tissues we examined were truly pre-metastatic, 4T1 cells expressing *Luciferase-zsGreen* and *hygromycin* (4T1-Luc-zsG-Hyg cells) were inoculated orthotopically into the $4^{th}$ mammary fat pad and lungs were harvested 6 days later. At this point we could not detect any 4T1-Luc-zsG-Hyg cancer cells in the lungs as assessed by qRT-PCR detecting *hygromycin* (Fig. 5B and Fig. 10). In addition, bioluminescence imaging did not detect any luciferase-positive 4T1 cells in the lungs at this stage (Fig 5C). Thus, any signal we detected in our gene expression analysis was not due to cancer cells.

In these studies, the period of tumor development without any detectable cancer cells in the lungs will be called the pre-metastatic phase, whereas a stage in which we could detect even single cancer cells or tumors in the lungs will be referred to as the metastatic phase.

Our analysis identified 260 genes specifically up-regulated and 274 genes downregulated more then 2-fold in lungs from mice bearing 4T1 tumors relative to lungs from Naive mice or bearing non-metastatic 67NR tumors (Fig. 1A). We focused on Bv8, as it was one of the top up-regulated genes (Fig. 1B). Bv8 is a secreted protein that has been previously characterized as a pro-angiogenic factor (LeCouter, J. et al. Proc Natl Acad Sci U S A 100, 2685-2690 (2003)), as a growth factor for hematopoietic progenitors (LeCouter, J. et al. Proc Natl Acad Sci U S A 101, 16813-16818 (2004)), and also as a neuromodulator (Cheng, M. Y. et al. Nature 417, 405-410 (2002); Matsumoto, S. et al. Proc Natl Acad Sci U S A 103, 4140-4145 (2006)). Also, Bv8 has been recently shown to be expressed by Cd11b+Gr1+ myeloid cells and to mediate growth of anti-VEGF resistant tumors (Shojaei, F. et al. Nature 450, 825-831 (2007)). qRT-PCR analysis of *Bv8* expression in lung tissues confirmed the microarray results (Fig. 5D). Moreover, for the first time, we found a strong correlation between high *Bv8* expression in the pre-metastatic lungs and metastatic potential of the tumors examined (Fig. 1C and Fig. 5E). Bv8 has been shown to be highly expressed by Cd11b+Gr1+ myeloid cells (Shojaei, F. et al. Nature 450, 825-831 (2007)) and thus we hypothesized that metastatic tumors are able to modify the lung microenvironment through mobilization of Cd11b+Gr1+ cells from the bone marrow and subsequent homing in the lungs. To test this hypothesis, we measured the frequency of Cd11b+Gr1+ cells infiltrating lung tissues during the pre-metastatic phase (Fig. 1D). We observed increased numbers of these cells in mice bearing the metastatic tumors 4TO7, 66c14 and 4T1, while lungs from mice bearing non-metastatic tumors 67NR and 168FARN did not show any increase in Cd11b+Gr1+ cells (Fig. 1D). Additionally, Cd11b+Gr1+ cells isolated from lungs of mice bearing 4T1 tumors strongly expressed Bv8 (Fig. 5F). However, we could detect only marginal Bv8 transcripts levels in Cd11b+Gr1- cells and essentially no transcript in Cd11b+Gr1+ cells isolated from mice without tumor, suggesting that primary tumor secretes factors up-regulating Bv8 in Cd11b+Gr1+ cells (Fig. 5F). Furthermore, we stained lung tissue with an anti-Bv8 antibody and we could detect Bv8-positive cells only in lungs isolated from mice bearing tumors that can metastasize (Fig. 5G). We also detected increased frequency of Cd11b+Gr1+ cells in pre-metastatic lungs of mice bearing additional metastatic tumors, such as B16F10, LLC and a genetic model of breast carcinoma, the MMTV-PyMT (Fig. 5H). Similarly, we detected increased expression of Bv8 in lungs from these mice (Fig. 5I). Thus, Cd11b+Gr1+ cells expressing Bv8 appear to be a major component of the pre-metastatic microenvironment. To further define the composition of such microenvironment, we performed FACS analysis to characterize Cd11b+Gr1+ cells and identify additional cell types in the pre-metastatic lungs (Fig. 11A). Such analysis revealed a specific accumulation of Ly6C+Ly6G+ granulocytes, and a modest accumulation of Ly6C+Ly6G- monocytes in the pre-metastatic lungs of 4T1-tumor bearing mice (Fig. 11A). We did not observed any significant changes in the frequency of macrophages (F4/80+ cells), eosinophils (SiglecF+ cells) or dendritic cells in response to the primary tumor *(see e.g.,* Fig. 11A). Also, we did not detect any mast cells in the lungs of naive or tumor-bearing mice. Moreover, we detected the presence of the previously described VEGFR1+CD117+ haematopoietic progenitor cells (Kaplan, RN et al., Nature 438, 820 (2005)), although their frequency was much lower than Ly6G+Ly6C+ cells (2.7% for HPC vs 27% for Ly6G+Ly6C+ cells) (Fig. 11A). We concluded that metastatic tumors induce a specific accumulation of granulocytes (defined as Ly6C+Ly6G+ cells, among Cd45+Cd11b+ population) and a modest accumulation of monocytes (Ly6C+Ly6G- cells) (Fig. 11A).

To determine which cytokine(s) secreted by tumor cells might be responsible for initiating the niche, we measured plasma levels of several candidates, namely vascular endothelial growth factor (VEGF)-A, placenta growth factor (PlGF), stromal derived factor (SDF)1$\alpha$, macrophage colony stimulating factor (M-CSF), granulocyte-macrophage (GM)-CSF, granulocyte (G)-CSF and Bv8 in naive or in tumor-bearing mice (Fig. 2A). Surprisingly, only G-CSF and Bv8 plasma levels correlated with the ability of tumor to metastasize. A similar correlation was found during the metastatic phase. We found G-CSF to be highly expressed in plasma of mice bearing MMTV-PyMT tumors as well. Interestingly, in mice bearing MDA-MB-231 tumors we detected elevated tumor and plasma levels of both human and mouse G-CSF, suggesting that host cells infiltrating the tumor are also a significant source of G-CSF (Fig. 13F). G-CSF is a major regulator of granulopoiesis, produced by a variety of cell types (Metcalf, D. Nature 339, 27-30 (1989)), plays a key role in neutrophil mobilization from the bone marrow (Christopher, M. J. & Link, D. C. Curr Opin Hematol 14, 3-8 (2007)) and has been recently shown to mediate tumor refractoriness to anti-VEGF therapy (Shojaei, F. et al. 106(16), 6742-6747 Proc Natl Acad Sci U S A (2009)). Another attractive feature of G-CSF as a potential regulator of a process distant from the tumor of origin, such as the development of the pre-metastatic niche, is its endocrine mode of action (Metcalf, D. Nature 339, 27-30 (1989)). We found that G-CSF is released by metastatic but not by non-metastatic tumor cells both *in vitro* (Fig. 6A and Fig. 11B) and *in vivo.* Also, treatment of 4T1-bearing mice with anti-G-CSF antibody prevented mobilization and homing of Cd11b+Gr1+ cells into the lungs (Fig. 2B), and also reduced Bv8 levels in lungs and plasma (Fig. 6B). These data suggest that G-CSF is a major cytokine secreted by metastatic tumors to initiate a pre-metastatic microenvironment enriched in Cd11b+Gr1+ cells. Treatment with anti-Bv8 antibody reduced mobilization of Cd11b+Gr1+ cells into the pre-metastatic lungs in mice bearing 4T1 tumors (Fig. 2B), although it did not have any significant effect during the metastatic phase (5.5 weeks after inoculation of tumor cells). Furthermore, treatment of MMTV-PyMT tumor-bearing mice with anti-G-CSF antibody prevented mobilization of Ly6G+Ly6C+ cells into the peripheral blood and homing of these cells into the lungs (Fig. 11C). The lack of response to anti-Bv8 treatment at the late stages of tumor development might be due to the very high amounts of G-CSF released by the primary tumors (Fig. 6C).

To address the significance of G-CSF and Bv8 in metastatic tumor progression, we inoculated 66c14 or 4T1 tumor cells orthotopically into the $4^{th}$ mammary fat pad of Balb/c or CB6F1 mice and we treated them with anti-Bv8, anti-G-CSF or both (combination) antibodies. Treatment was initiated 2 days after inoculation of the tumor cells and the number of metastatic lung nodules was evaluated 6 (for 66c14 model) or 5.5 (for 4T1 model) weeks after tumor cell inoculation. While we could not detect any significant changes in primary tumor growth in these tumor models (Fig. 6D and 6E), we observed a significant reduction in the number of lung tumors in mice treated with either anti-Bv8 or anti-G-CSF antibody. In mice bearing 4T1 tumors, anti-Bv8 antibody decreased number of visible tumors by 50%, anti-G-CSF by 65% and the combination by 75%, when compared to corresponding isotype control antibody groups (Fig. 2C). In mice bearing 66c14 tumors, anti-Bv8 antibody treatment reduced lung tumor numbers by 30% and anti-G-CSF by 43% (Fig. 6F). Similar results were obtained when micro-CT analysis was used to quantify detailed number of both micro- and macro-tumors in lungs from mice bearing 66c14 or 4T1 tumors treated with anti-Bv8 antibody (Fig. 2D). Importantly, metastasis of 4T1 or 66c14 orthotopic tumors was reduced to a level comparable with anti-Bv8 treatment when tumors were inoculated in mice lacking Bv8 in BMMNCs (Fig. 13A, 13B and 13C). Subsequently, we analyzed the efficacy of anti-Bv8 antibody and anti-G-CSF antibody in other breast cancer models, namely human MDA-MB-231 (Fig. 12A) and mouse MMTV-PyMT (Fig. 13D). In both models, anti-Bv8 antibody or anti-G-CSF antibody treatment resulted in reduction of lung metastasis (Fig. 12B and 13E).

To directly assess the role of Cd11b+Gr1+ cells in the development of metastasis, mice bearing orthotopical 66c14 tumors were treated with anti-Gr1 antibody (clone RB6-8C5 depleting both monocytes and neutrophils) or anti-Ly6G antibody (clone 1A8 specifically depleting neutrophils (Daley, JM et al., J Leukoc Biol 83, 64 (2008)) (Fig. 12C). Treatment with each antibody significantly reduced the number of lung metastases to a degree comparable with anti-Bv8 antibody or anti-G-CSF antibody. However, the anti-Gr1 antibody resulted in slightly more pronounced inhibition of metastasis compared to the anti-Ly6G treatment, indicating that, while Ly6G+Ly6C+ neutrophils are largely responsible for the metastasis in the tested models, monocytes also are likely to contribute to this process.

We sought to directly test the hypothesis that G-CSF positively regulates metastasis. First, we asked whether G-CSF administration is sufficient to initiate the pre-metastatic niche. We analyzed the frequency of Cd11b+Gr1+ cells in mice treated daily with recombinant G-CSF (rG-CSF) for 5 consecutive days (Fig. 3A). Delivery of rG-CSF induced mobilization of Cd11b+Gr1+ cells, as we detected marked increases in these cells in BM, peripheral blood and spleen. Remarkably, we detected a significant increase in the frequency of Cd11b+Gr1+ cells in the lungs (Fig. 3A) and this was followed by increased expression of Bv8 (Fig. 3B). In addition, we observed an increased frequency of Cd11b+Gr1+ cells and enhanced expression of Bv8 in liver, another organ that is a target for metastasis of the tested tumors. We did not detect increased Bv8 expression in tissues that are normally not targeted for metastasis, such as kidney (Fig. 3B). To determine whether these effects result in enhanced metastatic potential, mice were treated with rG-CSF for 5 consecutive days before and after injection of tumor cells through the tail vein. This enabled us to focus on the potential role of G-CSF and the G-CSF-initiated pre-metastatic niche at the final stages of metastasis, such as extravasation, survival and tumor growth at the distant organs. We injected mice with the non-metastatic tumor cell line 67NR or with a number of aggressive cell lines, 66c14, 4T1, and B16F10. Mice treated with rG-CSF exhibited significant increases in the number of lung tumors (Fig. 3C and 3D). That was followed by significant increases in lung mass, corresponding to the number of visible tumors (Fig. 7A). Remarkably, the non-metastatic cell line 67NR exhibited metastatic behavior in the lungs once mice had been pre-treated with G-CSF (Fig. 3E). Authenticity of the tumors in mice pre-treated with G-CSF was further confirmed by histological analysis of H&E stained sections (Fig. 7B).

To define the role of Bv8 in the G-CSF-induced pre-metastatic niche, mice were treated with rG-CSF and an anti-Bv8 antibody. Anti-Bv8 antibody treatment significantly reduced the increase in the number of Cd11b+Gr1+ cells in the lungs (Fig. 7C). Moreover, an anti-Gr1 antibody completely abolished the G-CSF effect on Cd11b+Gr1+ cells. Similarly, treatment with anti-G-CSF antibody completely prevented the G-CSF effect (Fig. 7C). Subsequently, to further investigate the role of Bv8 in G-CSF driven metastasis, we injected 66c14 or 4T1 cells through tail-vein into mice that were pre-treated with G-CSF in the presence or absence of anti-Bv8 antibody. Anti-Bv8 antibody treatment significantly reduced G-CSF-induced 66c14 (Fig. 3F), 4T1 (Fig. 7D) and MDA-MB-231 (Fig. 14A) lung metastases. Treatment with an anti-Gr1 antibody or anti-Ly6G antibody also markedly inhibited the G-CSF effect (Fig. 14B, Fig. 3F and Fig. 7D), indicating that neutrophils are predominantly responsible for the G-CSF-induced pre-metastatic priming and metastasis. We also tested whether an anti-VEGF antibody can prevent G-CSF-induced metastasis. In contrast to anti-Bv8 treatment, the anti-VEGF therapy did not prevent the G-CSF effect (Fig. 7E), suggesting that the G-CSF-promoted lung metastasis is VEGF-independent. It is noteworthy that we did not detect any increases in metastatic tumor burden in the presence of anti-VEGF antibody, as it was recently reported when other anti-angiogenic therapies were used (Paez-Ribes, M. et al. Cancer Cell 15, 220-231 (2009); Ebos, J. M. et al. Cancer Cell 15, 232-239 (2009)).

We further characterized the population of myeloid cells homing in the lungs in response to G-CSF with respect to expression of VEGF receptor (VEGFR)-1, as this receptor was previously reported to be expressed by hematopoietic cells within the pre-metastatic niche (Kaplan, R. N. et al. Nature 438, 820-827 (2005)). Following rG-CSF injection, we detected significant increases in frequency of Cd11b+Gr1+VEGFR1+ cells in lung (Fig. 7F) that matched the increase in frequency of Cd11b+Gr1+ cells (Fig. 7G). More detailed analysis revealed that about 44% percent of Cd11b+Gr1+ cells is VEGFR1+ (Fig. 7H). However, this value was not significantly affected by rG-CSF administration (Fig. 7H). Thus, our results show that Cd11b+Gr1+ cells in the G-CSF-induced pre-metastatic niche partially share characteristics of cells previously reported to initiate the niche (Kaplan, R. N. et al. Nature 438, 820-827 (2005)).

The microarray analysis showed that, in addition to Bv8, the pre-metastatic lungs are enriched in a number of factors which have been previously shown to promote metastasis, such as MMP-9 (Hiratsuka, S. et al. Cancer Cell 2, 289-300 (2002); Acuff, H. B. et al. Cancer Res 66, 259-266 (2006)), S100A8 and S100A9 (Hiratsuka, S. et al. Nat Cell Biol 8, 1369-1375 (2006)) (Fig. 1B). While MMP-9 has been shown to promote invasion (Hiratsuka, S. et al. Cancer Cell 2, 289-300 (2002)) and survival (Acuff, H. B. et al. Cancer Res 66, 259-266 (2006)) of tumor cells in the lung environment, S 100A8 and S100A9 have been shown to mediate recruitment of myeloid and tumor cells in the lungs (Hiratsuka, S. et al. Nat Cell Biol 8, 1369-1375 (2006)). To investigate whether the G-CSF/Bv8 axis might execute its pro-metastatic effect, at least in part, through regulation of those molecules, we measured their expression levels in lungs of mice treated with rG-CSF. We found that all three molecules were significantly up-regulated (Fig. 8A). Anti-Bv8 antibody treatment significantly reduced the G-CSF-induced expression of MMP-9, S100A8 and S100A9 (Fig. 8A). This correlated with decreased frequency of Cd11b+Gr1+ cells in lungs of mice treated with anti-Bv8 (Fig. 7C). Again, similar to the experiment with orthotopically inoculated tumors, the anti-Bv8 antibody effect was detected only when mice were treated with low doses of rG-CSF (Fig. 7C), but not when a maximal dose of rG-CSF was injected. Cd11b+Gr1+ cells sorted from lungs exposed to G-CSF expressed significant levels of MMP-9, S100A8 and S100A9, along with Bv8 (Fig. 8B). Similar results were obtained in mice orthotopically inoculated with 4T1 cells and treated with anti-Bv8 or anti-G-CSF antibody (Fig. 2C). MMP-9 expression was significantly reduced by anti-Bv8 antibody treatment in the pre-metastatic lungs (Fig. 8C), while anti-G-CSF antibody treatment reduced MMP-9 expression also in the metastatic lungs (Fig. 8C). Decreased MMP-9 expression correlated with reduced frequency of Cd11b+Gr1+ cells in the lungs (Fig. 2B). Thus, our data position G-CSF upstream of Bv8, MMP-9, S100A8 and S100A9 in the development of the pre-metastatic microenvironment.

Anti-Bv8 antibody treatment efficiently prevented the effects of G-CSF on the development of lung metastasis (Fig. 3F and Fig. 7D). However, the same treatment only partially inhibited G-CSF-induced mobilization and homing of Cd11b+Gr1+ cells into the pre-metastatic lungs (Fig. C). This finding suggests that the mechanism by which Bv8 promotes metastasis can only be partially explained by modulating mobilization of Cd11b+Gr1+ cells to the lung tissue. To determine whether Bv8 regulates metastasis by additional mechanisms, we asked whether Bv8 might have direct effects on tumor cells. We measured the expression levels of Bv8 receptors (PKR-1/GPR73 and PKR-2/GPR73L1) in both non-metastatic and metastatic tumor cells. We could detect significant expression of *PKR-1* in metastatic tumor cell lines (4TO7, 66c14, 4T1 as well as in B16F10 and LLC), whereas the non-metastatic cell lines (67NR and 168FARN) exhibited much lower or undetectable levels of *PKR-1* (Fig. 4A). We were unable to detect *PKR-2* in any of the cell lines tested, except for LLC (Fig. 4A). To determine whether PKR-1 is functional, we stimulated tumor cells with Bv8 and then we measured levels of phosphorylated ERK1/2 (Fig. 8D). We could detect significant ERK1/2 activation in response to Bv8 only in the metastatic cell lines, suggesting that Bv8 signaling in these cells is indeed functional (Fig. 8D). Bv8 has been shown to promote migration of myeloid cells (Chin, K. et al. Cancer Cell 10, 529-541 (2006)). Therefore, we examined the possibility Bv8 might also promote migration of metastatic tumor cells. Indeed, we detected increased migration in response to increasing concentration of Bv8 (Fig. 4B). In contrast, non-metastatic cell lines did not show any enhanced migration in response to Bv8. Hence, in addition to regulating mobilization and homing of Cd11b+Gr1+ cells into the pre-metastatic niche, Bv8 may regulate metastasis through its direct effect on tumor cells. Finally, to confirm that the G-CSF-induced pre-metastatic niche facilitates migration of tumor cells *in vivo,* we injected 4T1 or 66c14 cells fluorescently labeled with CellTracker Green (to enable *easy in* ***vivo*** tracking of the injected cells) into Balb/c nude mice pre-treated with G-CSF in the presence or absence of anti-Bv8, anti-Gr1 or anti-G-CSF antibodies. Thirty-six hours later, we assessed the numbers of tumor cells that were able to extravasate and seed the lung tissue. We detected significant increases in the number of tumor cells in the lungs of mice pre-treated with G-CSF compared to controls (Fig. 4C and 4D, and Fig. 8E). This effect was significantly reduced by administration of anti-Bv8 antibodies. Similar results were obtained when mice were treated with anti-Gr1 antibody or anti-G-CSF antibody (Fig. 4C and 4D, and Fig. 8E).

67NR cells exhibit metastatic properties when injected through the tail vein of mice pre-treated with G-CSF (Fig. 3E), likely due to the substantially increased expression of MMP9 and other pro-metastatic molecules in the G-CSF-primed lungs. We speculated that metastasis of 67NR cells might be enhanced by over-expression of PKR-1. Indeed, over-expression of *PKR-1* in 67NR cells to a level comparable with 66c14 cells (Fig. 16A), resulted in significant enhancement of 67NR metastasis in the G-CSF pre-treated mice (Fig. 15A). Conversely, PKR-1 deficient 66c14 cells (Fig. 16B) had significantly reduced number of G-CSF-induced metastasis when compared to cells expressing control shRNA (Fig. 16C). Thus, Bv8 can act as chemoattractant for tumor cells *in vivo* and promote their extravasation into the pre-metastatic lungs.

In order to determine whether expression of G-CSF or PKR-1 is maintained at higher levels in tumor cells that colonize the lungs. *in vivo* clonal selection for MDA-MB-231 cells that either reside at the primary tumor or are able to generate metastasis in the lungs was performed (Fig. 15C). A similar methodology has been previously used to identify gene sets that mediate breast cancer metastasis to the lung (Minn, AJ et al,, Nature 436, 518 (2005)) and other organs (Bos, PD et al,, Nature 459, 1005 (2009); Lu, X et al,, Genes Dev 23, 1882 (2009)). Gene expression profiling revealed that MDA-MB-231 cells isolated from the lungs express much higher levels of both *G-CSF* and *PKR-1,* along with *GM-CSF, MMP-9* and the previously identified *Cxcl1* and *MMP-1* (Minn, AJ et al,, Nature 436, 518 (2005)) when compared to cells isolated from the primary tumor or to parental cells (Fig. 15B). Importantly no increase in the expression of *PKR-*

*2, M-CSF, SDF1α, VEGF-A* or *PlGF* was detected, suggesting that in order to metastasize cancer cells selectively increase expression of a narrow group of genes, which include *G-CSF* and *PKR-1.*

We examined publicly available databases to identify a potential association between the expression of G-CSF in the primary tumors and the outcome of the disease. We analyzed three sets of microarray data: two from breast cancer (Pawitan Breast Cancer and Chin Breast Cancer datasets (Pawitan, Y. et al. Breast Cancer Res 7, R953-964 (2005); Chin, K. et al. Cancer Cell 10, 529-541 (2006)) and one from bladder carcinoma patients (Blaveri Bladder Cancer dataset (Blaveri, E. et al. Clin Cancer Res 11, 7012-7022 (2005)). We identified a strong negative correlation between survival of breast cancer patients and high expression of G-CSF (Fig. 4E and Fig. 9A). We identified a similar correlation in bladder carcinoma patients (Fig. 9B). Detailed statistical analysis is shown in Fig. 9C.

Cd11b+Gr1+ and other myeloid cell types have been shown to facilitate tumor growth in mouse models in a number of studies (Yang, L. et al. Cancer Cell 6, 409-421 (2004); Shojaei, F. et al. Nat Biotechnol 25, 911-920 (2007); De Palma, M. et al. Trends Immunol 28, 519-524 (2007); Murdoch, C. et al. Nat Rev Cancer 8, 618-631 (2008)). Importantly, their human counterparts have been found to be over-produced in cancer patients as well (Almand, B. et al. J Immunol 166, 678-689 (2001); Young, M. R. & Lathers, D. M. Int J Immunopharmacol 21, 241-252 (1999)). Recently, Cd11b+Gr1+ cells have been shown to promote invasion and metastasis at the primary tumor site through increased production of MMPs and TGF-β1 (Yang, L. et al. Cancer Cell 13, 23-35 (2008)).

The data herein suggest that the role of Cd11b+Gr1+ cells in metastasis is not limited to the primary tumor and we show for the first time that these cells are enriched in pre-metastatic lungs in order to create a microenvironment that supports the final steps of metastasis, such as extravasation, survival and growth of secondary tumors (Fig. 4F). Tumor-secreted G-CSF mobilizes Cd11b+Gr1+ cells from the BM and also induces Bv8 expression. Bv8 in turn acts as a chemoattractant for BM-derived Cd11b+Gr1+ cells and facilitates their homing into the lung before arrival of tumor cells. Once in the lungs, Cd11b+Gr1+ cells serve as a source of Bv8, MMP-9 and S100A8 and S100A9. These molecules can further facilitate metastasis by promoting invasion or further mobilization of BMDC to the lungs (Hiratsuka, S. et al. Cancer Cell 2, 289-300 (2002); Acuff, H. B. et al. Cancer Res 66, 259-266 (2006); Yang, L. et al. Cancer Cell 6, 409-421 (2004)). We also detected increased expression of Bv8 in livers of mice pre-treated with G-CSF, suggesting that the pro-metastatic effect of G-CSF and Bv8 might not be restricted to lung but present in other organs as well.

Throughout the present application, including the claims, the term "comprising" is used as an inclusive, open-ended transition phrase, which does not exclude additional, unrecited elements or method steps.

## Claims

1. An antagonistic anti-G-CSF antibody or an antigen-binding fragment thereof for use in a method of inhibiting or reducing tumor metastasis.

2. The antagonistic anti-G-CSF antibody or the antigen-binding fragment thereof for use of claim 1, wherein reducing tumor metastasis comprises reducing size or number of lung metastases.

3. The antagonistic anti-G-CSF antibody or the antigen-binding fragment thereof for use of claim 1 administered in combination with an antagonistic anti-Bv8 antibody or an antagonistic anti-PKR1 antibody or an antigen-binding fragment thereof.

4. The antagonistic anti-G-CSF antibody or the antigen-binding fragment thereof for use of claim 1 administered in combination with an antagonistic anti-VEGF antibody or an antigen-binding fragment thereof.

5. The antagonistic anti-G-CSF antibody or the antigen-binding fragment thereof for use of claim 4, wherein the antagonistic anti-VEGF antibody is bevacizumab or an antigen-binding fragment thereof.

6. The antagonistic anti-G-CSF antibody or the antigen-binding fragment thereof for use of claim 1, wherein the antagonistic anti-G-CSF antibody or the antigen-binding fragment thereof inhibits or reduces the spread of a primary tumor to a pre-metastatic organ of the subject, and wherein the pre-metastatic organ is lung or liver.

7. The antagonistic anti-G-CSF antibody or the antigen-binding fragment thereof for use of claim 1 administered in combination with a chemotherapeutic agent.

## Patentansprüche

1. Antagonistischer anti-G-CSF-Antikörper oder ein Antigen-bindendes Fragment davon zur Verwendung in einem Verfahren zur Hemmung oder Verringerung von Tumor-Metastasenbildung.

2. Antagonistischer anti-G-CSF-Antikörper oder das Antigen-bindende Fragment davon zur Verwendung nach Anspruch 1, wobei die Verringerung von Tumor-Metastasenbildung die Verringerung der Größe oder der Anzahl von Lungen-Metastasen umfasst.

3. Antagonistischer anti-G-CSF-Antikörper oder das Antigen-bindende Fragment davon zur Verwendung nach Anspruch 1, verabreicht in Kombination mit einem antagonistischen anti-Bv8-Antikörper oder einem antagonistischen anti-PKR1-Antikörper oder einem Antigen-bindenden Fragment davon.

4. Antagonistischer anti-G-CSF-Antikörper oder das Antigen-bindende Fragment davon zur Verwendung nach Anspruch 1, verabreicht in Kombination mit einem antagonistischen anti-VEGF-Antikörper oder einem Antigen-bindenden Fragment davon.

5. Antagonistischer anti-G-CSF-Antikörper oder das Antigen-bindende Fragment davon zur Verwendung nach Anspruch 4, wobei der antagonistische anti-VEGF-Antikörper Bevacizumab oder ein Antigen-bindendes Fragment davon ist.

6. Antagonistischer anti-G-CSF-Antikörper oder das Antigen-bindende Fragment davon zur Verwendung nach Anspruch 1, wobei der antagonistische anti-G-CSF-Antikörper oder das Antigen-bindende Fragment davon die Ausbreitung eines primären Tumors auf ein vor-metastatisches Organ des Individuums hemmt oder verringert und wobei das vor-metastatische Organ Lunge oder Leber ist.

7. Antagonistischer anti-G-CSF-Antikörper oder das Antigen-bindende Fragment davon zur Verwendung nach Anspruch 1, verabreicht in Kombination mit einem chemotherapeutischen Mittel.

**Revendications**

1. Anticorps anti-G-CSF antagoniste ou un de ses fragments de liaison à l'antigène, pour utilisation dans un procédé d'inhibition ou de réduction d'une métastase tumorale.

2. Anticorps anti-G-CSF antagoniste ou un de ses fragments de liaison à l'antigène pour une utilisation selon la revendication 1, dans lequel la réduction de la métastase tumorale comprend la réduction de la taille ou du nombre de métastases pulmonaires.

3. Anticorps anti-G-CSF antagoniste ou un de ses fragments de liaison à l'antigène pour une utilisation selon la revendication 1, administré en combinaison avec un anticorps anti-Bv8 antagoniste ou un anticorps anti-PKR1 antagoniste ou un de leurs fragments de liaison à l'antigène.

4. Anticorps anti-G-CSF antagoniste ou un de ses fragments de liaison à l'antigène pour une utilisation selon la revendication 1, administré en combinaison avec un anticorps anti-VGEF antagoniste ou un de ses fragments de liaison à l'antigène.

5. Anticorps anti-G-CSF antagoniste ou un de ses fragments de liaison à l'antigène pour une utilisation selon la revendication 4, dans lequel l'anticorps anti-VGEF antagoniste est le bévacizumab ou un de ses fragments de liaison à l'antigène.

6. Anticorps anti-G-CSF antagoniste ou un de ses fragments de liaison à l'antigène pour une utilisation selon la revendication 1, dans lequel l'anticorps anti-G-CSF antagoniste ou un de ses fragments de liaison à l'antigène inhibe ou réduit la propagation d'une tumeur primaire à un organe pré-métastatique du sujet, et dans lequel l'organe pré-métastatique est le poumon ou le foie.

7. Anticorps anti-G-CSF antagoniste ou un de ses fragment de liaison à l'antigène pour une utilisation selon la revendication 1, administré en combinaison avec un agent de chimiothérapie.

FIG. 1A

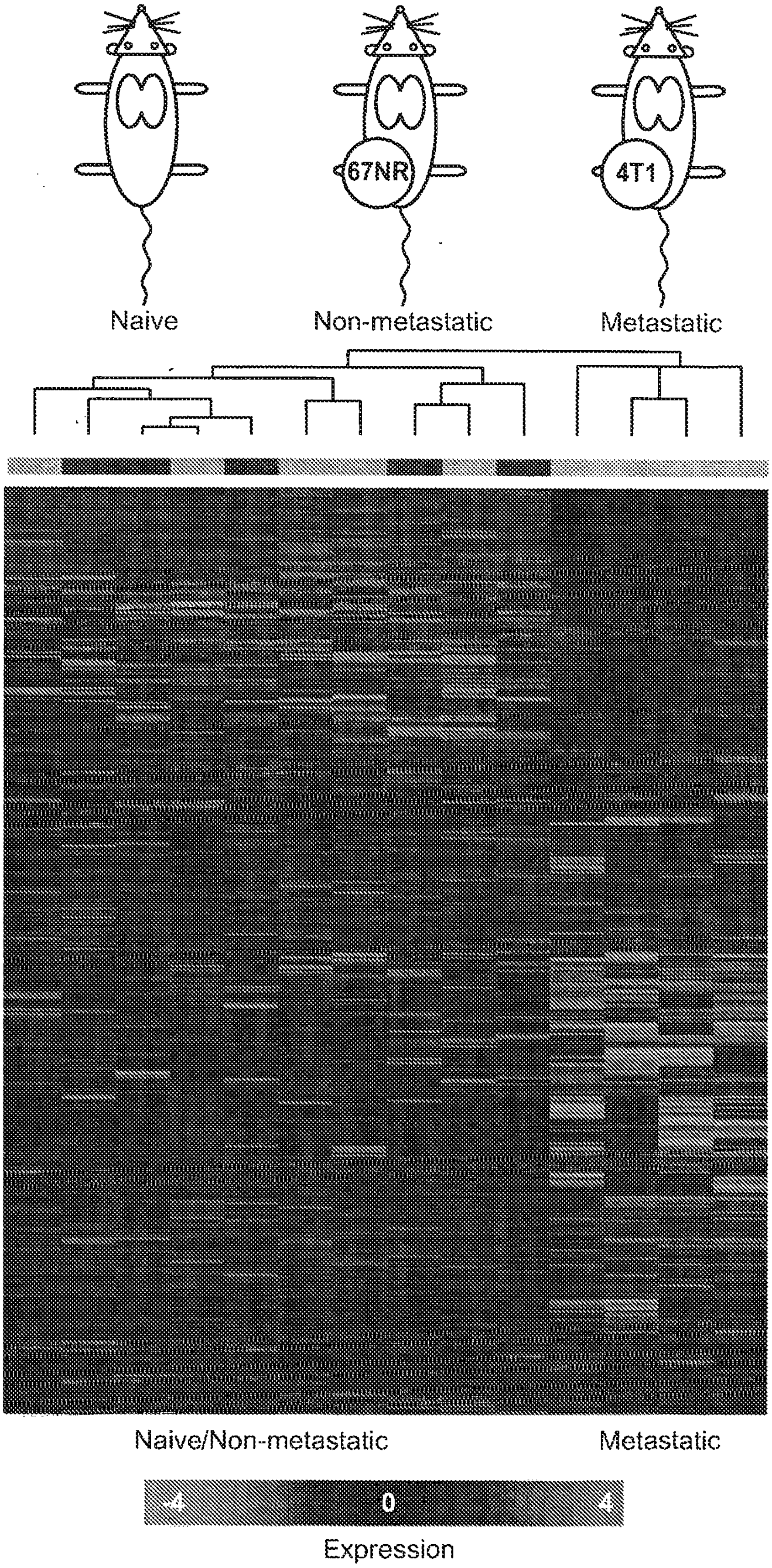
67NR
4T1
Naive
Non-metastatic
Metastatic
Naive/Non-metastatic
Metastatic
-4
0
4
Expression

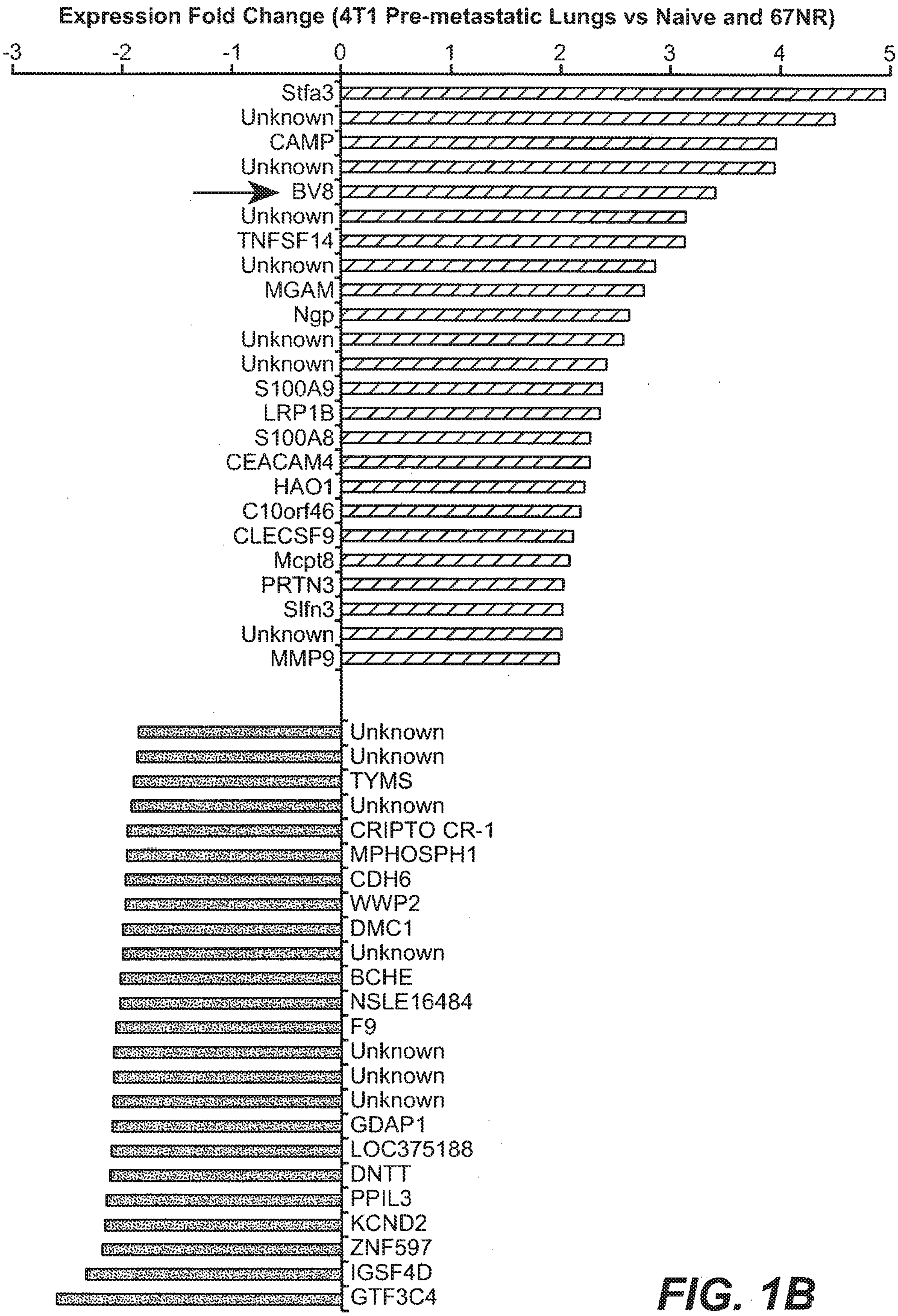

Expression Fold Change (4T1 Pre-metastatic Lungs vs Naive and 67NR)
-3
-2
-1
0
1
2
3
4
5
Stfa3
Unknown
CAMP
Unknown
BV8
Unknown
TNFSF14
Unknown
MGAM
Ngp
Unknown
Unknown
S100A9
LRP1B
S100A8
CEACAM4
HAO1
C10orf46
CLECSF9
Mcpt8
PRTN3
Slfn3
Unknown
MMP9
Unknown
Unknown
TYMS
Unknown
CRIPTO CR-1
MPHOSPH1
CDH6
WWP2
DMC1
Unknown
BCHE
NSLE16484
F9
Unknown
Unknown
Unknown
GDAP1
LOC375188
DNTT
PPIL3
KCND2
ZNF597
IGSF4D
GTF3C4


*FIG. 1B*

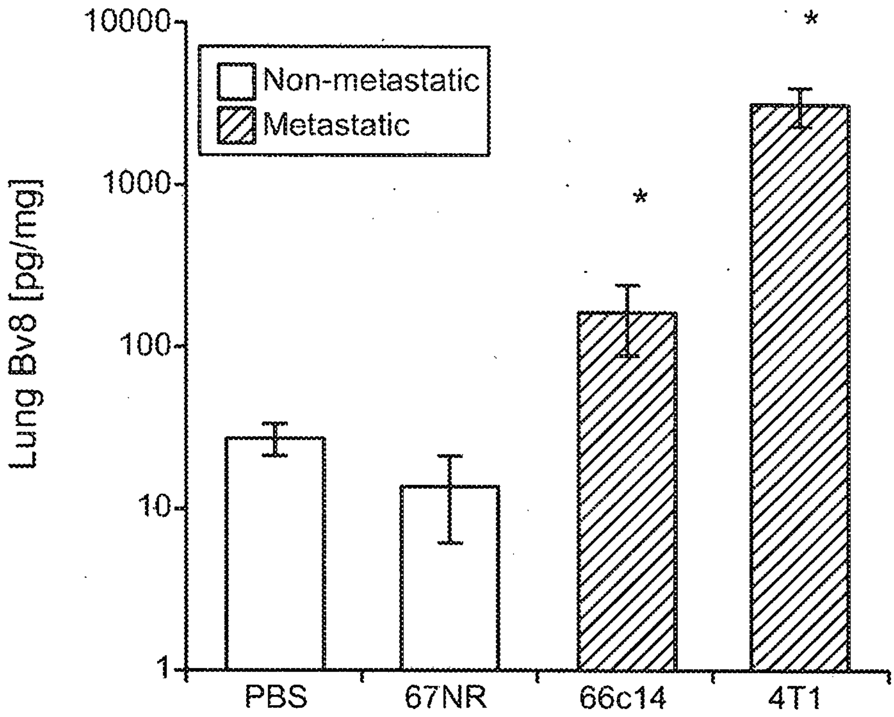

Lung Bv8 [pg/mg]
10000
1000
100
10
1
Non-metastatic
Metastatic
*
*
PBS
67NR
66c14
4T1


FIG. 1C

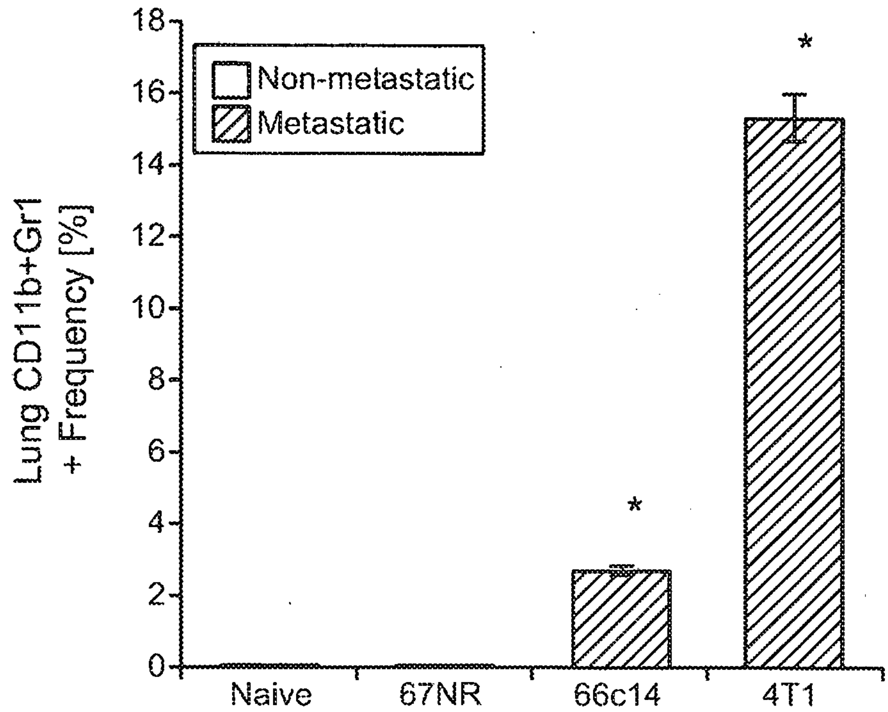

Lung CD11b+Gr1 + Frequency [%]
18
16
14
12
10
8
6
4
2
0
Non-metastatic
Metastatic
*
*
Naive
67NR
66c14
4T1


FIG. 1D

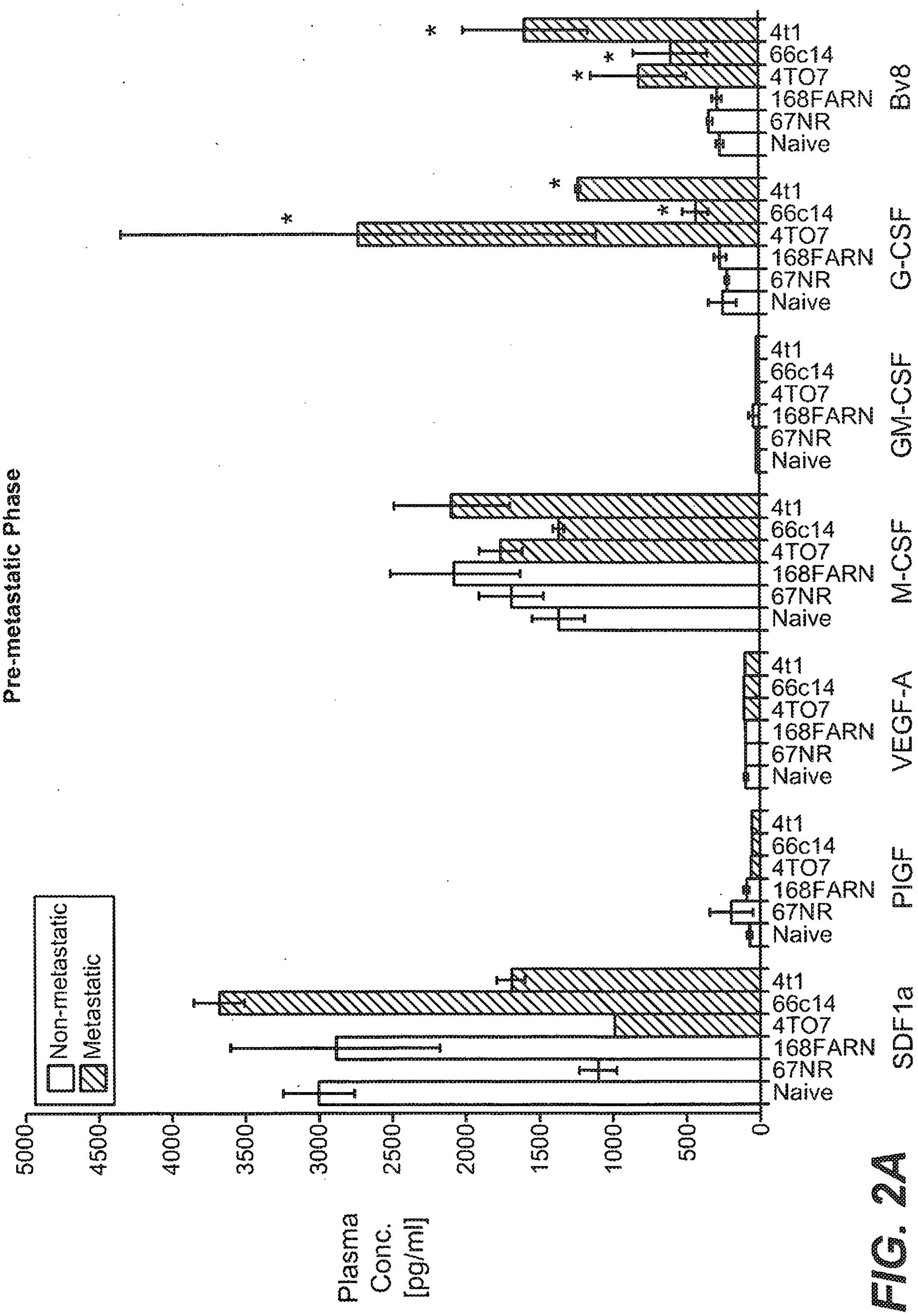
Pre-metastatic Phase
Non-metastatic
Metastatic
Plasma Conc. [pg/ml]
5000
4500
4000
3500
3000
2500
2000
1500
1000
500
0
Naive
67NR
168FARN
4TO7
66c14
4t1
SDF1a
PlGF
VEGF-A
M-CSF
GM-CSF
G-CSF
Bv8
*

FIG. 2A

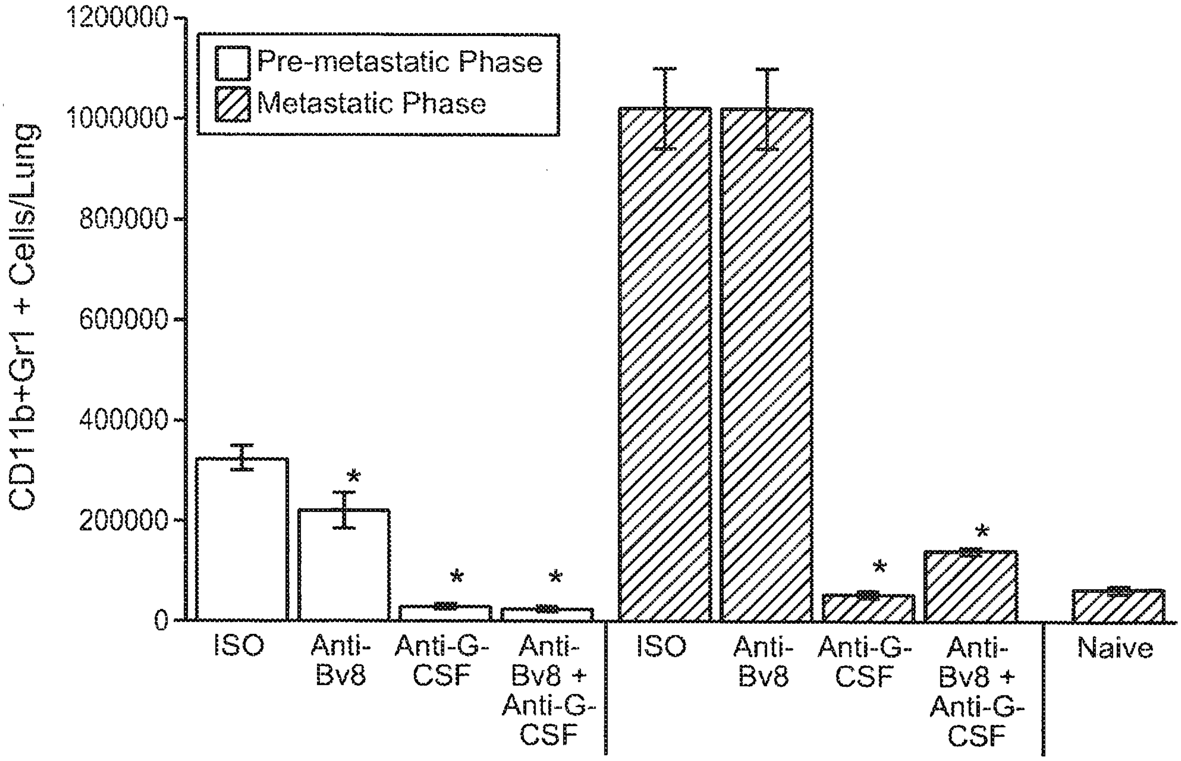
Pre-metastatic Phase
Metastatic Phase
CD11b+Gr1 + Cells/Lung
1200000
1000000
800000
600000
400000
200000
0
ISO
Anti-Bv8
Anti-G-CSF
Anti-Bv8 + Anti-G-CSF
ISO
Anti-Bv8
Anti-G-CSF
Anti-Bv8 + Anti-G-CSF
Naive
*

*FIG. 2B*

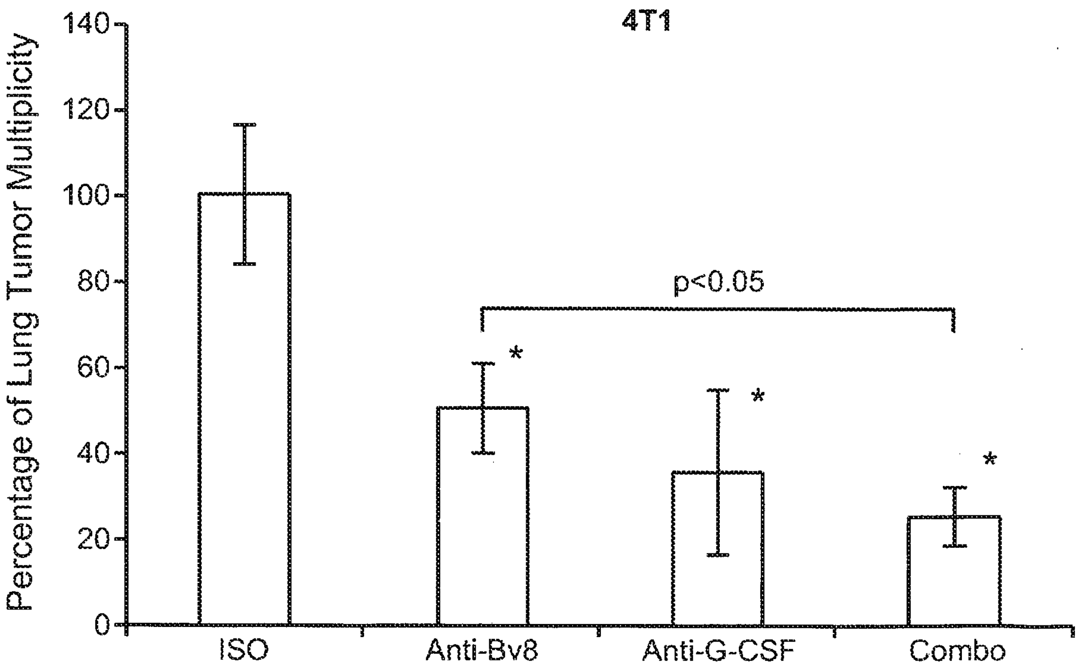
4T1
Percentage of Lung Tumor Multiplicity
140
120
100
80
60
40
20
0
p<0.05
*
ISO
Anti-Bv8
Anti-G-CSF
Combo

*FIG. 2C*

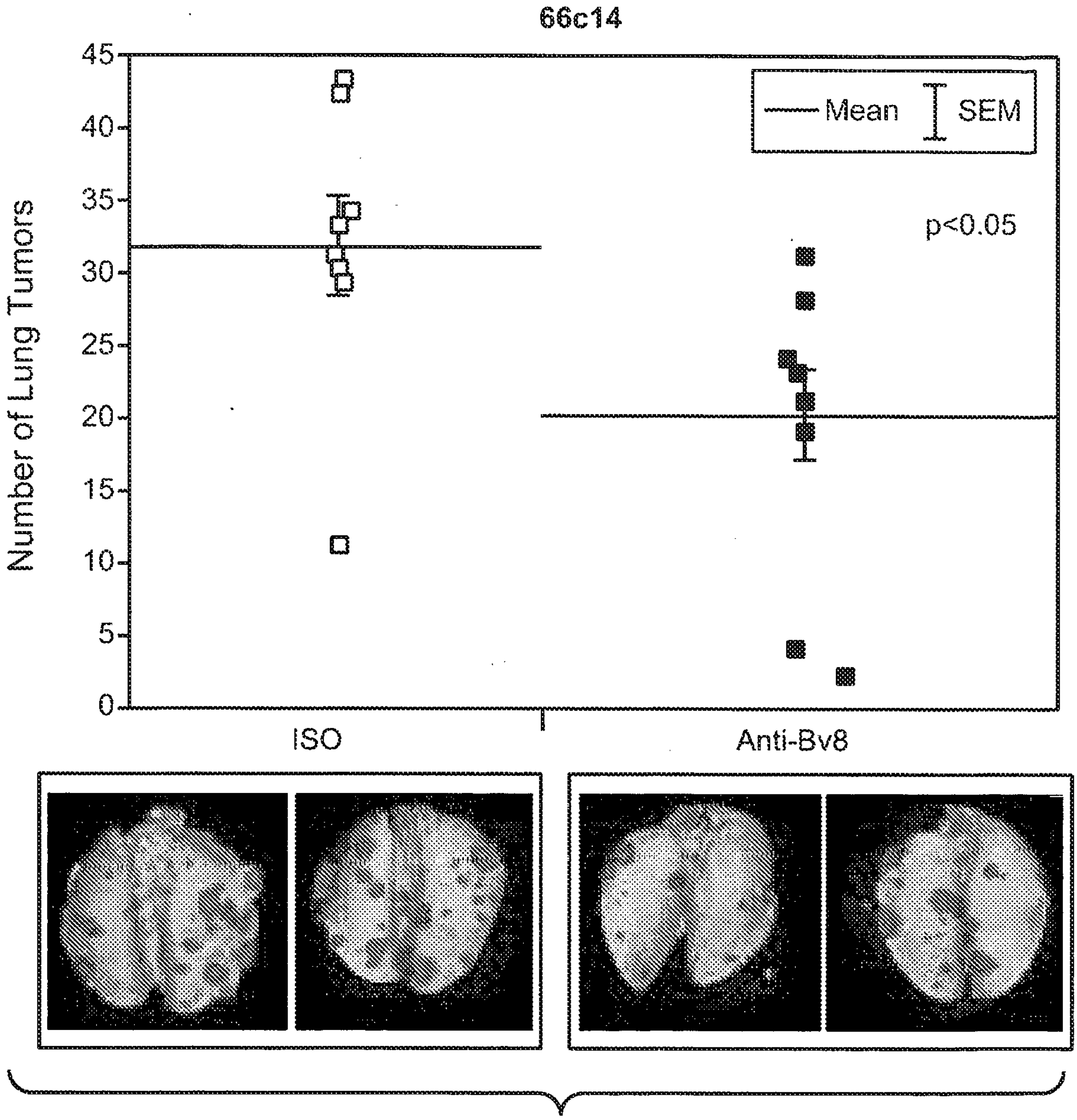
66c14
Number of Lung Tumors
45
40
35
30
25
20
15
10
5
0
Mean
SEM
p<0.05
ISO
Anti-Bv8

FIG. 2D

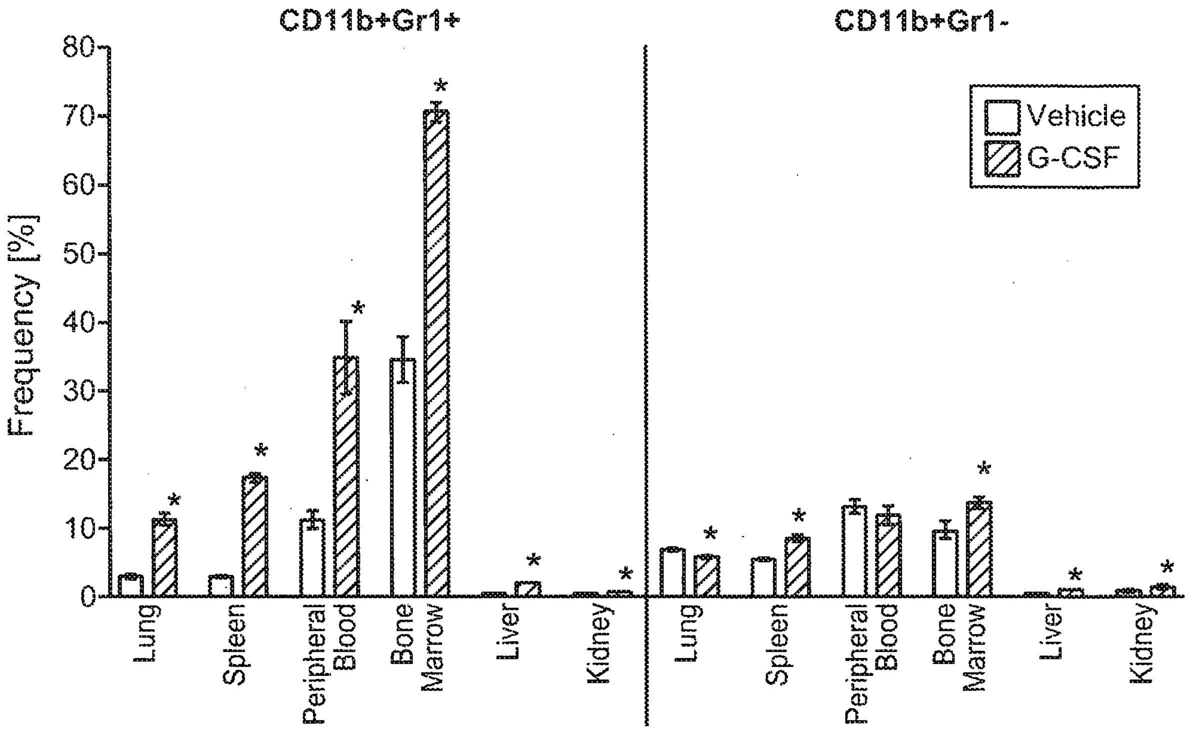
CD11b+Gr1+
CD11b+Gr1-
Vehicle
G-CSF
Frequency [%]
80
70
60
50
40
30
20
10
0
Lung
Spleen
Peripheral Blood
Bone Marrow
Liver
Kidney
Lung
Spleen
Peripheral Blood
Bone Marrow
Liver
Kidney

*FIG. 3A*

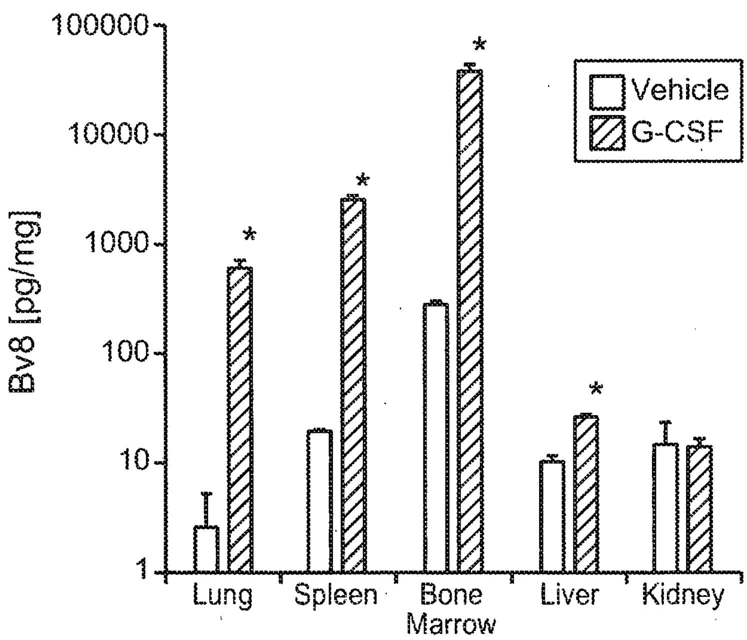
Vehicle
G-CSF
Bv8 [pg/mg]
100000
10000
1000
100
10
1
Lung
Spleen
Bone Marrow
Liver
Kidney

*FIG. 3B*

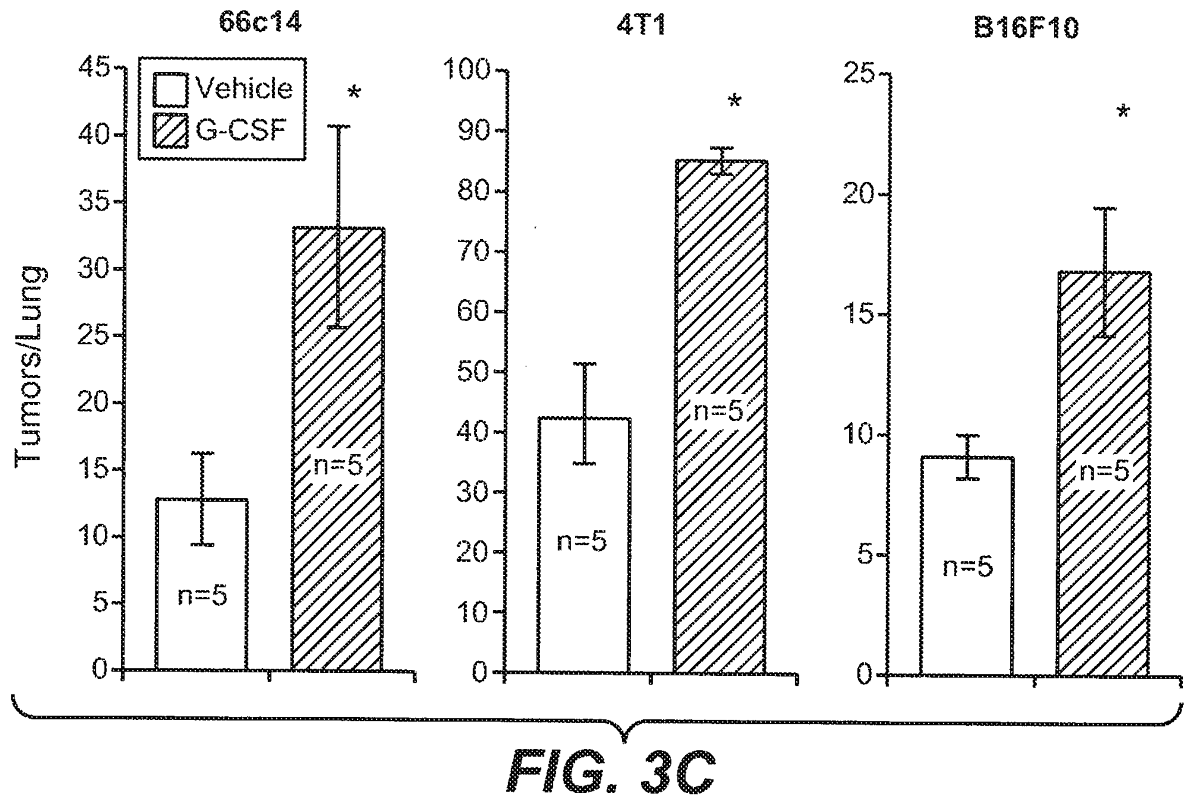
66c14
4T1
B16F10
Tumors/Lung
Vehicle
G-CSF
n=5
*
45
40
35
30
25
20
15
10
5
0
100
90
80
70
60
50
40
30
20
10
0
25
20
15
10
5
0

FIG. 3C

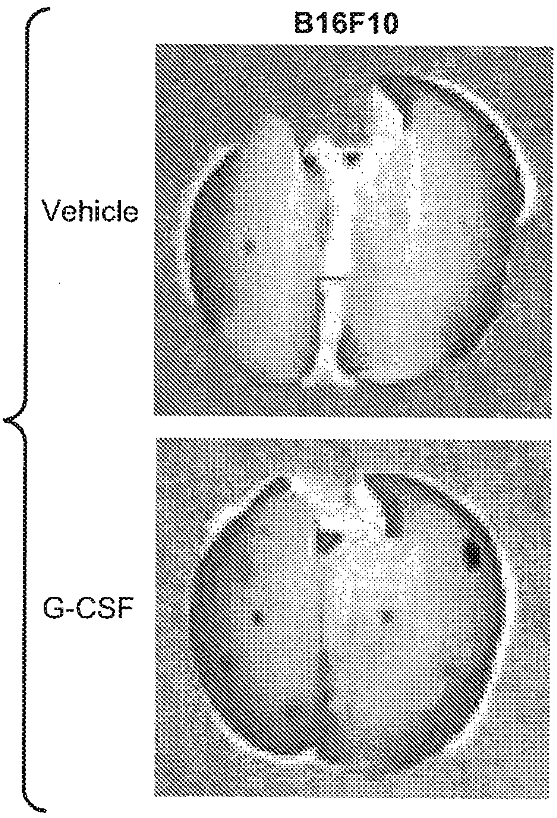
B16F10
Vehicle
G-CSF

FIG. 3D

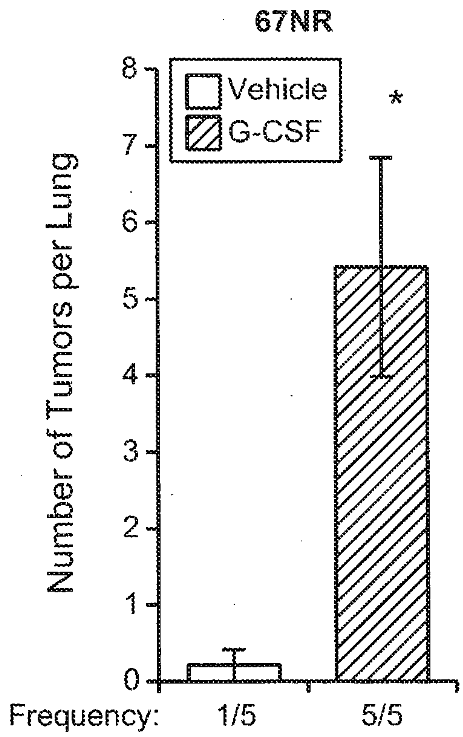
67NR
Vehicle
G-CSF
*
Number of Tumors per Lung
0
1
2
3
4
5
6
7
8
Frequency:
1/5
5/5

FIG. 3E

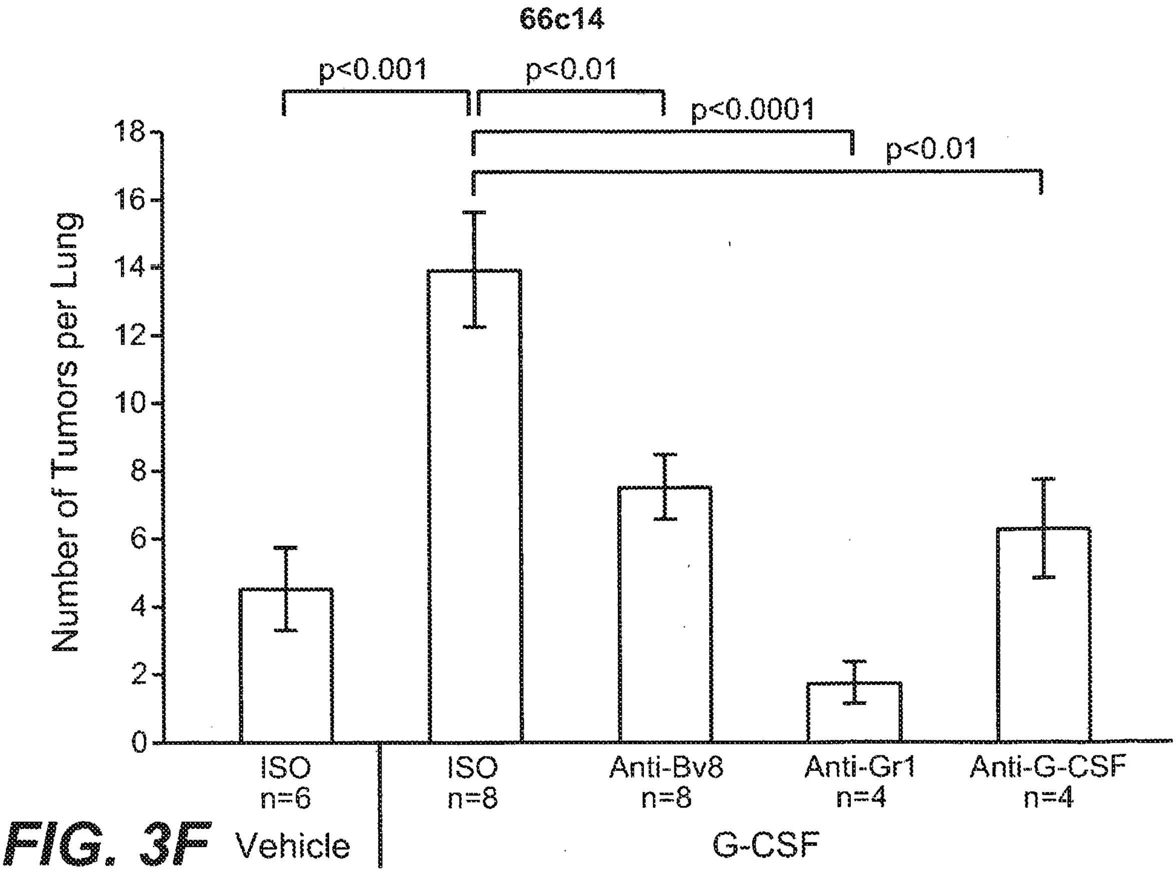
66c14
p<0.001
p<0.01
p<0.0001
p<0.01
Number of Tumors per Lung
0
2
4
6
8
10
12
14
16
18
ISO
n=6
ISO
n=8
Anti-Bv8
n=8
Anti-Gr1
n=4
Anti-G-CSF
n=4
Vehicle
G-CSF

FIG. 3F

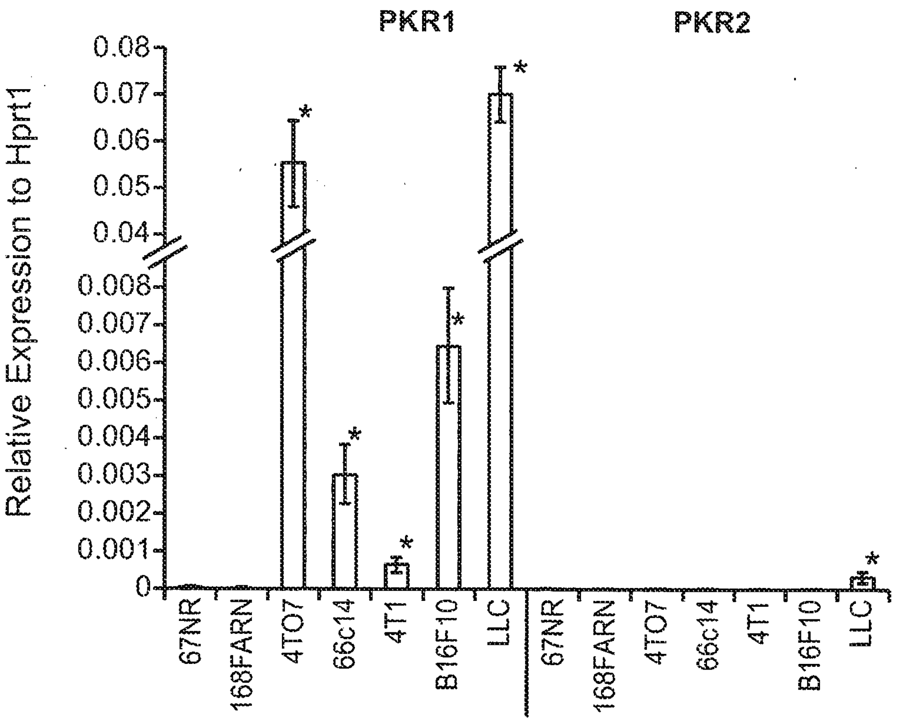
PKR1
PKR2
Relative Expression to Hprt1
0.08
0.07
0.06
0.05
0.04
0.008
0.007
0.006
0.005
0.004
0.003
0.002
0.001
0
67NR
168FARN
4TO7
66c14
4T1
B16F10
LLC
67NR
168FARN
4TO7
66c14
4T1
B16F10
LLC

*FIG. 4A*

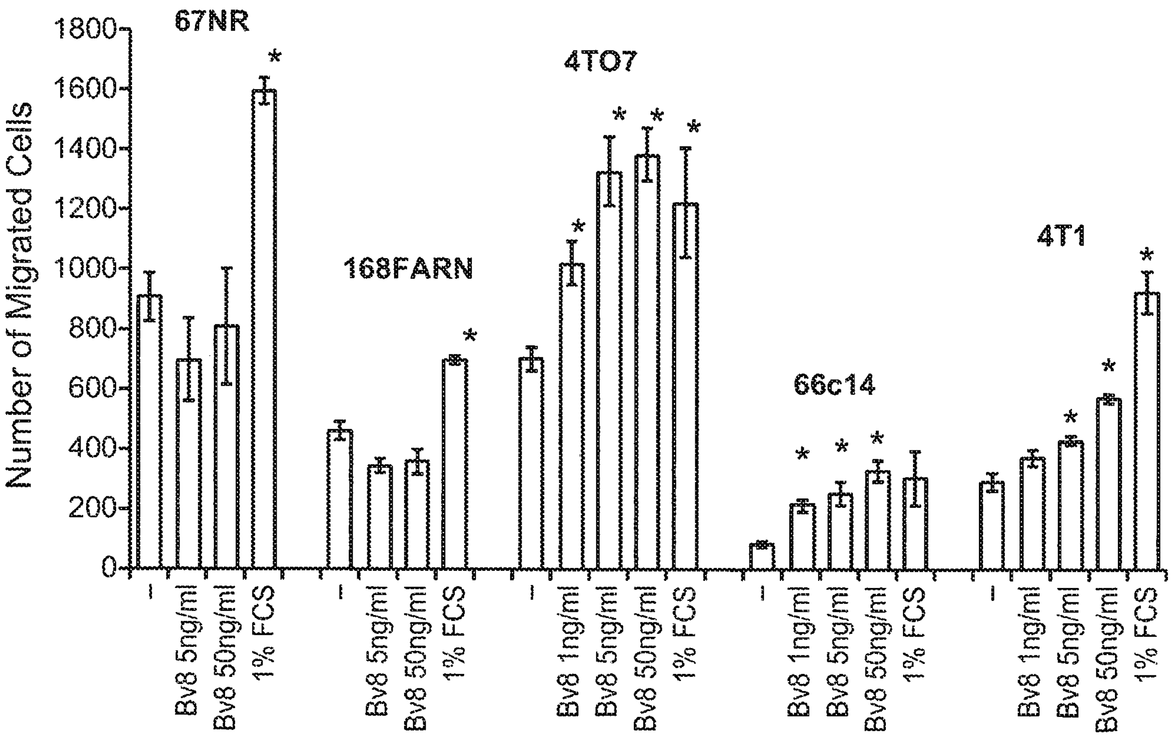
67NR
168FARN
4TO7
66c14
4T1
Number of Migrated Cells
1800
1600
1400
1200
1000
800
600
400
200
0
-
Bv8 5ng/ml
Bv8 50ng/ml
1% FCS
Bv8 1ng/ml

*FIG. 4B*

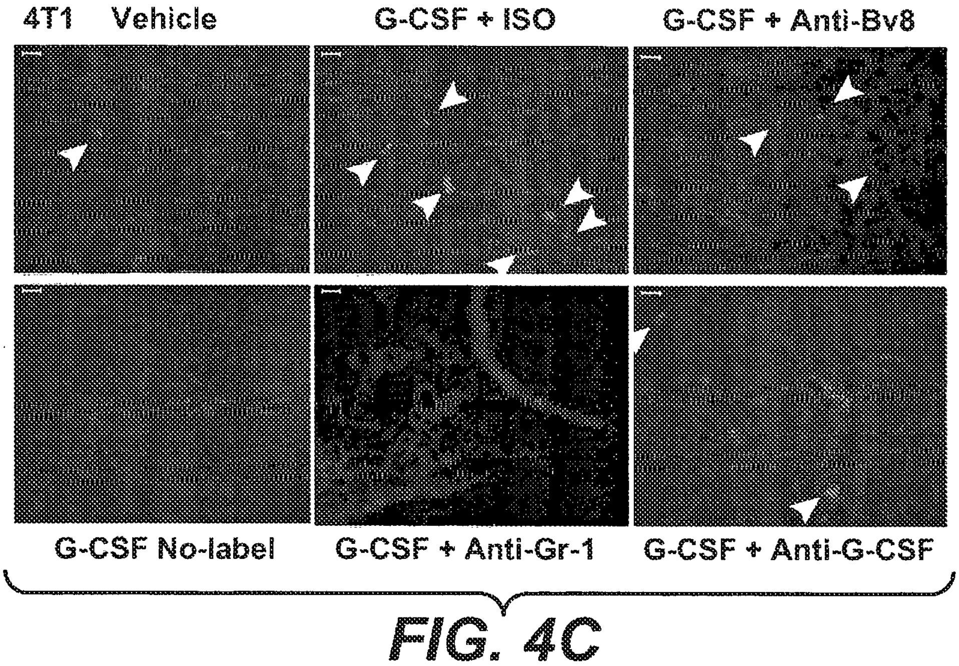
4T1 Vehicle
G-CSF + ISO
G-CSF + Anti-Bv8
G-CSF No-label
G-CSF + Anti-Gr-1
G-CSF + Anti-G-CSF

FIG. 4C

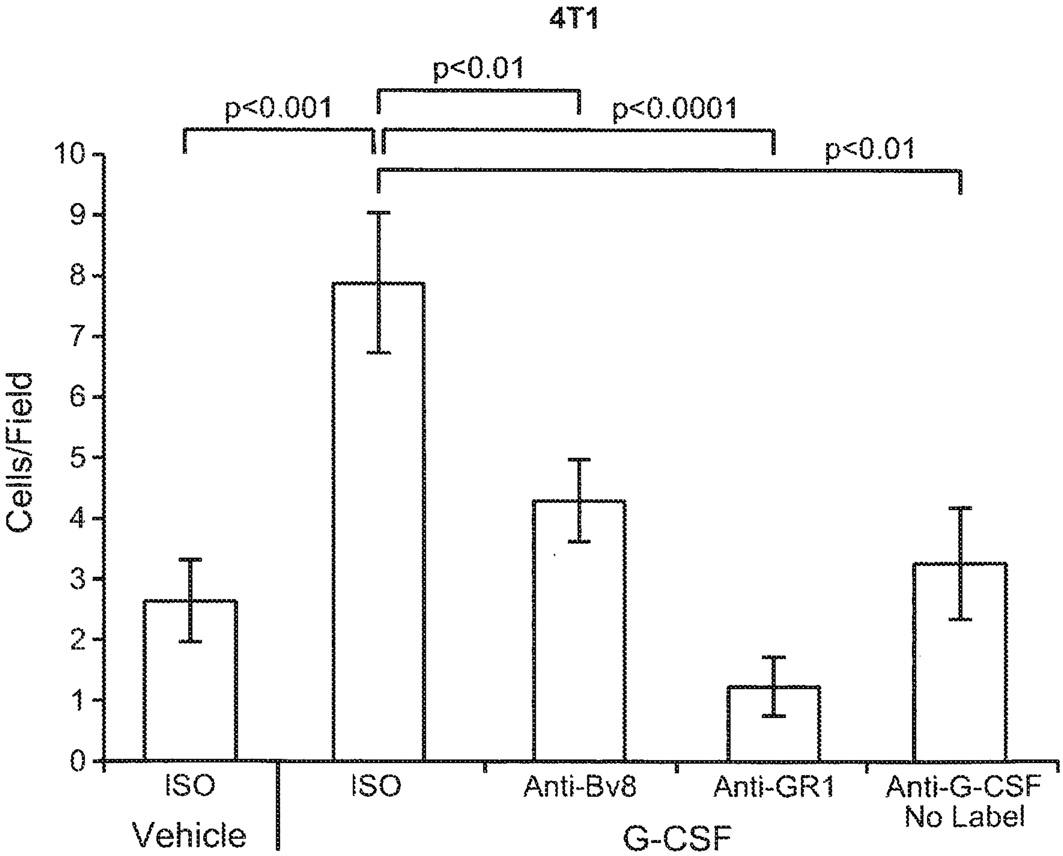
4T1
p<0.01
p<0.001
p<0.0001
p<0.01
Cells/Field
0
1
2
3
4
5
6
7
8
9
10
ISO
Vehicle
ISO
Anti-Bv8
Anti-GR1
Anti-G-CSF
No Label
G-CSF

FIG. 4D

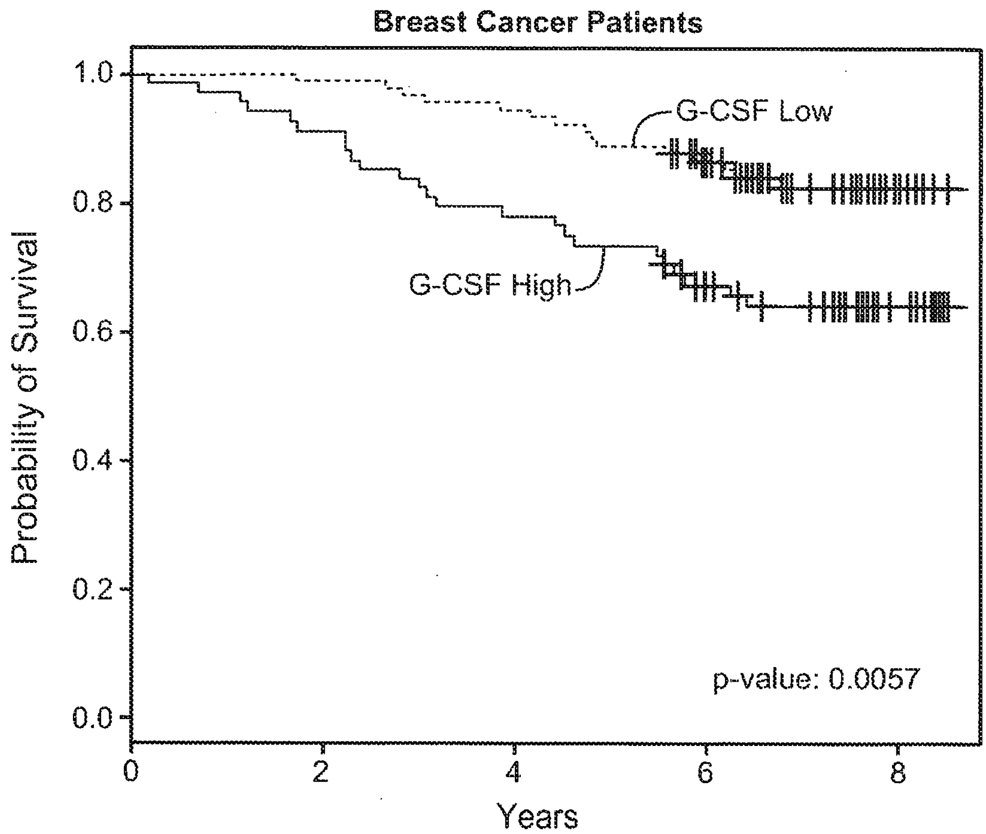
Breast Cancer Patients
1.0
0.8
0.6
0.4
0.2
0.0
Probability of Survival
G-CSF Low
G-CSF High
p-value: 0.0057
0
2
4
6
8
Years

*FIG. 4E*

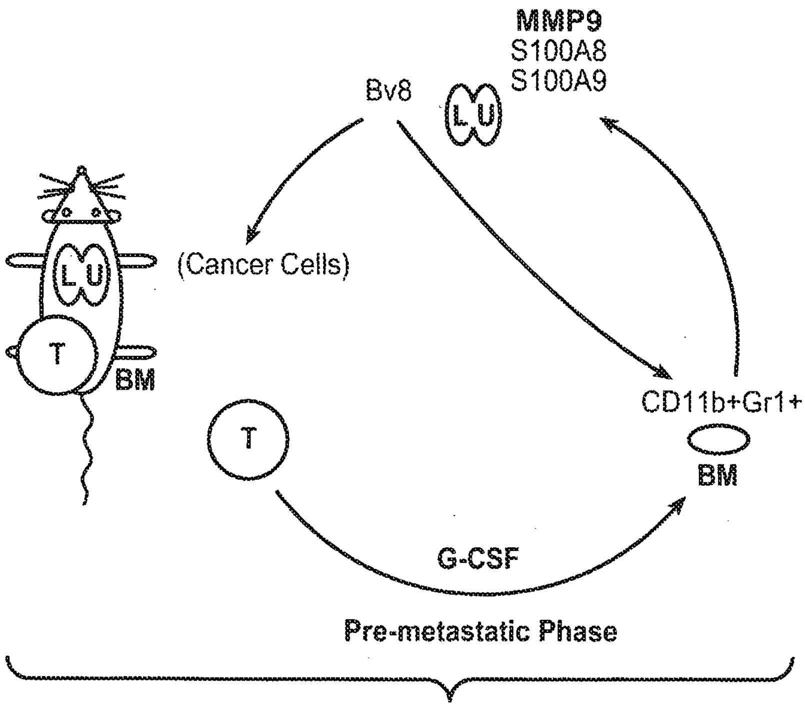
MMP9
S100A8
S100A9
Bv8
L U
(Cancer Cells)
T
BM
T
CD11b+Gr1+
BM
G-CSF
Pre-metastatic Phase

*FIG. 4F*

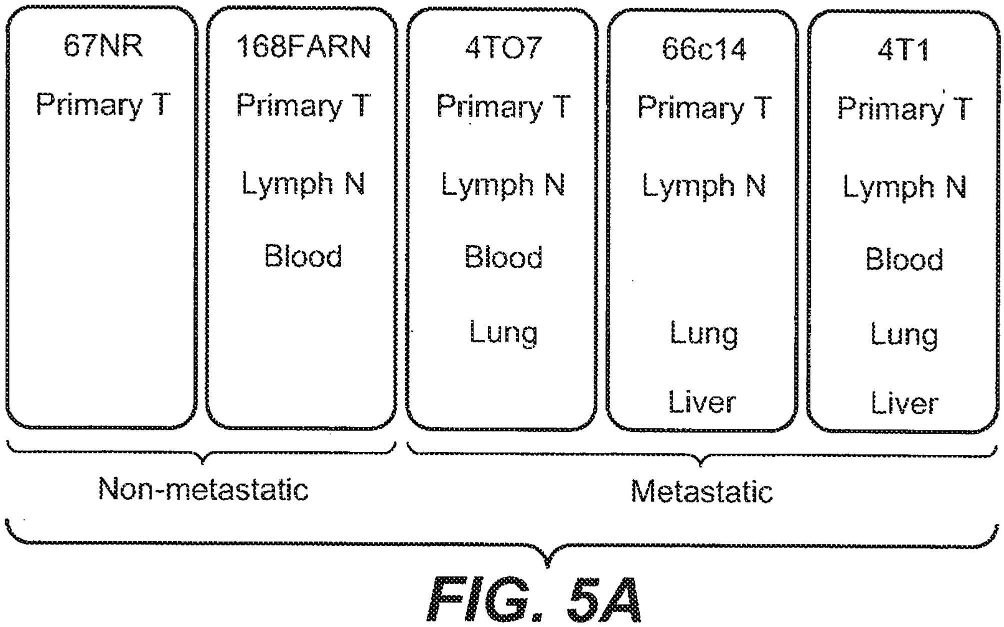
67NR
Primary T
168FARN
Primary T
Lymph N
Blood
4TO7
Primary T
Lymph N
Blood
Lung
66c14
Primary T
Lymph N
Lung
Liver
4T1
Primary T
Lymph N
Blood
Lung
Liver
Non-metastatic
Metastatic

*FIG. 5A*

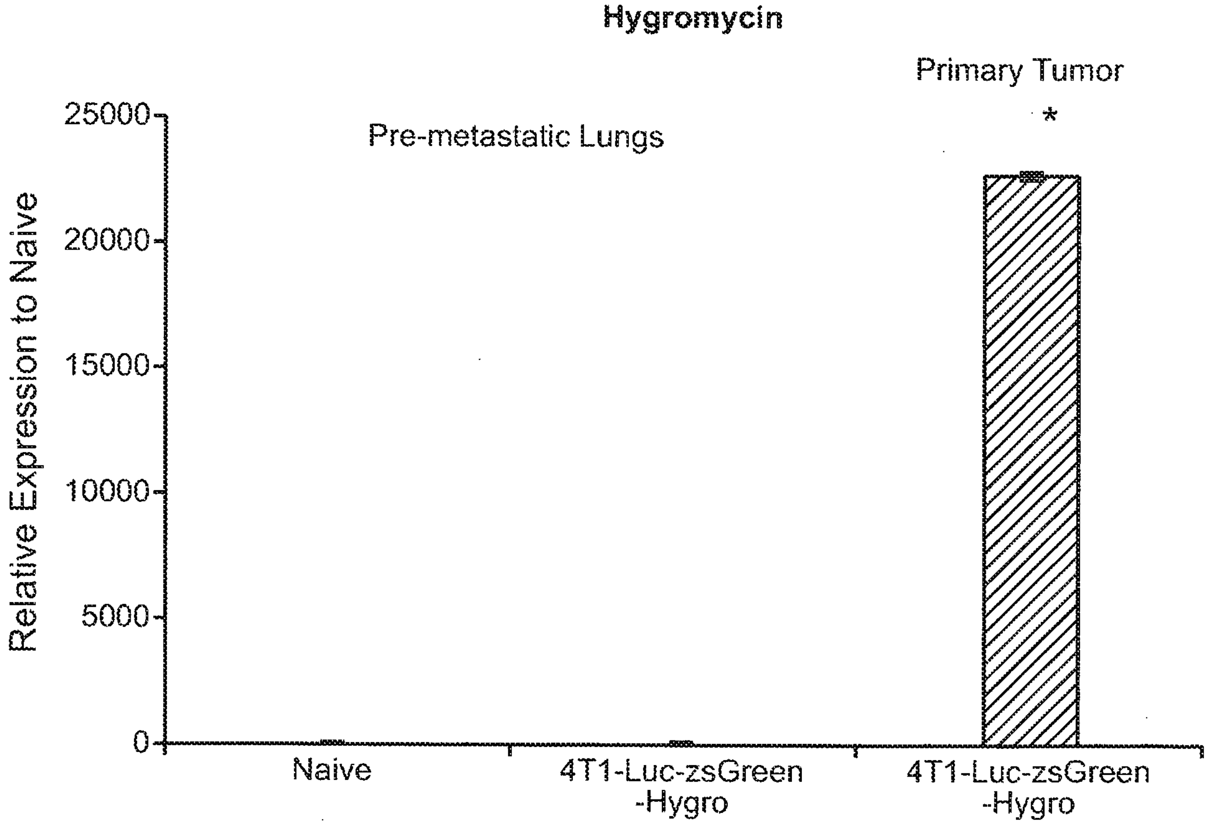
Hygromycin
Primary Tumor
*
Pre-metastatic Lungs
Relative Expression to Naive
25000
20000
15000
10000
5000
0
Naive
4T1-Luc-zsGreen -Hygro
4T1-Luc-zsGreen -Hygro

*FIG. 5B*

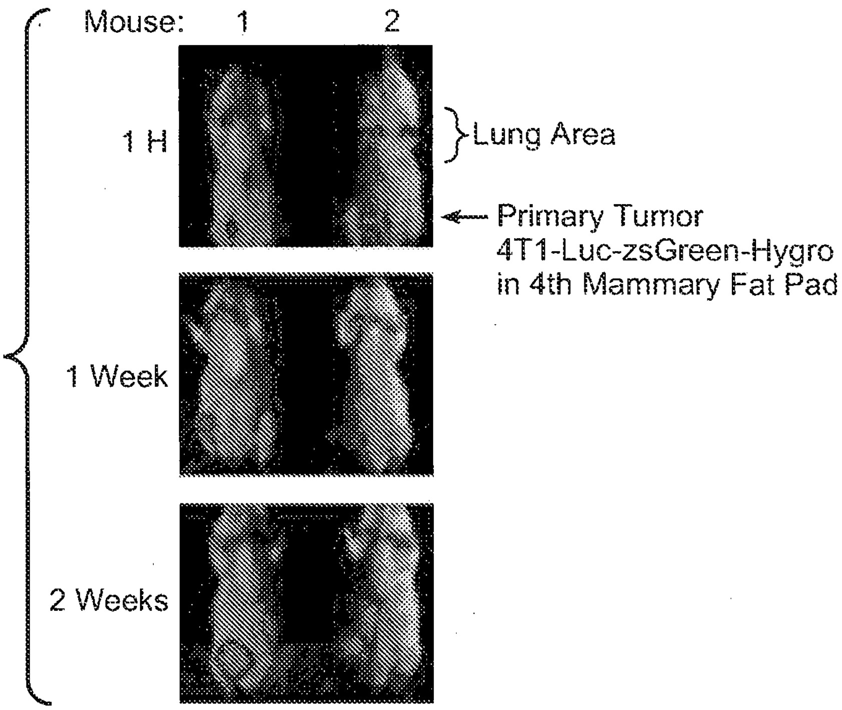
Mouse: 1 2
1 H
Lung Area
Primary Tumor
4T1-Luc-zsGreen-Hygro
in 4th Mammary Fat Pad
1 Week
2 Weeks

FIG. 5C

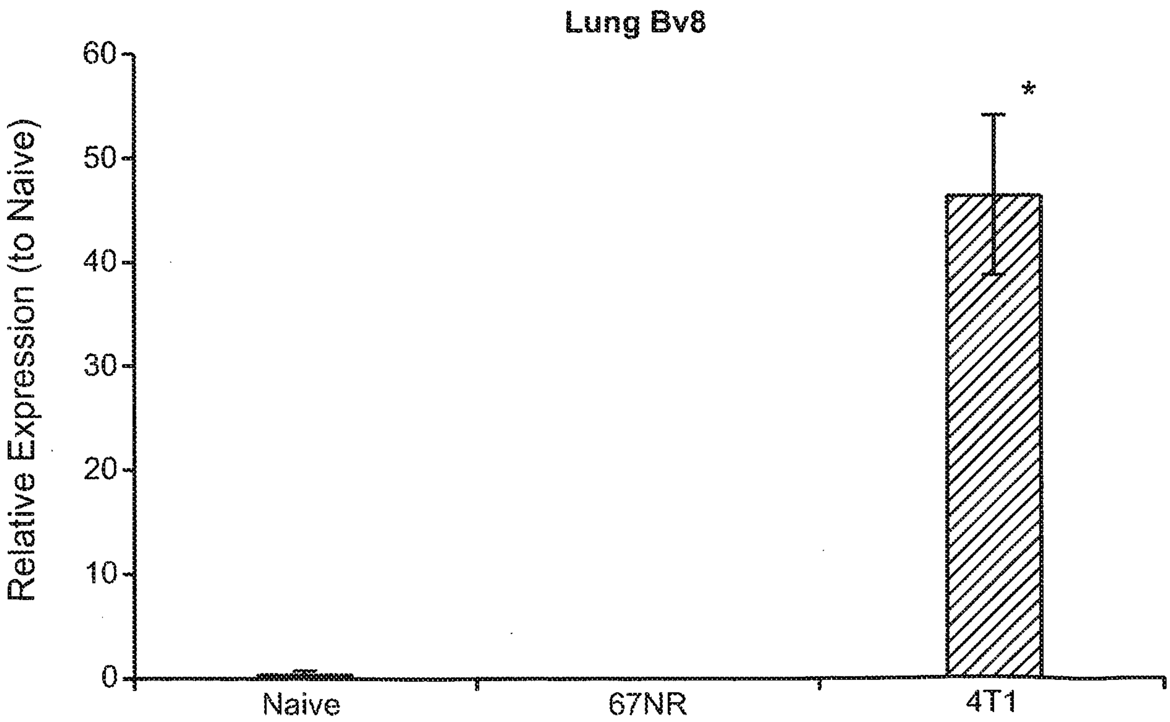
Lung Bv8
Relative Expression (to Naive)
60
50
40
30
20
10
0
*
Naive
67NR
4T1

FIG. 5D

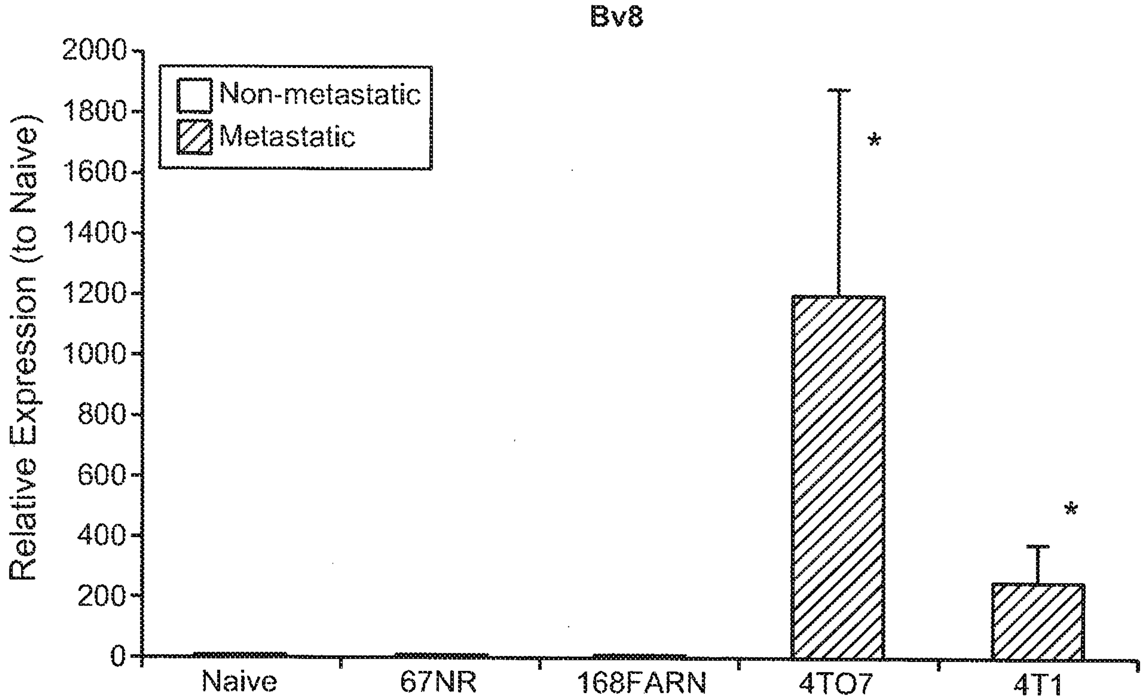
Bv8
Non-metastatic
Metastatic
Relative Expression (to Naive)
2000
1800
1600
1400
1200
1000
800
600
400
200
0
*
*
Naive
67NR
168FARN
4TO7
4T1

FIG. 5E

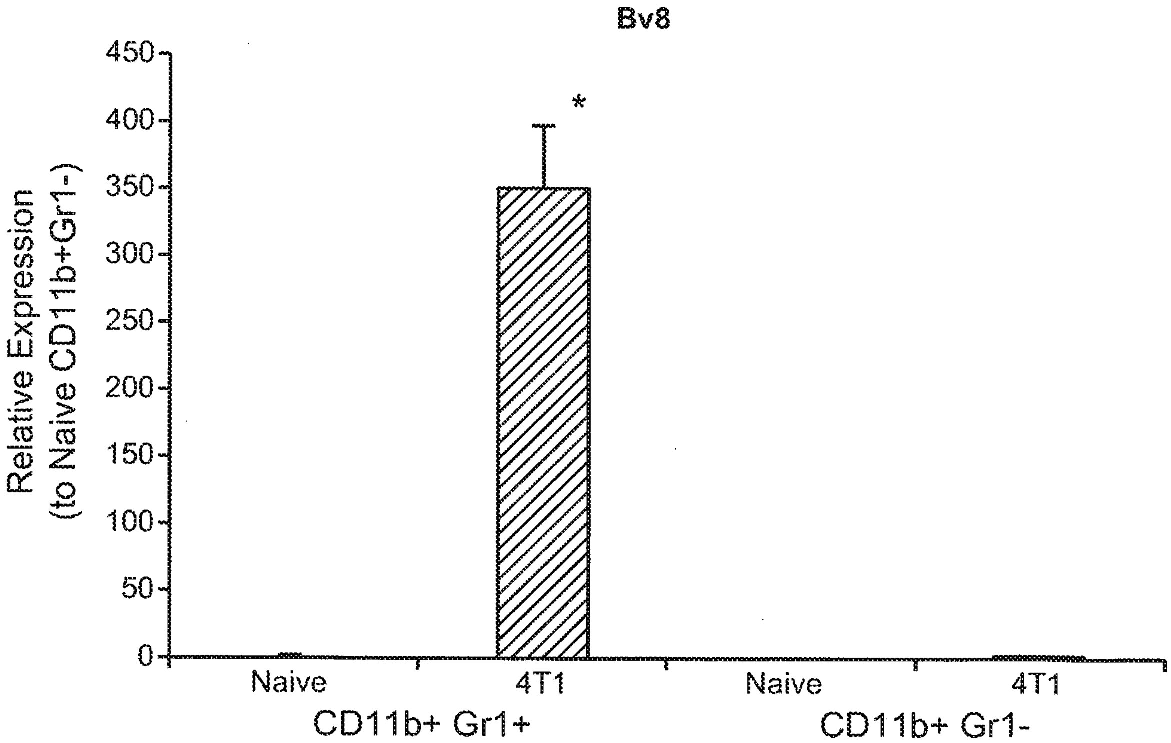
Bv8
Relative Expression (to Naive CD11b+Gr1-)
450
400
350
300
250
200
150
100
50
0
*
Naive
4T1
Naive
4T1
CD11b+ Gr1+
CD11b+ Gr1-

FIG. 5F

Bv8 IHC in the Pre-metastatic Lungs

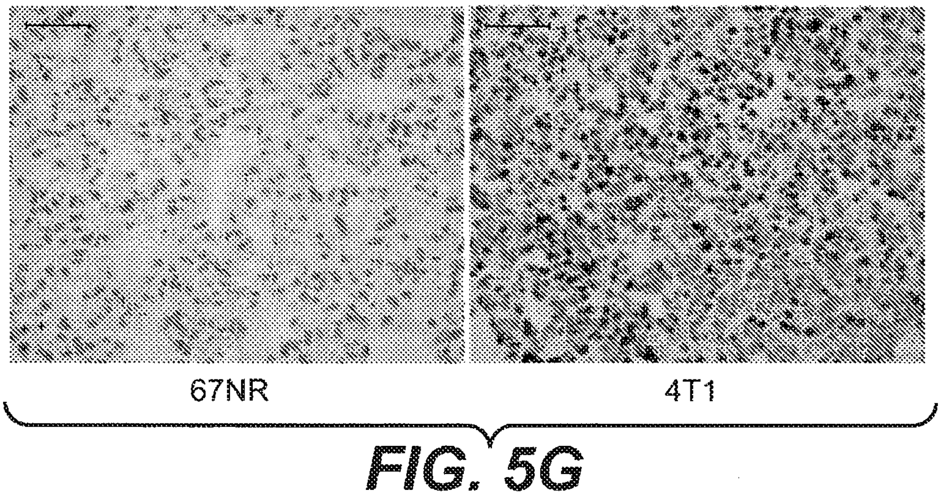


FIG. 5G

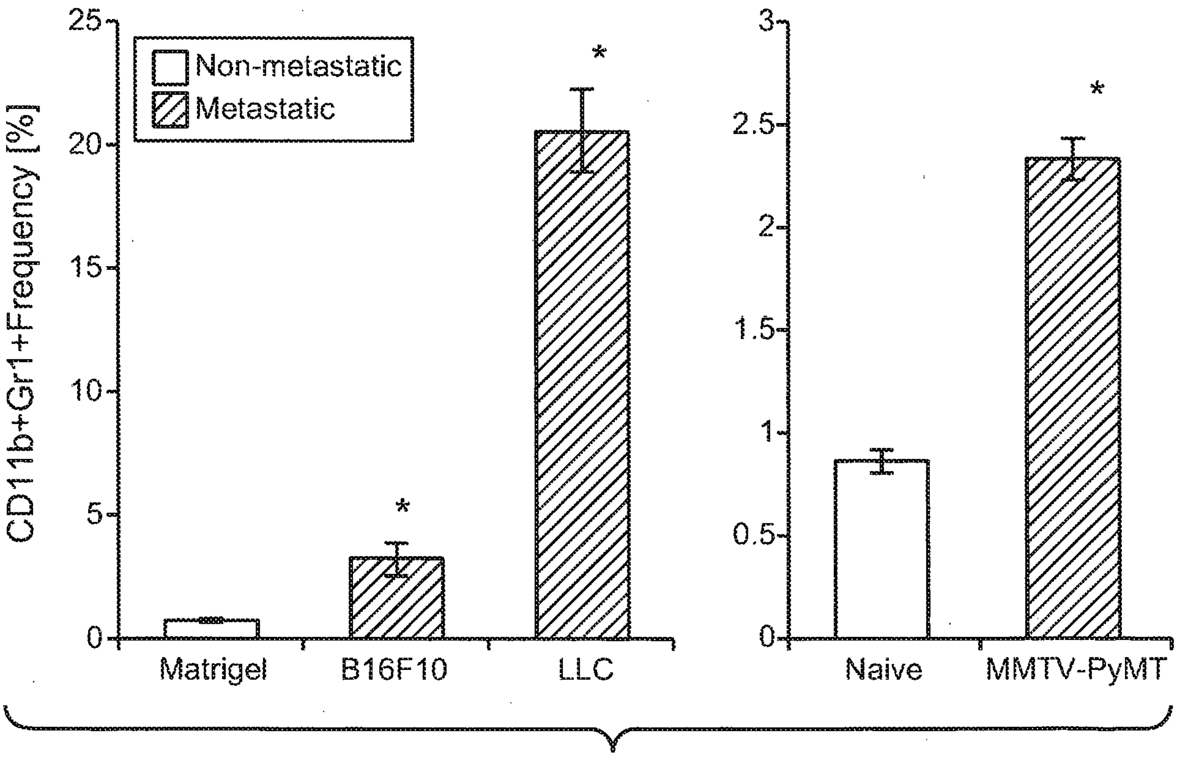


FIG. 5H

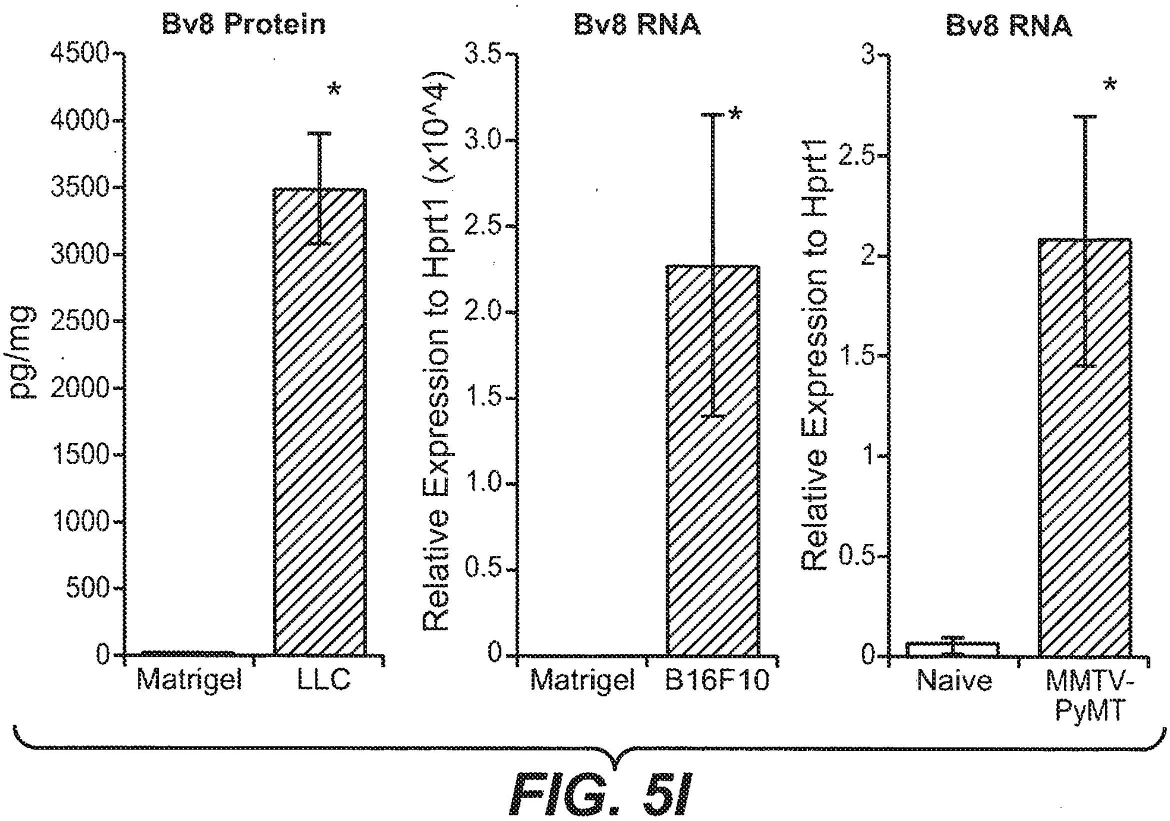
Bv8 Protein
4500
4000
3500
3000
2500
2000
1500
1000
500
0
pg/mg
*
Matrigel
LLC
Bv8 RNA
3.5
3.0
2.5
2.0
1.5
1.0
0.5
0
Relative Expression to Hprt1 (x10^4)
*
Matrigel
B16F10
Bv8 RNA
3
2.5
2
1.5
1
0.5
0
Relative Expression to Hprt1
*
Naive
MMTV-PyMT

FIG. 5I

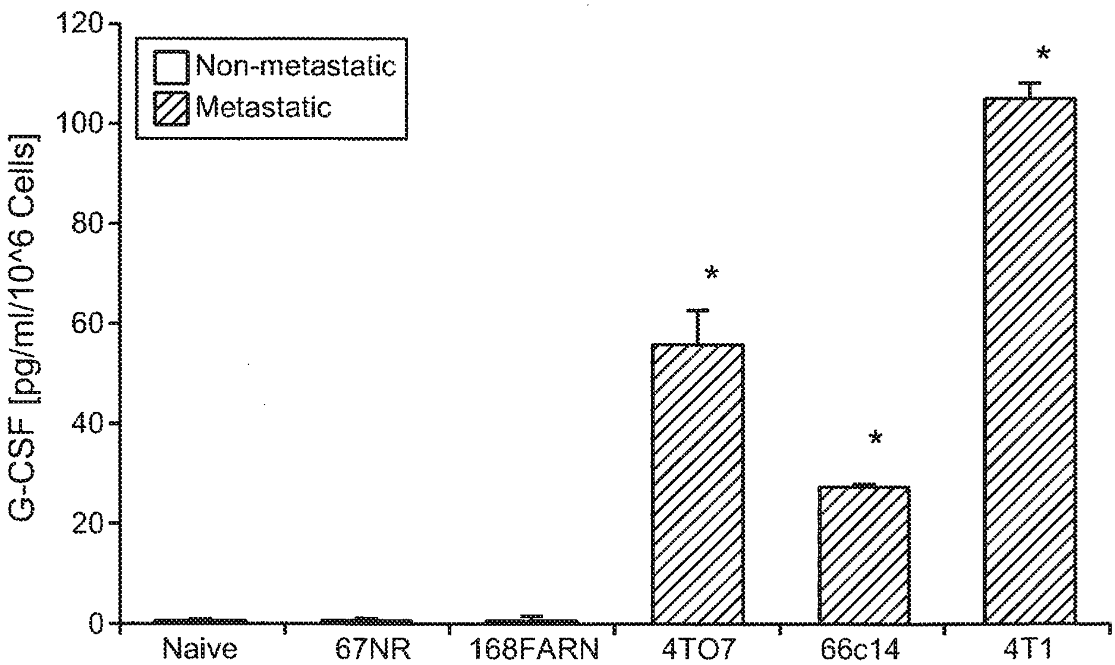
120
100
80
60
40
20
0
G-CSF [pg/ml/10^6 Cells]
Non-metastatic
Metastatic
*
*
*
Naive
67NR
168FARN
4TO7
66c14
4T1

FIG. 6A

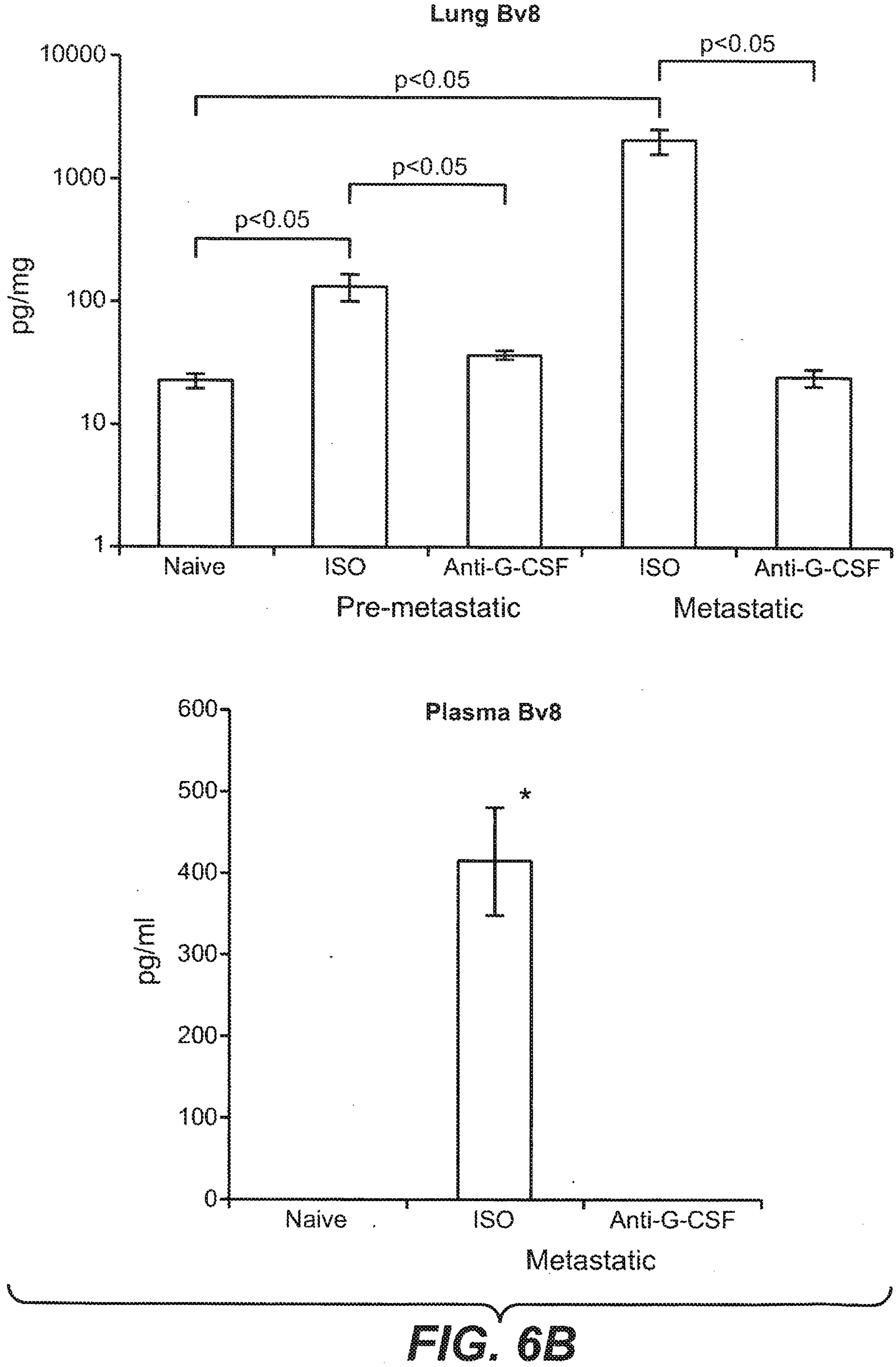
Lung Bv8
10000
1000
100
10
1
pg/mg
p<0.05
p<0.05
p<0.05
p<0.05
Naive
ISO
Anti-G-CSF
ISO
Anti-G-CSF
Pre-metastatic
Metastatic
Plasma Bv8
600
500
400
300
200
100
0
pg/ml
*
Naive
ISO
Anti-G-CSF
Metastatic

FIG. 6B

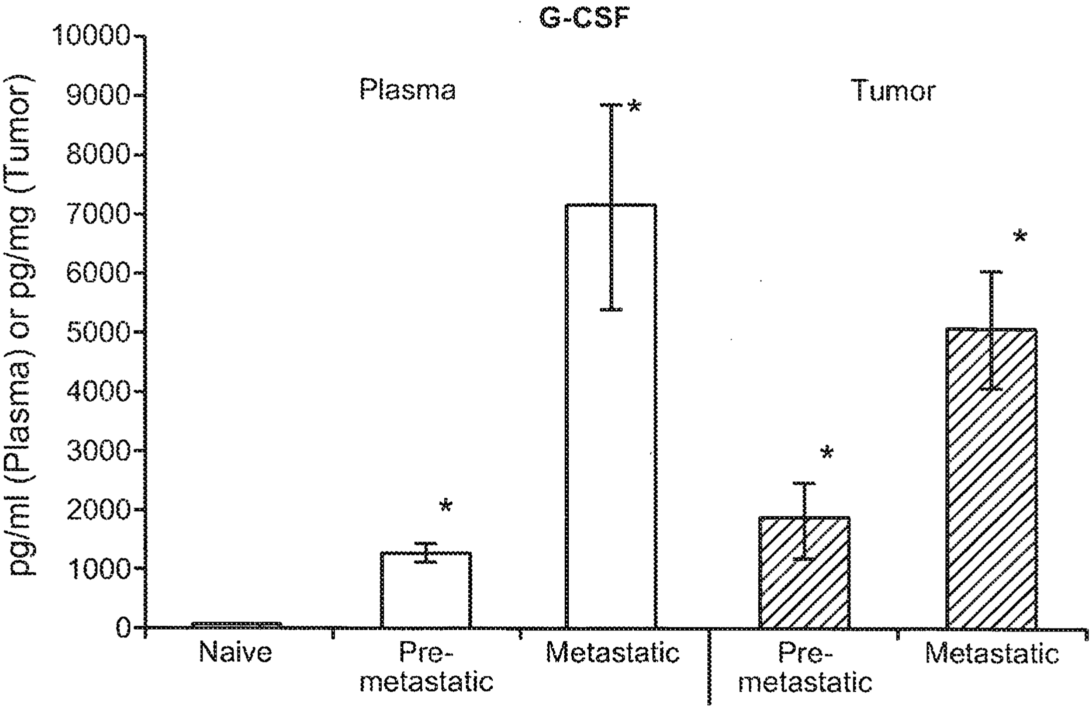
G-CSF
Plasma
Tumor
pg/ml (Plasma) or pg/mg (Tumor)
10000
9000
8000
7000
6000
5000
4000
3000
2000
1000
0
Naive
Pre-metastatic
Metastatic
Pre-metastatic
Metastatic
*

*FIG. 6C*

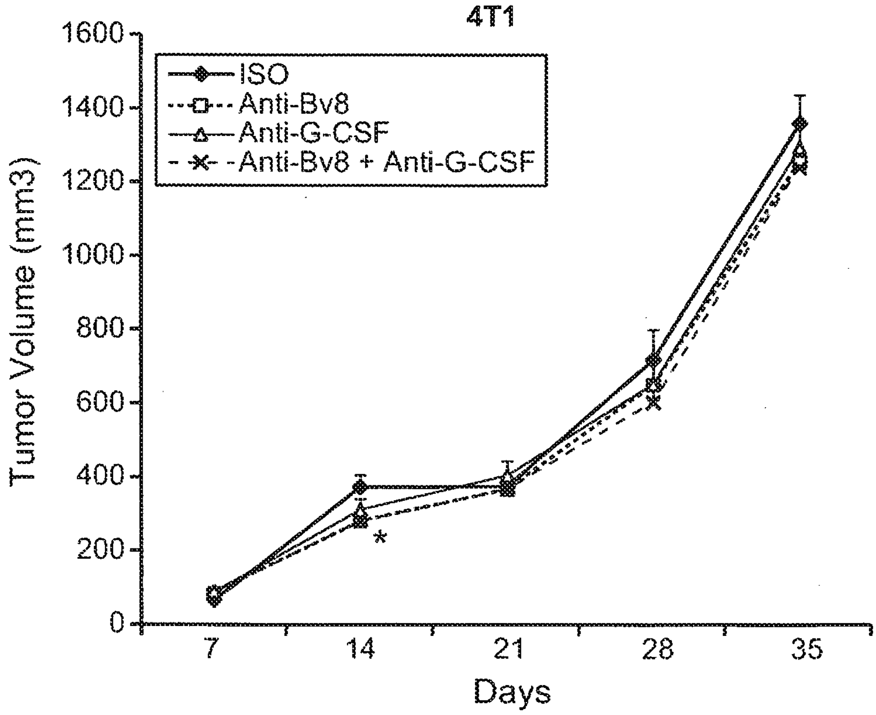
4T1
ISO
Anti-Bv8
Anti-G-CSF
Anti-Bv8 + Anti-G-CSF
Tumor Volume (mm3)
1600
1400
1200
1000
800
600
400
200
0
7
14
21
28
35
Days
*

*FIG. 6D*

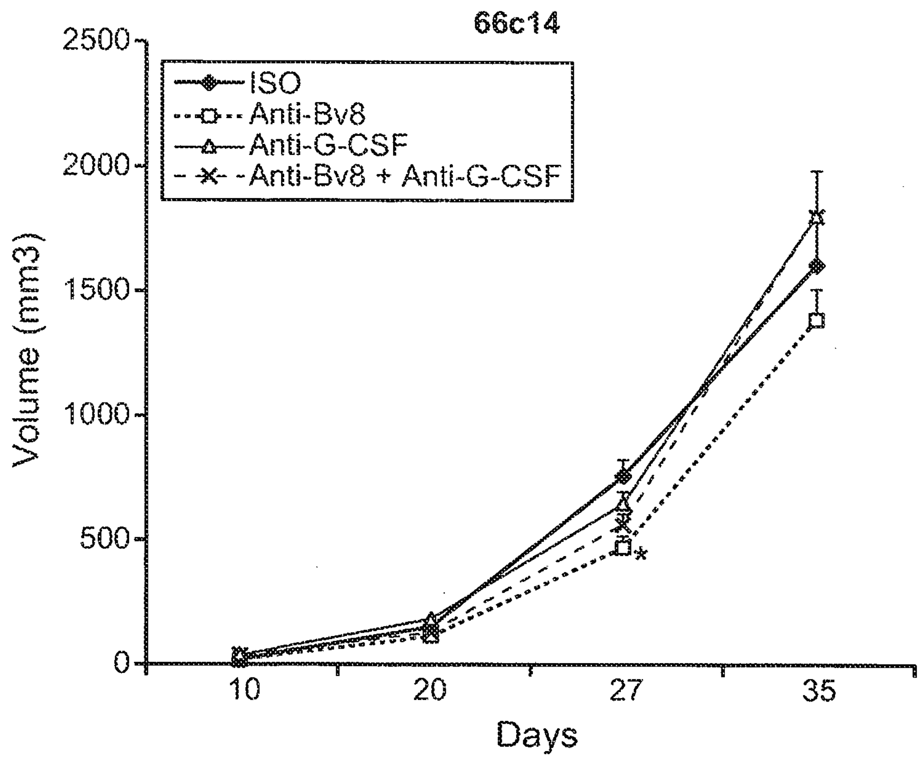
66c14
ISO
Anti-Bv8
Anti-G-CSF
Anti-Bv8 + Anti-G-CSF
2500
2000
1500
1000
500
0
Volume (mm3)
10
20
27
35
Days
*

FIG. 6E

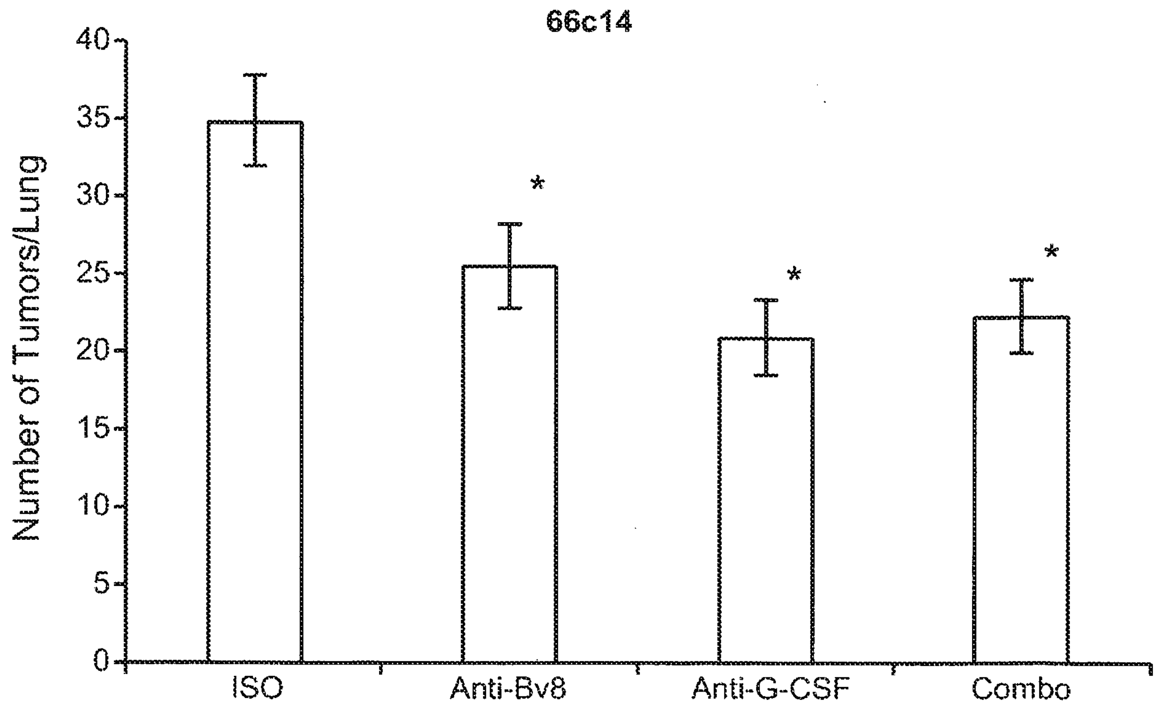
66c14
40
35
30
25
20
15
10
5
0
Number of Tumors/Lung
*
*
*
ISO
Anti-Bv8
Anti-G-CSF
Combo

FIG. 6F

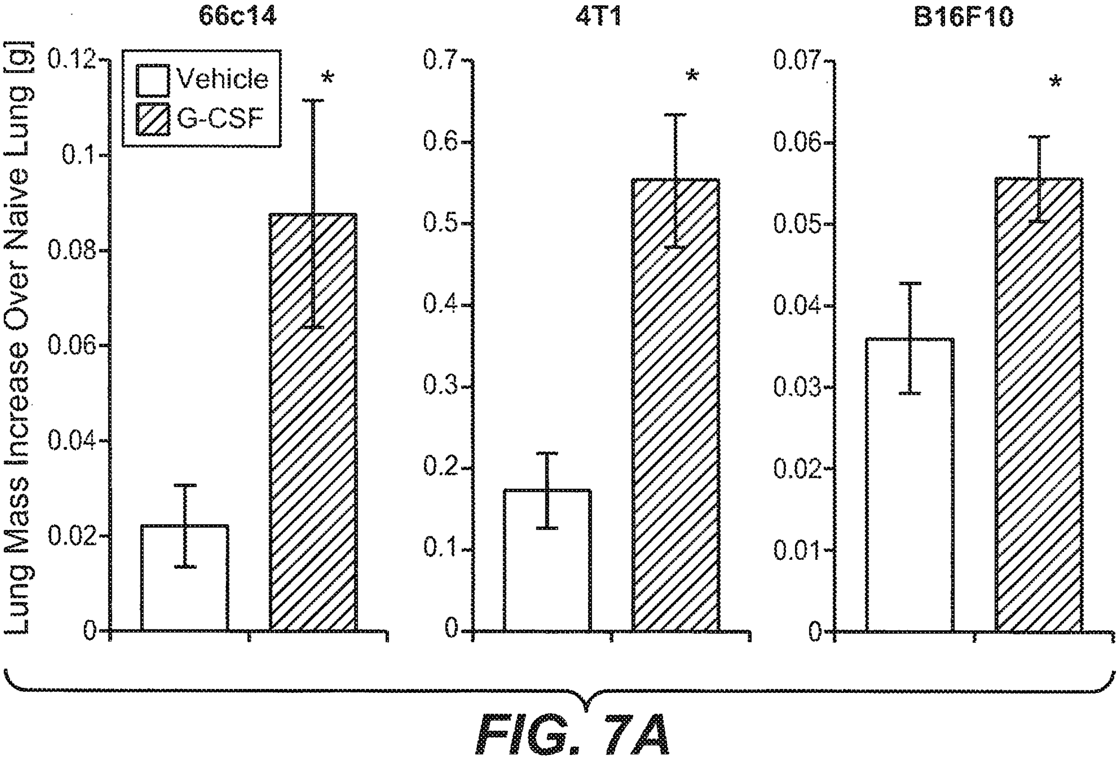
66c14
4T1
B16F10
Lung Mass Increase Over Naive Lung [g]
Vehicle
G-CSF
*
0.12
0.1
0.08
0.06
0.04
0.02
0
0.7
0.6
0.5
0.4
0.3
0.2
0.1
0.07
0.06
0.05
0.04
0.03
0.02
0.01

FIG. 7A

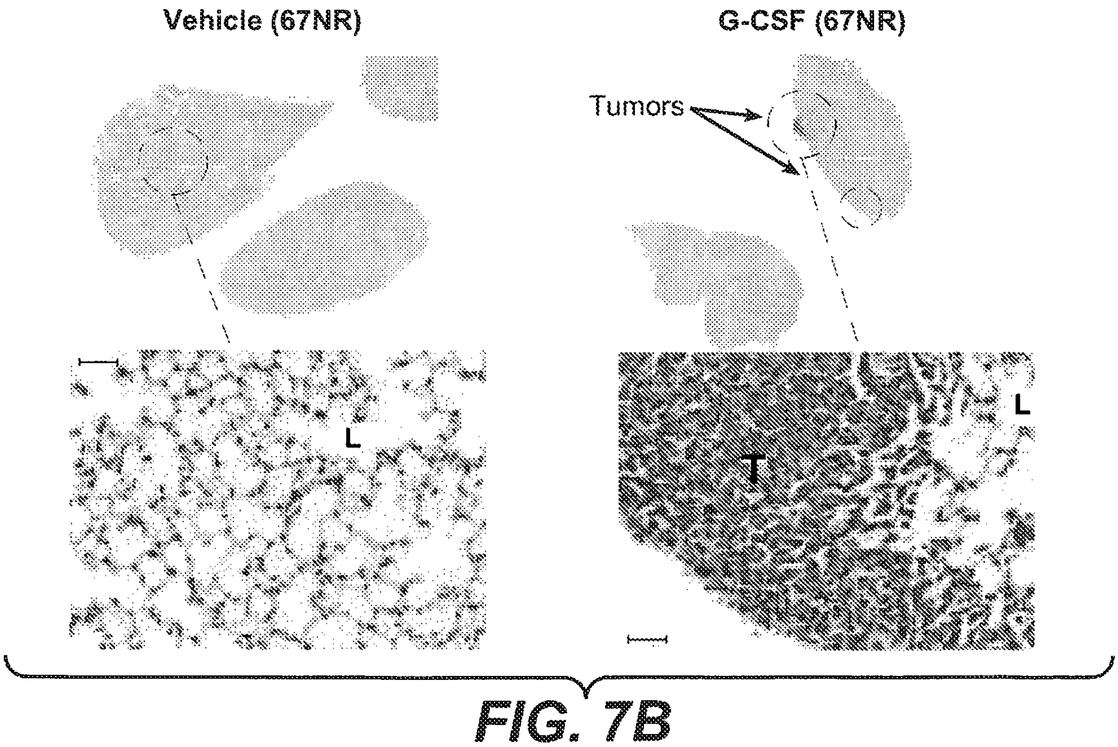
Vehicle (67NR)
G-CSF (67NR)
Tumors
L
T
L

FIG. 7B

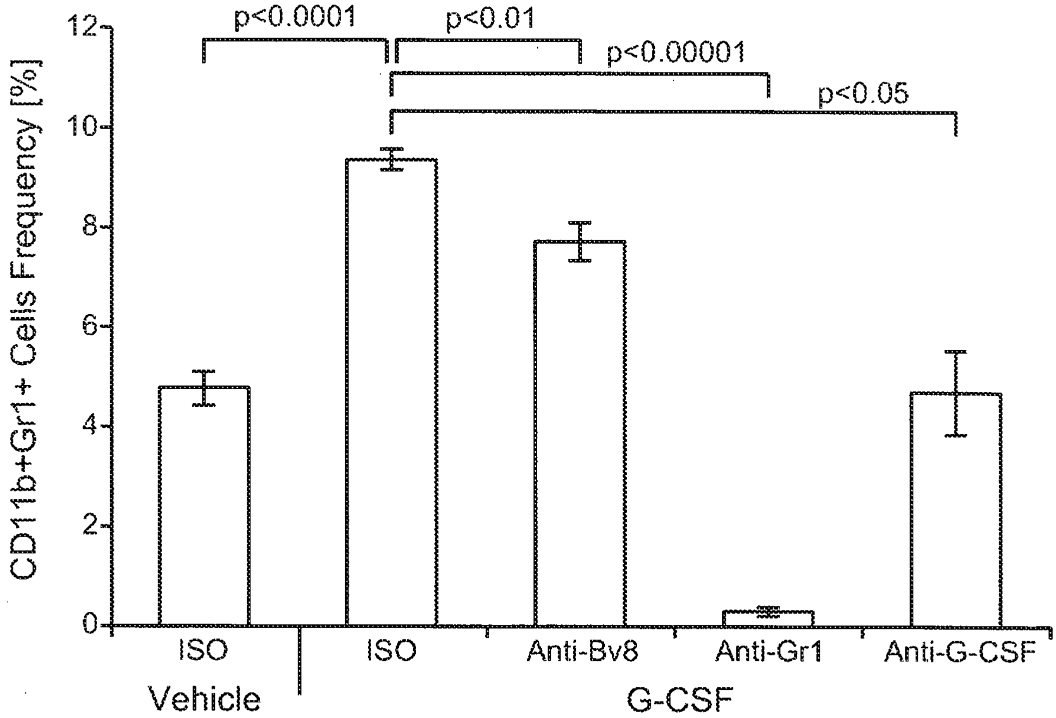
p<0.0001
p<0.01
p<0.00001
p<0.05
12
10
8
6
4
2
0
CD11b+Gr1+ Cells Frequency [%]
ISO
ISO
Anti-Bv8
Anti-Gr1
Anti-G-CSF
Vehicle
G-CSF

FIG. 7C

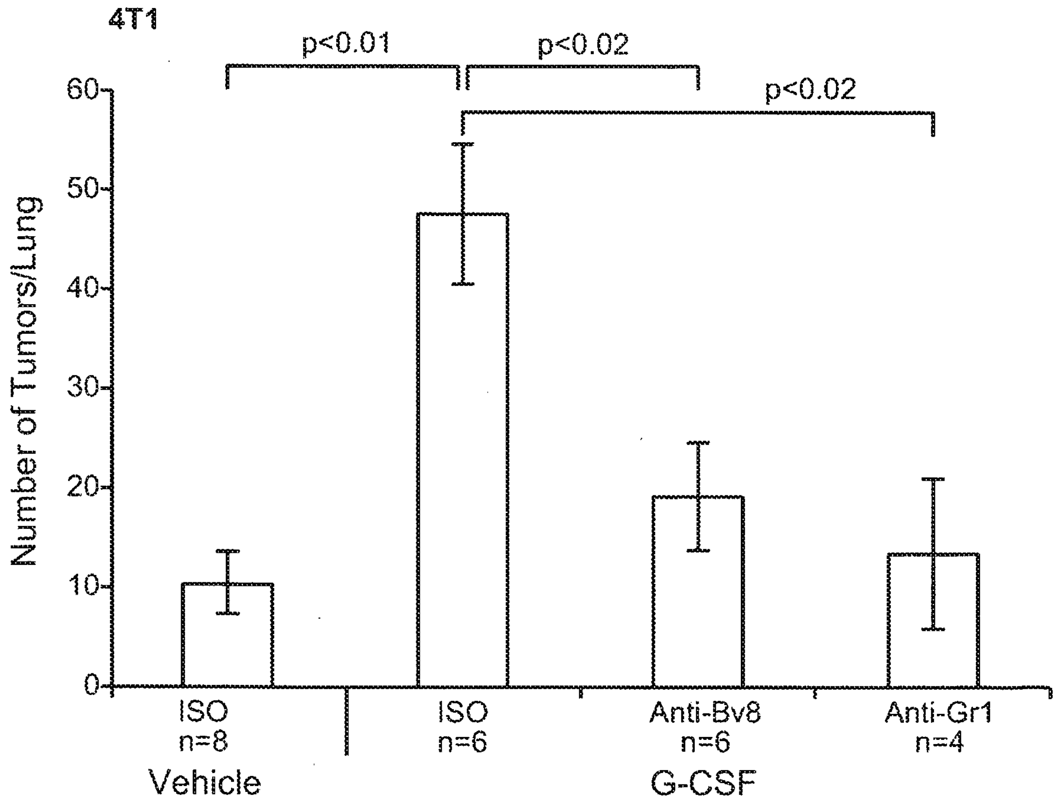
4T1
p<0.01
p<0.02
p<0.02
60
50
40
30
20
10
0
Number of Tumors/Lung
ISO n=8
ISO n=6
Anti-Bv8 n=6
Anti-Gr1 n=4
Vehicle
G-CSF

FIG. 7D

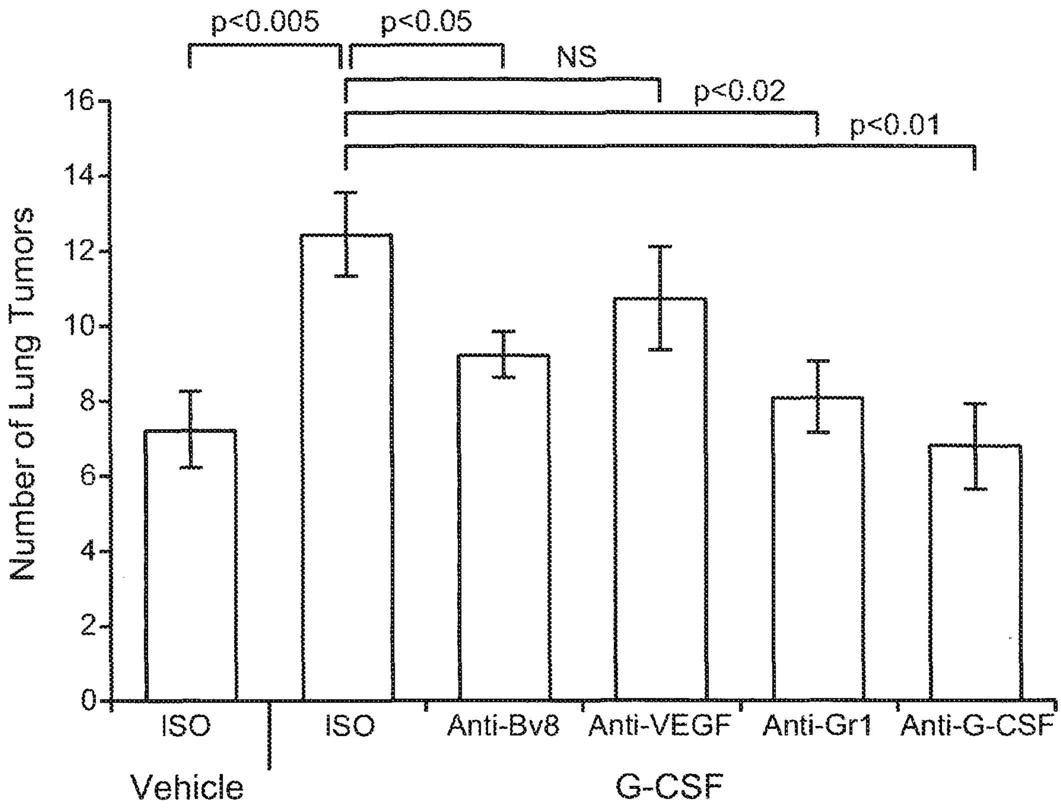
p<0.005
p<0.05
NS
p<0.02
p<0.01
Number of Lung Tumors
16
14
12
10
8
6
4
2
0
ISO
ISO
Anti-Bv8
Anti-VEGF
Anti-Gr1
Anti-G-CSF
Vehicle
G-CSF

*FIG. 7E*

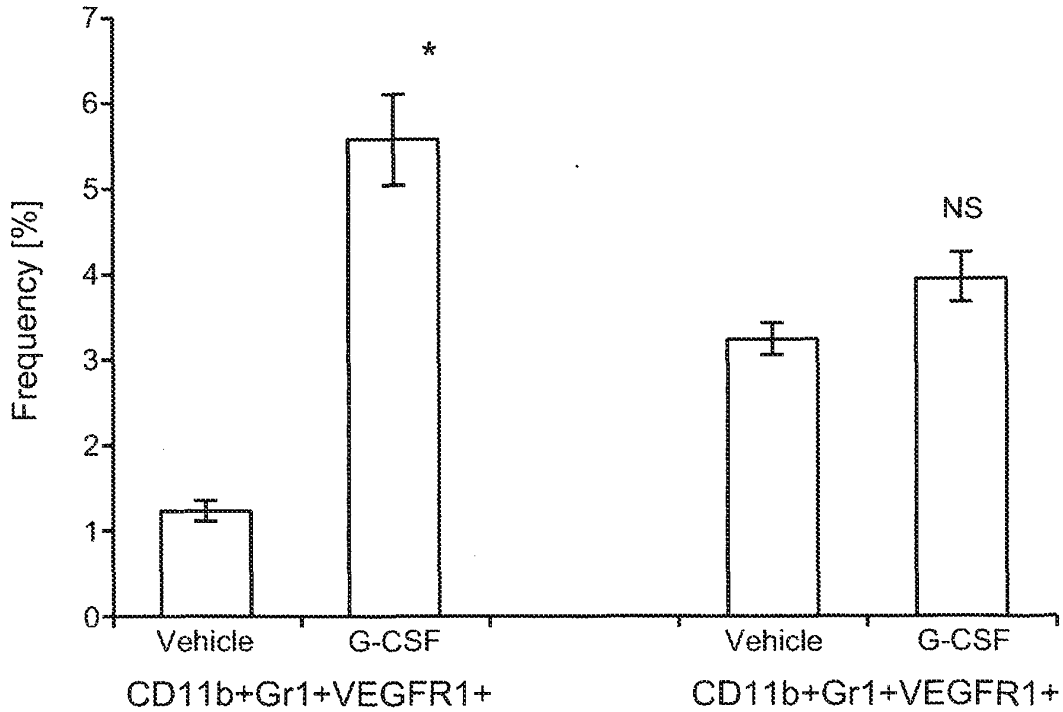
Frequency [%]
7
6
5
4
3
2
1
0
*
NS
Vehicle
G-CSF
Vehicle
G-CSF
CD11b+Gr1+VEGFR1+
CD11b+Gr1+VEGFR1+

*FIG. 7F*

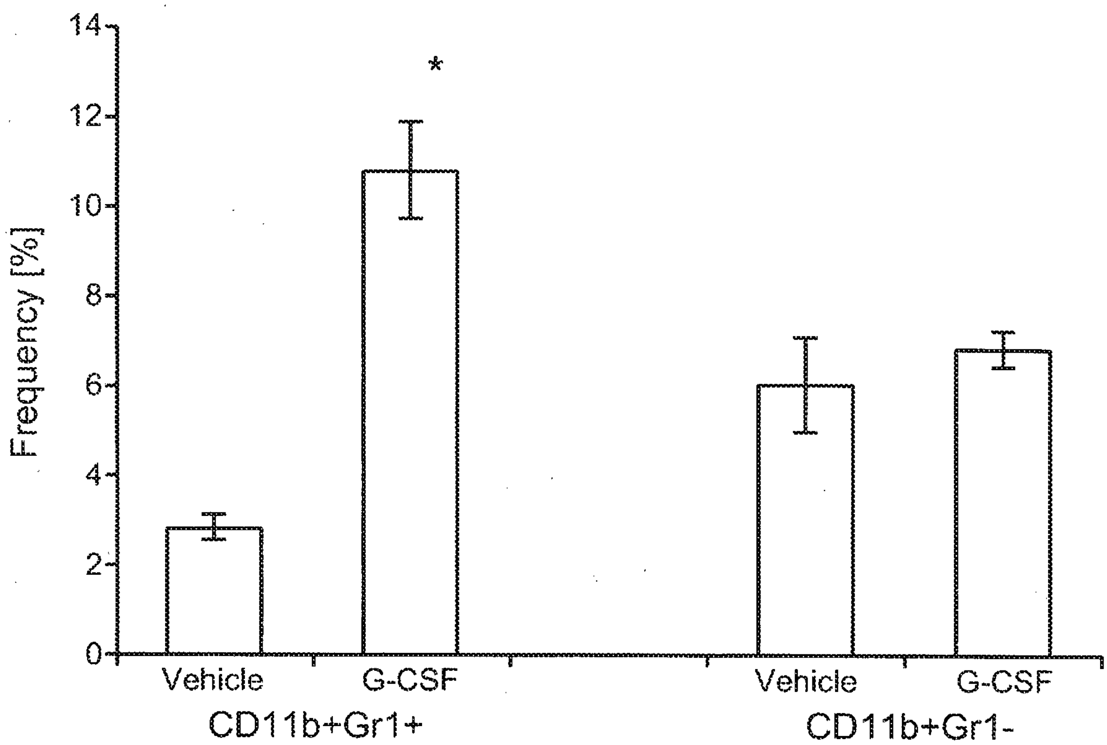
14
12
10
8
6
4
2
0
Frequency [%]
*
Vehicle
G-CSF
CD11b+Gr1+
Vehicle
G-CSF
CD11b+Gr1-

*FIG. 7G*

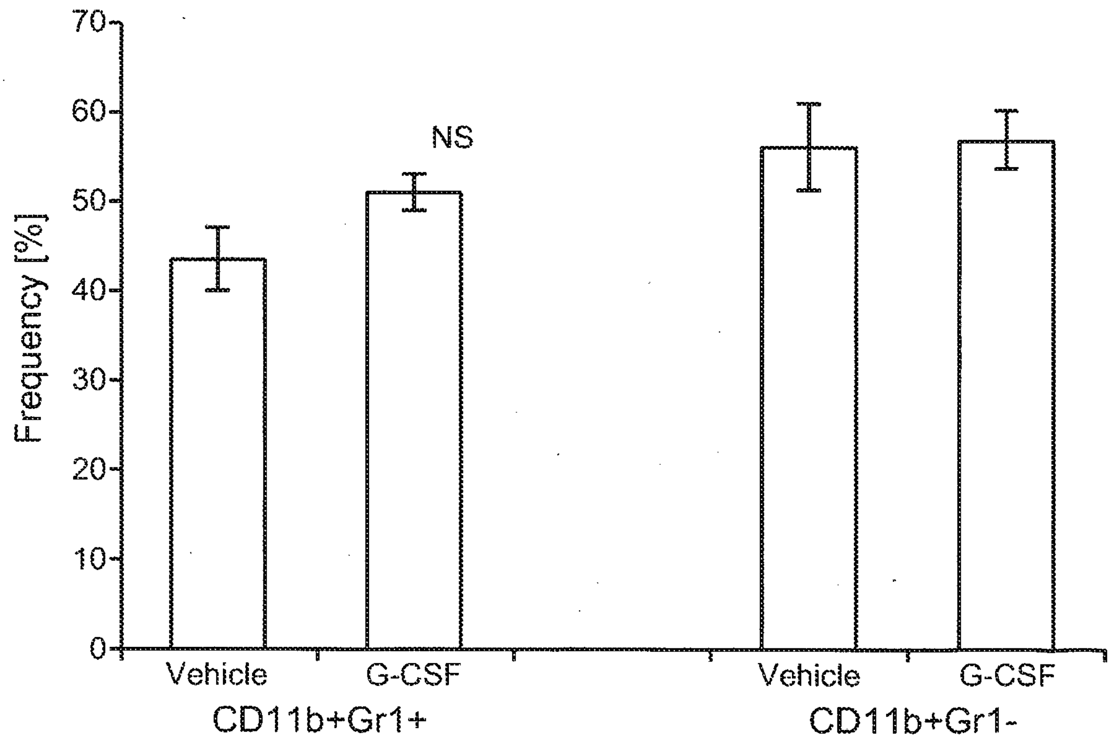
70
60
50
40
30
20
10
0
Frequency [%]
NS
Vehicle
G-CSF
CD11b+Gr1+
Vehicle
G-CSF
CD11b+Gr1-

*FIG. 7H*

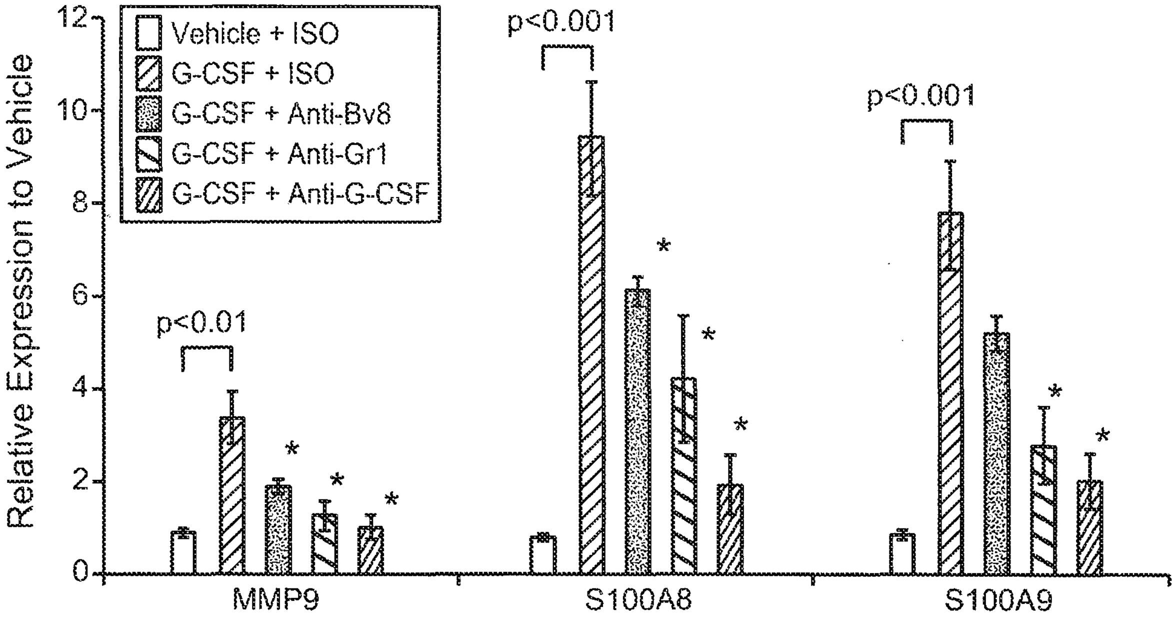

Relative Expression to Vehicle
0
2
4
6
8
10
12
Vehicle + ISO
G-CSF + ISO
G-CSF + Anti-Bv8
G-CSF + Anti-Gr1
G-CSF + Anti-G-CSF
p<0.01
p<0.001
p<0.001
*
MMP9
S100A8
S100A9


*FIG. 8A*

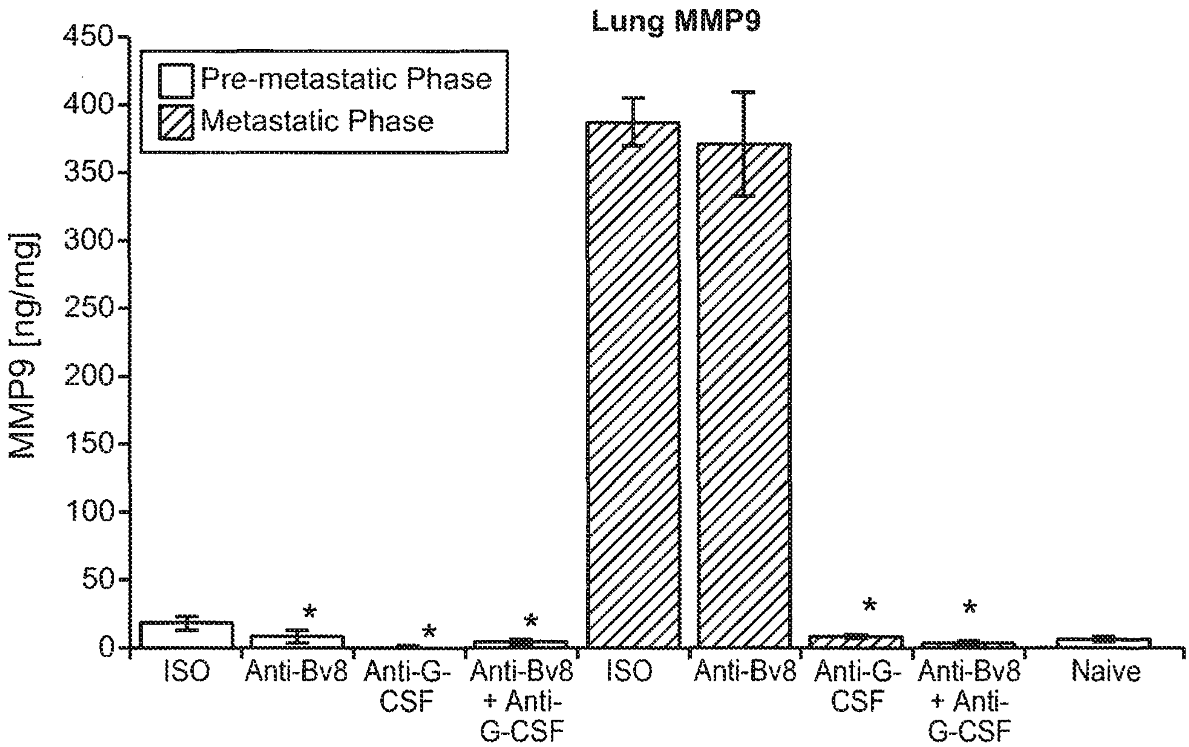

Lung MMP9
MMP9 [ng/mg]
0
50
100
150
200
250
300
350
400
450
Pre-metastatic Phase
Metastatic Phase
*
ISO
Anti-Bv8
Anti-G-CSF
Anti-Bv8 + Anti-G-CSF
ISO
Anti-Bv8
Anti-G-CSF
Anti-Bv8 + Anti-G-CSF
Naive


*FIG. 8C*

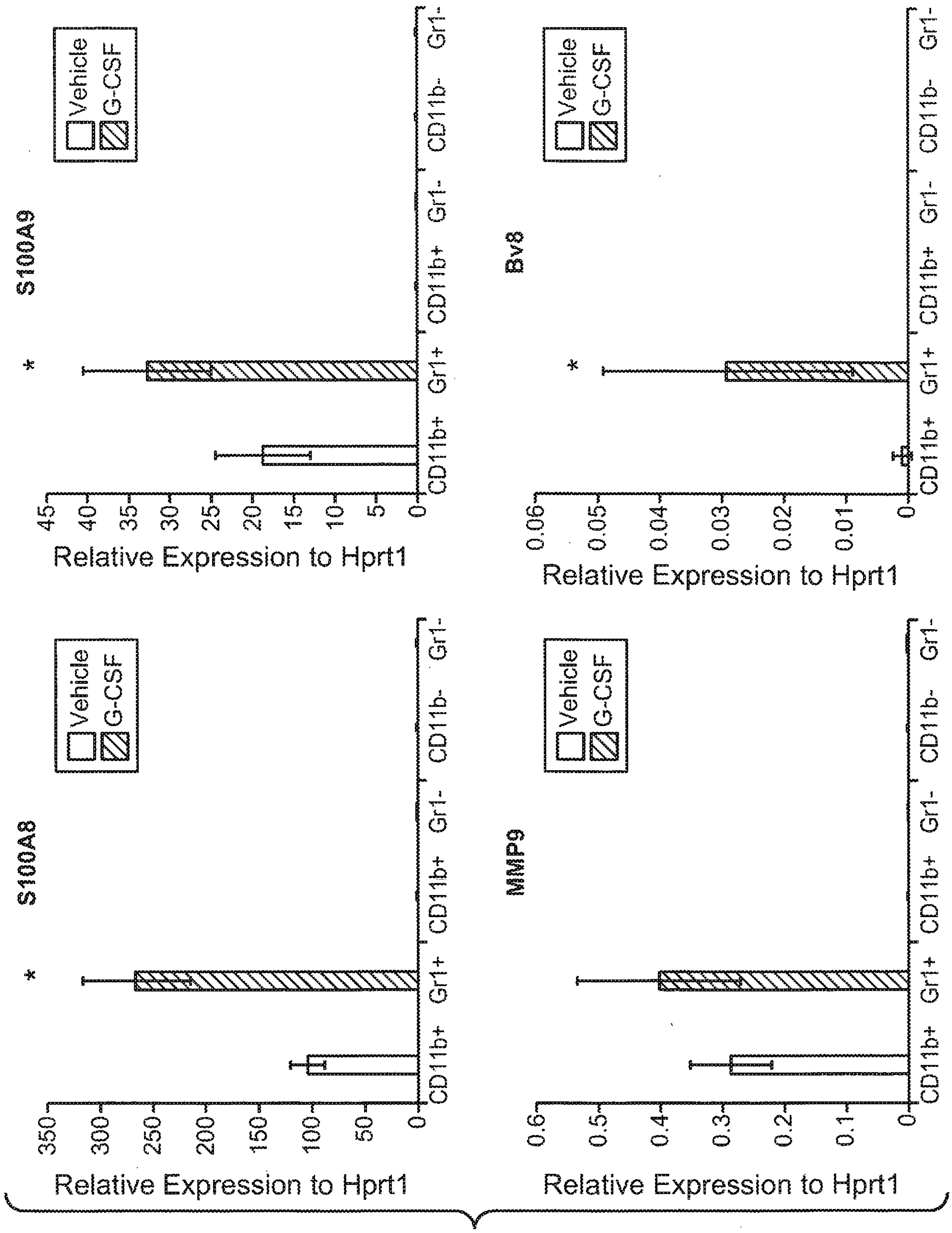
S100A8
S100A9
MMP9
Bv8
Vehicle
G-CSF
Relative Expression to Hprt1
CD11b+
Gr1+
CD11b+
Gr1-
CD11b-
Gr1-
*
350
300
250
200
150
100
50
0
45
40
35
30
25
20
15
10
5
0.6
0.5
0.4
0.3
0.2
0.1
0.06
0.05
0.04
0.03
0.02
0.01

*FIG. 8B*

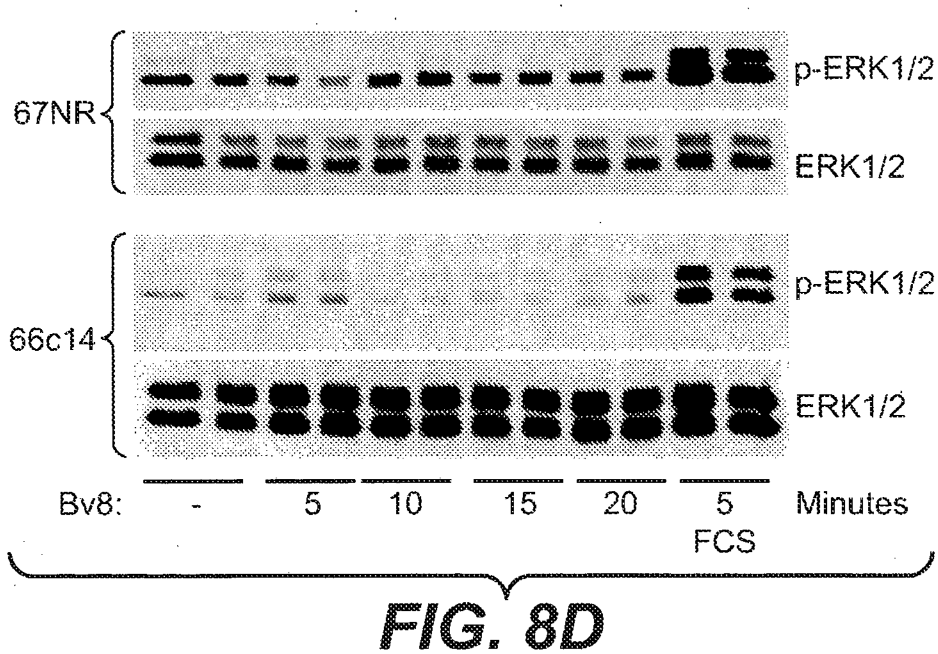
67NR
p-ERK1/2
ERK1/2
66c14
p-ERK1/2
ERK1/2
Bv8: - 5 10 15 20 5 Minutes
FCS

FIG. 8D

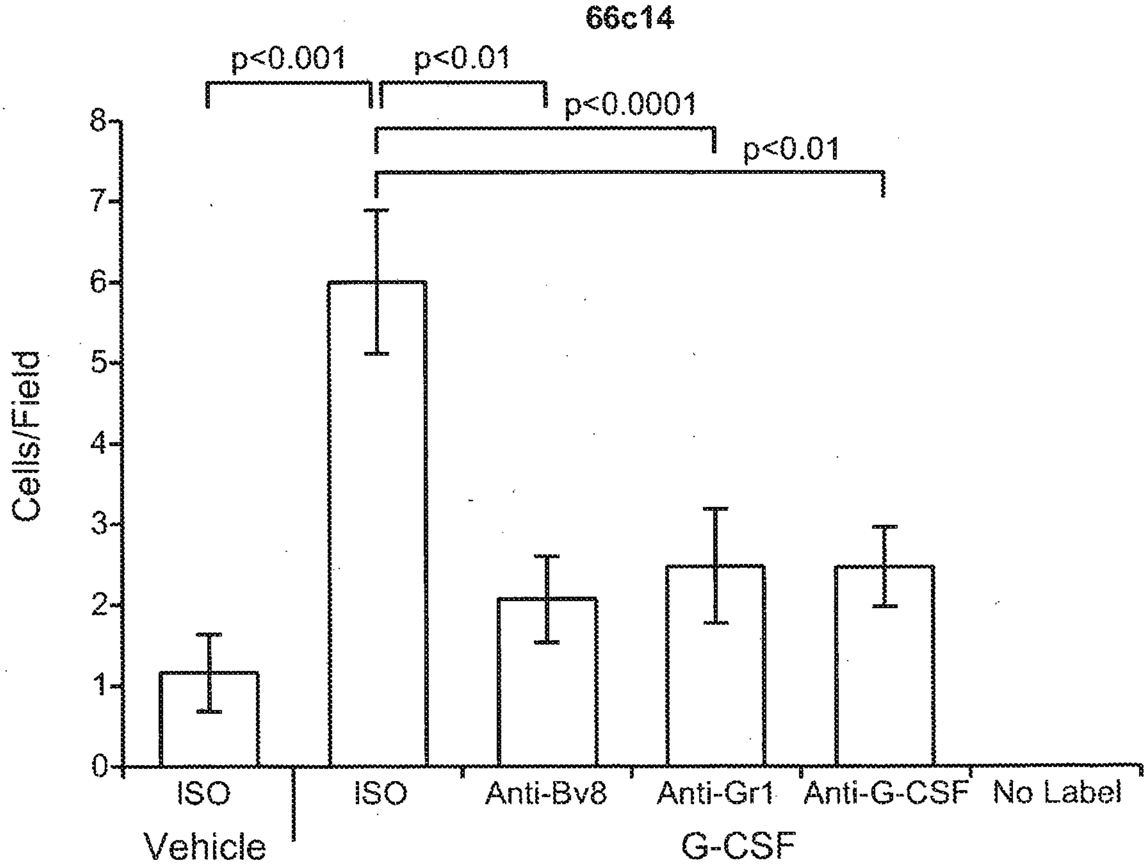
66c14
p<0.001
p<0.01
p<0.0001
p<0.01
Cells/Field
0
1
2
3
4
5
6
7
8
ISO
ISO
Anti-Bv8
Anti-Gr1
Anti-G-CSF
No Label
Vehicle
G-CSF

FIG. 8E

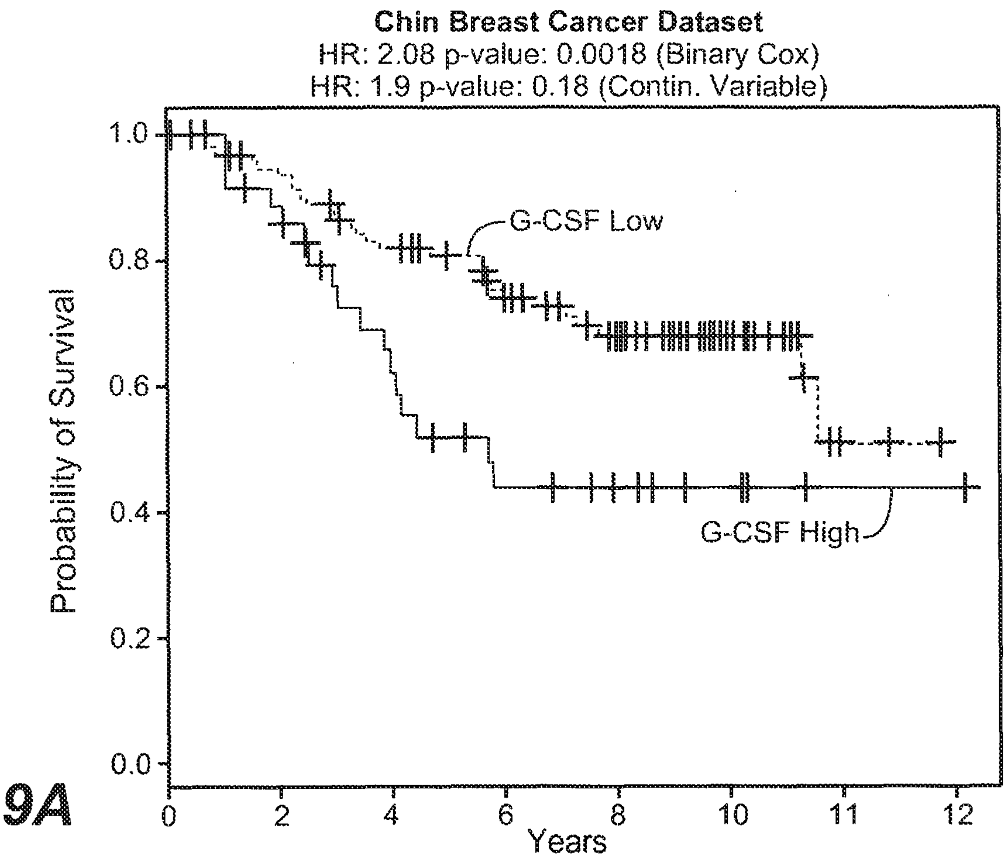
Chin Breast Cancer Dataset
HR: 2.08 p-value: 0.0018 (Binary Cox)
HR: 1.9 p-value: 0.18 (Contin. Variable)
G-CSF Low
G-CSF High
Probability of Survival
1.0
0.8
0.6
0.4
0.2
0.0
0
2
4
6
8
10
11
12
Years

FIG. 9A

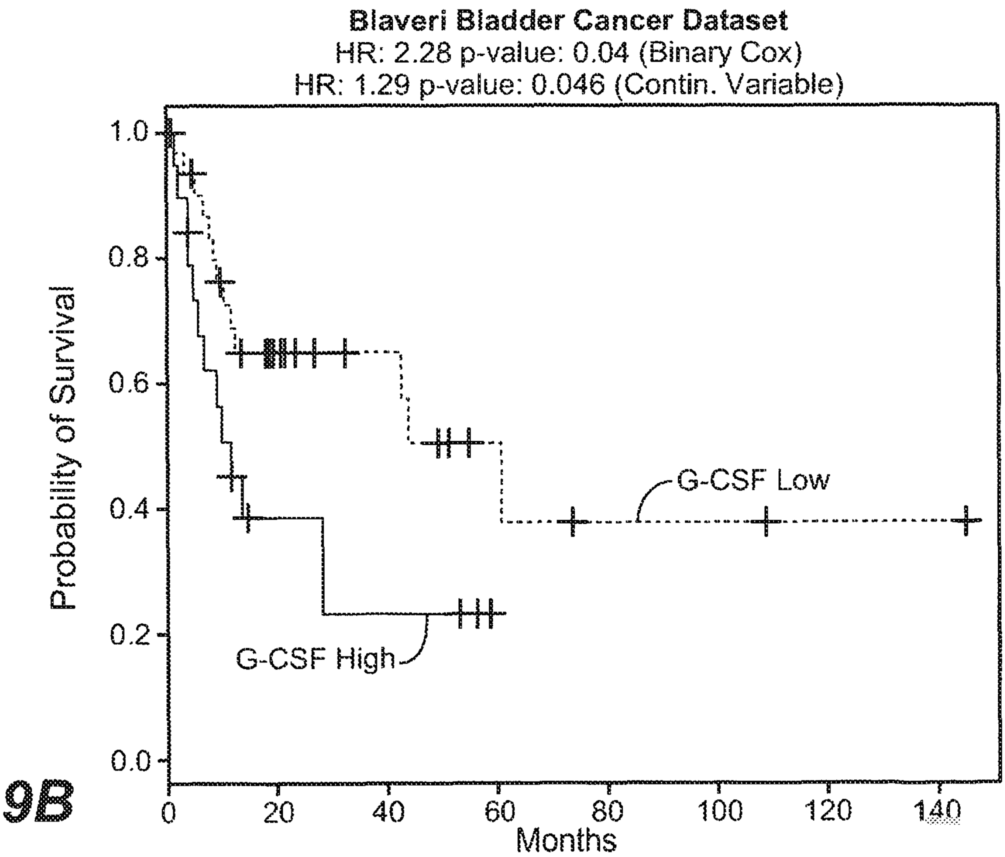
Blaveri Bladder Cancer Dataset
HR: 2.28 p-value: 0.04 (Binary Cox)
HR: 1.29 p-value: 0.046 (Contin. Variable)
G-CSF Low
G-CSF High
Probability of Survival
1.0
0.8
0.6
0.4
0.2
0.0
0
20
40
60
80
100
120
140
Months

FIG. 9B

| | No. of Patients | HR[1] | P-value[1] | HR[2] | P-value[2] |
|---|---|---|---|---|---|
| Blaveri Bladder | 98 | 2.28 | 0.04 | 1.29 | 0.046 |
| Pawitan BRC | 159 | 2.48 | 0.0057 | 1.7 | 0.059 |
| Chin BRC | 118 | 2.08 | 0.018 | 1.9 | 0.18 |

1: The hazard rations were estimated using the Cox model between the high and low G-CSF groups, where the cutoff was determined using the log rank statistics.
2: G-CSF expression was treated as a continuous variable in the Cox model

*FIG. 9C*

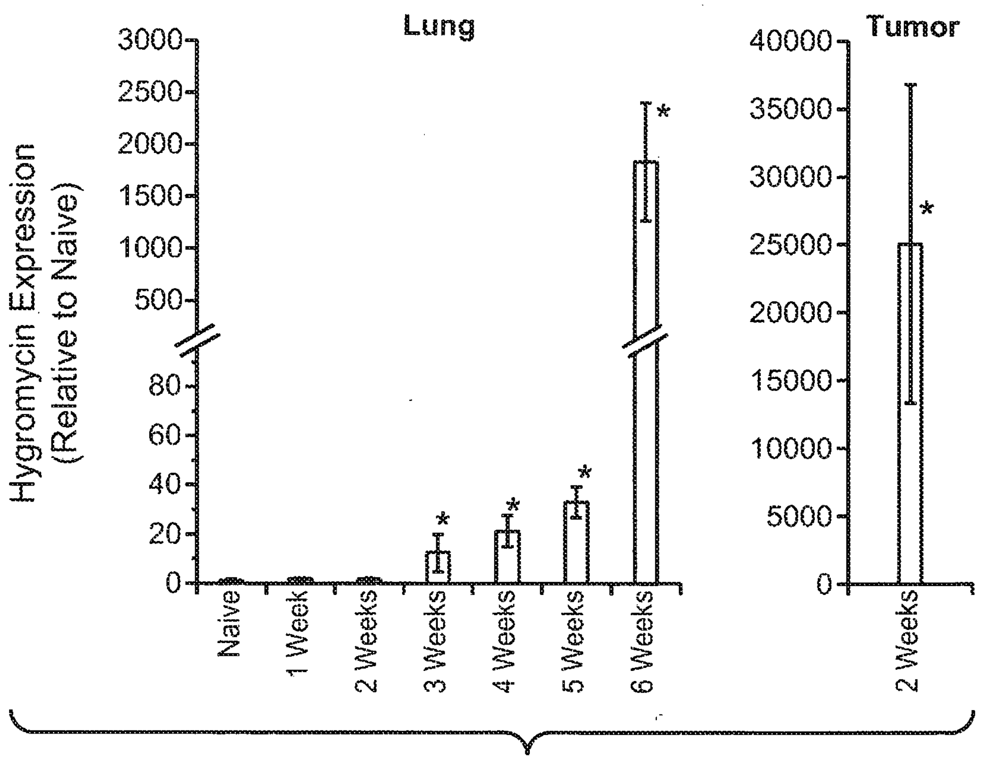


*FIG. 10*

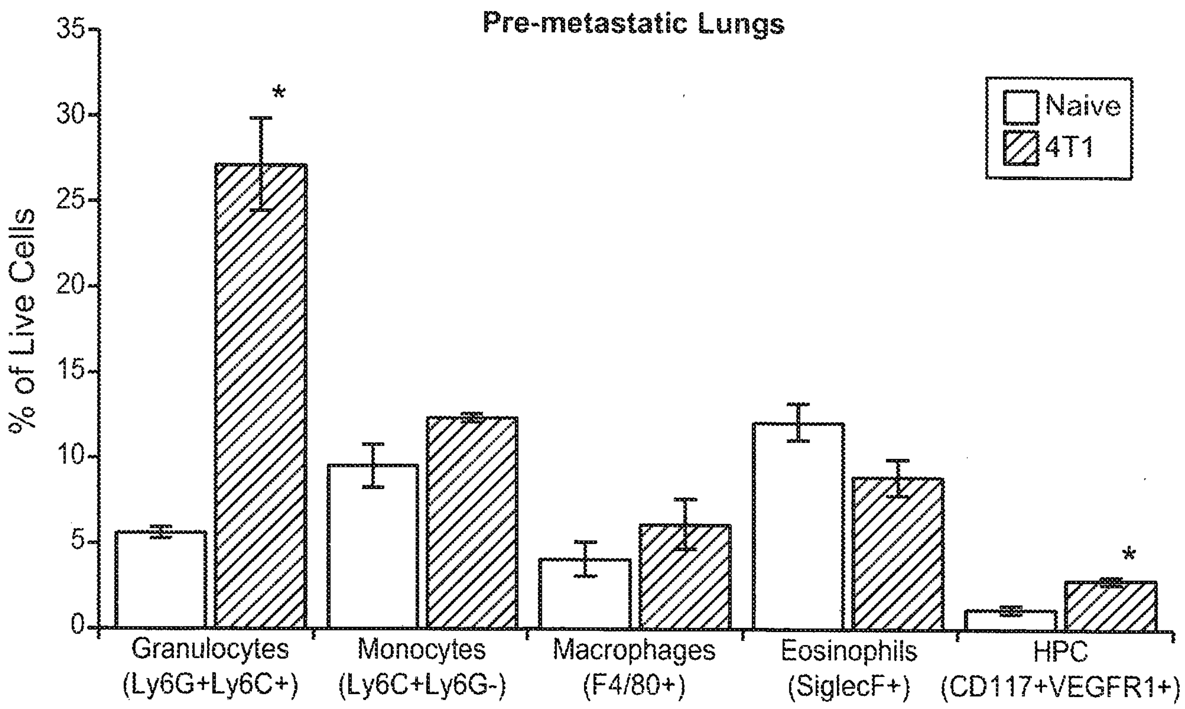


*FIG. 11A*

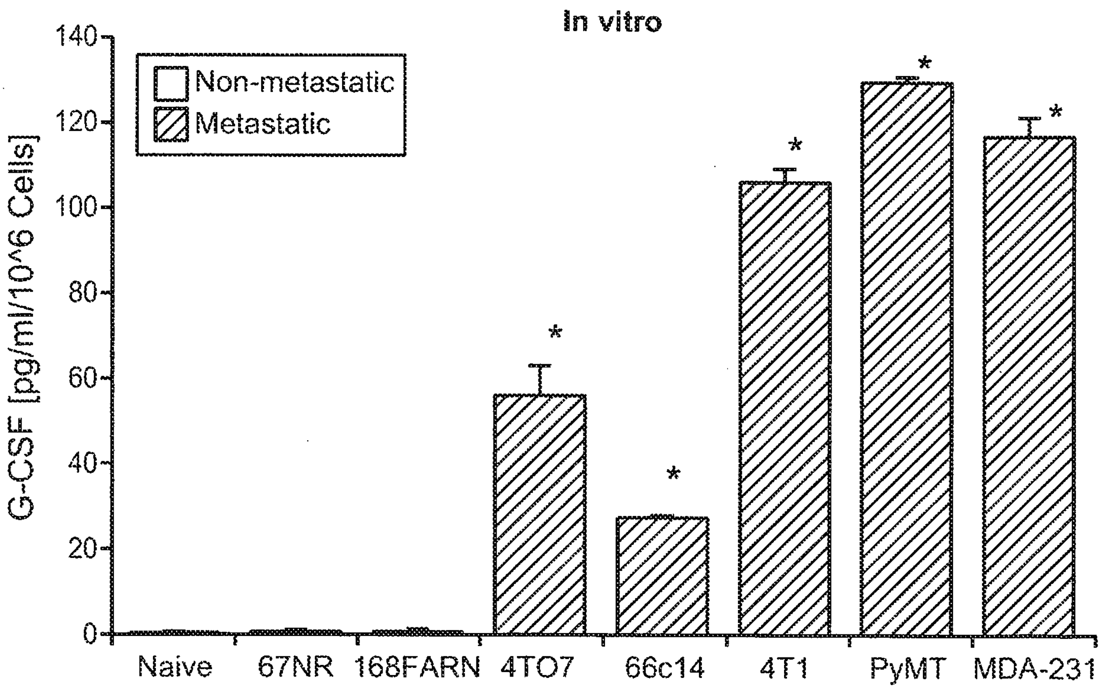


*FIG. 11B*

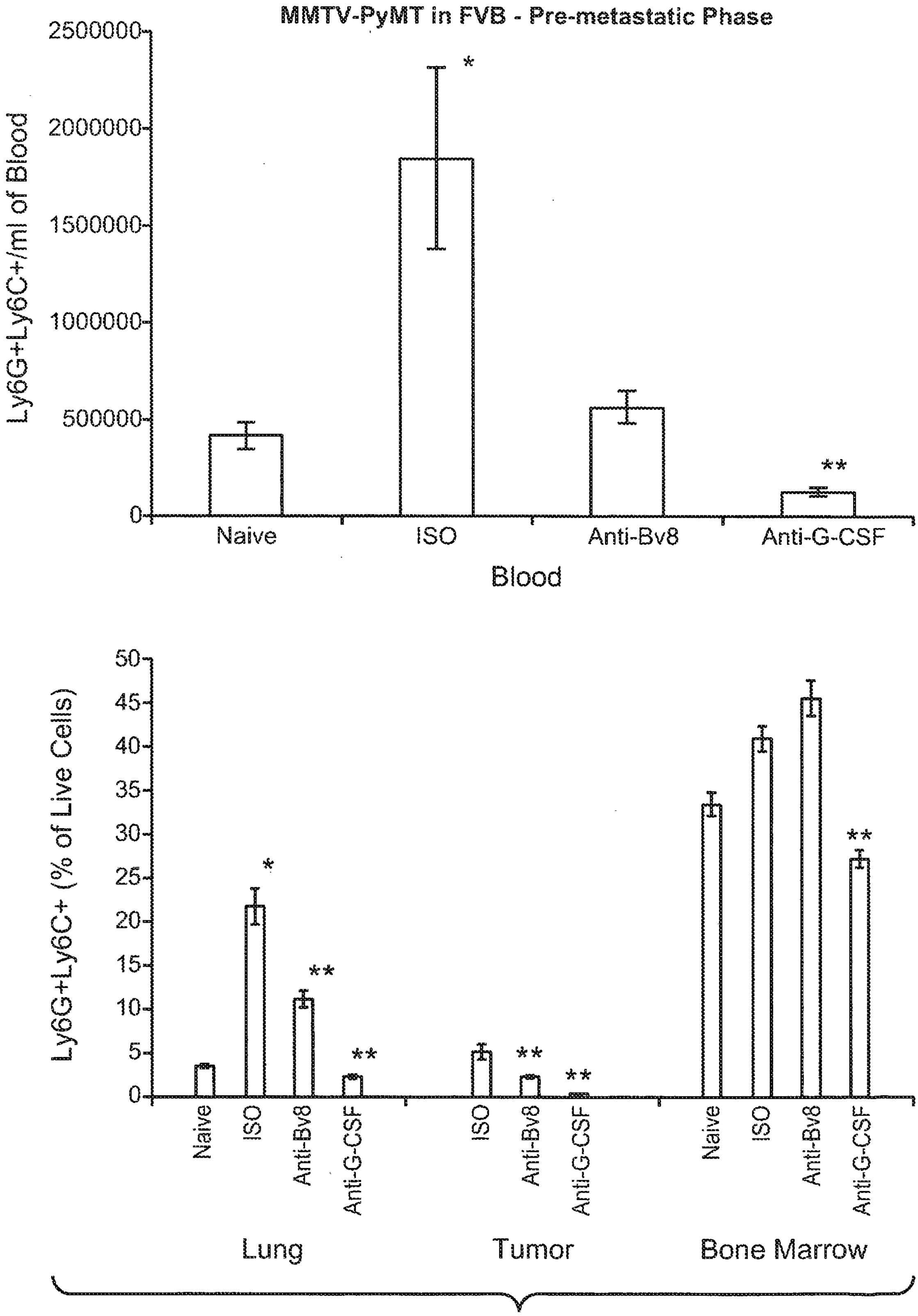
MMTV-PyMT in FVB - Pre-metastatic Phase
Ly6G+Ly6C+/ml of Blood
2500000
2000000
1500000
1000000
500000
0
*
**
Naive
ISO
Anti-Bv8
Anti-G-CSF
Blood
Ly6G+Ly6C+ (% of Live Cells)
50
45
40
35
30
25
20
15
10
5
0
Naive
ISO
Anti-Bv8
Anti-G-CSF
ISO
Anti-Bv8
Anti-G-CSF
Naive
ISO
Anti-Bv8
Anti-G-CSF
Lung
Tumor
Bone Marrow

*FIG. 11C*

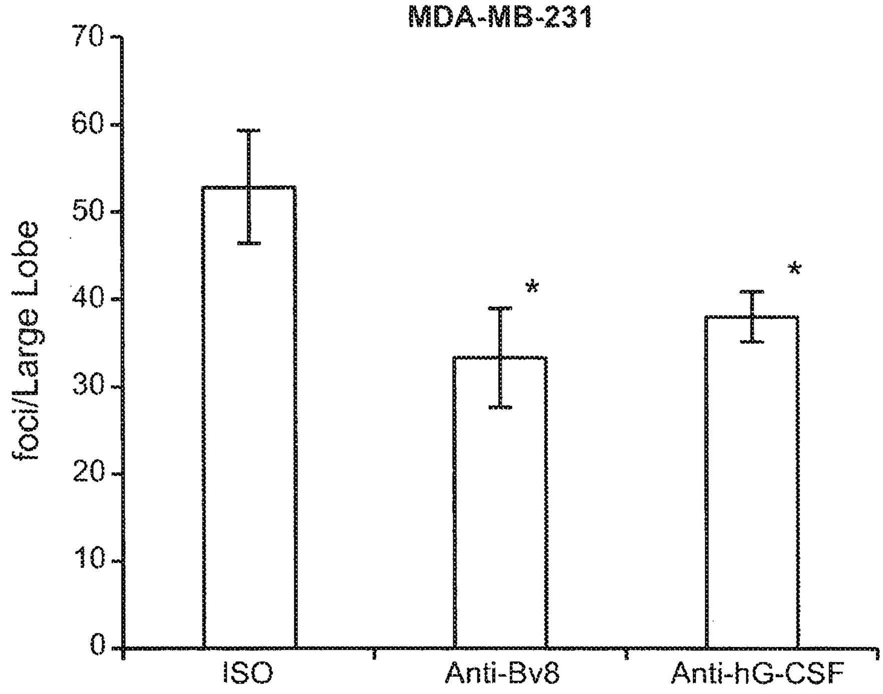


FIG. 12A

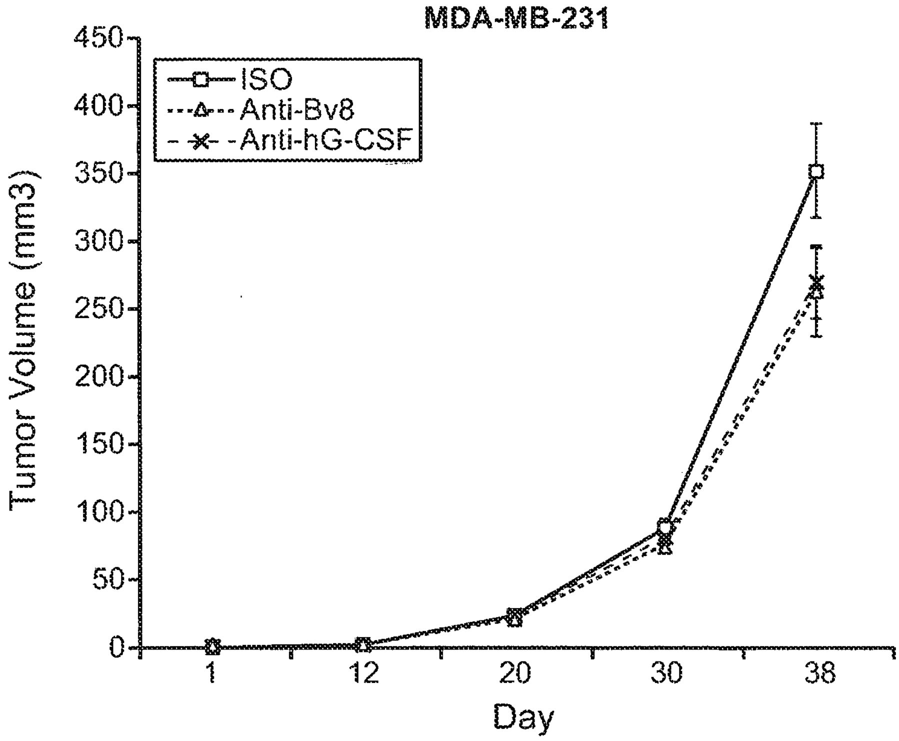


FIG. 12B

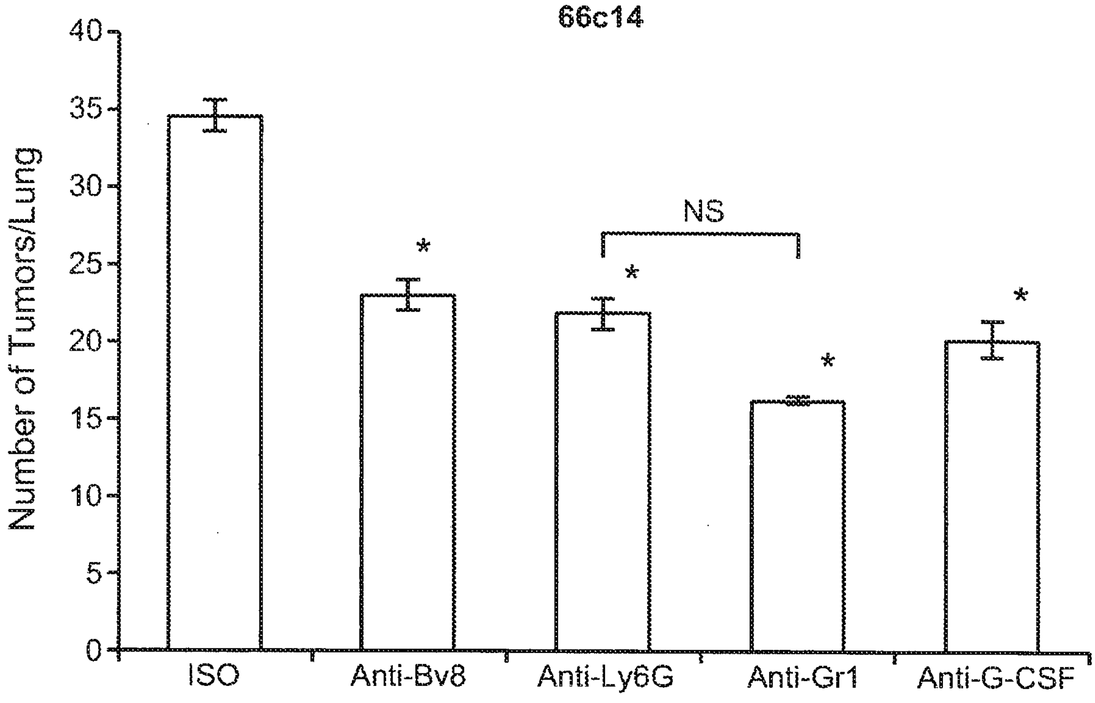


FIG. 12C

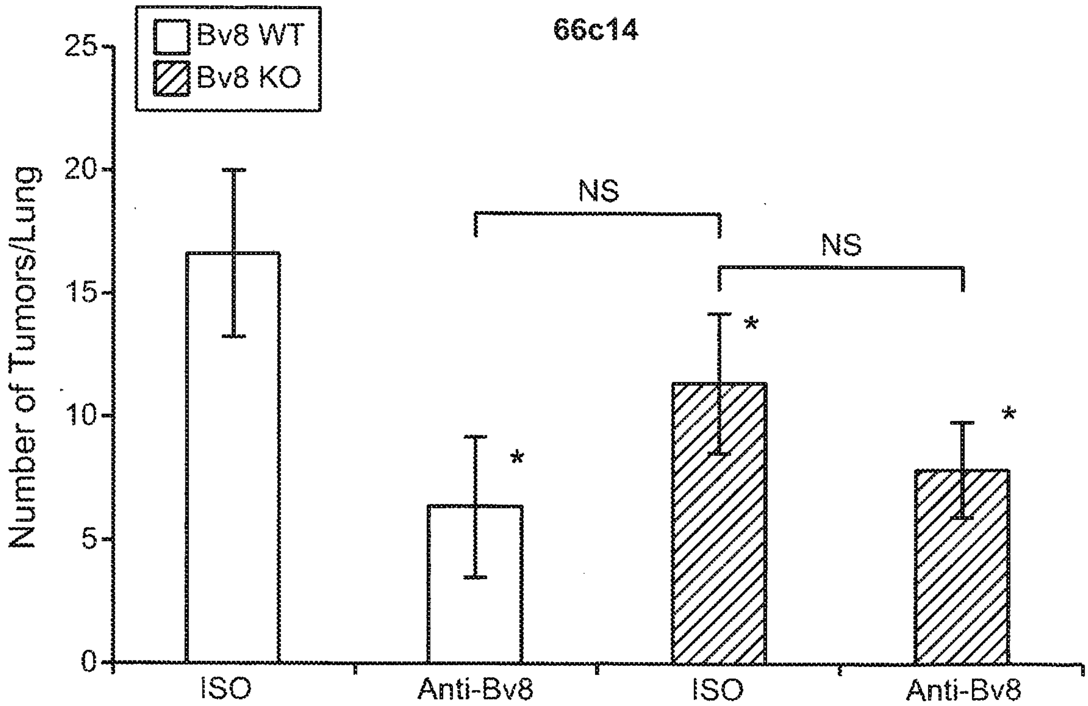


FIG. 13A

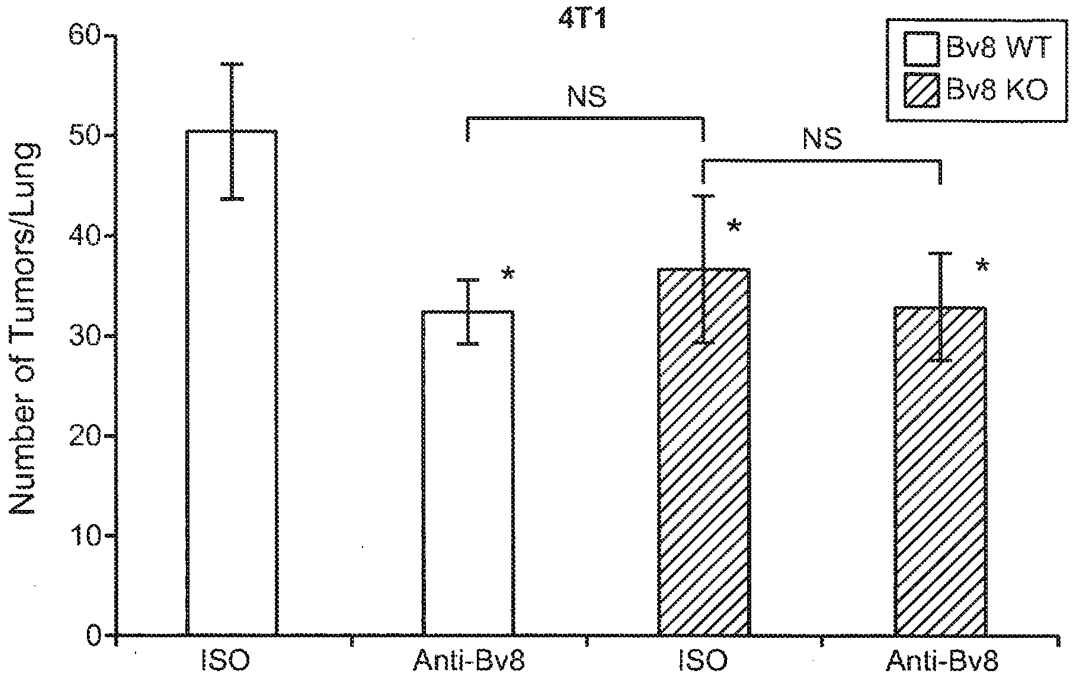


*FIG. 13B*

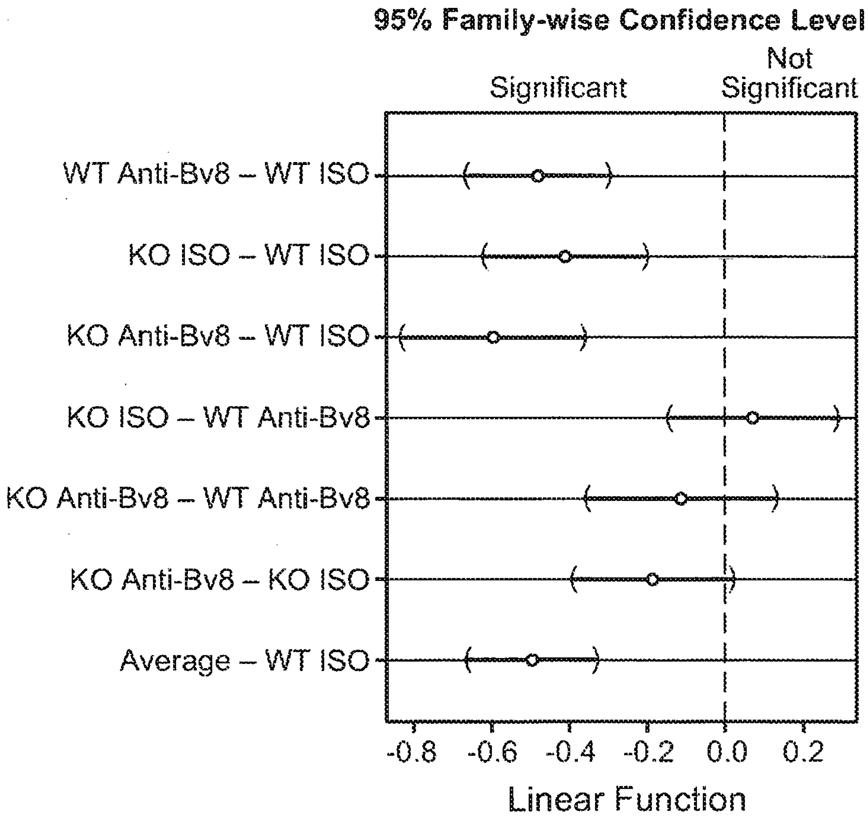


*FIG. 13C*

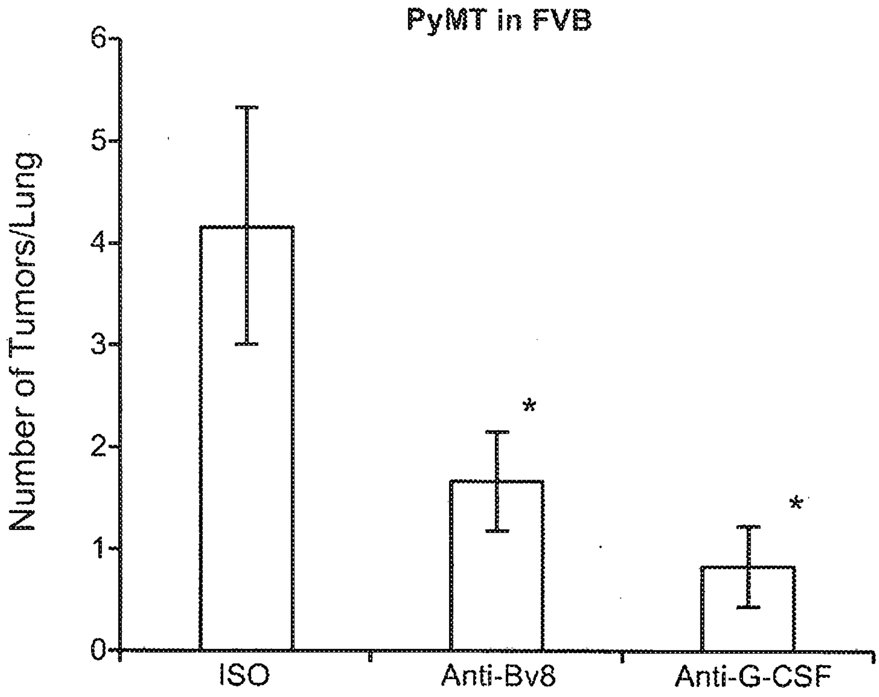


FIG. 13D

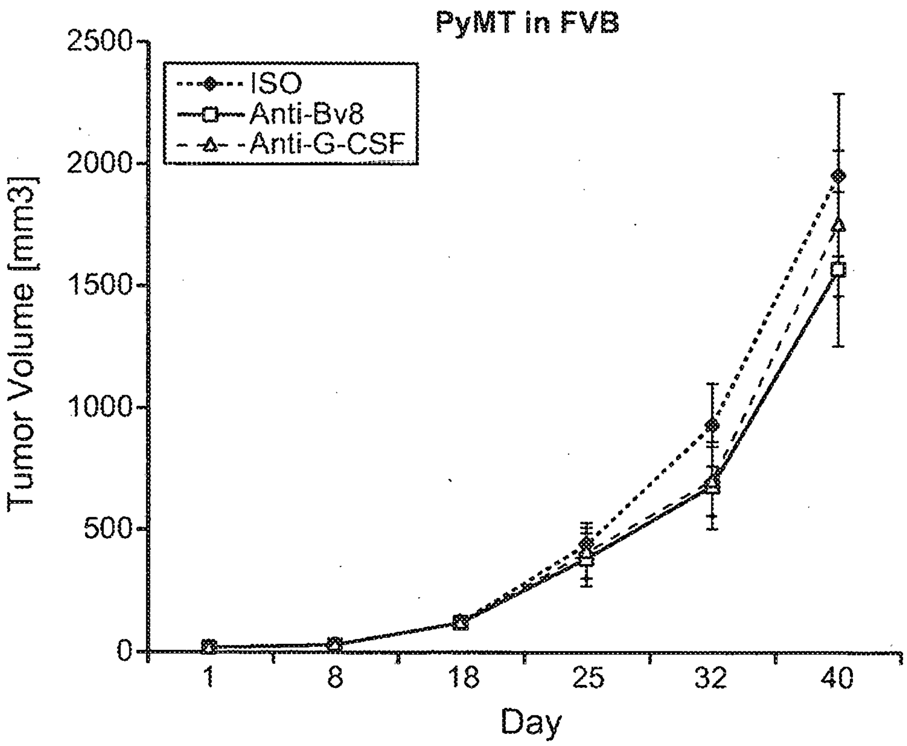


FIG. 13E

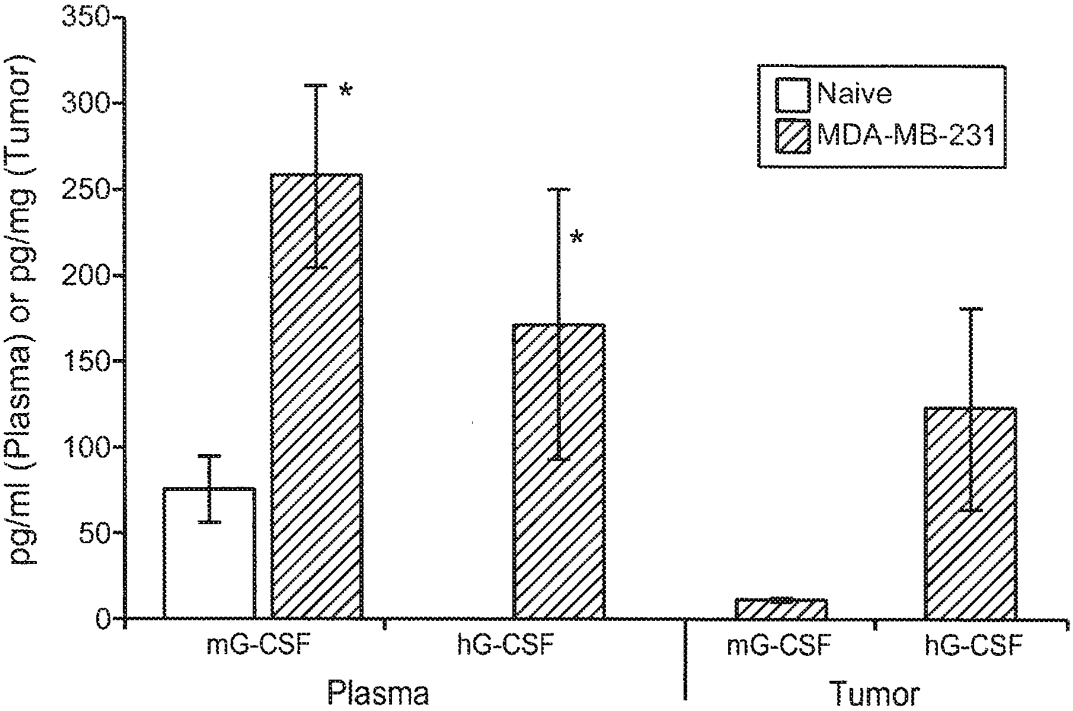


FIG. 13F

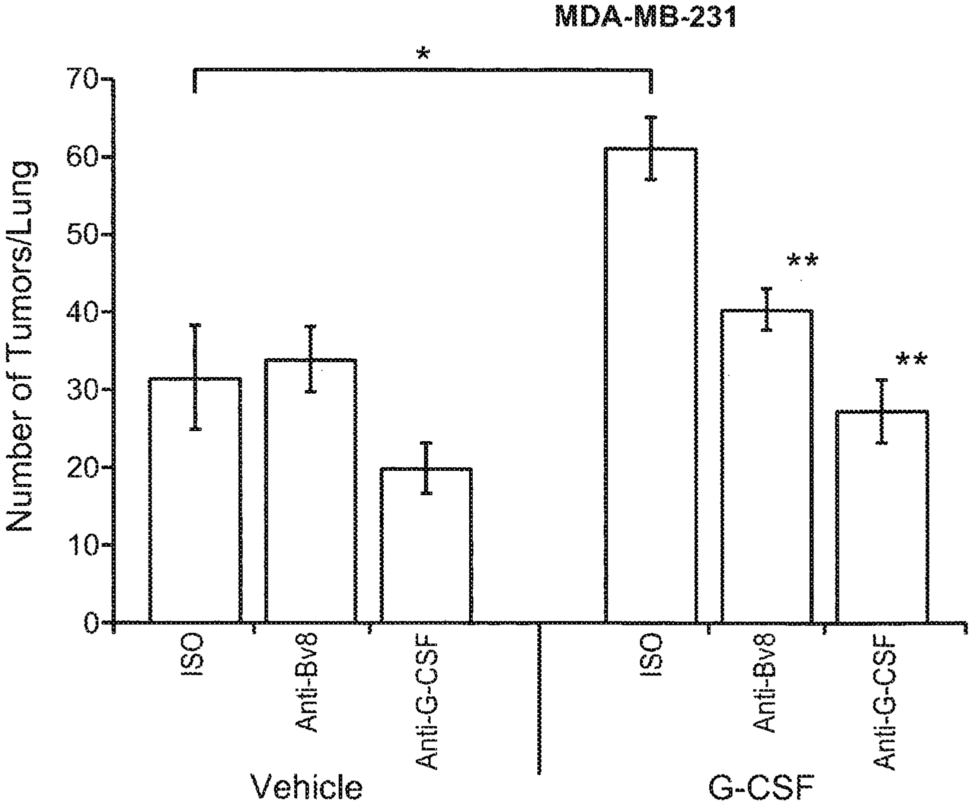


FIG. 14A

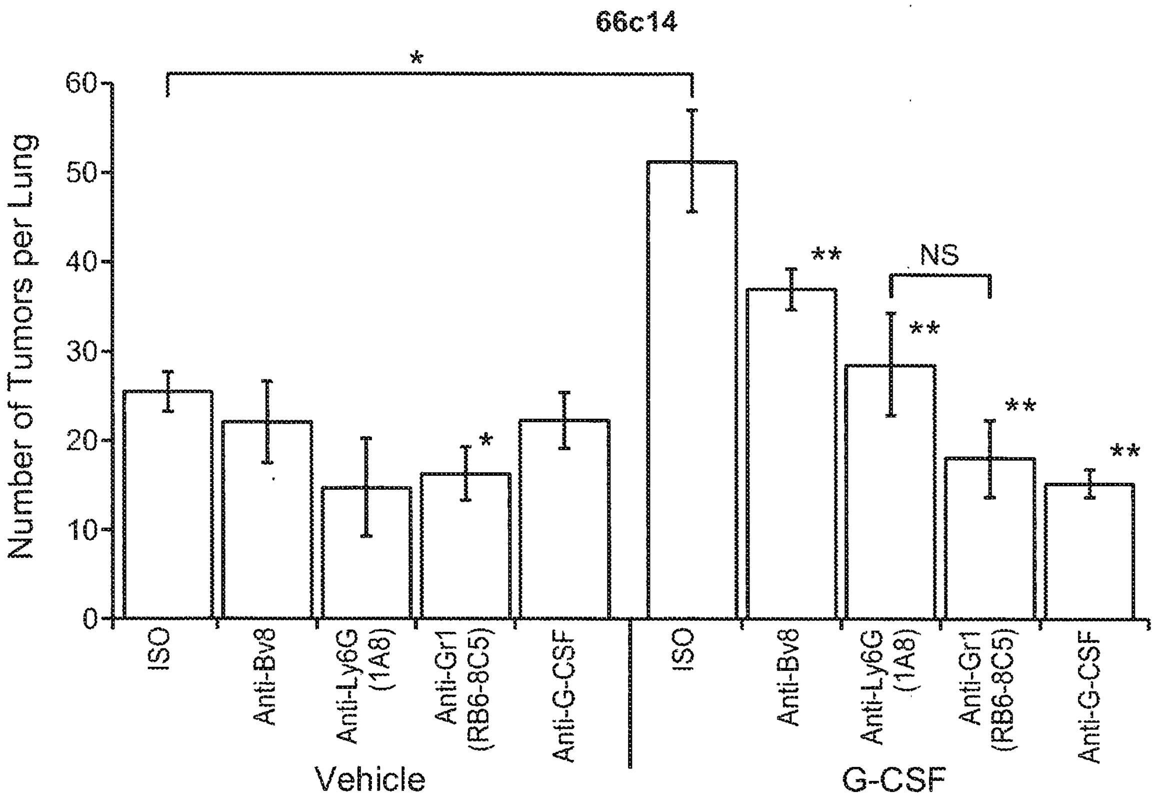


*FIG. 14B*

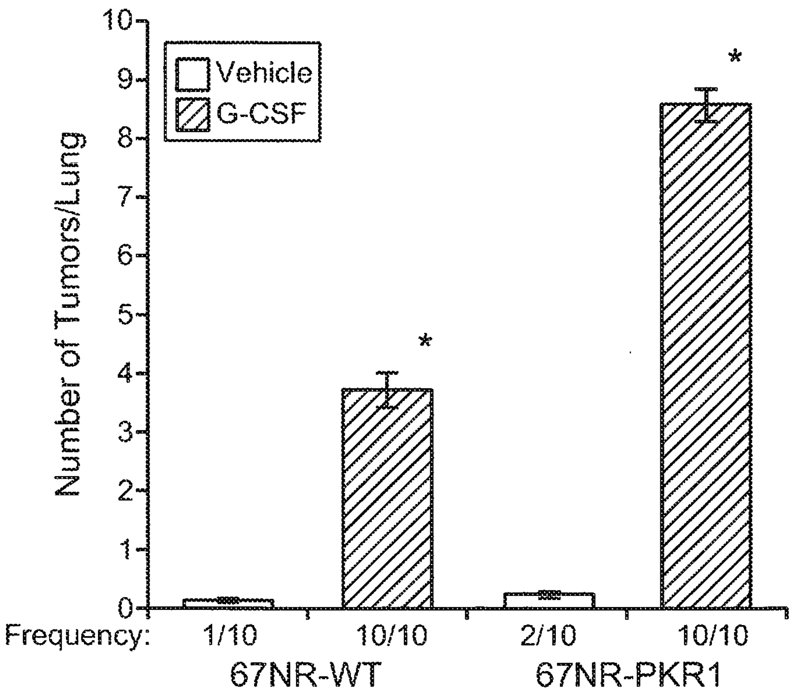


*FIG. 15A*

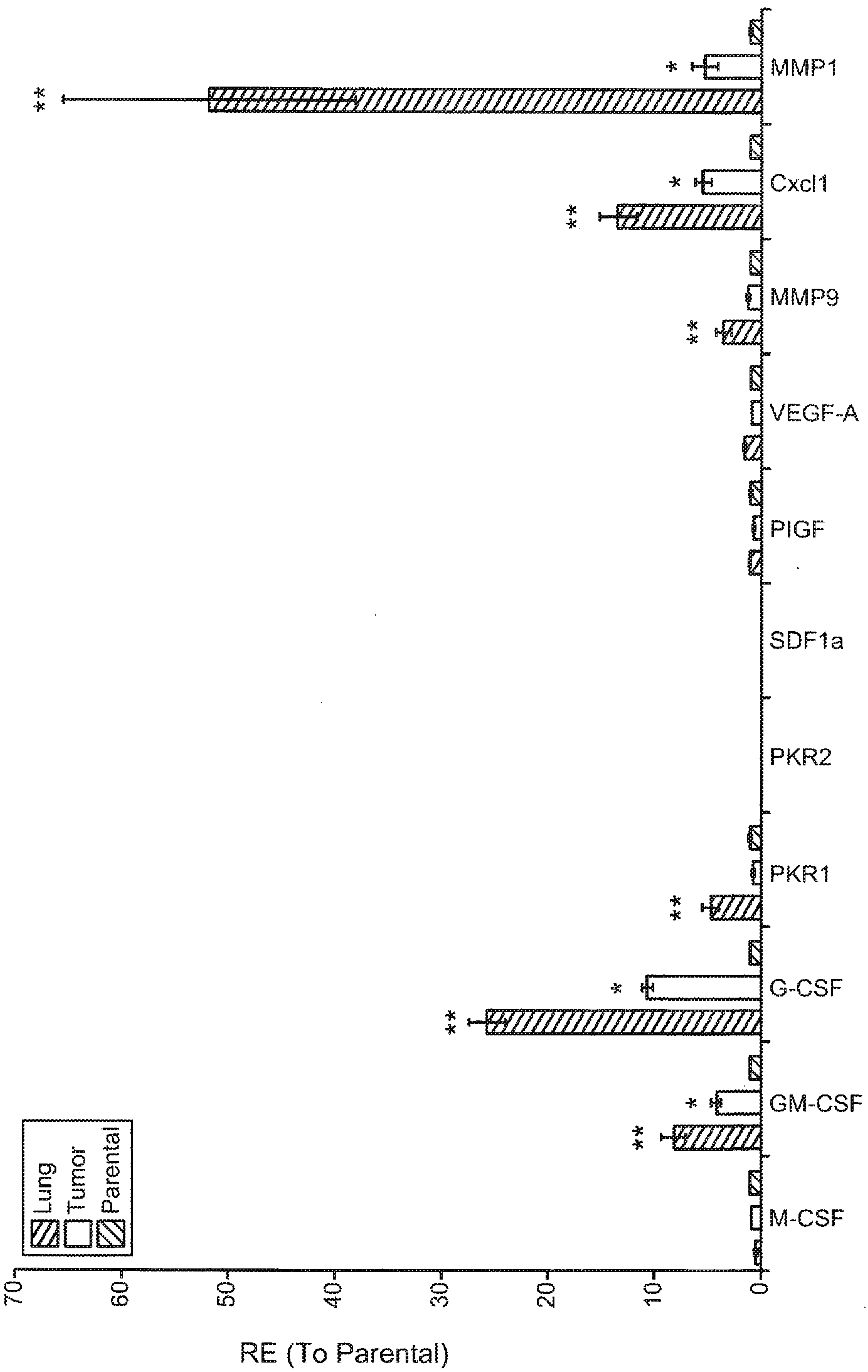
Lung
Tumor
Parental
M-CSF
GM-CSF
G-CSF
PKR1
PKR2
SDF1a
PIGF
VEGF-A
MMP9
Cxcl1
MMP1
0
10
20
30
40
50
60
70
RE (To Parental)

FIG. 15B

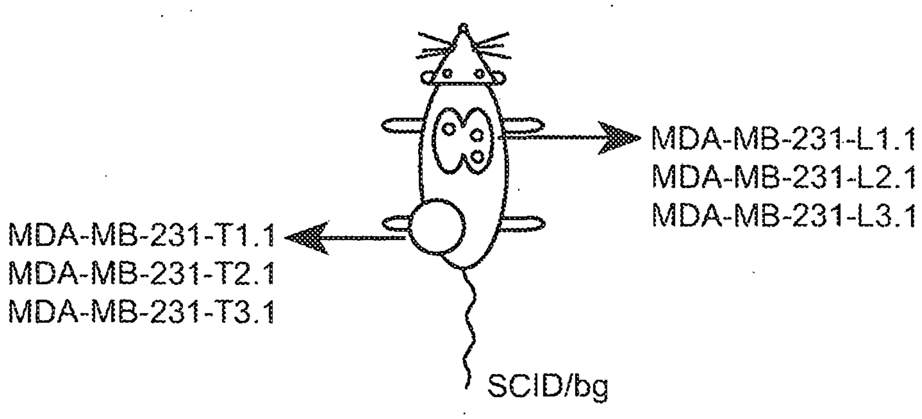


FIG. 15C

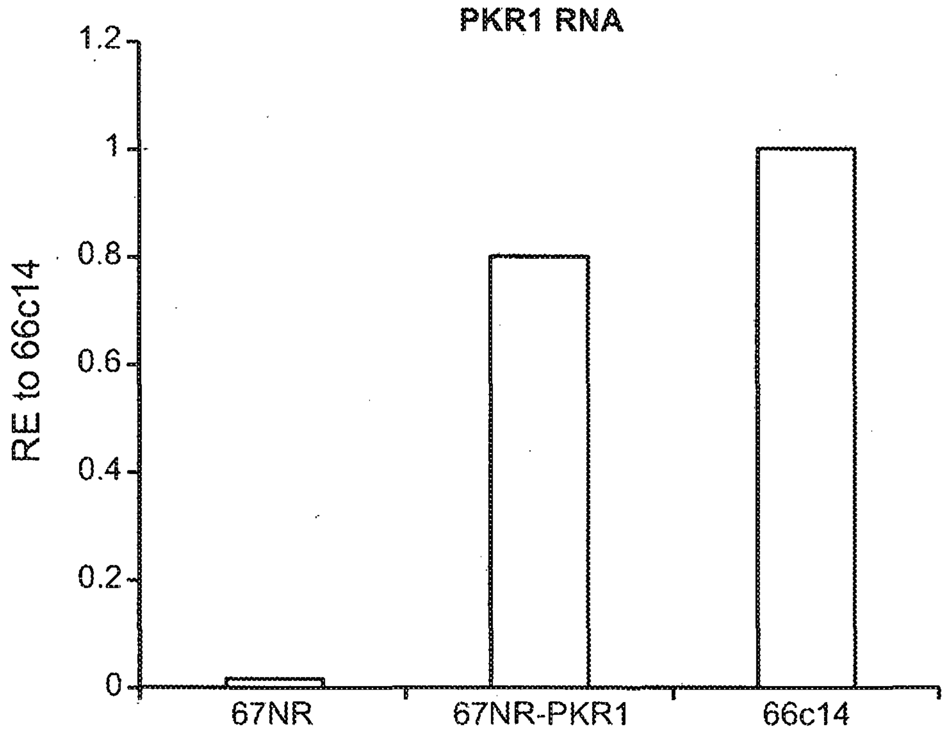


FIG. 16A

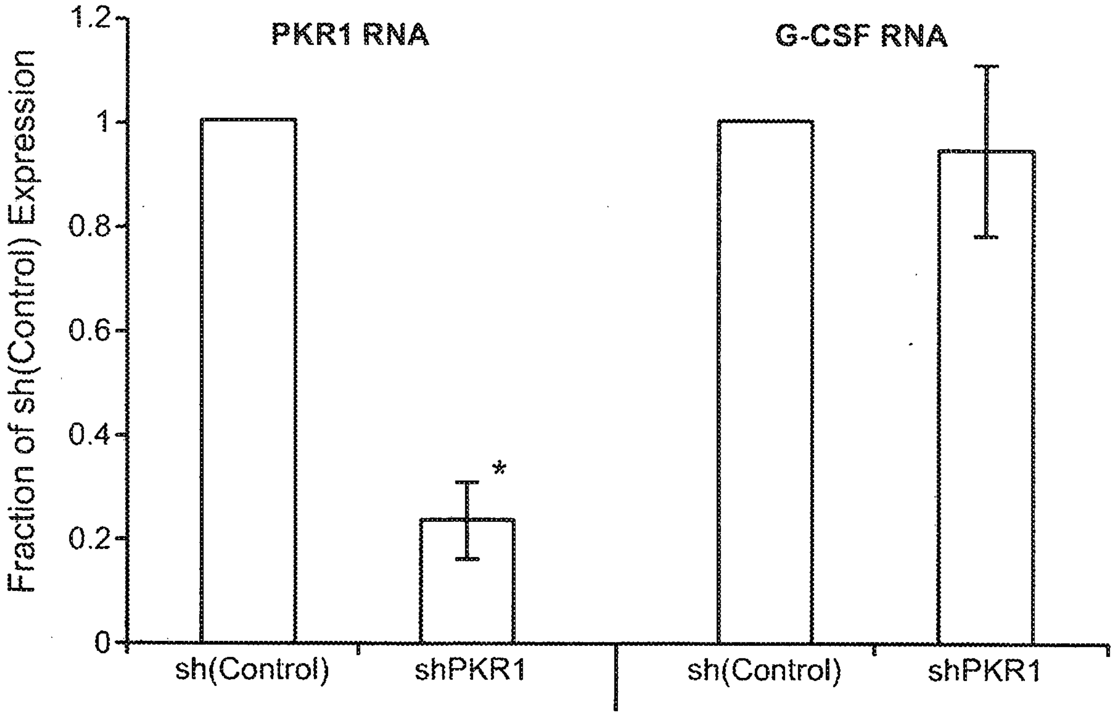


FIG. 16B

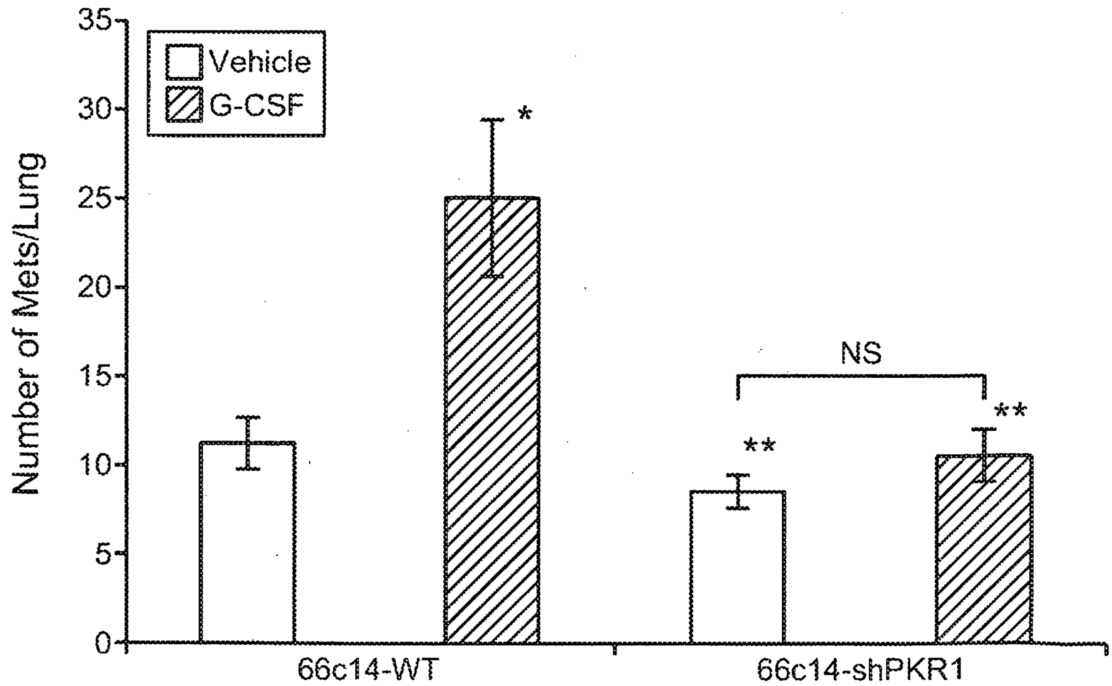


FIG. 16C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009039337 A
- US 6884879 B
- WO 2005012359 A
- US 7060269 B
- US 6582959 B
- US 6703020 B
- US 6054297 A
- WO 9845332 A
- WO 9630046 A
- WO 9410202 A
- EP 0666868 B1
- US 2006009360 A
- US 20050186208 A
- US 20030206899 A
- US 20030190317 A
- US 20030203409 A
- US 20050112126 A
- US 20060280747 A
- US 20070141065 A
- US 20070020267 A
- US 20090142343 A
- EP 75444 A
- EP 404097 A
- WO 9311161 A
- US 5641870 A
- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 6982321 B
- US 7087409 B
- US 6075181 A
- US 6150584 A
- US 5750373 A
- US 60640323 B
- US 5821333 A
- US 5500362 A
- US 5821337 A
- WO 200492219 A
- WO 200042072 A, Presta
- US 6194551 B1
- WO 199951642 A
- US 4665077 A
- US 4120649 A
- WO 199008187 A
- US 5114721 A, Cohen
- WO 199011294 A
- WO 199101133 A
- EP 340109 A
- US 20070264193 A
- WO 9316185 A
- WO 9308829 A
- WO 9404690 A
- WO 9627011 A
- US 4676980 A
- WO 9100360 A
- WO 92200373 A
- US 5534615 A
- US 4640835 A
- US 4496689 A
- US 4301144 A
- US 4670417 A
- US 4791192 A
- US 4179337 A
- US 5053394 A
- US 5770710 A
- US 5877296 A
- WO 9321232 A
- WO 9411026 A
- US 5208020 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A

- US 4362663 A
- US 4371533 A
- EP 0425235 B1
- US 5712374 A
- US 5714586 A
- US 5739116 A
- US 5767285 A
- US 5770701 A
- US 5773001 A
- WO 03102157 A
- EP 239400 B1
- US 20030157108 A, Presta, L.
- US 20040093621 A
- WO 2003011878 A
- US 6602684 B, Umana
- WO 199730087 A
- WO 199858964 A, Raju, S.
- WO 199922764 A, Raju, S.
- US 20050123546 A, Umana
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- US 20030157108 A1, Presta, L
- WO 2004056312 A1, Adams
- DD 266710
- EP 402226 A
- EP 183070 A
- EP 244234 A
- US 4767704 A
- US 4657866 A
- US 4927762 A
- US 4560655 A
- US 5122469 A
- WO 9003430 A
- WO 8700195 A
- US RE30985 E
- US 20030055006 A
- US 61230571 B
- US 61350558 B

**Non-patent literature cited in the description**


- **FOLKMAN.** *Nat Med,* 1995, vol. 1, 27-31
- **FERRARA ; KERBEL.** *Nature,* 2005, vol. 438, 967-974
- **CARMELIET.** *Nat Med,* 2003, vol. 9, 653-660
- **COUSSENS ; WERB.** *Nature,* 2002, vol. 420, 860-867
- **RAFII et al.** *Nat Rev Cancer,* 2002, vol. 2, 826-35
- **DE PALMA et al.** *Trends Immunol,* 2007, vol. 28, 519-524
- **SHOJAEI et al.** *Trends Cell Biol,* 2008, vol. 18, 372-378
- **YANG et al.** *Cancer Cell,* 2004, vol. 6, 409-421
- **BRONTE et al.** *Blood,* 2000, vol. 96, 3838-3846
- **LECOUTER et al.** *Proc Natl Acad Sci USA,* 2003, vol. 100, 2685-2690
- **LECOUTER et al.** *Proc Natl Acad Sci USA,* 2004, vol. 101, 16813-16818
- **CHENG et al.** *Nature,* 2002, vol. 417, 405-410
- **MATSUMOTO et al.** *Proc Natl Acad Sci USA,* 2006, vol. 103, 4140-4145
- **SHOJAEI et al.** *Nature,* 2007, vol. 450, 825-831
- **SHOJAEI et al.** *Proc Natl Acad Sci USA,* 2008, vol. 105, 2640-2645
- **SHOJAEI et al.** *Proc Natl Acad Sci USA,* 2009, vol. 106 (16), 6742-7
- **RAPOPORT et al.** *Blood Rev,* 1992, vol. 6, 43-57
- **LIESCHKE et al.** *Blood,* 1994, vol. 84, 1737-1746
- **NATORI.** *Biochem Biophys Res Commun,* 2002, vol. 297, 1058-1061
- **OKAZAKI, T. et al.** *Int Immunol,* 2006, vol. 18, 1-9
- **YANG, L. et al.** *Cancer Cell,* 2008, vol. 13, 23-35
- **NINOMIYA S. et al.** *Journal of Surgical Research,* 2009, vol. 154 (2), 196-202
- **NAGATA et al.** *Nature,* 1986, vol. 319, 415-418
- **NAGATA et al.** *EMBO J.,* 1986, vol. 5, 575-581
- **TSUCHIYA et al.** *Proc Natl Acad Sci,* 1986, vol. 83 (20), 7633-7637
- **WECHSELBERGER et al.** *FEBS Lett.,* 1999, vol. 462, 177-181
- **LI et al.** *Mol. Pharm.,* 2001, vol. 59, 692-698
- **LECOUTER et al.** *Proc. Natl. Acad. Sci. USA,* 2003, vol. 100, 2685-2690
- **LIN et al.** *J. Biol. Chem.,* 2002, vol. 277, 19276-19280
- **MASUDA et al.** *Biochem. Biophys. Res. Commun.,* 2002, vol. 293, 396-402
- **ALMAND, B. et al.** *J Immunol,* 2001, vol. 166, 678-689

- **YOUNG, M. R. ; LATHERS, D. M.** *Int J Immunopharmacol,* 1999, vol. 21, 241-252
- **LEUNG et al.** *Science,* 1989, vol. 246, 1306
- **HOUCK et al.** *Mol. Endocrin,* 1991, vol. 5, 1806
- **FERRARA ; DAVIS-SMYTH.** *Endocrine Rev.,* 1997, vol. 18, 4-25
- **FERRARA.** *J. Mol. Med.,* 1999, vol. 77, 527-543
- **CARMELIET et al.** *Nature,* 1996, vol. 380, 435-439
- **FERRARA et al.** *Nature,* 1996, vol. 380, 439-442
- **FERRARA et al.** *Nature Med.,* 1998, vol. 4, 336-340
- **GERBER et al.** *Nature Med.,* 1999, vol. 5, 623-628
- **CEBE-SUAREZ et al.** *Cell. Mol. Life Sci.,* 2006, vol. 63, 601-615
- **GUERRIN et al.** *J. Cell Physiol.,* 1995, vol. 164, 385-394
- **OBERG-WELSH et al.** *Mol. Cell. Endocrinol.,* 1997, vol. 126, 125-132
- **SONDELL et al.** *J. Neurosci.,* 1999, vol. 19, 5731-5740
- **PRESTA et al.** *Cancer Res.,* 1997, vol. 57, 4593-4599
- **POPKOV et al.** *Journal of Immunological Methods,* 2004, vol. 288, 149-164
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **WARD et al.** *Nature,* 1989, vol. 341, 544-546
- **BIRD et al.** *Science,* 1988, vol. 242, 423-426
- **HUSTON et al.** *PNAS (USA),* 1988, vol. 85, 5879-5883
- **HOLLINGER et al.** *Proc. Natl. Acad. Sci. USA,* 1993, vol. 90, 6444-6448
- **ZAPATA et al.** *Protein Eng.,* 1995, vol. 8 (10), 1057-1062
- **KOHLER ; MILSTEIN.** *Nature,* 1975, vol. 256, 495-97
- **HONGO et al.** *Hybridoma,* 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol.,* 1991, vol. 222, 581-597
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnol.,* 1996, vol. 14, 845-851
- **NEUBERGER.** *Nature Biotechnol.,* 1996, vol. 14, 826
- **LONBERG ; HUSZAR.** *Intem. Rev. Immunol.,* 1995, vol. 13, 65-93
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433
- **VAN DIJK ; VAN DE WINKEL.** *Curr. Opin. Pharmacol.,* 2001, vol. 5, 368-74
- **LI et al.** *Proc. Natl. Acad. Sci. USA,* 2006, vol. 103, 3557-3562
- **VAUGHAN et al.** *Nature Biotechnology,* 1996, vol. 14, 309-314
- **SHEETS et al.** *PNAS (USA),* 1998, vol. 95, 6157-6162
- **HOOGENBOOM ; WINTER.** *J. Mol. Biol.,* 1991, vol. 227, 381
- **MARKS et al.** *J. Mol. Biol.,* 1991, vol. 222, 581
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **COLE et al.** Monoclonal Antibodies and Cancer Therapy. Alan R. Liss, 1985, 77

- **BOERNER et al.** *J. Immunol.,* 1991, vol. 147 (1), 86-95
- **KABAT et al.** Sequences of Proteins of Immunological Interest. National Institutes of Health, 1991
- **XU et al.** *Immunity,* 2000, vol. 13, 37-45
- **JOHNSON ; WU.** Methods in Molecular Biology. Human Press, 2003, vol. 248, 1-25
- **HAMERS-CASTERMAN et al.** *Nature,* 1993, vol. 363, 446-448
- **SHERIFF et al.** *Nature Struct. Biol.,* 1996, vol. 3, 733-736
- **CHOTHIA ; LESK.** *J. Mol. Biol.,* 1987, vol. 196, 901-917
- **KABAT et al.** Sequences of Immunological Interest. National Institutes of Health, 1991
- **ABBAS et al.** Cellular and Mol. Immunology. W.B. Saunders, Co, 2000
- **BURTON.** *Molec. Immunol.,* 1985, vol. 22, 161-206
- **RAVETCH ; KINET.** *Annu. Rev. Immunol,* 1991, vol. 9, 457-92
- **CLYNES et al.** *PNAS (USA),* 1998, vol. 95, 652-656
- **DAËRON.** *Annu. Rev. Immunol.,* 1997, vol. 15, 203-234
- **CAPEL et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **SHIELDS et al.** *J. Biol. Chem.,* 2001, vol. 9 (2), 6591-6604
- **GAZZANO-SANTORO et al.** *J. Immunol. Methods,* 1996, vol. 202, 163
- **IDUSOGIE et al.** *J. Immunol.,* 2000, vol. 164, 4178-4184
- **BARBAS et al.** *Proc Nat. Acad. Sci, USA,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **KOSTELNEY et al.** *J. Immunol.,* 1992, vol. 148, 1547-1553
- **ARAKAWA et al.** *J. Biol. Chem.,* 1994, vol. 269 (45), 27833-27839
- **RADZIEJEWSKI et al.** *Biochem.,* 1993, vol. 32 (48), 1350
- **ED HARLOW ; DAVID LANE.** Antibodies, A Laboratory Manual. Cold Spring Harbor Laboratory, 1988
- Cell cycle regulation, oncogenes, and antineoplastic drugs. **MURAKAMI et al.** The Molecular Basis of Cancer. WB Saunders, 1995, 13
- **AGNEW.** *Chem Intl. Ed. Engl.,* 1994, vol. 33, 183-186
- **WILMAN.** Prodrugs in Cancer Chemotherapy. *Biochemical Society Transactions,* 1986, vol. 14, 375-382
- Prodrugs: A Chemical Approach to Targeted Drug Delivery. **STELLA et al.** Directed Drug Delivery,. Humana Press, 1985, 247-267
- **KLAGSBRUN ; D'AMORE.** *Annu. Rev. Physiol.,* 1991, vol. 53, 217-39
- **STREIT ; DETMAR.** *Oncogene,* 2003, vol. 22, 3172-3179
- **FERRARA ; ALITALO.** *Nature Medicine,* 1999, vol. 5 (12), 1359-1364
- **TONINI et al.** *Oncogene,* 2003, vol. 22, 6549-6556
- **SATO.** *Int. J. Clin. Oncol.,* 2003, vol. 8, 200-206
- **OFFNER et al.** *Science,* 1991, vol. 251, 430-432
- **IANEWAY.** *Nature,* 1989, vol. 341, 482
- **ELLMAN et al.** *Meth. Enzym.,* 1991, vol. 202, 301-336
- **NOREN et al.** *Science,* 1989, vol. 244, 182
- **CHEN et al.** *J. Mol. Biol.,* 1999, vol. 293, 865-881
- **ZACHER et al.** *Gene,* 1980, vol. 9, 127-140
- **SMITH et al.** *Science,* 1985, vol. 228, 1315-1317
- **PARMLEY ; SMITH.** *Gene,* 1988, vol. 73, 305-318
- **FIRE et al.** *Nature,* 1998, vol. 391, 806-811
- **MCMANUS et al.** *Nat. Rev. Genet.,* 2002, vol. 3 (10), 737-47
- **NGUYEN, D. X. ; MASSAGUE, J.** *Nat Rev Genet,* 2007, vol. 8, 341-352
- **PSAILA, B. ; LYDEN, D.** *Nat Rev Cancer,* 2009, vol. 9, 285-293
- **PAGET, S.** *Cancer Metastasis Rev.,* 1989, vol. 8 (2), 98-101
- **KAPLAN, R. N. et al.** *Nature,* 2005, vol. 438, 820-827
- **HIRATSUKA, S. et al.** *Nat Cell Biol,* 2006, vol. 8, 1369-1375
- **YAMAMOTO, M. et al.** *Cancer Res,* 2008, vol. 68, 9754-9762
- **KIM, S. et al.** *Nature,* 2009, vol. 457, 102-106
- **ERLER, J. T. et al.** *Cancer Cell,* 2009, vol. 15, 35-44

- **KOHLER et al.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **GODING.** MonoclonalAntibodies: Principles and Practice. Academic Press, 1986, 59-103
- **MCCAFFERTY et al.** *Nature,* 1990, vol. 348, 552-554
- **WATERHOUSE et al.** *Nuc. Acids. Res.,* 1993, vol. 21, 2265-2266
- **MORRISON et al.** *Proc. Natl. Acad. Sci. USA,* 1984, vol. 81, 6851
- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad Sci. USA,* 1992, vol. 89, 4285
- **PRESTA et al.** *J. Immnol.,* 1993, vol. 151, 2623
- **BRUGGERMANN et al.** *Year in Immuno.,* 1993, vol. 7, 33
- **DUCHOSAL et al.** *Nature,* vol. 355, 258
- **HOOGENBOOM et al.** *J. Mol. Biol.,* 1991, vol. 227, 381
- **MARKS et al.** *J. MoL Biol.,* 1991, vol. 222, 581-597
- **VAUGHAN et al.** *Nature Biotech,* 1996, vol. 14, 309
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **MILLSTEIN et al.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **SHALABY et al.** *J. Exp. Med.,* 1992, vol. 175, 217-225
- **KOSTELNY et al.** *J. Immunol.,* 1992, vol. 148 (5), 1547-1553
- **HOLLINGER et al.** *Proc. Nati. Acad. Sci. USA,* 1993, vol. 90, 6444-6448
- **GRUBER et al.** *J. Immunol,* 1994, vol. 152, 5368
- **TUFT et al.** *J. Immunol.,* 1991, vol. 147, 60
- **CARON et al.** *J. Exp Med.,* 1992, vol. 176, 1191-1195
- **SHOPES, B. J.** *Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **FRAKER et al.** *Biochem. Biophys. Res. Commun.,* 1978, vol. 80, 49-57
- Monoclonal Antibodies in Immunoscintigraphy. CRC Press, 1989
- **VITETTA et al.** *Science,* 1987, vol. 238, 1098
- **CHARI et al.** *Cancer Research,* 1992, vol. 52, 127-131
- **CARLSSON et al.** *Biochem. J.,* 1978, vol. 173, 723-737
- **HINMAN et al.** *Cancer Research,* 1993, vol. 53, 3336-3342
- **LODE et al.** *Cancer Research,* 1998, vol. 58, 2925-2928
- **AMIT et al.** *Science,* 1986, vol. 233, 747-753
- **CHOTHIA et al.** *J. Mol. Biol.,* 1987, vol. 196, 901-917
- **CUNNINGHAM ; WELLS.** *Science,* 1989, vol. 244, 1081-1085
- **KUNKEL.** *Proc. Natl. Acad. Sci. USA,* 1985, vol. 82, 488
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *Annals N.Y. Acad. Sci.,* 1982, vol. 383, 44-68
- **HAM et al.** *Meth. Enz.,* 1979, vol. 58, 44
- **BARNES et al.** *Anal. Biochem.,* 1980, vol. 102, 255
- **LINDMARK et al.** *J. Immunol. Meth.,* 1983, vol. 62, 1-13
- **GUSS et al.** *EMBO J.,* 1986, vol. 5, 1567-1575
- **CARMELIET ; JAIN.** *Nature,* 2000, vol. 407, 249-257
- **FERRARA et al.** *Nature Reviews:Drug Discovery,* 2004, vol. 3, 391-400
- Current Protocols In Molecular Biology. 1995
- Chemotherapy Service. Williams & Wilkins, 1992
- **DEXTER, DL et al.** *Cancer Res,* 1978, vol. 38, 3174
- **ASLAKSON, CJ ; MILLER, FR.** *Cancer Res,* 1992, vol. 52, 1399
- **SHOJAEI, F. et al.** *Nature,* 2007, vol. 450, 825-831

- **SHOJAEI, F. et al.** *Proc Natl Acad Sci U S A,* 2009
- **CHOE et al.** *Genome Biol,* 2005, vol. 6, R16
- **CAUNT, M. et al.** *Cancer Cell,* 2008, vol. 13, 331-342
- **BLAVERI, E. et al.** *Clin Cancer Res,* 2005, vol. 11, 7012-7022
- **CHIN, K. et al.** *Cancer Cell,* 2006, vol. 10, 529-541
- **PAWITAN, Y. et al.** *Breast Cancer Res,* 2005, vol. 7, R953-964
- **DEXTER, D. L. et al.** *Cancer Res,* 1978, vol. 38, 3174-3181
- **ASLAKSON, C. J. ; MILLER, F. R.** *Cancer Res,* 1992, vol. 52, 1399-1405
- **YANG, J. et al.** *Cell,* 2004, vol. 117, 927-939
- **LECOUTER, J. et al.** *Proc Natl Acad Sci U S A,* 2003, vol. 100, 2685-2690
- **LECOUTER, J. et al.** *Proc Natl Acad Sci U S A,* 2004, vol. 101, 16813-16818
- **CHENG, M. Y. et al.** *Nature,* 2002, vol. 417, 405-410
- **MATSUMOTO, S. et al.** *Proc Natl Acad Sci U S A,* 2006, vol. 103, 4140-4145
- **KAPLAN, RN et al.** *Nature,* 2005, vol. 438, 820
- **METCALF, D.** *Nature,* 1989, vol. 339, 27-30
- **CHRISTOPHER, M. J. ; LINK, D. C.** *Curr Opin Hematol,* 2007, vol. 14, 3-8
- **SHOJAEI, F. et al.** *Proc Natl Acad Sci U S A,* 2009, vol. 106 (16), 6742-6747
- **DALEY, JM et al.** *J Leukoc Biol,* 2008, vol. 83, 64
- **PAEZ-RIBES, M. et al.** *Cancer Cell,* 2009, vol. 15, 220-231
- **EBOS, J. M. et al.** *Cancer Cell,* 2009, vol. 15, 232-239
- **HIRATSUKA, S. et al.** *Cancer Cell,* 2002, vol. 2, 289-300
- **ACUFF, H. B. et al.** *Cancer Res,* 2006, vol. 66, 259-266
- **MINN, AJ et al.** *Nature,* 2005, vol. 436, 518
- **BOS, PD et al.** *Nature,* 2009, vol. 459, 1005
- **LU, X et al.** *Genes Dev,* 2009, vol. 23, 1882
- **YANG, L. et al.** *Cancer Cell,* 2004, vol. 6, 409-421
- **SHOJAEI, F. et al.** *Nat Biotechnol,* 2007, vol. 25, 911-920
- **DE PALMA, M. et al.** *Trends Immunol,* 2007, vol. 28, 519-524
- **MURDOCH, C. et al.** *Nat Rev Cancer,* 2008, vol. 8, 618-631